# EUROPEAN PATENT APPLICATION

(11) **EP 3 750 885 A1**
(43) Date of publication of application: **16.12.2020**
(21) Application number: 19751513.3
(22) Date of filing: 01.02.2019
(51) Int. Cl.: C07D 401/04, C07D 403/04, C07D 403/14, A61K 31/402, A61K 31/4025, A61P 21/00

(54) **COMPOUND HAVING BET INHIBITORY ACTIVITY AND PREPARATION METHOD AND USE THEREFOR**

(30) Priority: 06.02.2018 CN 201810118820
(71) Applicant: Shanghai Haihe Pharmaceutical Co., Ltd., Shanghai 201203 (CN); Shanghai Institute of Materia Medica, Chinese Academy of Sciences, Shanghai 201203 (CN)
(72) Inventor: XIA, Lin, Shanghai 201203 (CN); GENG, Meiyu, Shanghai 201203 (CN); YE, Yan, Shanghai 201203 (CN); DING, Jian, Shanghai 201203 (CN); ZHANG, Qiong, Shanghai 201203 (CN); SHEN, Aijun, Shanghai 201203 (CN); HUANG, Ying, Shanghai 201203 (CN); LIU, Hongchun, Shanghai 201203 (CN); YANG, Haoran, Shanghai 201203 (CN); AI, Jing, Shanghai 201203 (CN); ZHANG, Minmin, Shanghai 201203 (CN)
(74) Representative: Vossius & Partner Patentanwälte Rechtsanwälte mbB
(86) International application number: PCT/CN2019/074448
(87) International publication number: WO 2019/154329

(57) **Abstract**

The invention relates to the field of pharmaceutical chemistry. Specifically, the present invention relates to a series of BET (bromodomain and extra-terminal domain) inhibitors having a novel structure, particularly inhibitors targeting BRD4 (Bromodomain-containing protein 4), and a preparation method and use therefor. The structure thereof is shown in the following general formula (I). Said compounds or a stereoisomer, racemate, geometric isomer, tautomer, prodrug, hydrate, solvate, or crystal form thereof, or a pharmaceutically acceptable salt thereof, and the pharmaceutical compsosition thereof can be used for the treatment and/or prevention of related diseases mediated by bromodomain proteins.

## Description

### Field of the Invention

The invention belongs to the field of pharmaceutical chemistry. Specifically, the invention relates to novel compounds, or a stereoisomers, racemates, geometric isomers, tautomers, prodrugs, hydrates, solvates, or pharmaceutically acceptable salts thereof, and a pharmaceutical composition comprising the same, which are BET (bromodomain and extra-terminal domain) inhibitors, especially inhibitors targeting BRD4 (Bromodomain-containing protein 4) with novel structures.

### Background of the Invention

Bromodomain-containing proteins (BCPs) are divided into 8 families based on the structure and sequence similarity, the most extensively studied family among them being the BET family. The BET family includes a total of four members: BRD4, BRD3, BRD2 and BRDT, the first three of which are widely expressed in tissues, while BRDT is specifically expressed in testis. Each of these four proteins has two bromodomains capable of recognizing acetylated lysine residues, being BD1 and BD2 respectively. BRD4 and BRDT contain a C-terminal region that interacts with the positive transcription elongation factor (P-TEFb), which region has non-conserved sequence. BET family members regulate gene transcription, replication, cell cycle progression and other cellular activities through interaction with acetylated histones as well as non-histones, and are considered as epigenetic target proteins which are most likely to be made into drugs among the bromodomain-containing proteins (BCPs). BRD4 is further regarded as the most promising target for BET therapy, and has attracted considerable interest from the pharmaceutical and academic communities, particularly in the field of cancer and inflammation therapy. For example, NUT Midline Cancer (NMC), which is caused by c-MYC overexpression due to BRD4-NUT fusion protein produced by nuclear protein (NUT) translocation of BRD4 chromosome to testis genome, is an aggressive squamous cell malignant tumor unresponsive to conventional treatment. In human NMC-derived xenograft mouse model experiments, BET inhibitor JQ1 inhibited tumor squamous differentiation and growth and caused regression by blocking the binding of BRD4 protein with acetyl lysine on chromatin. Meanwhile, studies show that BET inhibitors can effectively down-regulate MYC transcription and MYC-dependent target genes in the whole genome. In view of the wide pathogenic role of MYC in cancer, BET inhibitors are promising for wide applications in the treatment of cancer. Recent studies have showed that BET inhibitors exhibit the best efficacy when the host possesses intact immune system in a MYC-driven B-cell lymphoma model. Through the genome-wide analysis of the transcription reaction induced by the BET inhibitor, the immune checkpoint ligand CD274 (PD-L1) was identified as the target gene of the BET inhibitor. In mouse and human tumor cell lines and in patient samples, BET inhibitors down-regulate not only the CD274 expression transcribed at a constant level, but also the CD274 expression induced by interferon-gamma (IFN-γ). In a MYC-driven lymphoma mouse model, the PD-1 antibody and the BET inhibitor JQ1 showed synergistic effects. These data provide a strong support for the clinical combination of BET inhibitors with the immune checkpoint inhibitor PD-1/PD-L antibody.

A large number of small molecule BRD4 inhibitors are currently in clinical research. For example, apabetalone (RVX-208, Hepalink) from Resverlogix, Canada has entered clinical phase III for the treatment of cardiovascular diseases. Molibresib (I-BET762, GSK525762), birabresib (OTX-015, MK8628), CPI-0610, and TEN-010/RG6146, which were developed based on JQ1 parent nuclear benzodiazepine, are mainly used for the treatment of cancer. Abbv-075, which was derived from fragment-based design, is currently in clinical phase I for the clinical indication solid tumor. Other BRD4 inhibitors that subsequently entered the clinical trial stage are BAY123 8097, CC90010, FT1101, ZEN3694, PLX51107, INCB54329, GS5829, BMS98618 and ODM207. According to literature research, the further clinical trials of birabresib (OTX-015, MK8628) and BAY1238097 have actually been terminated due to poor drug efficacy or toxicity. In view of the broad prospects of BRD4 inhibitors as single agent or in combinations in the field of tumor or cancer and other diseases, it is still crucial to find more effective and safe BRD4 inhibitors to meet the needs of the subjects.

### Description of the Invention

Through extensive experimental research, the inventors of the invention designed and synthesized a series of small molecule compounds with novel structures represented by the following general formula (I), which have high bromodomain protein inhibitory activity, particularly BET inhibitory activity, and more particularly inhibitory activity targeted to BRD4. These compounds or a stereoisomer, racemate, geometric isomer, tautomer, prodrug, hydrate, solvate, crystal form or pharmaceutically acceptable salt thereof and the pharmaceutical compositions comprising the same are useful for the treatment and/or prevention of the diseases mediated by bromodomain proteins. The invention also provides a method for preparing the compounds of the general formula (I), a pharmaceutical composition comprising the same and use of them in the manufacture of medicaments.

The compounds of the invention possess high bromodomain protein inhibitory activity, and provide a new treatment option for the treatment of such diseases as cancer, inflammation and the like.

### Embodiments

The invention provides the following embodiments:
Embodiment 1. A compound of formula (I), or a stereoisomer, racemate, geometric isomer, tautomer, prodrug, hydrate, solvate, or pharmaceutically acceptable salt thereof, 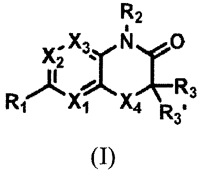 wherein,
   R₁ is selected from 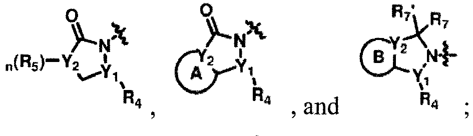 wherein,
   Y₁ is selected from CR₄' and N; wherein R₄' is selected from H, D, hydroxy, halo (e.g., F, Cl, Br, I), cyano and optionally substituted alkyl (e.g., C₁₋₆alkyl);
   R₄ is selected from optionally substituted aryl (e.g., C₆₋₁₄aryl), optionally substituted heteroaryl (e.g., 5- to 12-membered heteroaryl), optionally substituted cycloalkyl (e.g., C₃-₁₂cycloalkyl), optionally substituted arylalkylene (e.g., C₆₋₁₄arylC₁₋₃alkylene), optionally substituted heteroarylalkylene (e.g., 5- to 12-membered heteroarylC₁₋₃alkylene), and optionally substituted cycloalkylalkylene (e.g., C₃₋₁₂cycloalkylC₁₋₃alkylene);
   Y₂ is selected from C, O and N; n represents 1 or 2, and each R₅ is independently selected from H and optionally substituted alkyl (e.g., C₁₋₈alkyl);
   Ring A is selected from optionally substituted aromatic ring (e.g., C₆₋₁₄aromatic ring), optionally substituted heteroaromatic ring (e.g., 5- to 12-membered heteroaromatic ring), and optionally substituted heterocycle (e.g., 3- to 12-membered heterocycle);
   Ring B is selected from optionally substituted aromatic ring (e.g., C₆₋₁₄aromatic ring), optionally substituted heteroaromatic ring (e.g., 5- to 12-membered heteroaromatic ring), and optionally substituted heterocycle (e.g., 3- to 12-membered heterocycle);
   R₇ and R₇' are each independently selected from H, D, CN and optionally substituted alkyl (e.g., C₁₋₈alkyl);
   R₂ is selected from H, D, optionally substituted alkyl (e.g., C₁₋₈alkyl) and optionally substituted cycloalkyl (e.g., C₃₋₁₂cycloalkyl);
   R₃ and R₃' are each independently selected from H, D, and optionally substituted alkyl (e.g., C₁₋₈alkyl), and at least one of R₃ and R₃' is not H; or R₃ and R₃', together with the carbon to which they are bound, form a cycloalkyl (e.g., C₃₋₁₂cycloalkyl, e.g. C₃₋₈cycloalkyl, e.g., cyclopropyl);
   X₁ is selected from CR₆ₐ and N;
   X₂ is selected from CR_{6b} and N;
   X₃ is selected from CR_{6c} and N, and at most two of X₁, X₂ and X₃ are N;
   X₄ is selected from NR₈ and O;
   R₆ₐ, R_{6b} and R_{6c} are each independently selected from H, D, halo (e.g., F, Cl, Br, I) and optionally substituted alkyl (e.g., C₁₋₈alkyl);
   R₈ is selected from H, D, oxygen, hydroxy, optionally substituted alkyl (e.g., C₁₋₈alkyl), optionally substituted alkanoyl (e.g., C₁₋₈alkanoyl), optionally substituted alkoxycarbonyl (e.g., C₁₋₈alkoxycarbonyl), optionally substituted cycloalkyl (e.g., C₃₋₁₂cycloalkyl), optionally substituted heterocyclyl (e.g., 3- to 20-membered heterocyclyl), optionally substituted aryl (e.g., C₆₋₁₄aryl), optionally substituted heteroaryl (e.g., 5- to 12-membered heteroaryl), optionally substituted cycloalkylalkylene- (e.g., C₃₋₁₂cycloalkylC₁₋₃alkylene-), optionally substituted heterocyclylalkylene-(e.g., 3- to 20-membered heterocyclylC₁₋₃alkylene-), optionally substituted arylalkylene- (e.g., C₆₋₁₄arylC₁₋₃alkylene-), and optionally substituted heteroarylalkylene- (e.g., C₅₋₁₂heteroarylC₁₋₃alkylene-).
Embodiment 2. The compound according to Embodiment 1, or a stereoisomer, racemate, geometric isomer, tautomer, prodrug, hydrate, solvate, or pharmaceutically acceptable salt thereof, wherein: R₁ is selected from 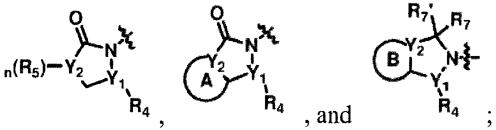 wherein,
   Y₁ is selected from CR₄' and N; wherein R₄' is selected from H, D, hydroxy, F, Cl, Br, I, cyano, and C₁₋₆alkyl (e.g., C₁₋₃alkyl) optionally substituted with 1-3 halo (e.g., F, Cl, Br, I); preferably, R₄' is selected from H, D, hydroxy, F, Cl, cyano, methyl, ethyl, methyl substituted with 1-3 fluoro and ethyl substituted with 1-3 fluoro; more preferably, R₄' is selected from H, D, hydroxy, F, cyano, methyl, and methyl substituted with 1-3 fluoro; most preferably, R₄' is selected from H, D and hydroxy;
   R₄ is selected from optionally substituted C₆₋₁₀aryl, optionally substituted 5- to 10-membered heteroaryl, optionally substituted C₃₋₈cycloalkyl, optionally substituted C₆₋₁₀arylC₁₋₃alkylene, optionally substituted 5- to 10-membered heteroarylC₁₋₃alkylene, and optionally substituted C₃₋₈cycloalkylC₁₋₃alkylene; preferably, R₄ is selected from optionally substituted phenyl, optionally substituted pyridinyl, optionally substituted pyrazinyl, optionally substituted pyridazinyl, optionally substituted pyrimidinyl, optionally substituted cyclopropyl, optionally substituted cyclobutyl, optionally substituted benzyl, optionally substituted pyridinylmethylene, optionally substituted pyrazinylmethylene, optionally substituted pyridazinylmethylene, optionally substituted pyrimidinylmethylene, optionally substituted cyclopropylmethylene, and optionally substituted cyclobutylmethylene; more preferably, R₄ is selected from optionally substituted phenyl and optionally substituted pyridinyl; for the substitution on R₄, it is preferable that the substituent is one or more groups independently selected from halo (e.g., F, Cl, Br, I), C₁₋₆alkyl (e.g., C₁₋₄alkyl), C₁₋₆alkoxy (e.g., C₁₋₄alkoxy), haloC₁₋₆alkyl (e.g., haloC₁₋₄alkyl), haloC₁₋₆alkoxy (e.g., haloC₁₋₄alkoxy), methanesulfonyl and cyano; more preferably, the substituent is one or more groups independently selected from halo (e.g., F, Cl, Br, I), C₁₋₂alkyl, C₁₋₂alkoxy, haloC₁₋₂alkyl, haloC₁₋₂alkoxy, methanesulfonyl and cyano; further preferably, the substituent is one or more groups independently selected from fluoro, chloro, methyl, ethyl, methoxy, ethoxy, methyl substituted with 1-3 fluoro, ethyl substituted with 1-3 fluoro, methoxy substituted with 1-3 fluoro, ethoxy substituted with 1-3 fluoro, methanesulfonyl and cyano;
   Most preferably, R₄ is selected from phenyl optionally substituted with one or more halo, C₁₋₆alkyl or cyano; and heteroaryl, such as pyridinyl, optionally substituted with one or more halo, C₁₋₆alkyl or cyano;
   Y₂ is selected from C, O and N; n represents 1 or 2, and each R₅ is independently selected from H and optionally substituted C₁₋₆alkyl; preferably, each R₅ is independently selected from H and optionally substituted C₁₋₃alkyl; more preferably, each R₅ is independently selected from H and methyl;
   Ring A is selected from optionally substituted 5- to 6-membered aromatic ring, optionally substituted 5- to 6-membered heteroaromatic ring, and optionally substituted 3- to 8-membered heterocycle; preferably, ring A is selected from optionally substituted benzene ring, optionally substituted pyridine, optionally substituted pyrrole, optionally substituted furan, optionally substituted thiophene, optionally substituted piperazine, and optionally substituted pyrazine;
   Ring B is selected from optionally substituted 5- to 6-membered aromatic ring, optionally substituted 5- to 6-membered heteroaromatic ring, and optionally substituted 3- to 8-membered heterocycle; preferably, ring B is selected from optionally substituted benzene ring, optionally substituted pyridine, optionally substituted pyrrole, optionally substituted furan, optionally substituted thiophene, optionally substituted piperazine, and optionally substituted pyrazine;
   For the substitution on ring A and ring B, it is preferable that the substituent is one or more, e.g, one or two groups independently selected from halo, carboxyl, C₁₋₈alkyl, -OR_{d}, 5- to 10-membered heterocyclylC₁₋₃alkylene unsubstituted or substituted with 1-3 C₁₋₃alkyl, C₁₋₃alkyleneNRₐR_{b}, 5- to 10-membered heterocyclylcarbonyl unsubstituted or substituted with 1-3 C₁₋₃alkyl, haloC₁₋₈alkyl, cyano, -C(O)NRₐR_{b}, -NRₐR_{b}, -S(O)₂C₁₋₆alkyl, -N(Rₐ)S(O)₂R_{b}, - N(Rₐ)C(O)R_{b} and -C(O)OC₁₋₆alkyl; R_{d} is H, C₆₋₁₀aryl, C₁₋₈alkyl, haloC₁₋₈alkyl, C₁₋₈alkyl substituted with -NRₐR_{b}, C₁₋₈alkyl substituted with hydroxy, or C₁₋₈alkyl substituted with C₁₋₆alkoxy; wherein Rₐ and R_{b} are each independently selected from hydrogen, C₁₋₈alkyl, and haloC₁₋₈alkyl;
   R₇ and R₇' are each independently selected from H, D, CN and optionally substituted C₁₋₆alkyl (e.g., C₁₋₃alkyl); preferably, R₇ and R₇' are each independently selected from H, D, CN, C₁₋₃alkyl and haloC₁₋₃alkyl; more preferably, R₇ and R₇' are each independently selected from H, D, CN, methyl, and methyl substituted with 1-3 halo such as fluoro;
   Preferably, R₁ is selected from

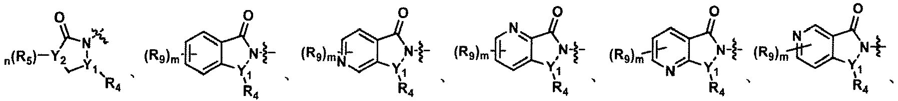

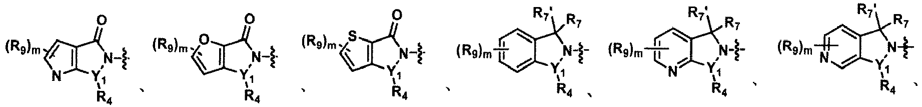

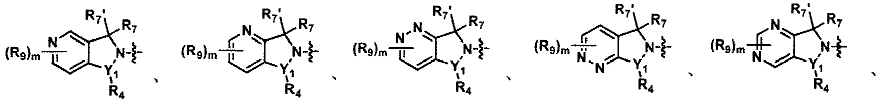

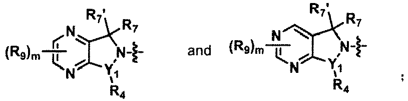

   More preferably, R₁ is selected from 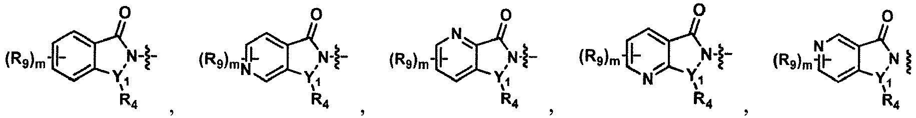 and 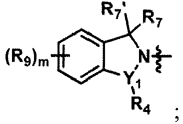 wherein,
   Y₁ is selected from CR₄' and N; wherein R₄' is selected from H, D, hydroxy, F, Cl, Br, I, cyano, and C₁₋₆alkyl (e.g., C₁₋₃alkyl) optionally substituted with 1-3 halo (e.g., F, Cl, Br, I); preferably, R₄' is selected from H, D, hydroxy, F, Cl, cyano, methyl, ethyl, methyl substituted with 1-3 fluoro and ethyl substituted with 1-3 fluoro; more preferably, R₄' is selected from H, D, hydroxy, F, cyano, methyl, and methyl substituted with 1-3 fluoro; most preferably, R₄' is selected from H, D and hydroxy;
   R₄ is selected from optionally substituted C₆₋₁₀aryl, optionally substituted 5- to 10-membered heteroaryl, optionally substituted C₃₋₈cycloalkyl, optionally substituted C₆₋₁₀arylC₁₋₃alkylene, optionally substituted 5- to 10-membered heteroarylC₁₋₃alkylene, and optionally substituted C₃₋₈cycloalkylC₁₋₃alkylene; preferably, R₄ is selected from optionally substituted phenyl, optionally substituted pyridinyl, optionally substituted pyrazinyl, optionally substituted pyridazinyl, optionally substituted pyrimidinyl, optionally substituted cyclopropyl, optionally substituted cyclobutyl, optionally substituted benzyl, optionally substituted pyridinylmethylene, optionally substituted pyrazinylmethylene, optionally substituted pyridazinylmethylene, optionally substituted pyrimidinylmethylene, optionally substituted cyclopropylmethylene, and optionally substituted cyclobutylmethylene; more preferably, R₄ is selected from optionally substituted phenyl and optionally substituted pyridinyl; for the substitution on R₄, it is preferable that the substituent is one or more groups independently selected from halo (e.g., F, Cl, Br, I), C₁₋₆alkyl (e.g., C₁₋₄alkyl), C₁₋₆alkoxy (e.g., C₁₋₄alkoxy), haloC₁₋₆alkyl (e.g., haloC₁₋₄alkyl), haloC₁₋₆alkoxy (e.g., haloC₁₋₄alkoxy), methanesulfonyl and cyano; more preferably, the substituent is one or more groups independently selected from halo (e.g., F, Cl, Br, I), C₁₋₂alkyl, C₁₋₂alkoxy, haloC₁₋₂alkyl, haloC₁₋₂alkoxy, methanesulfonyl and cyano; further preferably, the substituent is one or more groups independently selected from fluoro, chloro, methyl, ethyl, methoxy, ethoxy, methyl substituted with 1-3 fluoro, ethyl substituted with 1-3 fluoro, methoxy substituted with 1-3 fluoro, ethoxy substituted with 1-3 fluoro, methanesulfonyl and cyano;
   Most preferably, R₄ is selected from phenyl optionally substituted with one or more halo, C₁₋₆alkyl or cyano, and heteroaryl, such as pyridinyl, optionally substituted with one or more halo, C₁₋₆alkyl or cyano;
   Y₂ is selected from C, O and N; n represents 1 or 2, and each R₅ is independently selected from H and optionally substituted C₁₋₆alkyl; preferably, each R₅ is independently selected from H and optionally substituted C₁₋₃alkyl; more preferably, each R₅ is independently selected from H and methyl;
   R₉ is selected from halo, carboxyl, C₁₋₈alkyl, -OR_{d}, 5- to 10-membered heterocyclylC₁₋₃alkylene unsubstituted or substituted with 1-3 C₁₋₃alkyl, C₁₋₃alkyleneNRₐR_{b}, 5- to 10-membered heterocyclylcarbonyl unsubstituted or substituted with 1-3 C₁₋₃alkyl, haloC₁₋₈alkyl, cyano, - C(O)NRₐR_{b}, -NRₐR_{b}, -S(O)₂C₁₋₆alkyl, -N(Rₐ)S(O)₂R_{b}, -N(Rₐ)C(O)R_{b} and -C(O)OC₁₋₆alkyl; R_{d} is H, C₆₋₁₀aryl, C₁₋₈alkyl, haloC₁₋₈alkyl, C₁₋₈alkyl substituted with -NRaRb, C₁₋₈alkyl substituted with hydroxy, or C₁₋₈alkyl substituted with C₁₋₆alkoxy; wherein Rₐ and R_{b} are each independently selected from hydrogen, C₁₋₈alkyl, and haloC₁₋₈alkyl;
   Preferably, R₉ is selected from halo, carboxyl, C₁₋₆alkyl, -OR_{d}, 5- to 8-membered heterocyclylC₁₋₃alkylene unsubstituted or substituted with 1-3 C₁₋₃alkyl, C₁₋₃alkyleneNRₐR_{b}, 5-to 8-membered heterocyclylcarbonyl unsubstituted or substituted with 1-3 C₁₋₃alkyl, haloC₁₋₆alkyl, cyano, -C(O)NRₐR_{b}, -NRₐR_{b}, -S(O)₂C₁₋₄alkyl, -N(Rₐ)S(O)₂R_{b}, -N(Rₐ)C(O)R_{b} and - C(O)OC₁₋₄alkyl; R_{d} is H, C₆₋₁₀aryl, C₁₋₆alkyl, haloC₁₋₆alkyl, C₁₋₆alkyl substituted with -NRₐR_{b}, C₁₋₆alkyl substituted with hydroxy, or C₁₋₆alkyl substituted with C₁₋₃alkoxy; wherein Rₐ and R_{b} are each independently selected from hydrogen, C₁₋₆alkyl, and haloC₁₋₆alkyl;
   Preferably, R₉ is selected from halo, carboxyl, C₁₋₄alkyl, C₁₋₄alkoxy, C₁₋₄alkyl substituted with halo, C₁₋₄alkoxy substituted with halo, cyano, -C(O)NRₐR_{b}, -N(Rₐ)C(O)R_{b} and C(O)OC₁₋₄alkyl, Rₐ and R_{b} are each independently selected from hydrogen, C₁₋₄alkyl, and C₁₋₄alkyl substituted with halo,
   and m is the number of R₉, m is selected from 0, 1, 2 and 3; preferably, m is 0, 1 or 2;
   Most preferably, R₁ is selected from 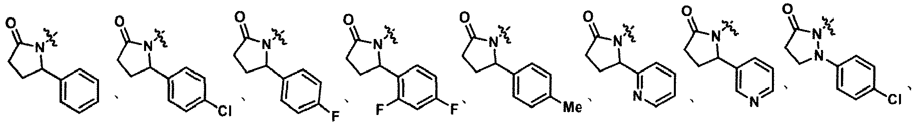 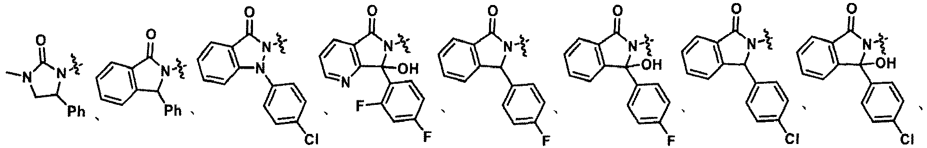 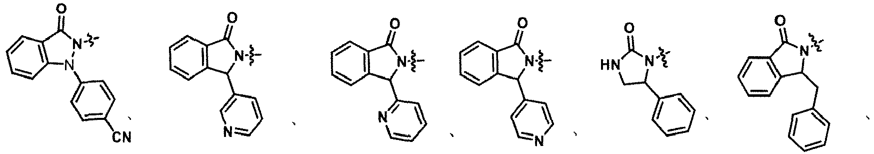 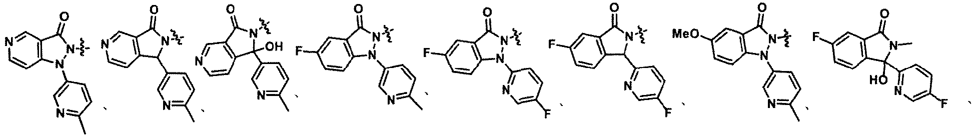 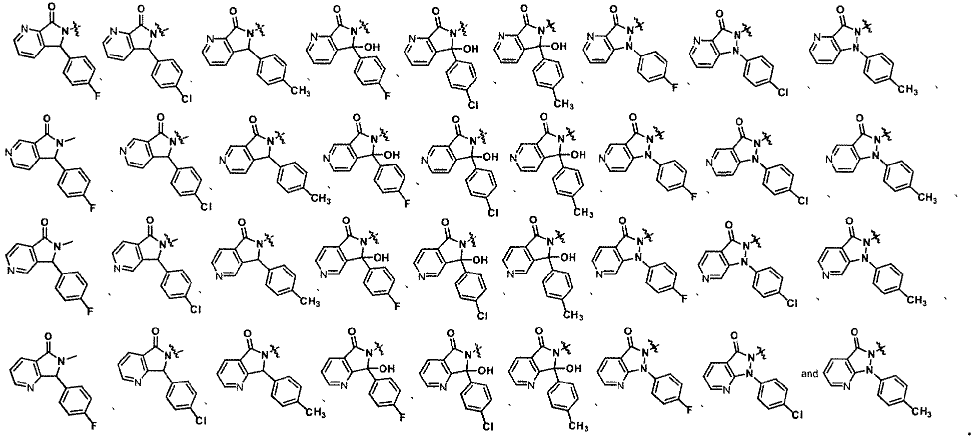
Embodiment 3. The compound according to any of previous embodiments or a stereoisomer, racemate, geometric isomer, tautomer, prodrug, hydrate, solvate, or pharmaceutically acceptable salt thereof, characterized in that:
   X₁ is N or CH;
   X₂ is CR_{6b} or N;
   X₃ is CR_{6c};
   R_{6b} and R_{6c} are each independently selected from H, D, halo and optionally substituted C₁₋₆alkyl such as C₁₋₃alkyl; preferably, R_{6b} and R_{6c} are each independently selected from H, D, fluoro, methyl, and methyl substituted with 1-3 halo such as fluoro; more preferably, R₆ₐ, R_{6b} and R_{6c} are each independently selected from H, D, fluoro and methyl.
Embodiment 4. The compound according to any of previous embodiments or a stereoisomer, racemate, geometric isomer, tautomer, prodrug, hydrate, solvate, or pharmaceutically acceptable salt thereof, characterized in that:
   X₄ is selected from NR₈ and O, wherein
   R₈ is selected from H, D, oxygen, hydroxy, optionally substituted C₁₋₄alkyl, optionally substituted C₁₋₄alkanoyl, optionally substituted C₁₋₄alkoxycarbonyl, optionally substituted C₃₋₈cycloalkyl, optionally substituted 3- to 8-membered heterocyclyl (e.g., 3- to 8-membered heterocyclyl containing oxygen or nitrogen as a heteroatom), optionally substituted C₆₋₁₀aryl, optionally substituted 5- to 10-membered heteroaryl, optionally substituted C₃₋₈cycloalkylC₁₋₃alkylene- (e.g., C₃₋₈cycloalkylmethylene), optionally substituted 3- to 8-membered heterocyclylC₁₋₃alkylene-, optionally substituted C₆₋₁₀arylalkylene- (e.g., benzyl), and optionally substituted 5- to 10-membered heteroarylC₁₋₃alkylene- (e.g., pyridinylC₁₋₃alkylene-, such as pyridinylmethylene-);
   Preferably, R₈ is selected from H, D, oxygen, hydroxy, methyl, ethyl, propyl such as isopropyl hydroxyethyl, propyl substituted with hydroxy, C₂₋₄alkyl substituted with C₁₋₂alkoxy, methoxycarbonyl, cyclopropyl, cyclobutyl, cyclopentyl, oxetanyl, oxolanyl (i.e., oxacyclopentyl), tetrahydropyranyl, cyclopropanemethylene-, benzyl, and pyridinylmethylene;
   It is preferable for the substitution on R₈ that the substituent is one or more groups independently selected from -OH, F, CN, -NH₂, C₁₋₆alkoxy (e.g., C₁₋₂alkoxy), -NH(C₁₋₃alkoxy), and -N(C₁₋₃alkoxy)₂.
Embodiment 5. The compound according to any of previous embodiments or a stereoisomer, racemate, geometric isomer, tautomer, prodrug, hydrate, solvate, or pharmaceutically acceptable salt thereof, characterized in that:
   R₂ is selected from H, D, optionally substituted C₁₋₆alkyl (e.g., C₁₋₄alkyl) and optionally substituted C₃₋₈cycloalkyl (e.g., C₃₋₆cycloalkyl); it is preferable for the substitution on R₂ that the substituent is one,two, three or more groups independently selected from halo (e.g., F, Cl, Br and I) and hydroxy;
   More preferably, R₂ is selected from H, D, C₁₋₃alkyl optionally substituted with 1-3 halo such as fluoro and/or hydroxy, and C₃₋₄cycloalkyl optionally substituted with 1-3 halo such as fluoro and/or hydroxy;
   Further preferably, R₂ is selected from H, D, methyl, ethyl, propyl, hydroxyethyl, -CHF₂, - CH₂F, -CF₃, CH₂CF₃ and cyclopropyl.
Embodiment 6. The compound according to any of previous embodiments or a stereoisomer, racemate, geometric isomer, tautomer, prodrug, hydrate, solvate, or pharmaceutically acceptable salt thereof, characterized in that:
   R₃ and R₃' are each independently selected from H, D, and optionally substituted C₁₋₆alkyl (e.g., C₁₋₃alkyl), and at least one of R₃ and R₃' is not H;
   Preferably, R₃ and R₃' are each independently selected from H, D, and C₁₋₃alkyl (e.g., methyl, ethyl) optionally substituted with one, two or more halo (e.g., F, Cl, Br, and I), and at least one of R₃ and R₃' is not H;
      Further preferably, R₃ and R₃' are each independently selected from H, methyl and ethyl, and at least one of R₃ and R₃' is not H;
      Or further preferably, either of R₃ and R₃' is methyl, and the other is H.
Embodiment 7. The compound according to embodiment 1, or a stereoisomer, racemate, geometric isomer, tautomer, prodrug, hydrate, solvate, or pharmaceutically acceptable salt thereof, wherein: R₁ is selected from 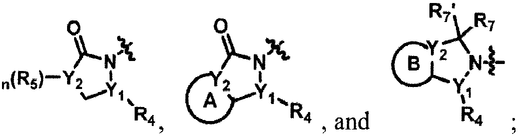 wherein,
   Y₁ is selected from CR₄' and N; wherein R₄' is selected from H, D, hydroxy, F, Cl, cyano, methyl, ethyl, methyl substituted with 1-3 fluoro and ethyl substituted with 1-3 fluoro;
   R₄ is selected from optionally substituted phenyl, optionally substituted heteroaryl, optionally substituted C₃₋₈cycloalkyl, optionally substituted benzyl, optionally substituted heteroarylalkylene, and optionally substituted C₃₋₈cycloalkylalkylene;
   Y₂ is selected from C, O and N; n represents 1 or 2, and each R₅ is independently selected from H and optionally substituted C₁₋₃alkyl;
   Ring A is selected from optionally substituted benzene ring, and optionally substituted 5- to 6-membered heteroaromatic ring;
   Ring B is selected from optionally substituted benzene ring, and optionally substituted 5- to 6-membered heteroaromatic ring such as pyridine ring;
   R₇ and R₇' are each independently selected from H, D, CN and optionally substituted C₁₋₃alkyl;
   R₂ is selected from H, D, optionally substituted C₁₋₄alkyl and optionally substituted C₃₋₄cycloalkyl;
   R₃ and R₃' are each independently selected from H, D, and optionally substituted C₁₋₆alkyl (e.g., C₁₋₃alkyl), preferably are each independently selected from H, D, methyl, and ethyl, and at least one of R₃ and R₃' is not H; or R₃ and R₃', together with the carbon to which they are bound, form a cyclopropyl;
   X₁ is selected from CR₆ₐ and N;
   X₂ is selected from CR_{6b} and N;
   X₃ is selected from CR_{6c} and N, and at most two of X₁, X₂ and X₃ are N;
   X₄ is selected from NR₈ and O;
   R₆ₐ, R_{6b} and R_{6c} are each independently selected from H, D, halo and optionally substituted C₁₋₃alkyl;
   R₈ is selected from H, D, oxygen, hydroxy, optionally substituted C₁₋₆alkyl, optionally substituted C₁₋₆alkanoyl, optionally substituted C₁₋₆alkylcarbonyl, optionally substituted C₃₋₈cycloalkyl, optionally substituted 3- to 8-membered heterocyclyl and optionally substituted benzyl.
Embodiment 8. The compound according to embodiment 1, or a stereoisomer, racemate, geometric isomer, tautomer, prodrug, hydrate, solvate, or pharmaceutically acceptable salt thereof, characterized in that: R₁ is selected from 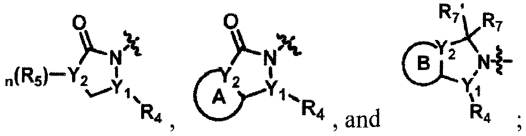 wherein,
   Y₁ is selected from CR₄' and N; wherein R₄' is selected from H, D, hydroxy, F, Cl, cyano, methyl, ethyl, methyl substituted with 1-3 fluoro, and ethyl substituted with 1-3 fluoro;
   R₄ is selected from optionally substituted phenyl, optionally substituted pyridinyl, optionally substituted pyrazinyl, optionally substituted pyridazinyl, optionally substituted pyrimidinyl, optionally substituted C₃₋₄cycloalkyl, optionally substituted pyridinylmethylene, and optionally substituted C₃₋₄cycloalkylmethylene; it is preferable for the substitution on R₄ that the substituent is one or more groups independently selected from halo, C₁₋₄alkyl, C₁₋₄alkoxy, haloC₁₋₄alkyl, haloC₁₋₄alkoxy, methanesulfonyl and -CN;
   Y₂ is selected from C, O and N; n represents 1 or 2, and each R₅ is independently selected from H and optionally substituted C₁₋₃alkyl;
   Ring A is selected from optionally substituted benzene ring, optionally substituted pyridine, optionally substituted pyrrole, optionally substituted furan and optionally substituted thiophene;
   Ring B is selected from optionally substituted benzene ring, and optionally substituted pyridine;
      It is preferable for the substitution on ring A and ring B that the substituent is one or more, e.g., one or two groups independently selected from halo, carboxyl, C₁₋₈alkyl, haloC₁₋₈alkyl, cyano, -C(O)NRₐR_{b}, -OR_{d}, 5- to 10-membered heterocyclylC₁₋₃alkylene unsubstituted or substituted with 1-3 C₁₋₃alkyl, C₁₋₃alkyleneNRₐR_{b}, 5- to 10-membered heteroarylcarbonyl unsubstituted or substituted with 1-3 C₁₋₃alkyl, -NRₐR_{b}, -S(O)₂C₁₋₆alkyl, -N(Rₐ)S(O)₂R_{b}, - N(Rₐ)C(O)R_{b} and -C(O)OC₁₋₈alkyl, wherein Rₐ and R_{b} are each independently selected from hydrogen, C₁₋₈alkyl, and haloC₁₋₈alkyl; R_{d} is H, C₆₋₁₀aryl, C₁₋₈alkyl, haloC₁₋₈alkyl, C₁₋₈alkyl substituted with -NRₐR_{b}, C₁₋₈alkyl substituted with hydroxy, or C₁₋₈alkyl substituted with C₁₋₃alkoxy; wherein Rₐ and R_{b} are each independently selected from hydrogen, C₁₋₆alkyl, and haloC₁₋₆alkyl;
   R₇ and R₇' are each independently selected from H, D, CN, C₁₋₃alkyl and haloC₁₋₃alkyl; R₂ is selected from H, D, C₁₋₂alkyl and C₁₋₂alkyl substituted with 1-3 fluoro;
   R₃ and R₃' are each independently selected from H, D, and optionally substituted C₁₋₆alkyl (e.g., C₁₋₃alkyl), preferably are each independently selected from H, D, methyl, and ethyl, and at least one of R₃ and R₃' is not H;
   X₁ is selected from CR₆ₐ and N;
   X₂ is selected from CR_{6b} and N;
   X₃ is selected from CR_{6c} and N, and at most two of X₁, X₂ and X₃ arc N;
   X₄ is selected from NR₈ and O;
   R₆ₐ, R_{6b} and R_{6c} are each independently selected from H, D, F, Cl, methyl and methyl substituted with 1-3 fluoro;
   R₈ is selected from H, D, oxygen, hydroxy, optionally substituted C₁₋₄alkyl, optionally substituted C₃₋₆cycloalkyl, optionally substituted 3- to 6-membered heterocyclyl containing oxygen or nitrogen as a heteroatom, optionally substituted C₃₋₆cycloalkylmethylene, optionally substituted benzyl, and optionally substituted pyridinylmethylene; it is preferable for the substituents on R₈ that the substituent is one or more, e.g., 1-3 groups independently selected from -OH, halo (e.g., F, Cl, Br and I), CN and C₁₋₆alkoxy (e.g., C₁₋₂alkoxy).
Embodiment 9. The compound according to embodiment 1, or a stereoisomer, racemate, geometric isomer, tautomer, prodrug, hydrate, solvate, or pharmaceutically acceptable salt thereof, characterized in that:
   R₁ is selected from 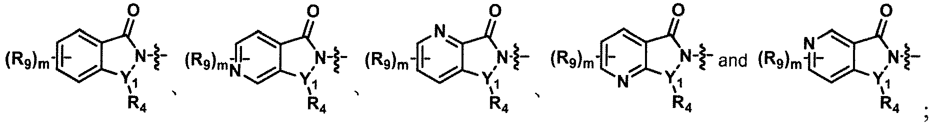
   Y₁ is selected from CR₄' and N; wherein R₄' is selected from H, D, and hydroxy;
   R₄ is selected from optionally substituted phenyl and optionally substituted pyridinyl; it is preferable for the substitution on R₄ that the substituent is one or more groups independently selected from F, Cl, C₁₋₂alkyl, C₁₋₂alkyl substituted with 1-3 fluoro, and -CN;
   R₉ is selected from fluoro, chloro, carboxyl, methyl, methoxy, fluoromethyl, and fluoromethoxy, and m is the number of R₉, and m is 0, 1 or 2;
   R₂ is selected from methyl, ethyl, -CHF₂, -CH₂F and -CF_{3;}
   R₃ and R₃' are each independently selected from H, D, methyl, and ethyl, and at least one of R₃ and R₃' is not H;
   X₁ is N;
   X₂ is CR_{6b} or N;
   X₃ is CR_{6c};
   X₄ is NR₈ or O;
   R_{6b} and R_{6c} are each independently selected from H and F;
   R₈ is selected from H, D, oxygen, hydroxy, methyl, ethyl, propyl such as isopropyl, hydroxyethyl, cyclopropyl, cyclobutyl, cyclopentyl, oxetanyl, furyl, pyranyl, cyclopropanemethylene, benzyl and pyridinylmethylene, It is preferable for the substitution on R₈ that the substituent is one or more groups independently selected from halo (e.g., F, Cl, Br and I), CN and C₁₋₆alkoxy such as methoxy.
Embodiment 10. The compound according to previous embodiments, or a stereoisomer, racemate, geometric isomer, tautomer, prodrug, hydrate, solvate, or pharmaceutically acceptable salt thereof, characterized in that: Formula (I) is: 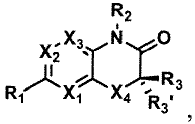 wherein each variable is correspondingly a defined in each claim;
   Preferably, R₃ and R₃' are each independently selected from H, D, and optionally substituted C₁₋₆alkyl (e.g., C₁₋₃alkyl), and at least one of R₃ and R₃' is not H;
   Preferably, R₃ and R₃' are each independently selected from H, D, and C₁₋₃alkyl (e.g., methyl, ethyl) optionally substituted with one, two or more halo (e.g., F, Cl, Br and I), and at least one of R₃ and R₃' is not H;
   Further preferably, R₃ is methyl or ethyl, and R₃' is H; more preferably, R₃ is methyl, and R₃' is H.
Embodiment 11. The compound according to any of previous embodiments or a stereoisomer, racemate, geometric isomer, tautomer, prodrug, hydrate, solvate, or pharmaceutically acceptable salt thereof, wherein the compound is selected from the compounds of the examples.
Embodiment 12. A pharmaceutical composition, comprising the compound according to any of Embodiments 1-11, or a stereoisomer, racemate, geometric isomer, tautomer, prodrug, hydrate, solvate, or pharmaceutically acceptable salt thereof, and optionally a pharmaceutically acceptable carrier.
Embodiment 13. Use of the compound according to any of Embodiments 1-11, or a stereoisomer, racemate, geometric isomer, tautomer, prodrug, hydrate, solvate, or pharmaceutically acceptable salt thereof, in the manufacture of a medicament for the treatment and/or prevention of a disease mediated by bromodomain proteins, or a product as a bromodomain protein inhibitor.
Embodiment 14. The use according to embodiment 13, wherein the disease mediated by bromodomain proteins include cancer such as hematological malignant tumor, midline carcinoma, inflammatory diseases, cardiovascular diseases, viral infections, fibrotic diseases, metabolic diseases, radiation poisoning, acute rejection of transplanted organs or multiorgan dysfunction syndrome and Alzheimer's disease.
Embodiment 15. A method for non-therapeutically inhibiting bromodomain protein activity, comprising contacting an effective amount of the compound according to any of embodiments 1-11, or a stereoisomer, racemate, geometric isomer, tautomer, prodrug, hydrate, solvate, or pharmaceutically acceptable salt thereof, with the bromodomain proteins, thereby inhibiting the bromodomain proteins.

### Definitions

The following terms and symbols used in the present application have the meanings as described below, unless otherwise specified in the context.

A dash ("-") that is not between two letters or symbols indicates a point of attachment of a substituent. For example, C₁₋₆alkylcarbonyl- refers to C₁₋₆ alkyl which is attached to the rest of the molecule through carbonyl. However, when the point of attachment of a substituent is apparent to those skilled in the art, for example, for a halo substituent, the dash "-" may be omitted.

When a group carries a wavy line " 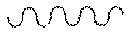 ", for example in 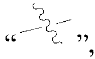 the wavy line indicates the point of attachment of the group to the rest of the molecule.

The term "hydrogen" as used herein refers to the group -H.

The symbol "D" as used herein refers to deuterium.

The term "hydroxyl" as used herein refers to the group -OH.

The term "halo" or "halogen" as used herein refers to fluoro (F), chloro (Cl), bromo (Br) and iodo (I), preferably fluoro and chloro, and most preferably fluoro.

The term "cyano" as used herein refers to the group -CN.

As used herein, the term "alkyl" refers to a straight or branched chain saturated monovalent hydrocarbon radical having from 1 to 8 carbon atoms, such as from 1 to 6 carbon atoms, such as from 1 to 4 carbon atoms, such as 1, 2 or 3 carbon atoms. For example, "C₁₋₈ alkyl" refers to alkyl having from 1 to 8 carbon atoms. Similarly, "C₁₋₆alkyl" refers to alkyl having from 1 to 6 carbon atoms; "C₁₋₄ alkyl" refers to alkyl having from 1 to 4 carbon atoms, and "C₁₋₃ alkyl" refers to alkyl having from 1 to 3 carbon atoms. Examples of alkyl include, but are not limited to, methyl ("Me"), ethyl ("Et"), propyl such as n-propyl ("n-Pr") or isoproyl ("i-Pr"), butyl such as n-butyl ("n-Bu"), isobutyl ("i-Bu"), sec-butyl ("s-Bu") or t-butyl ("t-Bu"), pentyl, hexyl, etc. Whether the term "alkyl" is used alone or as part of another group such as haloalkyl, alkoxy, etc., this definition applies.

As used herein, the term "alkylene" refers to a straight or branched chain saturated divalent hydrocarbon radical having from 1 to 8 carbon atoms, such as from 1 to 6 carbon atoms, such as from 1 to 4 carbon atoms, such as 1, 2 or 3 carbon atoms. For example, "C₁₋₈alkylene" refers to alkylene having from 1 to 8 carbon atoms. Similarly, "C₁₋₆alkylene" refers to alkylene having from 1 to 6 carbon atoms; "C₁₋₄alkylene" refers to alkylene having from 1 to 4 carbon atoms; "C₁₋₃alkylene" refers to alkylene having from 1 to 3 carbon atoms. Examples of alkylene include, but are not limited to, methylene, ethylene, propylene, butylene, etc. The term "cycloalkylalkylene" refers to cycloalkyl which is attached to the rest of the molecule through alkylene. Similarly, the term "heterocyclylalkylene" refers to heterocyclyl which is attached to the rest of the molecule through alkylene; the term "arylalkylene" refers to aryl which is attached to the rest of the molecule through alkylene; the term "heteroarylalkylene" refers to heteroaryl which is attached to the rest of the molecule through alkylene; and so on.

As used herein, the term "alkoxy" refers to the group -O-alkyl, wherein alkyl is as defined above. For example, "C₁₋₈alkoxy" refers to -O-C₁₋₈alkyl, i.e., alkoxy having from 1 to 8 carbon atoms. Similarly, "C₁₋₆alkoxy" refers to -O-C₁₋₆alkyl, i.e., alkoxy having from 1 to 6 carbon atoms; "C₁₋₄alkoxy" refers to -O-C₁₋₄alkyl, i.e., alkoxy having from 1 to 4 carbon atoms; "C₁₋₃alkoxy" refers to - O-C₁₋₃alkyl, i.e., alkoxy having from 1 to 3 carbon atoms. Examples of alkoxy include, but are not limited to, methoxy, ethoxy, propoxy such as n-propoxy or isopropoxy, butoxy such as n-butoxy, isobutoxy or t-butoxy, pentoxy and hexoxy etc. Whether the term "alkoxy" is used alone or as part of another group, this definition applies.

As used herein, the term "haloalkyl" refers to alkyl as defined herein wherein one or more hydrogen atoms, for example 1, 2, 3, 4 or 5 hydrogen atoms, are replaced by halogen; when more than one hydrogen atom is replaced by a halogen atom, said halogen atoms may be the same or different from one another. Examples of haloalkyl include, but are not limited to, -CF₃, -CHF₂ and - CH₂CF₃ etc.

As used herein, the term "hydroxylalkyl" or "hydroxyalkyl" refers to alkyl substituted with hydroxyl, wherein alkyl is as defined herein. Examples of such group include, but not limited to hydroxymethyl, hydroxyethyl (e.g., 2-hydroxyethyl, 1-hydroxyethyl), hydroxypropyl (e.g., 1-hydroxyprop-2-yl, 1-hydroxyprop-3-yl, 1-hydroxyprop-1-yl etc.), hydroxybutyl (e.g., 4-hydroxylbut-2-yl etc.).

As used herein, the term "cycloalkyl" refers to a saturated, monovalent monocyclic or bicyclic hydrocarbon radical having from 3 to 12 ring carbon atoms, such as from 3 to 8 ring carbon atoms, such as from 3 to 6 ring carbon atoms, such as from 3 to 4 ring carbon atoms. For example, "C₃₋₁₂cycloalkyl" refers to cycloalkyl having from 3 to 12 ring carbon atoms. Similarly, "C₃₋₈cycloalkyl" refers to cycloalkyl having from 3 to 8 ring carbon atoms; "C₃₋₆cycloalkyl" refers to cycloalkyl having from 3 to 6 ring carbon atoms; "C₃₋₄cycloalkyl" refers to cycloalkyl having from 3 to 4 ring carbon atoms. Examples of cycloalkyl include, but are not limited to, cyclopropyl, cyclobutyl, cyclopentyl, cyclohexyl, cycloheptyl and cyclooctyl etc.

As used herein, the term "heterocyclyl" or "heterocyclic" or "heterocycle" refers to monocyclic, bicyclic or tricyclic, saturated or partially unsaturated non-aromatic rings having from 3 to 20 ring atoms, such as from 3 to 12 ring atoms, such as from 3 to 8 ring atoms, such as from 3 to 6 ring atoms, which contain at least one carbon atom in addition to from 1 to 4, such as from 1 to 3, such as 1 or 2, such as 1, heteroatoms selected from O, S and N. In one example, said "heterocyclyl" or "heterocyclic" or "heterocycle" is monocyclic ring having from 3 to 8 ring atoms, such as 3, 4, 5 or 6 ring atoms, which contains at least one carbon atom in addition to from 1 to 4, such as from 1 to 3, such as 1 or 2, such as 1, heteroatoms selected from O, S and N. In one example, said "heterocyclyl" or "heterocyclic" or "heterocycle" contains 0, 1, 2 or 3 double bonds. Any nitrogen or sulfur heteroatom may be optionally oxidized (e.g., NO, SO, SO₂), and any nitrogen heteroatom may be optionally quaternized (e.g., [NR₄]⁺Cl⁻, [NR₄]⁺OH⁻). Heterocyclyl having from 3 to 8 ring atoms is also referred to simply as 3- to 8-membered heterocyclyl, and heterocyclyl having other numbers of ring atoms can be similarly abbreviated. Examples of heterocyclyl include, but are not limited to, oxiranyl, aziridinyl, thiiranyl, azetidinyl, oxetanyl, thietanyl, 1,2-dithietanyl, 1,3-dithietanyl, pyrrolidinyl (pyrrolidin-1-yl, pyrrolidin-2-yl, pyrrolidin-3-yl), dihydro-1H-pyrrolyl, dihydrofuranyl, tetrahydrofuranyl (e.g., tetrahydrofuran-2-yl, tetrahydrofuran-3-yl, tetrahydrofuran-4-yl), dihydrothienyl, tetrahydrothienyl, imidazolidinyl, piperidinyl, piperazinyl (e.g., piperazin-1-yl, piperazin-2-yl, piperazin-3-yl, piperazin-4-yl), isoquinolinyl, tetrahydroisoquinolinyl, morpholinyl (e.g., morpholino (i.e., morpholin-1-yl), morpholin-2-yl, morpholin-3-yl), thiomorpholinyl, 1,1-dioxo-thiomorpholinyl, dihydropyranyl, tetrahydropyranyl (e.g., tetrahydropyran-2-yl, tetrahydropyran-3-yl, tetrahydropyran-4-yl), hexahydrothiopyranyl, hexahydropyrimidyl, oxazitanyl, thiazinanyl, thioxanyl, homopiperazinyl, homopiperidinyl, azepanyl, oxepanyl, thiepanyl, oxazepinyl, oxazepanyl, diazepanyl, 1,4-diazepanyl, diazepinyl, thiazepinyl, thiazepanyl, tetrahydrothiopyranyl, oxazolidinyl, thiazolidinyl, isothiazolidinyl, 1,1-dioxoisothiazolidonyl, oxazolidinonyl, imidazolidinonyl, 4,5,6,7-tetrahydro[2H]indazolyl, tetrahydrobenzimidazolyl, 4,5,6,7-tetrahydrobenzo[d]imidazolyl, 1,6-dihydroimidazo[4,5-d]pyrrolo[2,3-b]pyridinyl, thiazinyl, oxazinyl, thiadiazinyl, oxadiazinyl, dithiazinyl, dioxazinyl, oxathiazinyl, thiatriazinyl, oxatriazinyl, dithiadiazinyl, imidazolinyl, dihydropyrimidyl, tetrahydropyrimidinyl, 1-pyrrolinyl, 2-pyrrolinyl, 3-pyrrolinyl, indolinyl, thiopyranyl, 2H-pyranyl, 4H-pyranyl, dioxanyl, 1,3-dioxolanyl, pyrazolinyl, pyrazolidinyl, dithianyl, dithiolanyl, pyrimidinonyl, pyrimidindionyl, pyrimidin-2,4-dionyl, piperazinonyl, piperazindionyl, pyrazolidinyl, imidazolinyl, 3-azabicyclo[3.1.0]hexanyl, 3,6-diazabicyclo[3.1.1]heptanyl, 6-azabicyclo[3.1.1]heptanyl, 3-azabicyclo[3.1.1]heptanyl, 3-azabicyclo[4.1.0]heptanyl, azabicyclo[2.2.2]hexanyl, 2-azabicyclo[3.2.1]octanyl, 8-azabicyclo[3.2.1]octanyl, 2-azabicyclo[2.2.2]octanyl, 8-azabicyclo[2.2.2]octanyl, 7-oxabicyclo[2.2.1]heptanyl, azaspiro[3.5]nonanyl, azaspiro[2.5]octanyl, azaspiro[4.5]decanyl, 1-azaspiro[4.5]decan-2-onyl, azaspiro[5.5]undecanyl, tetrahydroindolyl, octahydroindolyl, tetrahydroisoindolyl, tetrahydroindazolyl, 1,1-dioxohexahydrothiopyranyl. Examples of 5-membered heterocylyl containing sulfur or oxygen atom and from 1 to 3 nitrogen atoms are: thiazolyl, including thiazol-2-yl and thiazol-2-yl N-oxide; thiadiazolyl, including 1,3,4-thiadiazol-5-yl and 1,2,4-thiadiazol-5-yl; oxazolyl, such as oxazol-2-yl; and oxadiazolyl, such as 1,3,4-oxadiazol-5-yl and 1,2,4-oxadiazol-5-yl. Examples of 5-memberred heterocyclyl containing from 2 to 4 nitrogen atoms include: imidazolyl, such as imidazol-2-yl; triazolyl, such as 1,3,4-triazol-5-yl, 1,2,3-triazol-5-yl, 1,2,4-triazol-5-yl; and tetrazolyl, such as 1H-tetrazol-5-yl. Examples of benzo-fused 5-membered heterocyclyl are: benzoxazol-2-yl, benzothiazol-2-yl and benzimidazol-2-yl. Exemplary 6-membered heterocyclyl contains from 1 to 3 nitrogen atoms and optionally sulfur or oxygen atoms, such as: pyridinyl, such as pyridin-2-yl, pyridin-3-yl and pyridin-4-yl; pyrimidinyl, such as pyrimidin-2-yl and pyrimidin-4-yl; triazinyl, such as 1,3,4-triazin-2-yl and 1,3,5-triazin-4-yl; pyridazinyl, especially pyridazin-3-yl; and pyrazinyl. Further examples of heterocycyl are pyridine N-oxide and pyridazine N-oxide as well as pyridinyl, pyrimidin-2-yl, pyrimidin-4-yl, pyridazinyl and 1,3,4-triazin-2-yl.

As used herein, the term "aryl" refers to a carbocyclic hydrocarbon radical consisting of one or more fused rings, wherein at least one ring is aromatic, and having from 6 to 14 ring carbon atoms, such as from 6 to 12 ring carbon atoms, such as from 6 to 10 ring carbon atoms. Example of aryl include, but not limited to phenyl, naphthyl, 1,2,3,4-tetrahydronaphthyl, indenyl, preferably phenyl and naphthyl. Accordingly, the term "aromatic ring" as used herein refers to a ring of the aryl as defined above.

As used herein, the term "heteroaryl" refers to:
A monocyclic aromatic hydrocarbon radical having 5, 6 or 7 ring atoms, such as having 6 ring atoms, and containing one or more, such as 1, 2 or 3, such as 1 or 2, ring heteroatoms independently selected from N, O and S (e.g. N) in the ring, with the remaining ring atoms being carbon atoms; and
A bicyclic aromatic hydrocarbon radical having from 8 to 12 ring atoms, such as having 9 or 10 ring atoms, and containing one or more, such as 1, 2, 3 or 4, such as 1 or 2, ring heteroatoms independently selected from N, O and S (e.g. N) in the rings, with the remaining ring atoms being carbon atoms, wherein at least one of the rings is aromatic.

When the total number of S and O atoms in the heteroaryl group exceeds 1, those heteroatoms are not adjacent to one another.

The heteroaryl group also includes those wherein the N ring heteroatom occurs as N-oxide, such as pyrimidinyl N-oxides.

In some embodiments, the ring heteroatom(s) in the above heteroaryl is/are N atom(s), referred to as "nitrogen-containing heteroaryl". The nitrogen-containing heteroaryl also includes those wherein the N ring heteroatom occurs as N-oxide, such as pyridyl N-oxides.

Examples of heteroaryl include, but are not limited to, pyridyl (e.g., pyridin-2-yl, pyridin-3-yl, pyridin-4-yl, pyridin-5-yl, pyridin-6-yl), pyridyl N-oxide; pyrazinyl; pyrimidinyl; pyrazolyl (e.g., pyrazol-5-yl, pyrazol-1-yl, pyrazol-2-yl, pyrazol-3-yl, pyrazol-4-yl); imidazolyl; oxazolyl; isoxazolyl; thiazolyl; isothiazolyl; thiadiazolyl; tetrazolyl; triazolyl; thienyl; furyl; pyranyl; pyrrolyl; pyridazinyl; benzo[d]thiazolyl; bezodioxolyl, such as benzo[d][1,3]dioxolyl; benzoxazolyl, e.g., benzo[d]oxazolyl; imidazopyridyl, e.g., imidazo[1,2-a]pyridyl; triazolopyridyl, e.g., [1,2,4]triazolo[4,3-a]pyridyl and [1,2,4]triazolo[1,5-a]pyridyl; indazolyl, 2H-indazolyl; pyrrolopyrimidinyl, e.g., pyrrolo[3,4-d]pyrimidinyl, 7H-pyrrolo[2,3-d]pyrimidinyl; pyrazolopyrimidinyl, e.g., pyrazolo[1,5-a]pyrimidinyl; tetrazolopyridyl, e.g., tetrazolo[1,5-a]pyridyl; benzothienyl; benzofuryl; benzoimidazolinyl; indolyl; indolinyl; purinyl, e.g., 9H-purinyl and 7H-purinyl; quinolinyl; isoquinolinyl; 1,2,3,4-tetrahydroquinolinyl; and 5,6,7,8-tetrahydroisoquinolinyl.

Examples of the nitrogen-containing heteroaryl include, but are not limited to, pyrrolyl; pyrazolyl; imidazolyl; pyridyl; pyrazinyl; pyrimidinyl, pyrimidinyl N-oxide; pyridazinyl; pyrrolopyrimidinyl, e.g., pyrrolo[3,4-d]pyrimidinyl, 7H-pyrrolo[2,3-d]pyrimidinyl; purinyl, e.g., 9H-purinyl and 7H-purinyl; quinolinyl; indolyl; and indazolyl.

Accordingly, as used herein, the term "heteroaromatic ring" refers to a ring of the heteroaryl as defined above.

As used herein, "aryl" or "aromatic" follows Hückel's rule, wherein the number of π electrons is equal to 4n +2, wherein n is 0 or any positive integer up to 6.

As used herein, the term "carbonyl" refers to the group -C(O)-, i.e., -CO-.

As used herein, the term "alkylcarbonyl" refers to alkyl group attached to other group through carbonyl, i.e., alkyl-C(O)-, wherein alkyl is as defined herein.

As used herein, the term "alkoxycarbonyl" refers to alkoxy group attached to another group through carbonyl group, i.e., alkoxy-C(O)-, wherein alkoxy is as defined above.

As used herein, the term "alkanoyl" refers to the group alkyl-COO-, wherein alkyl is as defined above.

As used herein, the term "amino" refers to the group -NH₂.

As used herein, the term "alkylamino" or "monoalkylamino" refers to the group alkyl-NH-, wherein alkyl is as defined above.

As used herein, the term "dialkylamino" refers to the group (alkyl)₂-N-, wherein alkyl is as defined above.

As used herein, the term "oxo" refers to the group =O.

As used herein, the term "benzyl" refers to phenyl group attached through methylene group.

As used herein, the term "optional" means that the subsequently described event may or may not occur, and that the description includes instances where the event occurs and instances where it does not. For example, "optionally substituted alkyl" refers to both "unsubstituted alkyl" and "substituted alkyl", where alkyl is as defined herein. It will be understood by those skilled in the art, with respect to any group containing one or more substituents, that such groups are not intended to introduce any substitution or substitution patterns that are sterically impractical, chemically incorrect, synthetically non-feasible and/or inherently unstable.

As used herein, the term "substituted" or "substituted with......" means that one or more hydrogen atoms on a given atom or group are replaced, for example with one or more substituents selected from the indicated group of substituents, provided that the designated atom's normal valence is not exceeded. When a substituent is oxo (i.e., =O), then two hydrogen atoms on a single atom are replaced by the oxo. Combinations of substituents and/or variables are permissible only if such combinations result in a chemically correct and stable compound. A chemically correct and stable compound means a compound that is sufficiently robust to survive isolation from a reaction mixture for the identification of the chemical structure of the compound, and subsequently to be prepared into a formulation having at least one practical utility. For example, under the circumstances that the substituents are not specifically indicated, the term "substituted " or "substitution" as used herein means that one or more hydrogen atoms on a given atom or group are independently replaced by one or more, for example 1, 2, 3 or 4 substituents independently selected from: deuterium (D), halo, -OH, mercapto, cyano, -CD₃, alkyl (preferably C₁₋₆alkyl), alkoxy (preferably C₁₋₆alkoxy), alkylthio (preferably C₁₋₆alkylthio), haloalkyl (preferably haloC₁-C₆alkyl), haloalkoxy (preferably haloC₁-C₆alkoxy), -C(O)NRₐR_{b} and -N(Rₐ)C(O)R_{b} and -C(O)OC₁₋₄alkyl (wherein Rₐ and R_{b} are each independently selected from hydrogen, C₁₋₄alkyl, haloC₁₋₄alkyl and C₃₋₈cycloalkyl, or Rₐ and R_{b} together with the nitrogen atom carrying them form C₃₋₈heterocyclyl), carboxyl (-COOH), cycloalkyl (preferably 3- to 8-membered cycloalkyl), heterocyclyl (preferably 3- to 8-membered heterocyclyl), aryl, heteroaryl, aryl-C₁-C₆alkyl-, heteroaryl-C₁-C₆alkyl-, -OC₁-C₆alkylphenyl, -C₁-C₆alkyl-OH (preferably -C₁-C₄alkyl-OH), -C₁-C₆alkyl-SH, -C₁-C₆alkyl-O-C₁-C₆alkyl, -NH₂, -C₁-C₆alkyl-NH₂ (preferably -C₁-C₃alkyl-NH₂), -N(C₁-C₆alkyl)₂ (preferably -N(C₁-C₃alkyl)₂), -NH(C₁-C₆alkyl) (preferably -NH(C₁-C₃alkyl)), -N(C₁-C₆alkyl)(C₁-C₆alkylphenyl), -NH(C₁-C₆alkylphenyl), nitro, -C(O)OC₁-C₆alkyl (preferably -C(O)OC₁-C₃alkyl), -NHC(O)(C₁-C₆alkyl), -NHC(O)(phenyl), -N(C₁-C₆alkyl)C(O)(C₁-C₆alkyl), -N(C₁-C₆alkyl)C(O)(phenyl), -C(O)C₁-C₆alkyl, -C(O)heteroaryl (preferably -C(O)-5-7 membered heteroaryl), -C(O)C₁-C₆alkylphenyl, -C(O)C₁-C₆haloalkyl, -OC(O)C₁-C₆alkyl (preferably -OC(O)C₁-C₃alkyl), alkylsulfonyl (e.g., -S(O)₂-C₁-C₆alkyl), alkylsulfinyl (-S(O)-C₁-C₆alkyl), -S(O)₂-phenyl, -S(O)₂-C₁-C₆haloalkyl, -S(O)₂NH₂, -S(O)₂NH(C₁-C₆alkyl), -S(O)₂NH(phenyl), -NHS(O)₂(C₁-C₆alkyl), -NHS(O)₂(phenyl), and -NHS(O)₂(C₁-C₆haloalkyl), wherein said alkyl, cycloalkyl, phenyl, aryl, heterocyclyl and heteroaryl are each optionally further substituted with one or more substitutents selected from halo, -OH, -NH₂, cycloalkyl, 3- to 8-membered heterocyclyl, C₁-C₄alkyl, C₁-C₄haloalkyl-, -OC₁-C₄alkyl, -C₁-C₄alkyl-OH, -C₁-C₄alkyl-O-C₁-C₄alkyl, -OC₁-C₄haloalkyl, cyano, nitro, -C(O)-OH, -C(O)OC₁-C₆alkyl, -CON(C₁-C₆alkyl)₂, -CONH(C₁-C₆alkyl), -CONH₂, -NHC(O)(C₁-C₆alkyl), -NH(C₁-C₆alkyl)C(O)(C₁-C₆alkyl), -SO₂(C₁-C₆alkyl), -SO₂(phenyl), -SO₂(C₁-C₆haloalkyl), -SO₂NH₂, -SO₂NH(C₁-C₆alkyl), -SO₂NH(phenyl), -NHSO₂(C₁-C₆alkyl), -NHSO₂(phenyl), and -NHSO₂(C₁-C₆haloalkyl). When an atom or group is substituted with more than one substitutents, the substituents may be same or different.

As used herein, the term "pharmaceutically acceptable" means non-toxic, biologically tolerable and suitable for administration to a subject.

As used herein, the term "pharmaceutically acceptable salt" refers to an acid or base addition salt of the compound of formula (I) that is non-toxic, biologically tolerable and suitable for administration to a subject. The "Pharmaceutically acceptable salt" includes, but are not limited to, acid addition salts formed by the compound of formula (I) with an inorganic acid, such as hydrochloride, hydrobromide, carbonate, bicarbonate, phosphate, sulfate, sulfite, nitrate, and the like, as well as with an organic acid, such as formate, acetate, malate, maleate, fumarate, tartrate, succinate, citrate, lactate, methanesulfonate, p-toluenesulfonate, 2-hydroxyethanesulfonate, benzoate, salicylate, stearate, and salts with alkane-dicarboxylic acid of formlua HOOC-(CH₂)ₙ-COOH (wherein n is 0-4), etc. Also, "pharmaceutically acceptable salt" includes base addition salts formed by the compound of formula (I) carrying an acidic moiety with a pharmaceutically acceptable cations such as sodium, potassium, calcium, aluminum, lithium and ammonium.

In addition, if a compound described herein is obtained as an acid addition salt, the free base can be obtained by basifying a solution of the acid addition salt. Conversely, if the product is a free base, an acid addition salt, particularly a pharmaceutically acceptable acid addition salt, may be produced by dissolving the free base in a suitable solvent and treating the solution with an acid, in accordance with conventional procedures for preparing acid addition salts from base compounds. Those skilled in the art will be able to determine, without undue experimentation, a variety of synthetic methods, which are used to prepare non-toxic pharmaceutically acceptable acid addition salts.

The compounds of the invention may exist in the form of solvates. The term "solvates" means solvent addition forms that contain either stoichiometric or non-stoichiometric amounts of solvent. If the solvent is water, the solvate formed is a hydrate, and when the solvent is alcohol, the solvate formed is an alcoholate. Hydrates are formed by the combination of one or more molecules of water with one molecule of the substance in which the water retains its molecular state as H₂O. Such combination may form one or more kinds of hydrates, for example, hemihydrates, monohydrate, and dihydrate.

As used herein, the term "prodrug" refers to an active or inactive compound that will be chemically modified to form the compounds of the invention by physiological effects in vivo, such as hydrolysis, metabolism, and the like, upon administration to a subject. The suitability and techniques involved in preparing and using a prodrug are well known to those skilled in the art. Exemplary prodrugs are, for example, esters of free carboxylic acids, S-acyl derivatives of thiols and O-acyl derivatives of alcohols or phenols. Suitable prodrugs are generally pharmaceutically acceptable ester derivatives which are convertible under physiological conditions by solvolysis to the parent carboxylic acid, for example, lower alkyl esters, cycloalkyl esters, lower alkenyl esters, benzyl esters, mono-or di-substituted lower alkyl esters, such as ω-(amino, mono- or di-lower alkyl amino, carboxyl, lower alkoxycarbonyl)-lower alkyl esters, α-(lower alkanoyloxy, lower alkoxycarbonyl or di-lower alkylaminocarbonyl)-lower alkyl esters, for example, pivaloyloxymethyl ester and the like, which are conventional in the art.

It will be appreciated by those skilled in the art that some of the compounds of formula (I) may contain one or more chiral centers and therefore exist in two or more stereoisomeric forms. Thus, the compounds of the invention may exist as individual stereoisomers (e.g., enantiomers, diastereomers) and mixtures thereof in any proportion, such as racemate, and, where appropriate, as tautomers and geometric isomers.

As used herein, the term "stereoisomer" refers to compounds that have the same chemical constitution but are different in the spatial arrangement of the atoms or groups. Stereoisomers include enantiomers, diastereomers, conformers, and the like.

As used herein, the term "enantiomer" refers to two stereoisomers of a compound that are nonsuperimposable mirror images of each other.

As used herein, the term "diastereomer" refers to stereoisomers which have two or more chiral centers and whose molecules are not mirror images of one another. Diastereomers have different physical properties, e.g., melting points, boiling points, spectral properties, or biological activities. Mixtures of diastereomers may be separated under high resolution analytical procedures such as electrophoresis and chromatography such as HPLC.

Stereochemical definitions and conventions used herein follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. Many organic compounds exist in optically active forms, i.e., they have the ability to rotate the plane of plane-polarized light. In describing an optically active compound, the prefixes D and L, or R and S, are used to denote the absolute configuration of the molecule about its chiral center(s). The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The racemates can be used as such or can be resolved into their individual isomers. The resolution can afford stereochemically pure compounds or mixtures enriched in one or more isomers. Methods for separation of isomers are well known (*cf.* Allinger N. L. and Eliel E. L. in "Topics in Stereochemistry", Vol. 6, Wiley Interscience, 1971) and include physical methods such as chromatography using a chiral adsorbent. Individual isomers can be prepared in chiral form from chiral precursors. Alternatively individual isomers can be separated chemically from a mixture by forming diastereomeric salts with a chiral acid (such as the individual enantiomers of 10-camphorsulfonic acid, camphoric acid, alpha-bromocamphoric acid, tartaric acid, diacetyltartaric acid, malic acid, pyrrolidone-5-carboxylic acid, and the like), fractionally crystallizing the salts, and then freeing one or both of the resolved bases, optionally repeating the process, so as to obtain either or both substantially free of the other, i.e., in a form having an optical purity of, for example, at least 91%, 92%, 93%, 94%, 95%, 96%, 97%, 98%, 99% or 99.5% by weight of the desired stereoisomer. Alternatively the racemates can be covalently linked to a chiral compound (auxiliary) to produce diastereomers which can be separated by chromatography or by fractional crystallization after which time the chiral auxiliary is chemically removed to afford the pure enantiomers, as is known to those skilled in the art.

As used herein, the term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

As used herein, the term "geometric isomer" is an isomer caused by the inability of a double bond or a single bond of ring carbon atoms to rotate freely, also known as *cis-* and *trans*-isomers. The *cis*-isomer occurs when substituents are located on the same side of a plane, and the *trans*-isomer occurs when substituents are located on the opposite side of a plane.

As used herein, the terms "treating", "treat," or "treatment" of a disease refer to administering one or more pharmaceutical substances, especially a compound of formula (I) and/or a pharmaceutically acceptable salt thereof described herein to a subject that has the disease, or has a symptom of the disease, with the purpose to heal, alleviate, relieve, alter, remedy, ameliorate, improve, or affect the disease or the symptoms of the disease. In some embodiments, the disease is a disease associated with bromodomain recognition protein. In further embodiments, the disease is a disease associated with high expression or high activity of bromodomain proteins. In further embodiments, the disease is cancer such as hematological malignant tumor, midline carcinoma and inflammatory diseases.

As used herein, the terms "preventing", "prevent" or "prevention" of a disease refer to administering one or more pharmaceutical substances, especially a compound of formula (I) and/or a pharmaceutically acceptable salt thereof described herein to a subject that has a predisposition toward the disease, with the purpose to prevent the development of the disease in the subject. In some embodiments, the disease is a disease associated with bromodomain protein. In further embodiments, the disease is a disease associated with high expression or high activity of bromodomain proteins. In further embodiments, the disease is cancer such as hematological malignant tumor, midline carcinoma and inflammatory diseases.

The terms "treating", "contacting" and "reacting" in the context of a chemical reaction mean adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately lead to the formation of the indicated and/or the desired product.

As used herein, the term "effective amount" refers to an amount sufficient to generally bring about a beneficial effect in a subject. The effective amount of the compounds of the invention may be ascertained by conventional methods (such as modeling, dose escalation studies or clinical trials), and by taking into consideration conventional influencing factors (e.g., the mode of administration, the pharmacokinetics of the compound, the severity and course of the disease, the subject's medical history, the subject's health status and response to drugs).

The term "inhibition", "inhibitory" or "inhibiting" indicates a decrease in the baseline activity of a biological activity or process. The term "inhibition of bromodomain proteins" or "inhibition of BRDs" refers to a decrease in the activity of BRD as a direct or indirect response to the presence of the compound of formula (I) and/or the pharmaceutically acceptable salt thereof described herein, relative to the activity of BRD in the absence of the compound of formula (I) and/or the pharmaceutically acceptable salt thereof. The decrease in activity may be due to the direct interaction of the compound of formula (I) and/or the pharmaceutically acceptable salt thereof described herein with BRD, or due to the interaction of the compound of formula (I) and/or the pharmaceutically acceptable salt thereof described herein, with one or more other factors that in turn affect the BRD activity.

As used herein, the term "subject" refers to mammals and non-mammals. Mammals means any member of the mammalian class including, but not limited to, humans; non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex. In some embodiments, the subject is a human.

In general, the term "about" is used herein to modify a numerical value above and below the stated value by a variance of 20%, for example 10%, such as 5%.

Technical and scientific terms used herein and not specifically defined have the meaning commonly understood by those skilled in the art to which the invention pertains.

### General Synthesis Methods

The compounds of formula (I) of the invention, or a stereoisomer, racemate, geometric isomer, tautomer, prodrug, hydrate, solvate, crystal form or pharmaceutically acceptable salt thereof, may be prepared by a variety of methods, including those indicated below, those exemplified in the Examples, or methods analogous thereto. Suitable general synthetic schemes are depicted below. Suitable reaction conditions for each reaction step are known to those skilled in the art or may be ascertained by conventional methods. Starting materials for the preparation are commercially available or may be prepared using methods indicated below or analogous thereto, or well known to those skilled in the art. Variables in general formulae have the same meaning as above, unless otherwise indicated.

The compounds of the present invention are synthesized by the following general synthetic scheme A or general synthetic scheme B. 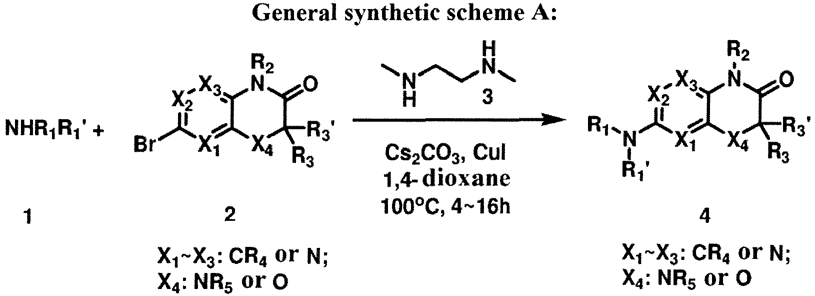 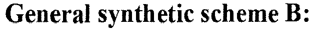 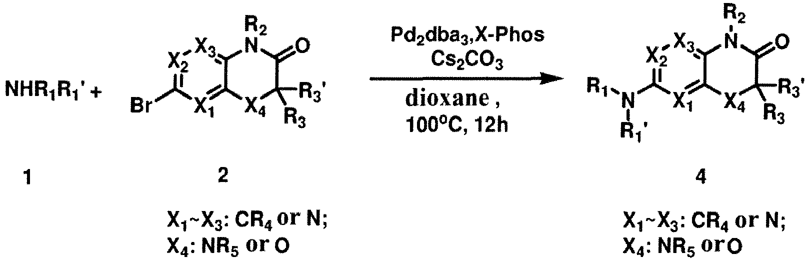 wherein the variables are as defined herein.

In these schemes, it is well understood that protecting groups for sensitive or reactive groups (e.g., amino, hydroxyl and carboxyl) are used, when necessary, according to common principles or chemistry to prevent undesired chemical reactions. The protecting groups are treated according to standard methods of organic synthesis (T. W. Greenee and P. G. M. Wuts, "Protective Groups in Organic Synthesis", 5nd Edition, Wiley, New York 2014.). These groups are removed at a convenient stage of synthesis of a compound using methods well known to those skilled in the art. The selection of the process, the reaction conditions and the performance sequence should be in accordance with the preparation of the compounds of formula (I).

Examples of amino-protecting groups include carbamates, amides, alkyl and aryl groups, imines, as well as many N-heteroatom derivatives which can be removed to regenerate the desired amine group. Specific amino protecting groups are Pmb (p-methoxybenzyl), Boc (t-butyloxycarbonyl), Fmoc (9-fluorenylmethyloxycarbonyl) and Cbz (carbobenzyloxy). Further examples of these groups are found in T. W. Greene and P. G. M. Wuts, "Protective Groups in Organic Synthesis", 5nd Edition, Wiley, New York 2014.

Examples of hydroxyl-protecting groups include tetrahydropyranyloxy, benzoyl, acetoxy, carbamoyloxy, benzyl, and silylethers (e.g. TBS, TBDPS) groups. Further examples of these groups are found in "Protective Groups in Organic Synthesis", 5nd Edition, Wiley, New York 2014.

Examples of carboxy protecting groups include, ester groups and heterocyclyl groups. Ester derivatives of the carboxylic acid group may be employed to block or protect the carboxylic acid group while reactions are carried out on other functional groups of the compound. Examples of such ester groups include substituted arylalkyl, including substituted benzyls, such as 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl; alkyl or substituted alkyl esters such as methyl, ethyl, t-butylallyl or t-amyl, triphenylmethyl (trityl), 4-methoxytrityl, 4,4'-dimethoxytrityl, 4,4',4"-trimethoxytrityl, 2-phenylprop-2-yl; thioesters such as t-butyl thioester; silyl esters such as trimethylsilyl esters, t-butyldimethylsilyl esters (TBSO), and the like. Further examples of these groups are found in T. W. Greene and P. G. M. Wuts, "Protective Groups in Organic Synthesis", 5th Edition, Wiley, New York, 2014.

Those skilled in the art will recognize whether a stereocentre is present in the compounds of formula (I). Accordingly, when a compound is desired as a single enantiomer or diastereomer, it can be obtained either by stereospecific synthesis or by resolution of the final product or any appropriate intermediate. The resolution of the final product, intermediates or starting materials may be carried out using any appropriate method known in the art. See, for example, E. L. Eliel, S. H. Wilen and L. N. Mander, "Stereochemistry of Organic Compounds" (Wiley-Interscience, 1994).

### Pharmacological efficacy

The compounds of the present invention have bromodomain proteins (BRDs) inhibitory activity, particularly BET inhibitory activity, more particularly inhibitory activity targeted to BRD4, and are therefore useful for the treatment or prevention of diseases associated with bromodomain proteins (BRDs), particularly BET-related diseases, more particularly BRD 4-related diseases, for example, diseases associated with high expression or high activity of bromodomain proteins (BRDs), for example, cancers such as hematological malignant tumor, midline carcinoma and inflammatory diseases.

For example, the compounds of the present invention are useful in the treatment or prevention of cancer. In particular, the cancer refers to the physiological condition of mammals that is typically characterized by unregulated cell growth. For example, the cancer is selected from hematological malignant tumor, lung cancer, multiple myeloma, neuroblastoma, colon cancer, testicular cancer, ovarian cancer. In particular, the cancer is selected from lung cancer (e.g., small cell lung cancer or non-small cell lung cancer), NUT midline carcinoma (e.g., BRD3-NUT midline carcinoma or BRD4-NUT midline carcinoma), leukemia, mixed Lineage Leukemia (MLL), acute granulocytic leukemia (AML), biphenotypic B myelomonocytic leukemia or erythroleukemia. In particular, the cancer is selected from burkitt's lymphoma, breast cancer, colon cancer, neuroblastoma, glioblastoma multiforme, chronic lymphocytic leukemia and squamous cell carcinoma.

The compounds of the invention may also be useful in the treatment or prevention of, for example, inflammatory diseases. In particular, the inflammatory diseases are diseases involving inflammatory response to bacterial, viral, fungal, parasitic and/or protozoal infections. In particular, the inflammatory disease is selected from osteoarthritis, acute gout, multiple sclerosis, inflammatory bowel disease (e.g. crohn's disease and ulcerative colitis), neuroinflammation, asthma, chronic obstructive airways disease, pneumonia, myositis, eczema, dermatitis, acne, cellulitis, occlusive disease, thrombosis, alopecia, nephritis, vasculitis, retinitis, uveitis, scleritis, sclerosing cholangitis, hypophysitis, thyroiditis, septic shock, Systemic Inflammatory Response Syndrome (SIRS), toxic shock syndrome, acute lung injury, ARDS (adult respiratory distress syndrome), acute renal failure, burns, pancreatitis (e.g. acute pancreatitis), post-operative syndrome, sarcoidosis, Herxheimer reaction, encephalitis, myelitis, meningitis and malaria. In particular, the inflammatory disease is acute or chronic pancreatitis. In particular, the inflammatory disease is burns. In particular, the inflammatory disease is inflammatory bowel disease. In particular, the inflammatory disease is neuroinflammation. In particular, the inflammatory disease is sepsis or sepsis syndrome. In particular, the inflammatory disease is graft versus host disease (GVHD).

The compounds of the present invention may also be useful in the treatment or prevention of cardiovascular diseases. In particular, the disease is selected from atherogenesis, atherosclerosis, arterial stent occlusion, heart failure (e.g., congestive heart failure), coronary artery disease, myocarditis, pericarditis, heart valve disease, stenosis, restenosis, intra-stent stenosis, angina pectoris, myocardial infarction, acute coronary syndrome, coronary bypass, cardiopulmonary bypass, endotoxemia, ischemia-reperfusion injury, cerebrovascular ischemia (stroke), renal reperfusion injury, embolism (e.g., pulmonary embolism, renal embolism, hepatic embolism, gastrointestinal embolism, or peripheral limb embolism), or myocardial ischemia.

The compounds of the present invention may also be useful in the treatment or prevention of viral infections. In particular, the disease is DNA viral infections. In particular, the disease is dsDNA viral infections, ssDNA viral infections, RNA viral infections and dsRNA viral infections. In particular, the disease is (+)ssRNA viral infections, (-)ssRNA viral infections, reverse transcription (RT) viral infections, ssRNA-RT viral infections and dsDNART viral infections. In particular, the disease is human immunodeficiency virus (HIV) infection, such as acquired immunodeficiency syndrome (AIDS), human papilloma virus (HPV) infection, hepatitis C virus (HCV) infection, herpes viral infection (e.g., herpes simplex virus (HSV) infection) and Ebola virus infection. In particular, the disease is severe acute respiratory syndrome (SARS) and influenza virus infection.

The compounds of the present invention may also be useful in the treatment or prevention of fibrotic diseases. In particular, the disease is selected from renal fibrosis, postoperative stenosis, keloid formation, cirrhosis, biliary cirrhosis, and cardiac fibrosis. In particular, tne disease is scleroderma. In particular, the disease is idiopathic pulmonary fibrosis.

The compounds of the present invention may also be useful in the treatment or prevention of metabolic diseases. In particular, the disease is endocrine disease. In particular, the disease is Addison's disease. In particular, the disease is diabetes. In particular, the disease is type I diabetes, type II diabetes or gestational diabetes. In particular, the disease is obesity. In particular, the disease is fatty liver (NASH or other), cachexia, hypercholesterolemia or disorder of lipid metabolism regulated by apolipoprotein A1 (APOA 1).

The compounds of the present invention may also be useful in the treatment or prevention of radiation poisoning. In particular, the disease is radiation injury.

The compounds of the present invention may also be useful in the treatment or prevention of acute rejection of transplanted organs or multiorgan dysfunction syndrome.

The compounds of the present invention may also be useful in the treatment or prevention of Alzheimer's disease.

### Pharmaceutical composition and administration

The compounds of the invention may be administered to a subject as a pharmaceutical composition, which may optionally comprise one or more pharmaceutically acceptable excipients.

The compounds of the invention can be administered by various known routes, including oral, rectal, intragastrical, intracranial and parenteral administration, e.g. intravenous, intramuscular, intranasal, intradermal, subcutaneous, and similar administration routes. Oral, intranasal and parenteral administration are particularly preferred. Depending on the route of administration, different pharmaceutical formulations are required. Some of the route of administration may require that protective coatings are applied to the pharmaceutical formulation to prevent degradation of a compound of the invention in, for example, the digestive tract.

The compounds of the invention may be formulated into a syrup, an infusion or injection solution, a spray, a tablet, a capsule, a lozenge, a liposome, or suppository etc.

Particular preferred pharmaceutical forms for the administration of the compounds of the invention are forms suitable for injection, including sterile aqueous solutions or dispersions and sterile powders for the extemporaneous preparation of sterile injectable solutions or dispersions. In all cases, the final solution or dispersion form must be sterile and fluid. Typically, such solution or dispersion will comprise solvent or dispersion medium, containing, for example, water-buffered aqueous solutions, e.g. biocompatible buffers, ethanol, or polyol such as glycerol, propylene glycol, polyethylene glycol, suitable mixtures thereof, surfactants or vegetable oils. The compounds of the invention can also be formulated into liposomes, in particular for parenteral administration. Liposomes provide the advantage of increased half life in the circulation, if compared to the free drug, and a prolonged, more even release of the enclosed drug.

Sterilization of infusion or injectable solutions can be accomplished by any techniques recognized in the art, including but not limited to addition of preservatives such as anti-bacterial or anti-fungal agents, e.g. parabens, chlorobutanol, phenol, sorbic acid or thimerosal. Further, isotonic agents, such as sugars or salts, in particular sodium chloride, may be incorporated in the infusion or injectable solutions.

Production of sterile injectable solutions containing one or more of the compounds of the invention is accomplished by incorporating the respective compound in the required amount in the appropriate solvent with various ingredients enumerated above as required followed by sterilization. To obtain a sterile powder, the above solutions are vacuum-dried or freeze-dried as necessary. Preferred diluents of the present invention are water, physiological acceptable buffers, physiological acceptable buffer salt solutions or salt solutions. Preferred carriers are cocoa butter and vitebesole.

Excipients which can be used with the various pharmaceutical forms of the compounds of the invention can be selected from the following non-limiting list:
a) binders such as lactose, mannitol, crystalline sorbitol, dibasic phosphates, sugars, microcrystalllne cellulose, carboxymethyl cellulose, hydroxyethyl cellulose, and/or polyvinyl pyrrolidone and the like;
b) lubricants such as magnesium stearate, talc, calcium stearate, zinc stearate, stearic acid, hydrogenated vegetable oil, leucine, glycerides and sodium stearyl fumarates,
c) disintegrants such as starches, croscarmellose, sodium methyl cellulose, agar, bentonite, alginic acid, carboxymethyl cellulose, and/or polyvinyl pyrrolidone and the like.

In one embodiment, the formulation is for oral administration and the formulation comprises one or more or all of the following ingredients: pregelatinized starch, talc, povidone K30, croscarmellose sodium, sodium stearyl fumarate, gelatin, titanium dioxide, sorbitol, monosodium citrate, xanthan gum, titanium dioxide, flavouring agent, sodium benzoate and saccharin sodium.

In one embodiment, the compounds of the invention are administered intranasally. It may be administered in the form of a dry powder inhaler or an aerosol spray from a pressurized container, pump, spray or nebulizer with the use of a suitable propellant, e.g., dichforodifluoromethane, trichlorofluoromethane, dichlorotetrafluoroethane, a hydrofluoro-alkane such as 1,1,1,2-tetrafluoroethane (HFA 134A™) or 1,1,1,2,3,3,3-heptafluoropropane (HFA 227EA™), carbon dioxide, or another suitable gas. The pressurized container, pump, spray or nebulizer may contain a solution or suspension of the compounds of the invention, e.g., using a mixture of ethanol and the propellant as the solvent, which may additionally contain a lubricant, e.g., sorbitan trioleate.

The typical dosage of the compounds of the invention will be in the range of 0.001 - 1000 mg/kg body weight/day. The daily dosage may be administered in a single dose or in multiple divided doses. The appropriate dosage is determined by the attending physician, as appropriate, depending on the type and severity of the disease being treated, the health status and past medical history of the individual, the co-drug, the specific compound to be administered, and the route of administration. If desired, the dosage of the compounds of the invention may go beyond said dosage range.

### Pharmaceutical combination

The compounds of the invention may be administered as the sole active ingredient or in combination with additional drugs or therapies which may have the same or different pharmacological effects. For example, the additional drug or therapy includes, but is not limited to, immunotherapeutic agents such as immunomodulators, e.g., immunosuppressants or immunopotentiators, other anticancer or anti-inflammatory agents, cardiovascular drugs, lipid-lowering drugs, antibacterial, antiviral, antidiabetic, antiproliferative, antiangiogenic, antiallergic agent or antineoplastic therapy such as tumor immunotherapy, and the like. Such drugs and therapies are known to those skilled in the art. The compounds of the invention and the additional drug or therapy may advantageously act, for example additively or synergistically.

Immunotherapeutic agents described herein refer to drugs used to transfer immunity of an immune donor, such as another human or animal, to a host by vaccination. Said term encompasses the use of serum or gamma globulin containing the formed antibodies produced by other subjects or animals, non-specific systemic stimulation, adjunctive agents, active specific immunotherapy and adoptive immunotherapy. For example, the term includes, but is not limited to, immunomodulators, e.g., immunosuppressants and immunopotentiators, and the like. The use of immunotherapeutic agents is determinable by one skilled in the art.

Immunosuppressants described herein include, but are not limited to: (1) glucocorticoids, e.g., cortisone and prednisone; (2) microbial metabolites, e.g., cyclosporine and fujimycin etc.; (3) antimetabolites, e.g., azathioprine and 6-mercaptopurine, etc.; (4) polyclonal and monoclonal anti-lymphocyte antibodies, e.g., anti-lymphocyte globulin and OKT3 etc.; (5) alkylating agents, e.g., cyclophosphamide and the like.

Immunopotentiators described herein include, but are not limited to: (1) drugs of microbial origin, e.g., BCG vaccination; (2) products of human or animal immune system, e.g., thymosins, transfer factors, interferons, interleukins, and the like; (3) chemically synthesized drugs, e.g., levamisole and polyinosinic acid; (4) fungal polysaccharides, e.g., lentinan and the like; (5) phytohemagglutinin, canavalin A, and placental polysaccharide.

Anti-inflammatory agents described herein include, but are not limited to steroids, such as corticosteroids. Suitable steroids include budesonide, beclomethasone (e.g., beclomethasone dipropionate), butixocort (e.g., propionate), CHF5188, ciclesonide, dexamethasone, flunisolide, fluticasone (e.g., propionate or furoate), GSK-685698, GSK-870086, LAS40369, methylprednisolone, mometasone (e.g., furoate), prednisolone, rofleponide and triamcinolone (e.g., triamcinolone acetonide). In some preferred embodiments, the steroid is a long-acting corticosteroid, such as budesonide, ciclesonide, fluticasone propionate, fluticasone furoate, or mometasone furoate.

When the compound of formula I is administered in combination with other drugs, the dosage of the other drug co-administered will, of course, vary depending upon factors such as the type of co-agents, the particular drug used, the condition to be treated, the general health of the patient, the judgment of the physician or veterinarian, and the like.

The compounds of the present invention may also be combined with anti-tumor therapies including, but not limited to, surgery, radiation therapy, transplantation (e.g., stem cell transplantation, bone marrow transplantation), tumor immunotherapy, chemotherapy, and the like. Preferably, the compounds of the invention may be combined with tumor immunotherapy. Tumor immunotherapy is a therapeutic method for restoring normal anti-tumor immune response by restarting and maintaining tumor-immune cycle, thereby controlling and eliminating tumor, and includes immune checkpoint inhibitors such as monoclonal antibody immune checkpoint inhibitors, therapeutic antibodies, cancer vaccines, cellular immunotherapy, small molecule inhibitors, and the like.

The agents used in combination with the present invention, i.e. the co-agents, may be administered simultaneously, separately or sequentially with the compounds of the present invention by the same or different routes of administration. They may be contained in the same pharmaceutical composition, or may be a combination product in separate form, for example in the form of kit. They may be prepared and/or formulated by the same or different manufacturers. Furthermore, the compounds of the invention together with the further medicament(s) may be introduced to the combination therapy (i) prior to distributing the combination product to a physician (e.g. in the case of a kit comprising the compounds of the invention and a further medicament); (ii) by the physician himself (or under the direction of the physician) immediately before administration; (iii) by the patient himself, for example during the sequential administration of the compounds of the invention and the further medicament.

It is understood that, within the scope of the invention, the technical features defined in the various technical solutions above and the technical features specified below (e.g., in the Examples) can be combined with each other to form a new or preferred technical solution. Such technical solutions are not reiterated one by one herein due to the length of the application.

The formula shall be relied on when there is discrepancy between the chemical name of any compound of the invention and its formula, unless the formula is obviously wrong.

### Examples

The invention will be further illustrated with reference to the following specific examples. It should be understood that these examples are for illustrative purposes only and are not intended to limit the scope of the present invention. Experimental procedures without specific conditions noted in the following examples generally follow the conventional conditions for such reactions, or follow the conditions recommended by the manufacturer. Unless otherwise indicated, percentages and parts are by weight. Unless otherwise indicated, the ratio of liquids is by volume.

The materials and reagents used in the following examples are commercially available, unless otherwise specified.

In the following examples, ¹H-NMR spectra were recorded on Bluker AVHD 400MHz or Bluker AVHD 500 MHz nuclear magnetic resonance spectrometer; ¹³C-NMR spectra were recorded on Bluker AVHD 500MHz or Bluker AVHD 600MHz nuclear magnetic resonance spectrometer, with chemical shift shown in δ (ppm); Mass spectra were recorded on Waters UPLC H-Class+QDa (ESI) and Agilent 1260_6120 (ESI) mass spectrometer; and reverse phase preparative HPLC separation was carried out on Waters UV guided full-automatic purification system (Xbridge Prep C18 5 µm OBD column). Chiral preparative HPLC is carried out on Elite P230 preparation gradient system (China) and SFC preparation system (Thar Prep-80).

The chemical names of the reagents represented by the chemical formula or abbreviation are as follows:
- AcOH: acetic acid
- AcONH₄: ammonium acetate
- AlMe₃: trimethyl aluminum
- BF₃-Et₂O: boron trifluoride-diethyl ether solution
- BOC: tert-butoxycarbonyl
- BOC₂O: di-tert-butyl dicarbonate
- degree Centigrade or °C: °C
- CAN: cerous ammonium nitrate
- CD₃OD: deuterated methanol
- CDI: N,N'-carbonyldiimidazole
- CH₃COOK or AcOK: potassium acetate
- CHCl₃: trichloromethane or chloroform
- CH₃I: methyl iodide
- CO₂: carbon dioxide
- conc.: concentrated
- Cs₂CO₃: cesium carbonate
- CuI: copper iodide
- DCM: dichloromethane
- DEA: diethanolamine
- DIAD: diisopropyl azodicarboxylate
- DIBAL-H: diisobutylaluminum hydride
- dioxane: dioxane
- DIPEA or DIEA: N,N-diisopropylethylamine
- DMAP: 4-dimethylaminopyridine or N,N-dimethyl-4-aminopyridine
- DMEA: N,N-dimethylethanolamine
- DMF: dimethylformamide
- DMSO: dimethyl sulfoxide
- EA or EtOAc: ethyl acetate
- EDCI: 1-ethyl-(3-dimethylaminopropyl)carbodiimide
- EtOH: ethanol
- Et₃N: triethylamine
- Et₃SiH: triethylsilane
- EtI: ethyl iodide
- FA: formic acid
- Fe: iron
- g: gram
- h: hour
- H₂: hydrogen gas
- H₂O: water
- H₂SO₄: sulfuric acid
- HCl: Hydrogen chloride or hydrochloric acid
- HCO₂H: formic acid
- HOBt: 1-hydroxybenzotriazole
- HPLC: high performance liquid chromatography
- *ⁱ*PrOH or IPA: isopropyl alcohol
- K₂CO₃: potassium carbonate
- K₃PO₄: potassium phosphate
- KOAc: potassium acetate
- LCMS: liquid chromatography-mass spectrometry
- MeCN, ACN or CH₃CN: acetonitrile
- MeI or Me₃I: methyl iodide
- MW: microwave
- MeOH: methanol
- min: minute
- mg: milligram
- ml: millilitre
- mmol: millimol
- mol: mole
- N₂: nitrogen gas
- N₂H₄: hydrazine
- Na₂CO₃: sodium carbonate
- NaBH₃CN or NaBH₃(CN): sodium cyanoborohydride
- NaBH₄: sodium borohydride
- NaH: sodium hydride
- NaHCO₃: sodium bicarbonate
- NaN₃: sodium azide
- NaOH: sodium hydroxide
- NBS: N-bromosuccinimide
- NH₄Cl: ammonium chloride
- Pd/C: Palladium/Charcoal
- Pd₂dba₃: Tris(dibenzylideneacetone) dipalladium
- Pd(dppf)Cl₂ or PdCl₂(dppf): 1,1'-bis(diphenylphosphino)ferrocene palladium dichloride
- PE: petroleum ether
- PhMgBr: phenyl magnesium bromide
- PPh₃: triphenylphosphine
- Py: pyridine
- r.t. or RT: room temperature
- RuPhos Palladacycle: Chloro(2-dicyclohexylphosphino-2',6'-diisopropoxy-1,1'-biphenyl) [2-(2-aminoethylphenyl)]palladium (II)
- SFC: Supercritical Fluid Chromatography
- ^{t}BuNC: 2-isocyano-2-methylpropane
- *t*-BuONa: sodium tert-butoxide
- T₃P: 1-propylphosphoric anhydride
- TEA: triethylamine
- Tf₂O: trifluoromethanesulfonic anhydride
- TLC: thin layer chromatography
- TFA or CF₃COOH: trifluoroacetic acid
- THF: tetrahydrofuran
- TMSI: trimethylsulfoxonium iodide
- Xantphos: 4,5-bisdiphenylphosphine-9,9-dimethylxanthene
- X-Phos: 2-dicyclohexylphosphine-2,4,6-triisopropylbiphenyl

### Synthesis of Intermediate

### Intermediate 5: Synthesis of 6-bromo-4-cyclopropyl-1,3-dimethyl-3,4-dihydropyrido[2,3-b] pyrazin-2(1H)-one (5)

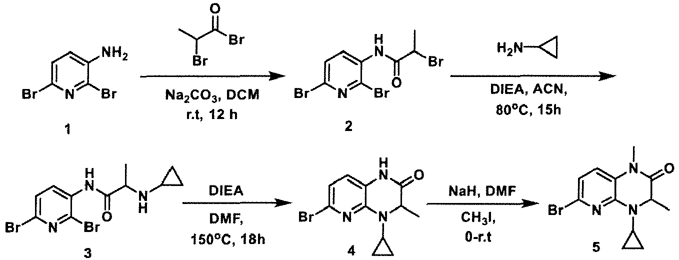

### Step 1: Synthesis of 2-bromo-N-(2,6-dibromopyridin-3-yl)propanamide (2)

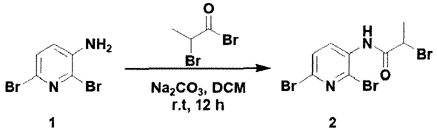

Into a dry 500 mL three-necked flask 2,6-dibromopyridin-3-amine **(1)** (20 g, 0.08 mol), sodium carbonate (8.4 g, 0.08 mol) and 200 mL dichloromethane were sequentially added, and then 2-bromopropionyl bromide (10 mL, 0.09 mol) was added dropwise. The reaction was carried out at RT for 12 h. After the completion of reaction detected by TLC, 150 mL saturated ammonium chloride solution was added slowly. The mixture was extracted with dichloromethane (150mL×3). The combined organic phase was dried over anhydrous Na₂SO₄ and filtered. The filtrate was concentrated under reduced pressure. The crude product was purified on column chromatography (eluted with dichloromethane) to yield 2-bromo-N-(2,6-dibromopyridin-3-yl)propanamide **(2)** (15.4 g, 0.04 mol). **LCMS:** *m*/*z* 386.8, 388.8 (M+H).

### Step 2: Synthesis of 2-(cyclopropylamino)-N-(2,6-dibromopyridin-3-yl)propanamide (3)

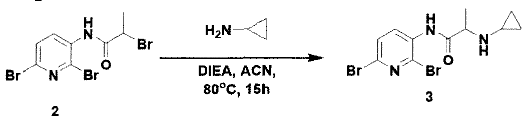

Into a dry 250 mL three-necked flask 2-bromo-N-(2,6-dibromopyridin-3-yl)propanamide **(2)** (5 g, 13 mmol), cyclopropylamine (1.1 g, 19 mmol) and 40 mL acetonitrile were sequentially added, followed by addition of DIEA (5 g, 33 mmol). Then the reaction was carried out at 80°C for 15 h. After the completion of reaction detected by TLC, the reaction mixture was concentrated. Water was added, and the mixture was extracted with dichloromethane (100mL×3). The combined organic phase was dried over anhydrous Na₂SO₄ and filtered. The filtrate was concentrated under reduced pressure. The crude product was purified on column chromatography (petroleum ether : ethyl acetate = 10:1 to 5:1) to yield 2-(cyclopropylamino)-N-(2,6-dibromopyridin-3-yl)propanamide **(3)** (2.5 g, 7 mmol). LCMS: *m*/*z* 361.8, 363.8, 365.7 (M+H).

### Step 3: Synthesis of 6-bromo-4-cyclopropyl-3-methyl-3,4-dihydropyrido[2,3-b]pyrazin-2(1 H)-one (4)

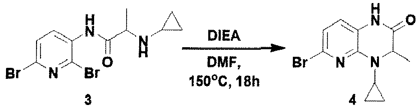

Into a dry 250 mL three-necked flask, 2-(cyclopropylamino)-N-(2,6-dibromopyridin-3-yl) propanamide **(3)** (2.5 g, 7 mmol), DIEA (1.8 g, 14 mmol) and 20 mL DMF were sequentially added, and the reaction was carried out at 150°C for 18 h. After the completion of reaction detected by TLC, the reaction mixture was concentrated. Water was added, and the mixture was extracted with dichloromethane (100mL×3). The combined organic phase was dried over anhydrous Na₂SO₄ and filtered. The filtrate was concentrated under reduced pressure to yield crude product 6-bromo-4-cyclopropyl-3-methyl-3,4-dihydropyrido [2,3-b]pyrazin-2(1H)-one **(4)** (1.8 g, 6 mmol) which was directly used in the next step. LCMS: *m*/*z* 281.9, 283.9 (M+H).

### Step 4: Synthesis of 6-bromo-4-cyclopropyl-1,3-dimethyl-3,4-dihydropyrido[2,3-b]pyrazin-2 (1H)-one (5)

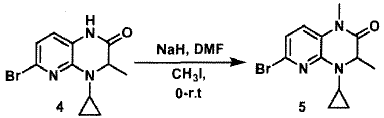

Into a dry 50 mL three-necked flask, 6-bromo-4-cyclopropyl-3-methyl-3,4-dihydropyrido [2,3-b]pyrazin-2(1H)-one **(4)** (1.8 g, 6 mmol) and 10 mL DMF were added, followed by addition of sodium hydride (0.72 g, 18 mmol), and the reaction was carried out at 0°C under nitrogen atmosphere for 0.5 h. Methyl iodide (0.8 mL, 12 mmol) was then added dropwise, and the reaction was carried out at RT for another 1 h. After the completion of reaction detected by TLC, 100 mL saturated ammonium chloride solution was added slowly, and the mixture was extracted with dichloromethane (100mL×3). The combined organic phase was dried over anhydrous Na₂SO₄ and filtered. The filtrate was concentrated under reduced pressure. The crude product was purified on column chromatography (petroleum ether : ethyl acetate = 3:1) to yield 6-bromo-4-cyclopropyl-1,3-dimethyl-3,4-dihydropyrido[2,3-b]pyrazin-2(1H)-one **(5)** (1.5 g, 5 mmol). LCMS: *m*/*z* 298.0 (M+H); ¹HNMR(400MHz, DMSO): δ7.23 (d, J= 8.1 Hz, 1H), 7.00 (d, J=8.1 Hz, 1H), 4.15 (q, J=6.9 Hz, 1H), 3.21 (s,3H), 2.70-2.56 (m, 1H), 1.19 (d, J=6.9 Hz, 3H), 1.01-0.87 (m, 1H), 0.75-0.55 (m, 2H), 0.52-0.36 (m, 2H).

### Intermediate 6: Synthesis of 6-bromo-4-cyclopentyl-3-methyl-3,4-dihydropyrido[2,3-b]pyra zin-2(1H)-one (6)

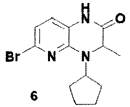

Intermediate **6** was prepared analogously to the synthetic procedure of Intermediate **4,** starting from 2,6-dibromopyridin-3-amine **(1)** and cyclopentylamine.

### Intermediate 7: Synthesis of 6-bromo-4-cyclopent-yl-1,3-dimethyl-3,4-dihydropyrido[2,3-b]p yrazin-2(1H)-one (7)

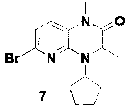

Intermediate **7** was prepared from Intermediate **6** analogously to the synthetic procedure of Intermediate **5.**

### Intermediate 11: Synthesis of (R)-6-bromo-3-methyl-3,4-dihydropyrido[2,3-b]pyrazin-2(1H) -one (11)

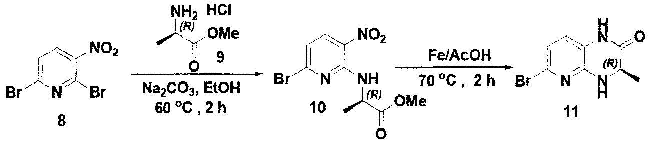

### Step 1. Synthesis of Methyl N-(6-bromo-3-nitropyridin-2-yl)-D-alanine ester (10)

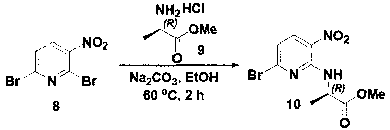

Into a dry 100 mL round-bottom flask, compound **8** (2.0 g, 7.0 mmol), compound **9** (1.47 g, 10.5 mmol), sodium carbonate (1.48 g, 14.0 mmol) and ethanol (30mL) were sequentially added at RT, and the reaction was heated to 60°C under stirring for 2 hours. After the completion of reaction as monitored on TLC plate, the mixture was cooled to RT and filtered. The filter cake was washed with ethanol (20mL), and the filtrate was concentrated under reduced pressure to yield methyl N-(6-bromo-3-nitropyridin-2-yl)-D-alanine ester **(10)** (2.12 g crude, yellow oil), yield: 100%, which was directly used in the next reaction.

### Step 2. Synthesis of (R)-6-bromo-3-methyl-3,4-dihydropyrido[2,3-b]pyrazin-2(1H)-one (11)

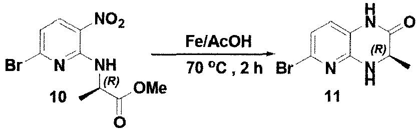

Into a dry 100 mL round-bottom flask, compound **10** (2.12 g, 7.0 mmol), iron powder for reduction (1.92 g, 35 mmol) and acetic acid (15mL) were sequentially added at RT, and the reaction was heated to 70°C under stirring for 2 hours. After the completion of reaction as monitored on TLC plate, the mixture was cooled to RT and filtered. The filter cake was washed with dichloromethane (40mL), and the filtrate was concentrated under reduced pressure to yield residue, which was purified on column chromatography (methanol:dichloromethane = 1:20) to yield the product (*R*)-6-bromo-3-methyl-3,4-dihydropyrido[2,3-b]pyrazin-2(1H)-one **(11)** (620 mg, yellow solid), yield: 37%. LCMS: *m*/*z* 241.9, 243.9 (M+H).

### Intermediate 13: Synthesis of Tert-butyl (R)-6-bromo-1,3-dimethyl-2-carbonyl-2,3-dihydro pyrido[2,3-b]pyrazine-4(1H)-carboxylate (13)

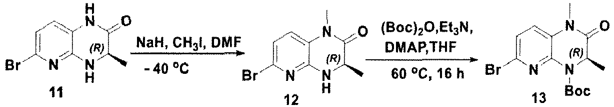

### Step 1. Synthesis of (R)-6-bromo-1,3-dimethyl-3,4-dihydropyrido[2,3-b]pyrazin-2(1H)-one (12)

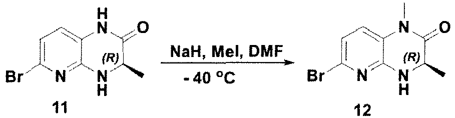

Into a dry 100 mL round-bottom flask, compound **11** (450 mg, 1.86 mmol) and DMF (4mL) were added at RT. The reaction was purged with nitrogen gas for three times, and cooled to -40°C. Sodium hydride (60%, 75 mg, 1.86 mmol) was added, and the reaction was stirred for 30 minutes. Methyl iodide (264 mg, 1.86 mmol) was added, and the reaction was continued at -40°C for 1 hour. After the completion of reaction as monitored on TLC plate, 10 mL water was added, and the mixture was extracted with ethyl acetate (20mL×2). The combined organic phase was dried over anhydrous Na₂SO₄ and filtered. The filtrate was concentrated under reduced pressure. The residue was purified by column chromatography (methanol:dichloromethane = 1:20) to yield (*R*)-6-bromo-1,3-dimethyl-3,4-dihydropyrido[2,3-b]pyrazin-2(1H)-one **(12)** (320 mg, white solid, ee: 100%), yield: 67%. LCMS: *m*/*z* 255.9, 257.9 (M+H).

### Step 2. Synthesis of Tert-butyl (R)-6-bromo-1,3-dimethyl-2-carbonyl-2,3-dihydropyrido[2, 3-b]pyrazine-4(1H)-carboxylate (13)

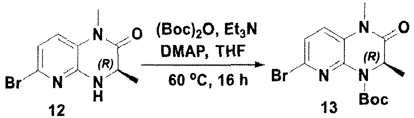

Into a dry 100 mL round-bottom flask, Intermediate **12** (270 mg, 1.05 mmol), di-tert-butyl dicarbonate (343 mg, 1.57 mmol), triethylamine (212 mg, 2.1 mmol), N,N-dimethyl-4-aminopyridine (64 mg, 0.52 mmol) and tetrahydrofuran (20mL) were sequentially added at RT, and the reaction was heated to 60°C under stirring for 16 hours. After the completion of reaction as monitored on TLC plate, the mixture was cooled to RT, and concentrated under reduced pressure. The residue was added to 10 mL water, and was extracted with ethyl acetate (20mL×2). The combined organic phase was dried over anhydrous Na₂SO₄ and filtered. The filtrate was concentrated under reduced pressure. The residue was purified by column chromatography (methanol:dichloromethane = 1:20) to yield tert-butyl (R)-6-bromo-1,3-dimethyl-2-carbonyl-2,3- dihydropyrido[2,3-b]pyrazine-4(1H)-carboxylate **(13)** (210 mg, white solid), yield: 56%. LCMS: *m*/*z* 299.9, 301.9 (M+H-56).

### Intermediate 14: Synthesis of (R)-4-acetyl-6-bromo-1,3-dimethyl-3,4-dihydropyrido[2,3-b]p yrazin-2(1H)-one (14)

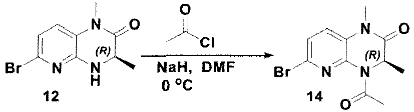

Into a dry 100 mL round-bottom flask, Intermediate **12** (250 mg, 0.97 mmol) and N,N-dimethylformamide (2mL) were added at RT. The reaction was purged with nitrogen gas for three times, and cooled to 0°C. Sodium hydride (60%, 59 mg, 1.45 mmol) was added, and the reaction was stirred for 1 hour. Acetyl chloride (83 mg, 1.07 mmol) was added, and the reaction was stirred at 0°C for 1 hour. After the completion of reaction as monitored on TLC plate, 10 mL water was added, and the mixture was extracted with ethyl acetate (20mL×2). The combined organic phase was dried over anhydrous Na₂SO₄ and filtered. The filtrate was concentrated under reduced pressure. The residue was purified by column chromatography (methanol: dichloromethane = 1:20) to yield (*R*)-4-acetyl-6-bromo-1,3-dimethyl-3,4-dihydropyrido[2,3-b]pyrazin-2(1H)-one **(14)** (190 mg, yellow solid), yield: 66%. LCMS: *m*/*z* 297.9, 299.9 (M+H).

### Intermediate 15: Synthesis of (R)-6-bromo-4-ethyl-1,3-dimethyl-3,4-dihydropyrido[2,3-b]py razin-2(1H)-one (15)

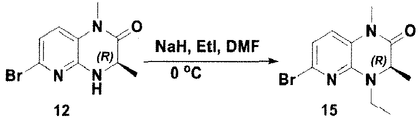

Into a dry 100 mL round-bottom flask, Intermediate **12** (250 mg, 0.97 mmol) and N,N-dimethylformamide (2mL) were added at RT. The reaction was purged with nitrogen gas for three times, and cooled to 0°C. Sodium hydride (60%, 78 mg, 1.94 mmol) was added. After 1 hour, ethyl iodide (2.1 g, 14.8 mmol) was added, and the reaction was stirred at 0°C for 1 hour. After the completion of reaction as monitored on TLC plate, 20 mL water was added, and the mixture was extracted with ethyl acetate (30mL×3). The combined organic phase was dried over anhydrous Na₂SO₄ and filtered. The filtrate was concentrated under reduced pressure. The residue was purified by column chromatography (methanol:dichloromethane = 1:20) to yield (*R*)-6-bromo-4-ethyl-1,3-dimethyl-3,4-dihydropyrido[2,3-b]pyrazin-2(1H)-one **(15)** (190 mg, yellow solid), yield: 69%. LCMS: *m*/*z* 284.0, 285.9 (M+H).

### Intermediate 16: Synthesis of (R)-6-bromo-1,3,4-trimethyl-3,4-dihydropyrido[2,3-b]pyrazin -2(1H)-one (16)

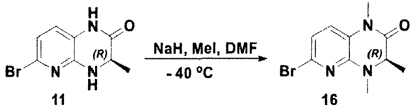

Into a dry 100 mL round-bottom flask Intermediate **11** (550 mg, 2.27 mmol) and DMF (6mL) were added at RT. The reaction was purged with nitrogen gas for three times, and cooled to -40°C. Sodium hydride (60%, 363 mg, 9.08 mmol) was added, and the mixture was stirred for 30 minutes. Methyl iodide (3.22 g, 22.7 mmol) was added, and the reaction was stirred at -40°C for 1 hour. After the completion of reaction detected by TLC, 10 mL water was added, and the mixture was extracted with ethyl acetate (30mL×2). The combined organic phase was dried over anhydrous Na₂SO₄ and filtered. The filtrate was concentrated under reduced pressure. The obtained residue was purified by column chromatography (ethyl acetate : petroleum ether = 1:5) to yield (*R*)-6-bromo-1,3,4-trimethyl-3,4-dihydropyrido[2,3-b]pyrazin-2(1H)-one **(16)** (505 mg, white solid, ee: 97.5%), yield: 82%. LCMS:*m*/*z* 269.9, 271.9 (M+H).

### Intermediate 22: Synthesis of (R)-6-bromo-1,3-dimethyl-4-(tetrahydro-2H-pyran-4-yl)-3,4-dihydropyrido[2,3-b]pyrazin-2(1H)-one

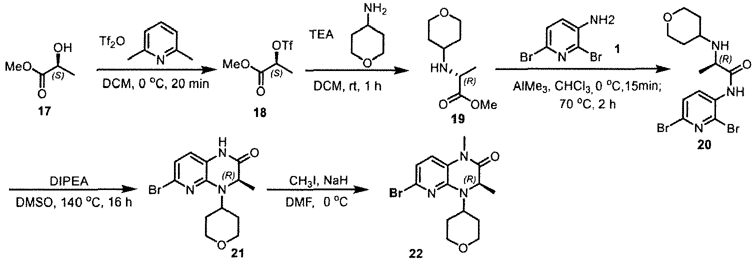

### Step 1. Synthesis of Methyl (S)-2-(((trifluoromethyl)sulfonyl)oxy)propionate (18)

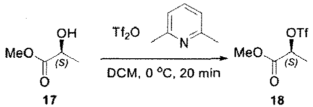

Into a dry 100 mL round-bottom flask, compound **17** (1.5 g, 6.36 mmol), anhydrous dichloromethane (50mL) were added, and trifluoromethanesulfonic anhydride (23 g, 80.8 mmol) and 2,6-dimethylpyridine (8.7 g, 80.8 mmol) were added dropwise at 0°C. After addition, the reaction mixture was stirred at 0°C for 20 min. Water (100mL) was added, and the mixture was extracted with dichloromethane (50mL×3). The combined organic phase was dried over anhydrous Na₂SO₄ and filtered. The filtrate was concentrated under reduced pressure. The obtained residue was purified by column chromatography (ethyl acetate : petroleum ether = 1:10) to yield methyl (S)-2-(((trifluoromethyl)sulfonyl)oxy)propionate **(18)** (8 g, brown oil), crude, yield: 44%. ¹H NMR (CDCl₃, 400mHz) 5.27 (q, *J* = 6.8 Hz, 1H), 3.88 (s,3H), 1.74 (d, *J* = 6.8 Hz, 3H).

### Step 2. Synthesis of methyl N-(tetrahydro-2H-pyran-4-yl)-D-alanine ester (19)

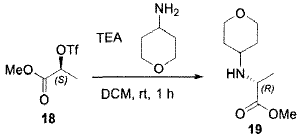

Into a dry 100 mL round-bottom flask, Intermediate **18** (8 g, 77 mmol), anhydrous dichloromethane (50mL), 4-aminotetrahydropyran (0.642 g, 6.36 mmol) and triethylamine (1.3 g, 12.72 mmol) were sequentially added at RT. The mixture was stirred for 1 hour. Water was added (100mL), and the mixture was extracted with ethyl acetate (50mL×3). The combined organic phase was dried over anhydrous Na₂SO₄ and filtered. The filtrate was concentrated under reduced pressure. The obtained residue was purified by column chromatography (ethyl acetate : petroleum ether =1:1) to yield methyl N-(tetrahydro-2H-pyran-4-yl)-D-alanine ester **(19)** (1.1 g, yellow oil), crude, yield: 92%. LCMS:*m*/*z* 188.0 (M+H).

### Step 3. Synthesis of (R)-N-(2,6-dibromopyridin-3-yl)-2-((tetrahydro-2H-pyran-4-yl)amino)p ropanamide (20)

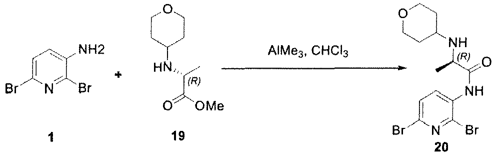

Under protection of nitrogen atmosphere, compound **1** (0.5 g, 1.98 mmol) and anhydrous chloroform (30mL) were added Into a dry 100 mL three-necked round-bottom flask, and the mixture was cooled to 0°C. Trimethyl aluminum (2 N, 5 mL, 10 mmol) was added dropwise, and the reaction mixture was stirred for 15 minutes. Intermediate **19** was added, and the reaction mixture was heated to 70°C under stirring for 2 hours. After the completion of reaction as monitored by LCMS, water (40mL) was added dropwise, and the mixture was extracted with ethyl acetate (50mL×3). The combined organic phase was dried over anhydrous Na₂SO₄ and filtered. The filtrate was concentrated under reduced pressure. The obtained residue was purified by column chromatography (ethyl acetate: petroleum ether = 1:1), to yield (*R*)-N-(2,6-dibromopyridin-3-yl)-2-((tetrahydro-2*H*-pyran-4-yl)amino)propanamide **(20)** (0.6 g, yellow oil), crude, yield: 74%. LCMS:*m*/*z* 405.9, 407.6 (M+H).

### Step 4. Synthesis of (R)-6-bromo-3-methyl-4-(4-tetrahydropyranyl)-3,4-pyrido[2,3-b]pyrazi n-2(1H)-one (21)

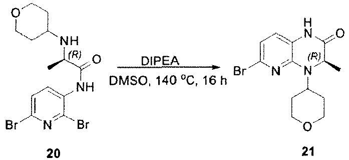

Into a dry 100 mL round-bottom flask, Intermediate **20** (0.6 g, 1.47 mmol), dimethyl sulfoxide (20mL) and *N*,*N*-diisopropylethylamine (1.9 g, 14.74 mmol) were added. The reaction mixture was warmed to 140°C under stirring for 16 hours. After the completion of reaction as monitored by LCMS, water (40mL) was added, and the mixture was extracted with ethyl acetate (20mL×3). The combined organic phase was dried over anhydrous Na₂SO₄ and filtered. The filtrate was concentrated under reduced pressure. The obtained residue was purified by column chromatography (ethyl acetate : petroleum ether = 1:1) to yield (*R*)-6-bromo-3-methyl-4-(4-tetrahydropyranyl)-3,4-pyrido[2,3-b]pyrazin-2(1H)-one **(21)** (0.31 g, yellow oil), crude, yield: 64%. LCMS:*m*/*z* 325.8, 327.8 (M+H).

### Step 5. Synthesis of (R)-6-bromo-1,3-dimethyl-4-(tetrahydro-2H-pyran-4-yl)-3,4-dihydropy rido[2,3-b]pyrazin-2(1H)-one (22)

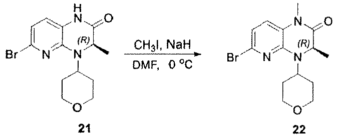

Into a dry 100 mL round-bottom flask, Intermediate **21** (0.3 g, 0.92 mmol), DMF (10mL) were added. The mixture was cooled to 0°C. Sodium hydride (60%, 55 mg, 1.38 mmol) was added, and the reaction mixture was stirred for 10 minutes. Methyl iodide (261 mg, 1.84 mmol) was added dropwise, and the reaction mixture was stirred at 0°C for 1 hour. Water was added (30mL), and the mixture was extracted with ethyl acetate (20mL×3). The combined organic phase was dried over anhydrous Na₂SO₄ and filtered. The filtrate was concentrated under reduced pressure. The obtained residue was purified by column chromatography (ethyl acetate : petroleum ether = 1:3) to yield (*R*)-6-bromo-1,3-dimethyl-4-(tetrahydro-2*H*-pyran-4-yl)-3,4-dihydropyrido[2,3-b]pyrazin-2(1H)-one **(22)** (0.3 g, brown oil), yield: 96%. LCMS:*m*/*z* 339.9,341.9 (M+H).

### Intermediate 25: Synthesis of 6-bromo-4-cyclopropyl-1,3,8-trimethyl-3,4-dihydropyrido[2,3 -b]pyrazin-2(1H)-one (25)

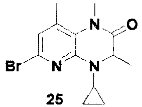

Intermediate **25** was prepared analogously to the synthetic procedure of Intermediate **5,** starting from 2,6-dibromo-4-methylpyridin-3-amine **(24).**

### Synthesis of 2,6-dibromo-4-methylpyridin-3-amine (24)

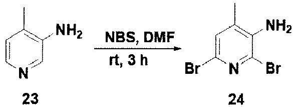

Into a dry 100 mL round-bottom flask, compound **23** (2.0 g, 18.5 mmol), NBS (6.58 g, 37 mmol) and DMF (10mL) were sequentially added at RT. The mixture was stirred at RT for 3 hours. After the completion of reaction as monitored on TLC plate, 30 mL water was added, and the mixture was extracted with EtOAc (50mL×3). The combined organic phase was dried over anhydrous Na₂SO₄ and filtered. The filtrate was concentrated under reduced pressure. The obtained residue was purified by column chromatography (ethyl acetate: petroleum ether = 1:5) to yield 2,6-dibromo-4-methylpyridin-3-amine **(24)** (2.89 g, pale yellow solid), yield: 58%. LCMS: *m*/*z* 266.8 (M+H).

### Intermediate 26: Synthesis of 6-bromo-4-cyclopropyl-8-fluoro-1,3-dimethyl-3,4-dihydroqui noxalin-2(1H)-one (26)

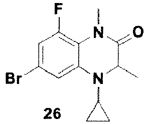

Intermediate **26** was prepared analogously to the synthetic procedure of Intermediate **5,** from 4-bromo-2,6-difluoroaniline.

### Intermediate 29: Synthesis of 6-bromo-1,3-dimethyl-1H-pyrido[2,3-B][1,4]oxazin-2(3H)-one (29)

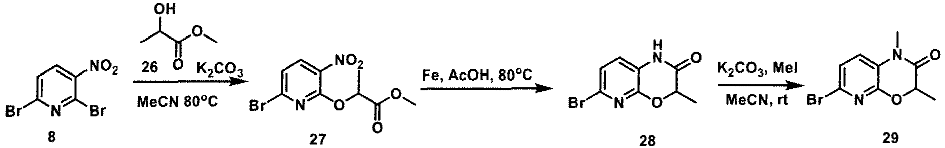

### Step 1: Synthesis of methyl 2-((6-bromo-3-nitropyridin-2-yl)oxy)propionate (27)

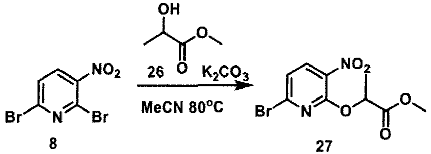

Into a dry 100 mL round-bottom flask, compound **8** (2 g, 7.14 mmol), compound **26** (743 mg, 7.14 mmol), potassium carbonate (1.97 g, 14.28 mmol) and acetonitrile (20mL) were sequentially added at RT. The mixture was heated to 80°C under stirring for 16 hours, cooled to RT, and concentrated under reduced pressure. The obtained residue was purified by column chromatography (petroleum ether : ethyl acetate = 20:1) to yield methyl 2-((6-bromo-3-nitropyridin-2-yl)oxy)propionate **(27)** (770 mg, pale yellow solid), yield: 35%. LCMS: *m*/*z* 306.8 (M+H).

### Step 2: Synthesis of 6-bromo-3-methyl-1H-pyrido[2,3-B][1,4]oxazin-2(3H)-one (28)

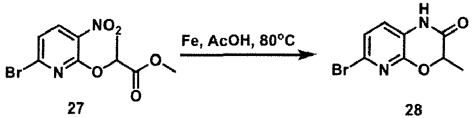

Into a dry 100 mL round-bottom flask, compound **27** (0.77 g, 2.53 mmol), acetic acid (20mL) and iron powder (0.71 g, 12.67 mmol) were sequentially added at RT. The mixture was heated to 80°C under stirring for 2 hours, and filtered hot. The filtrate was concentrated under reduced pressure, and the residue was dissolved in water. The mixture was adjusted to pH 9 with 5N aqueous sodium hydroxide solution, and was extracted with ethyl acetate (50mL×3). The combined organic phase was dried over anhydrous Na₂SO₄ and filtered. The filtrate was concentrated under reduced pressure. The obtained residue was purified by column chromatography (petroleum ether:ethyl acetate = 2:1) to yield 6-bromo-3-methyl-1*H*-pyrido[2,3-B][1,4]oxazin-2(3H)-one **(28)** (0.4 g, white solid), yield: 65%. LCMS: *m*/*z* 242.8 (M+H).

### Step 3: Synthesis of 6-bromo-1,3-dimethyl-1H-pyrido[2,3-B][1,4]oxazin-2(3H)-one (29)

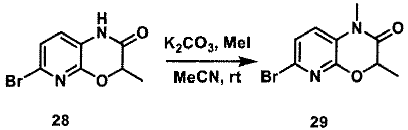

Into a dry 50 mL round-bottom flask, Intermediate **28** (0.35 g, 1.45 mmol), potassium carbonate (0.31 g, 2.17 mmol), methyl iodide (0.39 g, 2.89 mmol) and acetonitrile (15mL) were sequentially added at RT. The mixture was stirred at RT for 16 hours, and concentrated under reduced pressure. The residue was added to 50 mL water, and the mixture was extracted with ethyl acetate (20mL×3). The combined organic phase was dried over anhydrous Na₂SO₄ and filtered. The filtrate was concentrated under reduced pressure to yield 6-bromo-1,3-dimethyl-1*H-*pyrido[2,3-B][1,4]oxazin-2(3H)-one **(29)** (0.4 g, white solid, crude, purity: 93%). LCMS: *m*/*z* 258.7 (M+H).

### Intermediate 32: Synthesis of 5-(4-chlorophenyl)pyrrolidin-2-one (32)

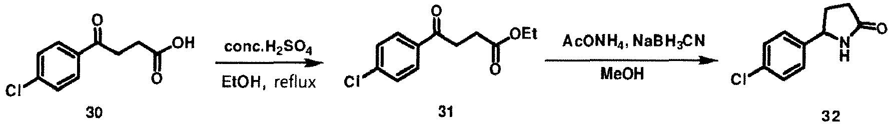

### Step 1: Synthesis of Ethyl 4-(4-chlorophenyl)-4-oxobutyrate (31)

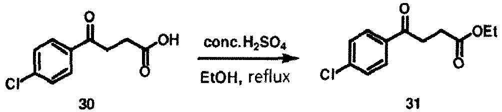

Into a dry 250 mL round-bottom flask, compound **30** (3 g, 14.15 mmol), 98% concentrated sulfuric acid (4 g, 42.45 mmol) and ethanol (20mL) were sequentially added at RT. The mixture was heated to reflux under stirring for 16 hours. The mixture was cooled to RT, and concentrated under reduced pressure. 50 mL water was added to the residue, and the mixture was extracted with ethyl acetate (30mL×3). The combined organic phase was dried over anhydrous Na₂SO₄ and filtered. The filtrate was concentrated under reduced pressure to yield ethyl 4-(4-chlorophenyl)-4-oxobutyrate **(31)** (3.3 g, white solid), yield: 97%. LCMS: *m*/*z* 240.06, 262.9 (M+Na).

### Step 2. Synthesis of 5-(4-chlorophenyl)pyrrolidin-2-one (32)

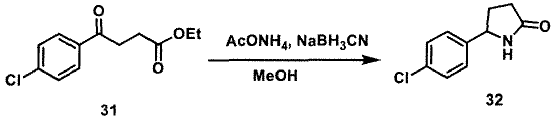

Into a dry 100 mL round-bottom flask, compound **31** (2.8 g, 11.67 mmol), ammonium acetate (9 g, 116.70 mmol) and methanol (80mL) were sequentially added at RT. The reaction mixture was stirred for 1 hour at RT, followed by addition of sodium cyanoborohydride (2.4 g, 38.50 mmol), heated to 50°C, and stirred for 16 hours. The mixture was cooled to RT, and concentrated under reduced pressure. 100 mL water was added to the residue, and the mixture was extracted with ethyl acetate (50mL×3). The combined organic phase was dried over anhydrous Na₂SO₄ and filtered. The filtrate was concentrated under reduced pressure. The obtained residue was purified by column chromatography (dichloromethane:methanol = 50:1) to yield 5-(4-chlorophenyl)pyrrolidin-2-one **(32)** (1.05 g, grey solid), yield: 46%. LCMS: *m*/*z* 195.9 (M+H).

### Intermediate 33: Synthesis of 5-(4-fluorophenyl)pyrrolidin-2-one (33)

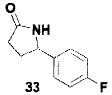

Intermediate **33** was prepared analogously to the synthetic procedure of Intermediate **32** 5-(4-chlorophenyl)pyrrolidin-2-one, starting from 4-(4-fluorophenyl)-4-oxobutyric acid.

### Intermediate 41: Synthesis of 5-(p-tolyl)pyrrolidin-2-one (41)

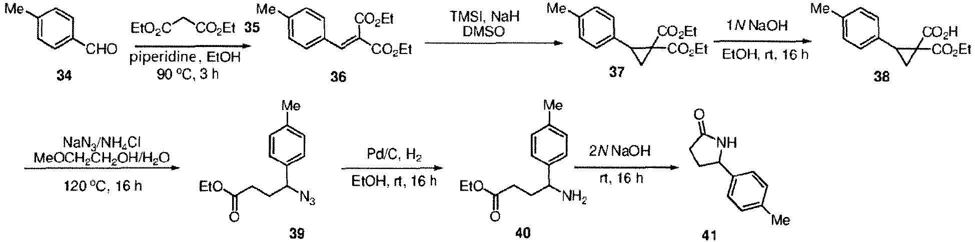

### Step 1: Synthesis of Diethyl 2-(4-methylbenzylidene)malonate (36)

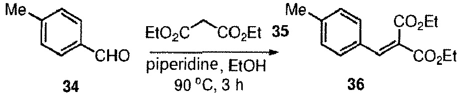

Into a dry 500 mL round-bottom flask, compound **34** (10.0 g, 83.3 mmol), compound **35** (14.67 g, 91.67 mmol), piperidine (0.708 g, 8.33 mmol), acetic acid (1 g, 16.67 mmol) and ethanol (150mL) were sequentially added at RT. The mixture was heated to 90°C under stirring for 3 hours, cooled to RT, and concentrated under reduced pressure. The obtained residue was purified by column chromatography (ethyl acetate : petroleum ether = 1:20) to yield diethyl 2-(4-methylbenzylidene)malonate **(36)** (15.1 g, colorless liquid), yield: 69%. LCMS:*m*/*z* 262.9 (M+H).

### Step 2. Synthesis of Diethyl 2-(4-methylphenyl)cyclopropane-1,1-dicarboxylate (37)

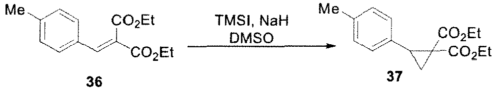

Into a dry 500 mL round-bottom flask, trimethylsulfoxonium iodide (12.68 g, 57.63 mmol) and DMSO (100mL) were sequentially added at RT. Sodium hydride (2.3 g, 60%, 57.63 mmol) was added slowly. The reaction mixture was stirred for 30 minutes while the temperature was controlled between 0 and 10°C. Then the mixture was warmed to RT. Intermediate **36** (15.1 g, 57.63 mmol) was added dropwise, and the mixture was stirred for 16 hours. The reaction mixture was quenched by addition of water (200mL), and extracted with ethyl acetate (150mL×3). The combined organic phase was dried over anhydrous Na₂SO₄ and filtered. The filtrate was concentrated under reduced pressure. The obtained residue was purified by column chromatography (petroleum ether:ethyl acetate = 10:1) to yield the product diethyl 2-(4-methylphenyl)cyclopropane-1,1-dicarboxylate **(37)** (9.8 g, colorless liquid), yield: 62%. LCMS: *m*/*z* 276.9 (M+H).

### Step 3. Synthesis of 2-(4-methylphenyl)-1-(ethoxycarbonyl)cyclopropane-1-carboxylic acid (38)

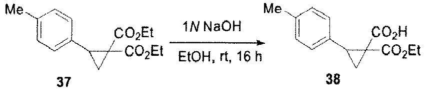

Into a dry 500 mL round-bottom flask, Intermediate **37** (9.8 g, 35.5 mmol), ethanol (100mL) and 1*N* aqueous sodium hydroxide solution (46 mL, 46.16 mmol) were added at RT. The reaction mixture was stirred for 16 hours at RT and then concentrated under reduced pressure. Water (50mL) was added to the residue. The mixture was adjusted to pH 3-4 with 6 *N* hydrochloric acid, and extracted with ethyl acetate (50mL×3). The combined organic phase was dried over anhydrous Na₂SO₄ and filtered. The filtrate was concentrated under reduced pressure to yield 2-(4-methylphenyl)-1-(ethoxycarbonyl)cyclopropane-1-carboxylic acid **(38)** (8.3 g, white solid), yield: 94%. LCMS: *m*/*z* 248.9 (M+H).

### Step 4. Synthesis of Ethyl 4-azido-4-(4-methylphenyl)butyrate (39)

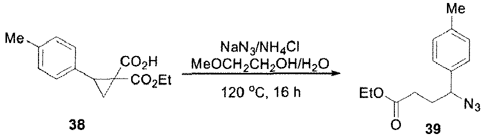

Into a dry 250 mL round-bottom flask, Intermediate **38** (4 g, 16.13 mmol), sodium azide (1.26 g, 19.35 mmol), ammonium chloride (1.2 g, 22.58 mmol), 2-methoxyethanol and water (10:1, 22mL) were sequentially added at RT. The mixture was heated to 120°C under stirring for 16 hours. Water (100mL) was added, and the mixture was extracted with ethyl acetate (50mL×3). The combined organic phase was dried over anhydrous Na₂SO4 and filtered. The filtrate was concentrated under reduced pressure. The obtained residue was purified by column chromatography (petroleum ether:ethyl acetate = 10:1) to yield ethyl 4-azido-4-(4-methylphenyl)butyrate **(39)** (3.37 g, colorless liquid), yield: 84%. LCMS: *m*/*z* 205.0 (M-42).

### Step 5. Synthesis of Ethyl 4-amino-4-(4-methylphenyl)butyrate (40)

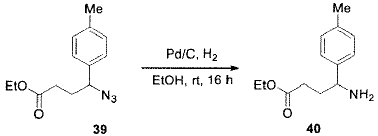

Into a dry 100 mL round-bottom flask, Intermediate **39** (2 g, 8.1 mmol), 10% Pd/C (400 mg) and ethanol (10mL) were sequentially added at RT. The mixture was stirred at RT for 16 hours under 1 atmosphere of hydrogen, and filtered. The filtrate was concentrated under reduced pressure to yield ethyl 4-amino-4-(4-methylphenyl)butyrate **(40)** (1.63 g, colorless liquid), yield: 91%. LCMS: *m*/*z* 222.0 (M+H).

### Step 6. Synthesis of 5-(4-methylphenyl)pyrrolidin-2-one (41)

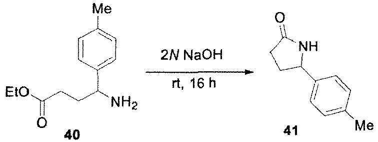

Into a dry 100 mL round-bottom flask, Intermediate **40** (1.63 g, 7.37 mmol), 2N sodium hydroxide solution (5.5 mL, 11.06 mmol) and methanol (20mL) were sequentially added at RT. The mixture was stirred at RT for 16 hours. 50 mL water was added. The mixture was extracted with ethyl acetate (20mL×3). The combined organic phase was dried over anhydrous Na₂SO₄ and filtered. The filtrate was concentrated under reduced pressure. The obtained residue was purified by column chromatography (dichloromethane:methanol = 30:1) to yield 5-(4-methylphenyl)pyrrolidin-2-one **(41)** (1 g, white solid), yield: 77%. LCMS: *m*/*z* 176.0 (M+H).

### Intermediate 44: Synthesis of 5-(2,4-difluorophen-yl)pyrrolidin-2-one (44)

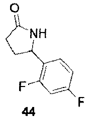

Intermediate **44** was prepared analogously to the synthetic procedure of Intermediate **41** 5-(p-tolyl)pyrrolidin-2-one, starting from 2,4-difluorobenzaldehyde, except that the reaction conditions in the following azide reduction step are different.

### Synthesis of Ethyl 4-amino-4-(2,4-difluorophenyl)butyrate (43)

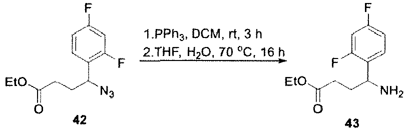

Into a dry 100 mL round-bottom flask, compound **42** (3 g, 11.14 mmol), triphenylphosphine (3.5 g, 13.37 mmol) and DCM (50mL) were sequentially added at RT. The mixture was stirred at RT for 3 hours and then concentrated under reduced pressure. THF (10mL) and water (1mL) were added to the residue. The reaction mixture was warmed to 70°C under stirring for 16 hours, and then filtered. The filtrate was concentrated under reduced pressure. The obtained residue was purified by column chromatography (ethyl acetate : petroleum ether = 1:1) to yield ethyl 4-amino-4-(2,4-difluorophenyl)butyrate **(43)** (6 g, white solid), purity: 20%. LCMS: *m*/*z* 243.9 (M+H).

### Intermediate 45: Synthesis of 5-(pyridin-2-yl)pyrrolidin-2-one (45)

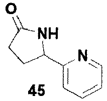

Intermediate **45** was prepared analogously to the synthetic procedure of Intermediate **44** 5-(2,4-difluorophenyl)pyrrolidin-2-one, starting from pyridylaldehyde.

### Intermediate 46: Synthesis of 5-(pyridin-3-yl)pyrrolidin-2-one (46)

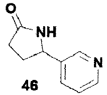

Intermediate **46** was prepared analogously to the synthetic procedure of Intermediate **44** 5-(2,4-difluorophenyl)pyrrolidin-2-one, starting from nicotinealdehyde.

### Intermediate 47: Synthesis of 5-(pyridin-4-yl)pyrrolidin-2-one (47)

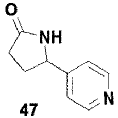

Intermediate **47** was prepared analogously to the synthetic procedure of Intermediate **44** 5-(2,4-difluorophenyl)pyrrolidin-2-one, starting from isonicotinic aldehyde.

### Intermediate 53: Synthesis of Tert-butyl 2-oxo-4-phenylimidazolidine-1-carboxylate

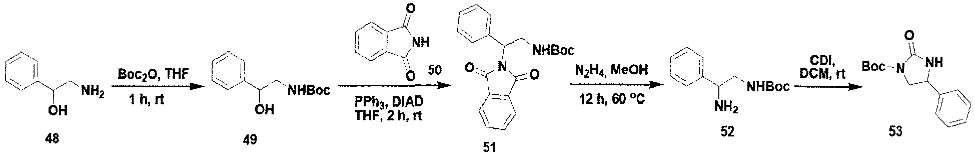

### Step 1. Synthesis of Tert-butyl (2-hydroxy-2-phenethyl)carbamate (49)

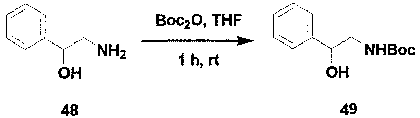

Into a dry 250 mL single-neck reaction flask compound **48** (5 g, 36 mmol) and anhydrous tetrahydrofuran (50mL) were added, and then di-tert-butyl dicarbonate (8.75 g, 40 mmol) was slowly added dropwise at 0°C. The mixture was warmed to RT, and stirred for 1 hour. After the completion of the reaction, the mixture was concentrated under reduced pressure to yield crude tert-butyl (2-hydroxy-2-phenethyl)carbamate **49** (8.64 g, yellow liquid), which was directly used in the next step. LCMS: m/*z* 259.9 (M+23).

### Step 2. Synthesis of Tert-butyl 2-(1,3-dioxoisoindolin-2-yl)-2-phenylethylcarbamate (51)

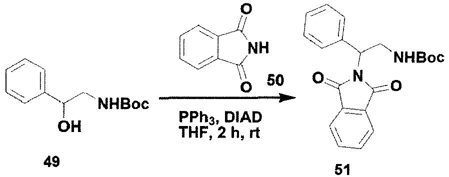

Into a dry 250 mL three-necked flask, Intermediate **49** (8.64 g, 36 mmol), anhydrous tetrahydrofuran (100mL), triphenylphosphine (12.42 g, 47 mmol) and phthalimide **(50)** (5.36 g, 36 mmol) were added. The mixture was purged with argon gas for three times, and diisopropyl azodicarboxylate (9.58 g, 47 mmol) was slowly added dropwise at 0°C. The reaction mixture was warmed to RT under stirring for 2 hours. After the completion of the reaction, the mixture was concentrated under reduced pressure. The obtained residue was purified by column chromatography (ethyl acetate : petroleum ether = 1:5) to yield crude tert-butyl 2-(1,3-dioxoisoindolin-2-yl)-2-phenylethylcarbamate **(51)** (6.7 g, white solid), which was directly used in the next step. LCMS: m/*z* 267.0 (M-99).

### Step 3. Synthesis of Tert-butyl (2-amino-2-phenethyl)carbamate (52)

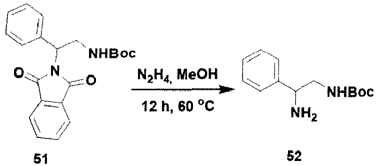

Into a dry 250 mL round-bottom flask, compound **51** (6.7 g, 18 mmol), methanol (30mL) and hydrazine hydrate (3.6 g, 91mmol, 80wt%) were sequentially added. The mixture was warmed to 60°C under stirring for 12 hours. After the completion of the reaction, the mixture was concentrated under reduced pressure to yield tert-butyl (2-amino-2-phenethyl)carbamate **52** (4.3 g, yellow liquid, crude), which was directly used in the next step. LCMS: m/*z* 237.0.

### Step 4. Synthesis of Tert-butyl 2-carbonyl-4-phenylimidazolidine-1-carboxylate (53)

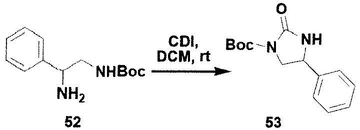

Into a dry 100 mL round-bottom flask, Intermediate **52** (4.3 g, 18 mmol), dry dichloromethane (40mL) and N,N'-carbonyldiimidazole (4.13 g, 25 mmol) were added. The reaction mixture was stirred at RT for 12 hours. After the completion of the reaction, the mixture was concentrated under reduced pressure. The obtained residue was purified by preparative high-performance liquid chromatography (column: Gemini-C18, 150×21.2 mm, 5µm, mobile phase: ACN-H₂O (0.1% FA), gradient: 40-50) to yield tert-butyl 2-carbonyl-4-phenylimidazolidine-1-carboxylate **(53)** (1.7 g, white solid), total yield over 4 steps: 17%. LCMS: m/*z* 284.8 (M+23). ¹H NMR (DMSO-*d₆*, 400mHz): δ 7.92 (s,1H), 7.44-7.31 (m, 5H), 4.77-4.71 (m, 1H), 4.18 (t, *J =* 10Hz, 1H), 3.42 (dd, *J=* 10.4, 6.4 Hz, 1H), 1.45 (s, 9H).

### Intermediate 56: Synthesis of 1-(4-chlorophenyl)pyrazolidin-3-one (56)

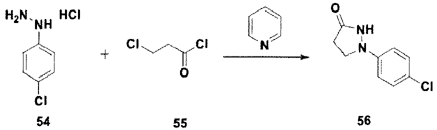

Into a dry 100 mL single-neck reaction flask, compound **54** (3 g, 16.07 mmol) and anhydrous pyridine (50mL) were added, and compound **55** (2.34 g, 18.4 mmol) was slowly added dropwise at 0°C. The mixture was warmed to RT under stirring for 4 hours, and then to 100°C under stirring for 8 hours. After the completion of the reaction, the mixture was diluted with dichloromethane, and 2N hydrochloric acid solution was slowly added dropwise under ice bath to adjust pH to 2. The organic phase was separated, washed with saturated brine (40mL×2), dried over anhydrous Na₂SO₄, and filtered. The filtrate was concentrated under reduced pressure. The obtained residue was purified by column chromatography (ethyl acetate : petroleum ether = 1:1) to yield 1-(4-chlorophenyl) pyrazolidin-3-one **(56)** (0.6 g, brown solid), yield: 18.7%. LCMS :m/*z* 196.9 (M+H).

### Intermediate 57: Synthesis of 3-hydroxy-3-phenylisoindolin-1-one (57)

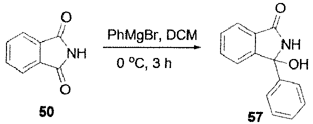

Into a dry 250 mL round-bottom flask, phthalimide **(50)** (3.0 g, 20.4 mmol) and dichloromethane (100mL) were sequentially added at RT. The mixture was cooled to 0°C, and phenyl magnesium bromide solution (IN solution in THF, 61 mL, 61 mmol) was slowly added dropwise. The mixture was stirred at 0°C for 3 hours. Water (300mL) was added, and the mixture was extracted with ethyl acetate (200mL×3). The combined organic phase was dried over anhydrous Na₂SO₄ and filtered. The filtrate was concentrated under reduced pressure. The obtained residue was purified by column chromatography (petroleum ether:ethyl acetate = 1:1) to yield 3-hydroxy-3-phenylisoindolin-1-one **(57)** (4.3 g, white solid), yield: 93%. LCMS: *m*/*z* 248.0 (M+23).

### Intermediate 58: Synthesis of 3-phenylisoindolin-1-one (58)

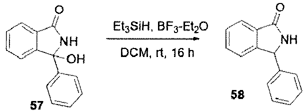

Into a dry 100 mL round-bottom flask, Intermediate **57** (4.3 g, 19.11 mmol) and dichloromethane (50mL) were sequentially added at RT. The mixture was cooled to -40°C, and BF₃-Et₂O (10mL) and Et3SiH (6.65 g, 57.33 mmol) were added slowly. After the addition, the mixture was warmed to RT, and stirred for 16 hours. After the completion of reaction, the mixture was adjusted to pH 6 with saturated sodium bicarbonate solution, and extracted with ethyl acetate (100mL×3). The combined organic phase was dried over anhydrous Na₂SO₄ and filtered. The filtrate was concentrated under reduced pressure to yield the product 3-phenylisoindolin-1-one **(58)** (1.2 g, white solid), yield: 30%. LCMS: *m*/*z* 210.0 (M+H).

### Intermediate 61: Synthesis of 1-(4-chlorophenyl)-1,2-dihydro-3H-indazol-3-one (61)

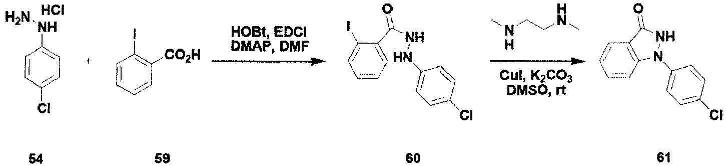

### Step 1. Synthesis of N'-(4-chlorophenyl)-2-iodobenzohydrazide (60)

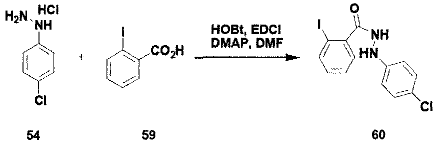

Into a dry 100 mL single-neck reaction flask, compound **54** (1.44 g, 8.06 mmol), anhydrous DMF (40mL), compound **59** (2 g, 8.06 mmol), 1-hydroxybenzotriazole (1.19 g, 8.86 mmol), 1-ethyl-(3-dimethylaminopropyl)carbodiimide hydrochloride (1.7 g, 8.86 mmol) and 4-dimethylaminopyridine (0.983 g, 8.06 mmol) were added. The mixture was stirred at RT for 5 hours. After the completion of the reaction as monitored by LCMS, the reaction mixture was poured into ice-water mixture, precipitating a large amount of white solid, and was filted. The filter cake was washed with water (50mL×2). The resulting crude solid was washed with a mixture solution of ethyl acetate (10mL) and petroleum ether (30mL) to yield N'-(4-chlorophenyl)-2- iodobenzohydrazide **(60)** (1.6 g, white solid), yield: 55%. LCMS: m/*z* 372.8, 374.8 (M+H).

### Step 2. Synthesis of 1-(4-chlorophenyl)-1,2-dihydro-3H-indazol-3-one (61)

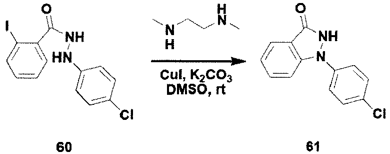

Into a dry 100 mL round-bottom flask, Intermediate **60** (0.6 g, 1.61 mmol), DMSO (20mL), N,N'-dimethylethylenediamine (0.283 g, 3.22 mmol), potassium carbonate (0.444 g, 3.22 mmol) and copper iodide (0.306 g, 1.61 mmol) were added. The mixture was stirred at RT for 4 hours. After the completion of the reaction as monitored by LCMS, the mixture was diluted with water, and extracted with ethyl acetate (50mL×2). The organic phases were combined, washed with saturated brine (50mL×2), dried over anhydrous Na₂SO₄ and filtered. The filtrate was concentrated under reduced pressure. The obtained residue was washed with a mixture solution of ethyl acetate (10mL) and petroleum ether (30mL) to yield 1-(4-chlorophenyl)-1,2-dihydro-3*H*-indazol-3-one **(61)** (0.17 g, white solid), yield: 18.4%. LCMS m/*z* 244.9, 246.9 (M+H).

### Intermediate 66: Synthesis of 7-(2,4-difluorophenyl)-7-hydroxy-6,7-dihydro-5H-pyrrolo[3,4 -b]pyridin-5-one (66)

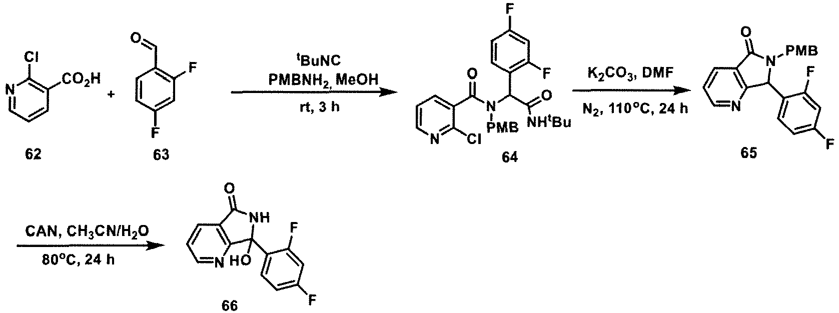

### Step 1: Synthesis of N-(2-(tert-butylamino)-1-(2,4-difluorophenyl)-2-carbonylethyl)-2-chlor o-N-(4-methoxybenzyl)niacinamide (64)

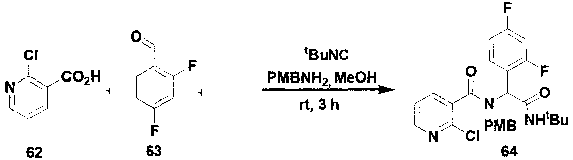

Into a dry 100 mL round-bottom flask, compound **62** (1.58 g, 10 mmol), compound **63** (1.42 g, 10 mmol), 2-isocyano-2-methylpropane (830 mg, 10 mmol), (4-methoxyphenyl)methylamine (1.37 g, 10 mmol) and methanol (60mL) were sequentially added at RT. The mixture was stirred at RT for 3 hours. After the completion of reaction as monitored on TLC plate, the mixture was concentrated under reduced pressure to yield N-(2-(tert-butylamino)-1-(2,4-difluorophenyl)-2-carbonylethyl)-2-chloro-N-(4-methoxybenzyl)niacinamide **(64)** (crude 5.02 g, white solid), yield: 100%, which was directly used in the next reaction. LCMS: *m*/*z* 501.9 (M+H).

### Step 2: Synthesis of 7-(2,4-difluorophenyl)-6-(4-methoxybenzyl)-6,7-dihydro-5H-pyrrolo[3,4 -b]pyridin-5-one (65)

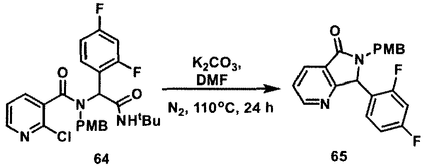

Into a dry 250 mL round-bottom flask, Intermediate **64** (5.02 g, 10 mmol), potassium carbonate (2.76 g, 20 mmol) and N,N-dimethylformamide (10mL) were sequentially added at RT. The mixture was purged with nitrogen gas for three times, and heated to 110°C under stirring for 24 hours. After the completion of reaction as monitored on TLC plate, the mixture was cooled to RT, 30 mL water was added, and the mixture was extracted with ethyl acetate (50mL×3). The combined organic phase was dried over anhydrous Na₂SO₄ and filtered. The filtrate was concentrated under reduced pressure. The obtained residue was purified by column chromatography (methanol : dichloromethane = 1:20) to yield 7-(2,4-difluorophenyl)-6-(4-methoxybenzyl)-6,7-dihydro-5*H*-pyrrolo[3,4-b]pyridin-5-one **(65)** (1.9 g, white solid), yield: 52%. LCMS: *m*/*z* 367.0 (M+H).

### Step 3: Synthesis of 7-(2,4-difluorophenyl)-7-hydroxy-6,7-dihydro-5H-pyrrolo[3,4-b]pyridin -5-one (66)

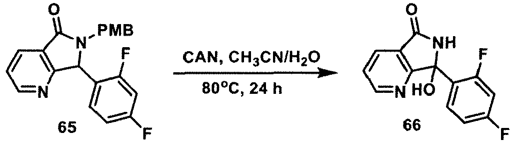

Into a dry 100 mL round-bottom flask, Intermediate **65** (900 mg, 2.46 mmol), cerous ammonium nitrate (3.37 g, 6.15 mmol) and acetonitrile/water (10 mL /10mL) were added at RT. The mixture was heated to 80°C under stirring for 24 hours. After the completion of reaction as monitored on TLC plate, the mixture was extracted with ethyl acetate (40mL×3). The combined organic phase was dried over anhydrous Na₂SO₄ and filtered. The filtrate was concentrated under reduced pressure. The obtained residue was purified by reverse phase chromatography (0.1% trifluoroacetic acid, mobile phase: acetonitrile and water) to yield 7-(2,4-difluorophenyl)-7-hydroxy-6,7-dihydro-5*H-*pyrrolo[3,4-b]pyridin-5-one **(66)** (230 mg, white solid), yield: 35%. LCMS: *m*/*z* 263.0 (M+H).

### Example A1: Synthesis of 4-cyclopropyl-1,3-dimethyl-6-(2-oxo-5-phenyl-pyrrolidin-1-yl)-3, 4-dihydropyrido[2,3-b]pyrazin-2(1H)-one (A1)

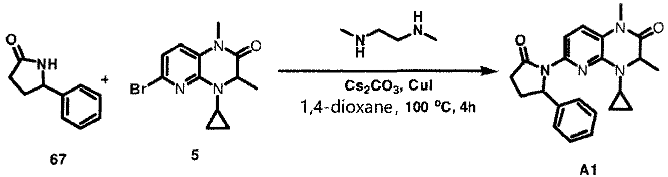

Into a dry 100 mL round-bottom flask, 5-phenylpyrrolidin-2-one **(67)** (100 mg, 0.62 mmol, purchased from Shanghai Shuya Pharmaceutical Technology Co., Ltd.), Intermediate **5** (184 mg, 0.62 mmol), N,N'-dimethylethylenediamine (110 mg, 1.24 mmol), Copper iodide (118 mg, 0.62 mmol), cesium carbonate (403 mg, 1.24 mmol) and 1,4-dioxane (10mL) were sequentially added at RT. The mixture was purged with nitrogen gas for three times, and then was heated to 100°C under stirring for 4 hours. After the completion of reaction as monitored on TLC plate, the mixture was concentrated under reduced pressure. 10 mL water was added, and the mixture was extracted with ethyl acetate (30mL×3). The organic phases were combined, dried over anhydrous Na₂SO₄ and filtered. The filtrate was concentrated under reduced pressure. The obtained residue was purified by column chromatography (methanol:dichloromethane = 1:20) to yield the title product of Example **A1** (200 mg, yellow oil), yield: 85.8%.

Then chiral separation (Supercritical Fluid Chromatography, SFC) was performed: diastereomers were first separated (chiral column: OD-H, eluent: 70% CO₂ + 30% MeOH (0.2% DEA)); then the enantiomers were separated (chiral column: AS-H, eluent: 70% CO₂ + 30% IPA (0.2% DEA)). Four isomers were obtained:
**A1-P1-1:** LCMS:*m*/*z* 377.0 (M+H); ¹H-NMR (CDCl₃, 400mHz): 7.71 (d, *J* = 8.4 Hz, 1H), 7.26-7.18 (m, 5H), 7.06 (d, *J* = 8.5 Hz, 1H), 5.75 (dd,*J* = 8.0,3.6 Hz, 1H), 4.04 (q, *J =* 6.8 Hz, 1H), 3.25 (s,3H), 2.90-2.79 (m, 1H), 2.69-2.58 (m, 2H), 2.08-1.95 (m, 2H), 1.00 (d, *J* = 6.8 Hz, 3H), 0.94-0.86 (m, 1H), 0.66-0.57 (m, 1H), 0.55-0.42 (m, 2H).
**A1-P1-2:** LCMS:*m*/*z* 377.0 (M+H); ¹H-NMR (CDCl₃, 400mHz): 7.81 (d, *J* = 8.4 Hz, 1H), 7.27-7.17 (m, 5H), 7.08 (d, *J* = 8.5 Hz, 1H), 5.78 (dd,*J* = 8.0,3.5 Hz, 1H), 4.05 (q, *J=* 6.8 Hz, 1H), 3.27 (s,3H), 2.94-2.77 (m, 1H), 2.71-2.56 (m, 2H), 2.08-1.94 (m, 2H), 0.99 (d, *J =* 6.8 Hz, 3H), 0.92-0.83 (m, 1H), 0.65-0.57 (m, 1H), 0.55-0.42 (m, 2H).
**A1-P2-1:** LCMS:*m*/*z* 377.0 (M+H); ¹H-NMR (CDCl₃, 400mHz): 7.80 (d,J= 8.4 Hz, 1H), 7.29-7.14 (m, 5H), 7.09 (d, *J* = 8.5 Hz, 1H), 5.95 (m, 1H), 4.09 (q, *J* = 6.8 Hz, 1H), 3.26 (s, 3H), 2.81-2.69 (m, 1H), 2.68-2.53 (m, 2H), 2.48-2.41 (m, 1H), 2.04-1.96 (m, 1H), 1.21 (d, *J* = 6.8 Hz, 3H), 0.58-0.50 (m, 1H), 0.44-0.36 (m, 1H), 0.35-0.26 (m, 1H), -0.14- -0.23 (m, 1H).
**A1-P2-2:** LCMS:*m*/*z* 377.0 (M+H); ¹H-NMR (CDCl₃, 400mHz): 7.78 (d, *J* = 8.4 Hz, 1H), 7.29-7.14 (m, 5H), 7.10 (d, *J* = 8.5 Hz, 1H), 5.96 (m, 1H), 4.10 (q, *J* = 6.8 Hz, 1H), 3.26 (s, 3H), 2.82 - 2.71 (m, 1H), 2.68-2.54 (m, 2H), 2.51-2.42 (m, 1H), 2.05-1.96 (m, 1H), 1.22 (d, *J* = 6.8 Hz, 3H), 0.60-0.52 (m, 1H), 0.46-0.37 (m, 1H), 0.37-0.28 (m, 1H), -0.12- -0.22 (m, 1H).

Analogously to the synthesis of Example A1, the following examples were synthesized according to the general synthesis scheme A:

| Ex. | Structure | Name | Analysis data |
|---|---|---|---|
| A2 | 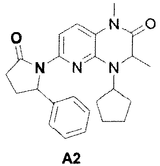 | 4-cyclopentyl-1,3-dimethyl-6-(2-oxo-5-phenylpyrrolidin-1-yl)-3,4-dihydropyrido[2,3-b]pyrazin-2(1H)-one | **A2-P1-1:** LCMS:*m*/*z* 405.0 (M+H); ¹H NMR (CDCl₃, 400mHz) 7.75 (d, *J* = 8.4 Hz, 1H), 7.32-7.26 (m, 2H), 7.18 (t,*J* = 8.0 Hz, 3H), 7.07 (d, *J* = 8.4 Hz, 1H), 5.70 (dd,*J* = 8.4 Hz, 2.8 Hz, 1H), 4.15 (q,*J* = 6.8 Hz, 1H), 3.89-3.78 (m, 1H), 3.30 (s,3H), 2.86-2.75 (m, 1H), 2.66-2.56 (m, 1H), 2.06-1.90 (m, 2H), 1.73-1.55 (m, 4H), 1.54-1.41 (m, 2H), 1.40-1.26 (m, 2H), 0.93 (d, *J* = 6.8 Hz, 3H). |
| | | | **A2-P2-1:** LCMS:*m*/*z* 405.1 (M+H); ¹H NMR (CDCl₃, 400mHz) 7.81 (d, *J* = 8.4 Hz, 1H), 7.40-7.25 (m, 2H), 7.22 (d, *J* = 7.6 Hz, 1H), 7.17 (d, *J* = 7.6 Hz, 2H), 7.10 (d, *J* = 8.4 Hz, 1H), 5.77-5.72 (m, 1H), 4.18 (q,*J* = 6.8 Hz, 1H), 3.95-3.84 (m, 1H), 3.29 (s,3H), 2.86-2.74 (m, 1H), 2.65-2.57 (m, 1H), 2.04-1.89 (m, 2H), 1.75-1.52 (m, 4H), 1.51-1.37 (m, 2H), 1.34-1.26 (m, 2H), 1.16 (d, *J* = 6.8 Hz, 3H). |
| | | | **A2-P1-2:** LCMS: m/z 405.1 (M+H); ¹H NMR (CDCl3, 400 MHz) 7.74 (d, *J* = 8.4 Hz, 1H), 7.23-7.25 (m, 2H), 7.20 (t,*J* = 8.0 Hz, 3H), 7.09 (d, *J* = 8.4 Hz, 1H), 5.70 (dd,*J* = 7.6 Hz, 2.8 Hz, 1H), 4.15 (q,*J* = 6.8 Hz, 1H), 3.88-3.80 (m, 1H), 3.29 (s,3H), 2.83-2.74 (m, 1H), 2.68-2.57 (m, 2H), 2.05-1.90 (m, 2H), 1.70-1.57 (m, 2H), 1.53-1.40 (m, 3H), 1.37-1.22 (m, 2H), 0.93 (d, *J* = 6.8 Hz, 3H). |
| | | | **A2-P2-2:** LCMS:*m*/*z* 405.0 (M+H); ¹H NMR (CDCl₃, 400mHz) 7.78 (d, *J* = 8.0 Hz, 1H), 7.30-7.25 (m, 2H), 7.23 (d, *J* = 7.2 Hz, 1H), 7.17 (d, *J* = 7.2 Hz, 2H), 7.11 (d, *J* = 8.4 Hz, 1H), 5.82-5.75 (m, 1H), 4.22 (q, *J* = 6.8 Hz, 1H), 3.95-3.86 (m, 1H), 3.30 (s, 3H), 2.89-2.77 (m, 1H), 2.73-2.60 (m, 2H), 2.06-1.90 (m, 2H), 1.75-1.51 (m, 4H), 1.48-1.40 (m, 2H), 1.35-1.24 (m, 2H), 1.17 (d, *J* = 6.8 Hz, 3H). |
| A3 | 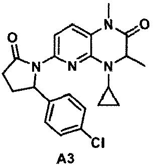 | 4-cyclopropyl-6-(2-(4-chlorophenyl)-5-oxopyrrolidin -1-yl)-1,3-dimethyl-3,4-dihydropyrido[2,3-b]pyrazin-2(1 H)-one | **A3-P1:** LCMS: *m*/*z* 410.8 (M+H); ¹H NMR (CD₃OD, 400 MHz) δ 7.70 (d, *J* = 8.4 Hz, 1H), 7.24-7.32 (m, 5H), 5.81 (dd, *J* = 8.0, 4.2 Hz, 1H), 4.06 (q, *J* = 6.8 Hz, 1H), 3.28 (s, 3H), 2.89-2.75 (m, 1H), 2.74-2.59 (m, 2H), 2.11-2.09 (m, 1H), 2.03-1.93 (m, 1H), 0.98 (d, *J =* 6.8 Hz, 4H), 0.69 (d, *J* = 4.4 Hz, 1H), 0.54-0.43 (m, 2H). |
| | | | **A3-P2:** LCMS: *m*/*z* 410.8 (M+H); ¹H NMR (CD₃OD, 400 MHz) δ 7.70 (d, *J* = 8.4 Hz, 1H), 7.24-7.32 (m, 5H), 5.81 (dd, *J* = 8.0, 4.2 Hz, 1H), 4.06 (q, *J* = 6.8 Hz, 1H), 3.28 (s, 3H), 2.88-2.74 (m, 1H), 2.73-2.61 (m, 2H), 2.16-2.06 (m, 1H), 2.05-1.93 (m, 1H), 1.00-0.97 (m, 4H), 0.75-0.65 (m, 1H), 0.55-0.42 (m, 2H). |
| | | | **A3-P3:** LCMS: *m*/*z* 410.8 (M+H); ¹H NMR (CD₃OD, 400 MHz) δ 7.72 (d, *J* = 8.4 Hz, 1H), 7.35 (d, *J* = 8.4 Hz, 1H), 7.30 (d, *J* = 8.4 Hz, 2H), 7.20 (d, *J* = 8.4 Hz, 2H), 5.96 (dd, *J* = 8.0, 3.0 Hz, 1H), 4.10 (q, *J* = 6.8 Hz, 1H), 3.29 (s, 3H), 2.80-2.56 (m, 3H), 2.54-2.48 (m, 1H), 2.04-1.92 (m, 1H), 1.20 (d, *J* = 6.8 Hz, 3H), 0.65-0.59 (m, 1H), 0.55-0.44 (m, 1H), 0.41-0.35 (m, 1H), -0.17- -0.20 (m, 1H). |
| | | | **A3-P4:** LCMS: 410.8 (M+H); ¹H NMR (CD₃OD, 400 MHz) δ 7.72 (d, *J* = 8.4 Hz, 1H), 7.35 (d, *J* = 8.4 Hz, 1H), 7.30 (d, *J* = 8.4 Hz, 2H), 7.20 (d, *J* = 8.4 Hz, 2H), 5.96 (dd,*J* = 8.0,3.0 Hz, 1H), 4.10 (q, *J* = 6.8 Hz, 1H), 3.29 (s,3H), 2.82-2.56 (m, 3H), 2.54-2.48 (m, 1H), 2.06-1.92 (m, 1H), 1.20 (d, *J* = 6.8 Hz, 3H), 0.68-0.56 (m, 1H), 0.53-0.46 (m, 1H), 0.41-0.35 (m, 1H), -0.17- -0.23 (m, 1H). |
| A4 | 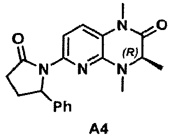 | (3*R*)-1,3,4-trimethyl-6-(2-oxo-5-phenylpyrrolidin-1-yl)-3,4dihydropyrido[2,3-b]pyrazin-2(1H)-one | **A4-P1**: LCMS:*m*/*z* 351.0 (M+H); ¹H NMR (CDCl₃, 400mHz) 7.68 (d, *J* = 8.4 Hz, 1H), 7.32-7.26 (m, 2H), 7.25-7.18 (m, 3H), 7.04 (d, *J* = 8.4 Hz, 1H), 5.77-5.70 (m, 1H), 4.00 (q, *J* = 6.8 Hz, 1H), 3.29 (s, 3H), 2.88-2.77 (m, 1H), 2.68-2.55 (m, 2H), 2.63 (s, 3H), 2.05-1.95 (m, 1H), 1.05 (d, *J* = 6.8 Hz, 3H). |
| | | | **A4-P2:** LCMS:*m*/*z* 351.0 (M+H); ¹H NMR (CDCl₃, 400mHz) 7.74 (d, *J* = 8.4 Hz, 1H), 7.33-7.25 (m, 2H), 7.24-7.18 (m, 3H), 7.05 (d, *J* = 8.4 Hz, 1H), 5.83-5.76 (m, 1H), 4.00 (q, *J* = 6.8 Hz, 1H), 3.28 (s, 3H), 2.89-2.77 (m, 1H), 2.68-2.57 (m, 2H), 2.66 (s, 3H), 2.04-1.95 (m, 1H), 1.21 (d, *J =* 6.8 Hz, 3H). |
| A5 | 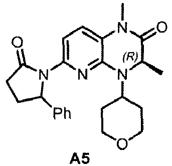 | (3*R*)-1,3-dimethyl-6-(2-oxo-5-phenylpyrrol-1 -yl)-4-(tetrahydro-2*H*-pyran-4-yl)-3,4-dihydropyrido[2,3-b]pyrazin-2(1H)-one | **A5-P1**: LCMS:*m*/*z* 421.1 (M+H); ¹H NMR (CD₃OD, 400mHz) 7.78 (d, *J* = 8.4 Hz, 1H), 7.36 (d, *J* = 12.8 Hz, 1H), 7.33 (t, *J* = 7.2 Hz, 2H), 7.22 (t, *J* = 8.4 Hz, 3H), 5.80 (dd, *J* = 7.6 Hz, 3.2 Hz, 1H), 4.20 (q, *J* = 6.8 Hz, 1H), 4.00-3.96 (m, 1H), 3.91-3.82 (m, 2H), 3.45 (td, *J* = 12.0 Hz, 2.4 Hz, 1H), 3.30 (s, 3H), 3.22 (td*, J* = 12.0 Hz, 1.6 Hz, 1H), 2.78-2.58 (m, 3H), 2.03-1.95 (m, 1H), 1.84-1.66 (m, 3H), 1.14(d, *J* = 6.8 Hz, 3H). |
| | | | **A5-P2:** LCMS:*m*/*z* 420.9 (M+H); ¹H NMR (CD₃OD, 400mHz) 7.78 (d, *J* = 8.4 Hz, 1H), 7.37 (d, *J* = 8.8 Hz, 1H), 7.30 (t, *J* = 8.4 Hz, 2H), 7.21 (t, *J* = 6.4 Hz, 3H), 5.82 (dd, *J* = 7.6 Hz, 2.8 Hz, 1H), 4.18 (q, *J* = 6.4 Hz, 1H), 3.99 (td, *J* = 11.6 Hz, 4.4 Hz, 1H), 3.82 (td, *J* = 12.4 Hz, 4.8 Hz, 1H), 3.52 (td, *J* = 12.0 Hz, 2.0 Hz, 1H), 3.36 (m, 1H), 3.31 (s, 3H), 2.76-2.56 (m, 3H), 2.03-1.88 (m, 3H), 1.57-1.49 (m, 2H), 0.96 (d, *J* = 6.8 Hz, 3H). |
| A6 | 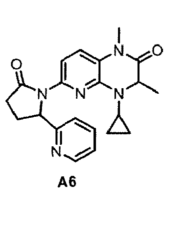 | 4-cyclopropyl-1,3-dimethyl-6-(2-oxo-5-pyridin-2-yl)pyrrolidin-1-yl)-3,4-dihydropyrido[2,3-b]pyrazin-2(1 H)-one | **A6-P1:** LCMS:*m*/*z* 377.9 (M+H); ¹H NMR (CD₃OD, 400mHz) 8.54 (d, *J* = 4.4 Hz, 1H), 7.89 (d, *J* = 8.4 Hz, 1H), 7.73 (t, *J* = 7.6 Hz, 1H), 7.37 (d, *J* = 8.8 Hz, 1H), 7.30 (t, *J* = 6.8 Hz, 1H), 7.15 (d, *J* = 8.0 Hz, 1H), 6.01 (dd, *J* = 7.6 Hz, 1.6 Hz, 1H), 4.08 (q, *J* = 6.8 Hz, 1H), 3.30 (s, 3H), 2.79-2.56 (m, 3H), 2.51-2.45 (m, 1H), 2.15-2.07 (m, 1H), 1.19(d, *J* = 6.8 Hz, 3H), 0.61-0.56 (m, 1H), 0.37-0.26 (m, 2H), -0.39- -0.42 (m, 1H). |
| | | | **A6-P2:** LCMS:*m*/*z* 377.9 (M+H); ¹H NMR (CD₃OD, 400mHz) 8.45 (d, *J* = 4.8 Hz, 1H), 7.83 (d, *J* = 8.4 Hz, 1H), 7.73 (t, *J* = 8.0 Hz, 1H), 7.33 (d, *J* = 8.4 Hz, 1H), 7.32 (d, *J* = 8.0 Hz, 1H), 7.26 (dd, *J* = 7.2 Hz, 4.8 Hz, 1H), 5.86 (dd, *J* = 8.0 Hz, 3.6 Hz, 1H), 4.04 (q, *J* = 7.2 Hz, 1H), 3.28 (s, 3H), 2.90-2.80 (m, 1H), 2.73-2.63 (m, 2H), 2.11-2.05 (m, 1H), 1.93-1.88 (m, 1H), 1.02-0.99 (m, 1H), 0.95(d, *J* = 6.8 Hz, 3H), 0.67-0.61 (m, 1H), 0.50-0.44 (m, 2H). |
| | | | **A6-P3:** LCMS:*m*/*z* 378.0 (M+H); ¹H NMR (CD₃OD, 400mHz) 8.45 (d, *J* = 4.8 Hz, 1H), 7.83 (d, *J* = 8.4 Hz, 1H), 7.73 (t, *J* = 8.0 Hz, 1H), 7.33 (d, *J* = 8.4 Hz, 1H), 7.32 (d, *J* = 8.0 Hz, 1H), 7.26 (dd, *J* = 7.2 Hz, 4.8 Hz, 1H), 5.86 (dd, *J* = 8.0 Hz, 3.6 Hz, 1H), 4.04 (q, *J* = 7.2 Hz, 1H), 3.28 (s, 3H), 2.90-2.80 (m, 1H), 2.73-2.63 (m, 2H), 2.11-2.05 (m, 1H), 1.93-1.88 (m, 1H), 1.02-0.99 (m, 1H), 0.95(d, *J* = 6.8 Hz, 3H), 0.67-0.61 (m, 1H), 0.50-0.44 (m, 2H). |
| | | | **A6-P4:** LCMS:*m*/*z* 377.9 (M+H); ¹H NMR (CD₃OD, 400mHz) 8.54 (d, *J* = 4.4 Hz, 1H), 7.89 (d, *J* = 8.4 Hz, 1H), 7.73 (t, *J* = 7.6 Hz, 1H), 7.37 (d, *J* = 8.8 Hz, 1H), 7.30 (t, *J* = 6.8 Hz, 1H), 7.15 (d, *J* = 8.0 Hz, 1H), 6.01 (dd, *J* = 7.6 Hz, 1.6 Hz, 1H), 4.08 (q, *J* = 6.8 Hz, 1H), 3.30 (s, 3H), 2.79-2.56 (m, 3H), 2.51-2.45 (m, 1H), 2.15-2.07 (m, 1H), 1.19(d,*J* = 6.8 Hz, 3H), 0.61-0.56 (m, 1H), 0.37-0.26 (m, 2H), -0.39-0.42 (m, 1H). |
| A7 | 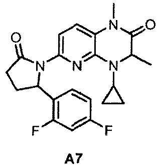 | 4-cyclopropyl-6-(2-(2,4-difluorophenyl)-5-oxopyrrolidin -1-yl)-1,3-dimethyl-3,4-dihydropyrido[2,3-b]pyrazin-2(1 H)-one | **A7-P1**: LCMS:*m*/*z* 412.9 (M+H); ¹H NMR (CD₃OD, 400mHz) 7.76 (d, *J =* 8.8 Hz, 1H), 7.33 (d, *J =* 8.4 Hz, 1H), 7.15 (td, *J =* 8.8 Hz, 6.4 Hz, 1H), 6.96 (m, 1H), 6.85 (td, *J* = 8.4 Hz, 2.4 Hz, 1H), 6.05 (dd, *J =* 8.0 Hz, 3.2 Hz, 1H), 4.07 (q, *J* = 7.2 Hz, 1H), 3.29 (s, 3H), 2.84-2.66 (m, 3H), 2.10-1.99 (m, 2H), 2.15-2.07 (m, 1H), 0.99 (d, *J* = 6.8 Hz, 3H), 0.96-0.93 (m, 1H), 0.71-0.66 (m, 1H), 0.49 (bs, 2H). |
| | | | **A7-P2:** LCMS:*m*/*z* 413.0 (M+H); ¹H NMR (CD₃OD, 400mHz) 7.76 (d, *J* = 8.8 Hz, 1H), 7.33 (d, *J =* 8.4 Hz, 1H), 7.15 (td, *J =* 8.8 Hz, 6.4 Hz, 1H), 6.96 (m, 1H), 6.85 (td, *J* = 8.4 Hz, 2.4 Hz, 1H), 6.05 (dd, *J=* 8.0 Hz, 3.2 Hz, 1H), 4.07 (q, *J* = 7.2 Hz, 1H), 3.29 (s, 3H), 2.84-2.66 (m, 3H), 2.10-1.99 (m, 2H), 2.15-2.07 (m, 1H), 0.99 (d, *J =* 6.8 Hz, 3H), 0.96-0.93 (m, 1H), 0.71-0.66 (m, 1H), 0.49 (bs, 2H). |
| | | | **A7-P3:** LCMS:*m*/*z* 412.9 (M+H); ¹H NMR (CD₃OD, 400mHz) 7.82 (d, *J =* 8.4 Hz, 1H), 7.37 (d, *J =* 8.4 Hz, 1H), 7.05-6.97 (m, 2H), 6.83 (td, *J=* 8.4 Hz, 2.0 Hz, 1H), 6.19(d, *J* = 6.0 Hz, 1H), 4.11 (q, *J=* 6.8 Hz, 1H), 3.30 (s, 3H), 2.79-2.50 (m, 4H), 2.05-1.98 (m, 1H), 2.11-2.05 (m, 1H), 1.93-1.88 (m, 1H), 1.21(d, *J* = 6.8 Hz, 3H), 0.67-0.60 (m, 1H), 0.55-0.48 (m, 1H), 0.42-0.35 (m, 1H), -0.20-0.26 (m, 1H). |
| | | | **A7-P4:** LCMS:*m*/*z* 412.9 (M+H); ¹H NMR (CD₃OD, 400mHz) 7.82 (d, *J =* 8.4 Hz, 1H), 7.37 (d, *J =* 8.4 Hz, 1H), 7.05-6.97 (m, 2H), 6.83 (td, *J* = 8.4 Hz, 2.0 Hz, 1H), 6.19(d, *J* = 6.0 Hz, 1H), 4.11 (q, *J* = 6.8 Hz, 1H), 3.30 (s, 3H), 2.79-2.50 (m, 4H), 2.05-1.98 (m, 1H), 2.11-2.05 (m, 1H), 1.93-1.88 (m, 1H), 1.21(d, *J* = 6.8 Hz, 3H), 0.67-0.60 (m, 1H), 0.55-0.48 (m, 1H), 0.42-0.35 (m, 1H), -0.20--0.26 (m, 1H). |
| A8 | 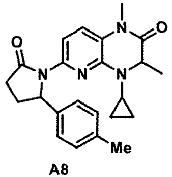 | 4-cyclopropyl-6-(2-(4-methylphenyl)-5-oxopyrrolidin-1-yl)-1,3-dimethyl-3,4-dihydropyrido[2,3-b]pyrazin-2(1 H)-one | **A8-P1:** LCMS:*m*/*z* 390.9 (M+H); ¹H NMR (CD₃OD, 400mHz)δ 7.67 (d, *J =* 8.4 Hz, 1H), 7.30 (d, *J =* 8.4 Hz, 1H), 7.10 (dd, *J =* 18.6, 8.0 Hz, 4H), 5.81 (dd, *J =* 8.0, 4.2 Hz, 1H), 4.05 (q, *J =* 6.8 Hz, 1H), 3.28 (s, 3H), 2.80 (dt, *J =* 11.8, 9.0 Hz, 1H), 2.70-2.58 (m, 2H), 2.27 (s, 3H), 2.14-2.19 (m, 1H), 2.04-1.94 (m, 1H), 1.05-0.92 (m, 4H), 0.66-0.7 (m, 1H), 0.49 (s, 2H). |
| | | | **A8-P2:** LCMS:*m*/*z* 390.9 (M+H); ¹H NMR (CD₃OD, 400mHz) δ 7.67 (d, *J* = 8.4 Hz, 1H), 7.30 (d, *J* = 8.4 Hz, 1H), 7.10 (dd, *J* = 18.6, 8.0 Hz, 4H), 5.81 (dd, *J* = 8.0, 4.2 Hz, 1H), 4.05 (q, *J* = 6.8 Hz, 1H), 3.28 (s, 3H), 2.87-2.75 (m, 1H), 2.70-2.57 (m, 2H), 2.27 (s, 3H), 2.20-2.13 (m, 1H), 2.03-1.92 (m, 1H), 1.05-0.90 (m, 4H), 0.72-0.62 (m, 1H), 0.49 (s, 2H). |
| | | | **A8-P3:** LCMS:*m*/*z* 390.9 (M+H); ¹H NMR (CD₃OD, 400mHz)δ 7.67 (d, *J =* 8.4 Hz, 1H), 7.32 (t, *J* = 7.2 Hz, 1H), 7.09 (q, *J =* 8.4 Hz, 4H), 5.93 (dd, *J* = 8.0, 2.8 Hz, 1H), 4.10 (q, *J* = 6.8 Hz, 1H), 3.28 (s, 3H), 2.76-2.49 (m, 4H), 2.29 (s, 3H), 2.03-1.93 (m, 1H), 1.20 (d, *J* = 6.8 Hz, 3H), 0.68-0.51 (m, 2H), 0.39-0.33 (m, 1H), -0.13-0.19 (m, 1H). |
| | | | **A8-P4:** LCMS:*m*/*z* 390.9 (M+H); ¹H NMR (CD₃OD, 400mHz) δ 7.67 (d, *J* = 8.4 Hz, 1H), 7.33 (d, *J* = 8.4 Hz, 1H), 7.09 (q, *J* = 8.4 Hz, 4H), 5.93 (dd, *J* = 8.0, 2.8 Hz, 1H), 4.10 (q, *J* = 6.8 Hz, 1H), 3.28 (s, 3H), 2.78-2.49 (m, 4H), 2.29 (s, 3H), 2.01-1.94 (m, 1H), 1.20 (d, *J* = 6.8 Hz, 3H), 0.71-0.50 (m, 2H), 0.41-0.31 (m, 1H), -0.13-0.19 (m, 1H). |
| A9 | 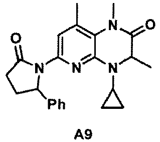 | 4-cyclopropyl-1,3,8-trimethyl-6-(2-oxo-5-phenylpyrrolidin-1-yl)-3,4-dihydropyrido[2,3-b]pyrazin-2(1 H)-one | **A9-P1-1:** LCMS:*m*/*z* 391.1 (M+H); ¹H NMR (CDCl₃, 400mHz) 7.69 (s, 1H), 7.30 - 7.18 (m, 3H), 7.14 (d, *J* = 7.6 Hz, 2H), 5.94 (d, *J* = 7.2 Hz, 1H), 4.01 (q, *J* = 7.0 Hz, 1H), 3.26 (s, 3H), 2.83 - 2.69 (m, 1H), 2.67 - 2.53 (m, 2H), 2.45 - 2.35 (m, 1H), 2.40 (s, 3H), 2.02 - 1.95 (m, 1H), 1.09 (d, *J* = 7.2 Hz, 3H), 0.58 - 0.50 (m, 1H), 0.47 - 0.39 (m, 1H), 0.37 - 0.28 (m, 1H), - 0.05 - -0.12 (m, 1H). |
| | | | **A9-P1-2:** LCMS:*m*/*z* 391.1 (M+H); ¹H NMR (CDCl₃, 400mHz) 7.72 (s, 1H), 7.32 - 7.20 (m, 3H), 7.16 (d, *J* = 7.4 Hz, 2H), 5.96 (d, *J* = 7.2 Hz, 1H), 4.03 (q, *J* = 7.0 Hz, 1H), 3.28 (s, 3H), 2.84 - 2.73 (m, 1H), 2.70 - 2.56 (m, 2H), 2.46 - 2.36 (m, 1H), 2.42 (s, 3H), 2.03 - 1.97 (m, 1H), 1.11 (d, *J* = 7.0 Hz, 3H), 0.60 - 0.53 (m, 1H), 0.49 - 0.41 (m, 1H), 0.38 - 0.32 (m, 1H), - 0.03 - -0.10 (m, 1H). |
| | | | **A9-P2-1:** LCMS:*m*/*z* 391.0 (M+H); ¹H NMR (CDCl₃, 400mHz) 7.60 (s, 1H), 7.26 - 7.13 (m, 5H), 5.79 - 5.71 (m, 1H), 3.93 (q, *J* = 6.8 Hz, 1H), 3.26 (s, 3H), 2.87 - 2.75 (m, 1H), 2.68 - 2.56 (m, 2H), 2.37 (s, 3H), 2.03 - 1.94 (m, 1H), 1.89 - 1.81 (m, 1H), 0.81 (d, *J* = 6.8 Hz, 3H), 0.81 - 0.76 (m, 1H), 0.58 - 0.47 (m, 2H), 0.47 - 0.37 (m, 1H). |
| | | | **A9-P2-2:** LCMS:*m*/*z* 391.1 (M+H); ¹H NMR (CDCl3, 400mHz) 7.60 (s, 1H), 7.26 - 7.13 (m, 5H), 5.78 - 5.72 (m, 1H), 3.92 (q, *J* = 6.8 Hz, 1H), 3.26 (s, 3H), 2.87 - 2.76 (m, 1H), 2.68 - 2.55 (m, 2H), 2.37 (s, 3H), 2.05 - 1.95 (m, 1H), 1.88 - 1.82 (m, 1H), 0.81 (d, *J* = 6.8 Hz, 3H), 0.80 - 0.77 (m, 1H), 0.59 - 0.47 (m, 2H), 0.46 - 0.38 (m, 1H). |
| A10 | 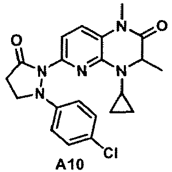 | 6-(2-(4-chlorophenyl)-5-oxopyrazolidin-1-yl)-4-cyclopropyl-1,3-dimethyl-3,4-dihydropyrido[2,3-b]pyrazin-2(1 H)-one | **A10-P1:** LCMS:*m*/*z* 411.7 (M+H); ¹H NMR (DMSO-*d₆*, 400mHz): δ7.66 (d, *J* = 8.4 Hz, 1H), 7.38 (d, *J* = 8.4 Hz, 1H), 7.31 (d, *J* = 8.8 Hz, 2H), 6.90 (d, *J* = 8.8 Hz, 2H), 4.10 - 3.96 (m, 2H), 3.93 - 3.86 (m, 1H), 3.20 (s, 3H), 2.87 - 2.77 (m, 1H), 2.60 - 2.54 (m, 1H), 2.25 - 2.18 (m, 1H), 1.09 (d, *J* = 6.8 Hz, 3H), 0.47 - 0.37 (m, 1H), 0.36 - 0.27 (m, 1H), -0.04 - -0.14 (m, 1H), -0.23 - -0.32 (m, 1H). |
| | | | **A10-P2:** LCMS:m/*z* 411.7 (M+H); ¹H NMR (DMSO-*d₆*, 400mHz): δ7.66 (d, *J* = 8.4 Hz, 1H), 7.38 (d, *J* = 8.4 Hz, 1H), 7.31 (d, *J* = 8.8 Hz, 2H), 6.90 (d, *J* = 8.8 Hz, 2H), 4.10 - 3.96 (m, 2H), 3.93 - 3.86 (m, 1H), 3.20 (s, 3H), 2.87 - 2.77 (m, 1H), 2.60 - 2.54 (m, 1H), 2.25 - 2.18 (m, 1H), 1.09 (d, *J* = 6.8 Hz, 3H), 0.47 - 0.37 (m, 1H), 0.36 - 0.27 (m, 1H), -0.04 - -0.14 (m, 1H), -0.23 - -0.32 (m, 1H). |
| A11 | 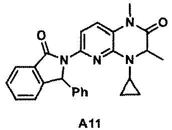 | 4-cyclopropyl-1,3-dimethyl-6-(1-oxo-3-phenylisoindolin-2-yl)-3,4-dihydropyrido[2,3-b]pyrazin-2(1 H)-one | **A11-P1:** LCMS:*m*/*z* 424.9 (M+H); ¹H NMR (CD₃OD, 400mHz) 7.93 (d, *J* = 7.2 Hz, 1H), 7.92 (d, *J* = 8.4 Hz, 1H), 7.63 (t, *J* = 7.2 Hz, 1H), 7.56 (t, *J* = 6.8 Hz, 1H), 7.40 (d, *J* = 7.6 Hz, 1H), 7.36 (d, *J* = 8.4 Hz, 1H), 7.30-7.19 (m, 5H), 6.68 (s, 1H), 4.10 (q, *J* = 6.8 Hz, 1H), 3.30 (s, 3H), 2.35 (m, 1H), 1.24-1.18 (m, 1H), 0.98 (d, *J* = 6.8 Hz, 3H), 0.81-0.74 (m, 1H), 0.63-0.53 (m, 2H). |
| | | | **A11-P2:** LCMS:*m*/*z* 424.9 (M+H); ¹H NMR (CD₃OD, 400mHz) 7.93 (d, *J* = 7.2 Hz, 1H), 7.92 (d, *J* = 8.4 Hz, 1H), 7.63 (t, *J* = 7.2 Hz, 1H), 7.56 (t, *J* = 6.8 Hz, 1H), 7.40 (d, *J* = 7.6 Hz, 1H), 7.36 (d, *J* = 8.4 Hz, 1H), 7.30-7.19 (m, 5H), 6.68 (s, 1H), 4.10 (q, *J* = 6.8 Hz, 1H), 3.30 (s, 3H), 2.35 (m, 1H), 1.24-1.18 (m, 1H), 0.98 (d, *J* = 6.8 Hz, 3H), 0.81-0.74 (m, 1H), 0.63-0.53 (m, 2H). |
| | | | **A11-P3:** LCMS:*m*/*z* 424.9 (M+H); ¹H NMR (CD₃OD, 400mHz) 7.91 (d, *J* = 7.6 Hz, 1H), 7.80 (d, *J* = 8.4 Hz, 1H), 7.62 (t, *J* = 7.2 Hz, 1H), 7.55 (t, *J* = 7.2 Hz, 1H), 7.46 (d, *J* = 7.6 Hz, 1H), 7.40 (d, *J* = 8.4 Hz, 1H), 7.33-7.20 (m, 5H), 6.80 (s, 1H), 4.14 (q, *J* = 6.8 Hz, 1H), 3.30 (s, 3H), 2.66 (m, 1H), 1.23 (d, *J* = 6.8 Hz, 1H), 0.95-0.88 (m, 1H), 0.75-0.68 (m, 1H), 0.48-0.42 (m, 1H), 0.05-0.01 (m, 1H). |
| | | | **A11-P4:** LCMS:*m*/*z* 424.9 (M+H); ¹H NMR (CD₃OD, 400mHz) 7.91 (d, *J* = 7.6 Hz, 1H), 7.80 (d, *J* = 8.4 Hz, 1H), 7.62 (t, *J* = 7.2 Hz, 1H), 7.55 (t, *J* = 7.2 Hz, 1H), 7.46 (d, *J* = 7.6 Hz, 1H), 7.40 (d, *J* = 8.4 Hz, 1H), 7.33-7.20 (m, 5H), 6.80 (s, 1H), 4.14 (q, *J* = 6.8 Hz, 1H), 3.30 (s, 3H), 2.66 (m, 1H), 1.23 (d, *J* = 6.8 Hz, 1H), 0.95-0.88 (m, 1H), 0.75-0.68 (m, 1H), 0.48-0.42 (m, 1H), 0.05-0.01 (m, 1H). |
| A12 | 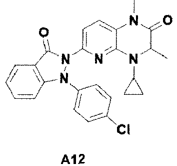 | 6-(1-(4-chlorophenyl)-3-oxo-1,3-dihydro-2*H-*indazol-2-yl)-4-cyclopropyl-1,3-dimethyl-3,4-dihydropyrido[2,3-b]pyrazin-2(1H) -one | **A12-P1:** LCMS:m/z 459.6 (M+H); ¹H NMR (DMSO-*d₆*, 400mHz): δ7.89 (d, *J* = 7.6 Hz, 1H), 7.67 (t, *J* = 7.8 Hz, 1H), 7.53 - 7.41 (m, 6H), 7.41 - 7.30 (m, 2H), 4.05 - 4.00 (m, 1H), 3.21 (s, 3H), 2.32- 2.23 (m, 1H), 1.04 (d, *J* = 6.8 Hz, 3H), 0.83 - 0.71 (m, 1H), 0.64 - 0.53 (m, 1H), 0.49 - 0.41 (m, 1H), -0.04 - -0.13(m, 1H). |
| | | | **A12-P2:** LCMS:m/*z* 460.0 (M+H); ¹H NMR (DMSO-*d₆*, 400mHz): δ7.89 (d, *J* = 7.6 Hz, 1H), 7.67 (t, *J* = 7.8 Hz, 1H), 7.53 - 7.41 (m, 6H), 7.41 - 7.30 (m, 2H), 4.05 - 4.00 (m, 1H), 3.21 (s, 3H), 2.32 - 2.23 (m, 1H), 1.04 (d, *J* = 6.8 Hz, 3H), 0.83 - 0.71(m, 1H), 0.64 - 0.53 (m, 1H), 0.49 - 0.41(m, 1H), -0.04 - -0.13(m, 1H). |
| A13 | 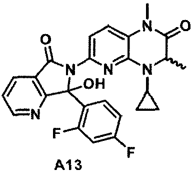 | 4-cyclopropyl-6-(7-(2,4-difluorophenyl)-7-hydroxy-5-oxo-5,7-dihydro-6*H-*pyrrolo[3,4-b]pyridin-6-yl)-1,3-dimethyl-3,4-dihydropyrido[2,3-b]pyrazin-2(1H)-one | **A13-P1:** LCMS: *m*/*z* 478.0 (M+H); ¹H NMR (DMSO-*d*₆, 400 MHz) 8.79 - 8.75 (m, 1H), 8.36 - 8.28 (m, 1H), 8.15 - 7.99 (m, 1H), 7.82 (s, 1H), 7.68 - 7.62 (m, 1H), 7.45 - 7.36 (m, 1H), 7.22 (d*, J* = 8.4 Hz, 1H), 7.18 - 7.11 (m, 1H), 7.08 - 6.97 (m, 1H), 4.11 - 4.03 (m, 1H), 3.20 (d, *J* = 2.0 Hz, 3H), 2.39 - 2.27 (m, 1H), 1.04 (dd, *J* = 50.6, 6.8 Hz, 3H), 0.93 - 0.52 (m, 3H), 0.38 - 0.09 (m, 1H). |
| | | | **A13-P2:** LCMS: *m*/*z* 478.0 (M+H); ¹H NMR (CDCl₃, 400 MHz) 8.77 (d, *J* = 3.0 Hz, 1H), 8.35-8.27 (m, 1H), 8.15 - 8.00 (m, 1H), 7.82 (s, 1H), 7.69 - 7.62 (m, 1H), 7.45 - 7.38 (m, 1.5H), 7.22 (d, *J* = 8.4 Hz, 0.5H), 7.14 (t, *J* = 7.6 Hz, 1H), 7.08 - 6.97 (m, 1H), 4.10 - 4.03 (m, 1H), 3.20 (d, *J* = 1.6 Hz, 3H), 2.40 - 2.27 (m, 1H), 1.11 (d, *J* = 6.8 Hz, 1.5H), 0.98 (d*, J* = 6.8 Hz, 1.5H), 0.93 - 0.52 (m, 3H), 0.38 - 0.10 (m, 1H). |
| A14 | 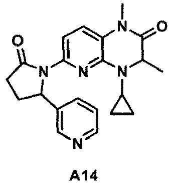 | 4-cyclopropyl-1,3-dimethyl-6-(2-oxo-5-(pyridin-3-yl)pyrrolidin-1-yl)-3,4-dihydropyrido[2,3-b]pyrazin-2(1 H)-one | **A14-P1:** LCMS: *m*/*z* 377.9 (M+H); ¹H NMR (CD₃OD, 400 MHz) 8.48 (s, 1H), 8.42 (d, *J* = 4.4 Hz, 1H), 7.76 (d, *J* = 8.8 Hz, 1H), 7.67 (d, *J* = 8.0 Hz, 1H), 7.39 (t, *J* = 4.8 Hz, 1H), 7.37 (d, *J* = 8.8 Hz, 1H), 6.03 (dd, *J* = 7.6 Hz, 3.2 Hz, 1H), 4.08 (q, *J* = 6.8 Hz, 1H), 3.29 (s, 3H), 2.82-2.64 (m, 3H), 2.55-2.50 (m, 1H), 2.05-1.99 (m, 1H), 1.19(d, *J* = 6.8 Hz, 3H), 0.64-0.58 (m, 1H), 0.42-0.35 (m, 2H), -0.27-0.33 (m, 1H). |
| | | | **A14-P2:** LCMS: *m*/*z* 377.9 (M+H); ¹H NMR (CD₃OD, 400 MHz) 8.48 (s, 1H), 8.42 (d, *J* = 4.4 Hz, 1H), 7.76 (d, *J* = 8.8 Hz, 1H), 7.67 (d, *J* = 8.0 Hz, 1H), 7.39 (t, *J* = 4.8 Hz, 1H), 7.37 (d, *J* = 8.8 Hz, 1H), 6.03 (dd, *J* = 7.6 Hz, 3.2 Hz, 1H), 4.08 (q, *J* = 6.8 Hz, 1H), 3.29 (s, 3H), 2.82-2.64 (m, 3H), 2.55-2.50 (m, 1H), 2.05-1.99 (m, 1H), 1.19(d, *J* = 6.8 Hz, 3H), 0.64-0.58 (m, 1H), 0.42-0.35 (m, 2H), -0.27-0.33 (m, 1H). |
| | | | **A14-P3:** LCMS: *m*/*z* 377.9 (M+H); ¹H NMR (CD₃OD, 400 MHz) 8.55 (s, 1H), 8.41 (d, *J* = 4.4 Hz, 1H), 7.78 (d, *J* = 7.2 Hz, 1H), 7.72 (d, *J* = 8.4 Hz, 1H), 7.39 (t, *J* = 7.6 Hz, 1H), 7.32 (d, *J* = 8.4 Hz, 1H), 5.85 (dd, *J* = 8.0 Hz, 4.4 Hz, 1H), 4.05 (q, *J* = 6.8 Hz, 1H), 3.28 (s, 3H), 2.91-2.68 (m, 3H), 2.09-2.01 (m, 2H), 1.04-0.99 (m, 1H), 0.95 (d, *J* = 7.2 Hz, 3H), 0.71-0.66 (m, 1H), 0.42-0.35 (m, 2H), 0.55-0.47 (m, 2H). |
| | | | **A14-P4:** LCMS: *m*/*z* 378.1 (M+H); ¹H NMR (CD₃OD, 400 MHz) ¹H NMR (CD₃OD, 400 MHz) 8.55 (s, 1H), 8.41 (d, *J* = 4.4 Hz, 1H), 7.78 (d*, J* = 7.2 Hz, 1H), 7.72 (d, *J* = 8.4 Hz, 1H), 7.39 (t, *J* = 7.6 Hz, 1H), 7.32 (d, *J* = 8.4 Hz, 1H), 5.85 (dd, *J* = 8.0 Hz, 4.4 Hz, 1H), 4.05 (q, *J* = 6.8 Hz, 1H), 3.28 (s, 3H), 2.91-2.68 (m, 3H), 2.09-2.01 (m, 2H), 1.04-0.99 (m, 1H), 0.95 (d, *J* = 7.2 Hz, 3H), 0.71-0.66 (m, 1H), 0.42-0.35 (m, 2H), 0.55-0.47 (m, 2H). |
| A15 | 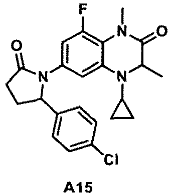 | 6-(2-(4-chlorophenyl)-5-oxopyrrolidin-1-yl)-4-cyclopropyl-8-fluoro-1,3-dimethyl-3,4-dihydroquinoxalin-2(1H)-one | **A15-P1-1:** LCMS: *m*/*z* 428.0 (M+H); ¹H NMR (CDCl₃, 300 MHz) 7.32 (d, *J* = 7.8 Hz, 2H), 7.23 - 7.07 (m, 3H), 6.66 (d, *J* = 14.4 Hz, 1H), 5.19 - 5.11 (m, 1H), 4.01 (q, *J* = 6.6Hz, 1H), 3.34 (d, *J* = 6.0 Hz, 3H), 2.79 - 2.56 (m, 3H), 2.23 - 2.12 (m, 1H), 2.04 - 1.92 (m, 1H), 1.05 (d, *J* = 6.6 Hz, 3H), 0.92 - 0.67 (m, 2H), 0.64 - 0.42 (m, 2H). |
| | | | **A15-P1-2:** LCMS: *m*/*z* 428.0 (M+H); ¹H NMR (CDCl₃, 300 MHz) 7.32 (d, *J* = 8.1 Hz, 2H), 7.22 - 7.06 (m, 3H), 6.66 (d, *J* = 13.0 Hz, 1H), 5.20 - 5.10 (m, 1H), 4.00 (q, *J* = 6.8 Hz, 1H), 3.34 (d, *J* = 6.2 Hz, 3H), 2.11-2.51 (m, 3H), 2.23 - 2.13 (m, 1H), 2.04 - 1.93 (m, 1H), 1.05 (d, *J* = 6.6 Hz, 3H), 0.93 - 0.69 (m, 2H), 0.64 - 0.42 (m, 2H). |
| | | | **A15-P2-1:** LCMS: *m*/*z* 428.0 (M+H); ¹H NMR (CDCl₃, 300 MHz) 7.32 (d, *J* = 8.0 Hz, 2H), 7.16 (d, *J* = 8.0 Hz, 2H), 7.09 (d, *J* = 15.6 Hz, 1H), 6.76 (s, 1H), 5.27 - 5.16 (m, 1H), 4.00 (q, *J* = 6.8 Hz, 1H), 3.36 (d, *J* = 6.2 Hz, 3H), 2.76 - 2.53 (m, 3H), 2.27 - 2.16 (m, 1H), 2.03 - 1.92 (m, 1H), 1.08 (d, *J* = 6.7 Hz, 3H), 0.75 - 0.64 (m, 1H), 0.62 - 0.48 (m, 2H), 0.23 - 0.11 (m, 1H). |
| | | | **A15-P2-2:** LCMS: *m*/*z* 427.9 (M+H); ¹H NMR (CDCl₃, 300 MHz) 7.32 (d, *J* = 7.8 Hz, 2H), 7.16 (d, *J* = 7.8 Hz, 2H), 7.09 (d, *J* = 14.7 Hz, 1H), 6.76 (s, 1H), 5.25 - 5.17 (s, 1H), 4.00 (d, *J* = 6.8 Hz, 1H), 3.36 (d, *J* = 6.0 Hz, 3H), 2.76 - 2.55 (m, 3H), 2.26 - 2.17 (m, 1H), 2.02 - 1.91 (m, 1H), 1.08 (d, *J* = 6.6 Hz, 3H), 0.75 - 0.64 (m, 1H), 0.62 - 0.48 (m, 2H), 0.21 - 0.11 (m, 1H). |
| A16 | 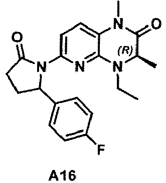 | (3*R*)-4-ethyl-6-(2-(4-fluorophenyl)-5-oxopyrrolidin-1-yl)-1,3-dimethyl-3,4-dihydropyrido[2,3-b]pyrazin-2(1 H)-one | **A16-P1:** LCMS: *m*/*z* 383.0 (M+H); ¹H NMR (CDCl₃, 400 MHz) 7.71 (d, *J* = 8.4 Hz, 1H), 7.17 -7.10 (m, 2H), 7.04 (d, *J* = 8.4 Hz, 1H), 6.96 (t, *J* = 8.6 Hz, 2H), 5.73 (dd, *J* = 7.6, 2.4 Hz, 1H), 4.08 (q, *J* = 6.8 Hz, 1H), 3.63 - 3.52 (m, 1H), 3.27 (s, 3H), 2.85 - 2.71 (m, 2H), 2.67 - 2.52 (m, 2H), 1.98 - 1.86 (m, 1H), 1.21 (d, *J* = 6.8 Hz, 3H), 0.79 (t, *J* = 7.2 Hz, 3H). |
| | | | **A16-P2:** LCMS: *m*/*z* 383.0 (M+H); ¹H NMR (CDCl₃, 400 MHz) 7.68 (d, *J* = 8.4 Hz, 1H), 7.22 - 7.16 (m, 2H), 7.06 (d, *J* = 8.4 Hz, 1H), 6.99 (t, *J* = 8.6 Hz, 2H), 5.68 (dd, *J* = 7.6, 2.8 Hz, 1H), 4.09 (q, *J* = 6.8 Hz, 1H), 3.44 - 3.35 (m, 1H), 3.29 (s, 3H), 2.89 - 2.75 (m, 2H), 2.66 - 2.55 (m, 2H), 2 . 01 - 1.91 (m, 1H), 1.11 (d, *J* = 6.8 Hz, 3H), 1.04 (t, *J* = 7.2 Hz, 3H). |
| A17 | 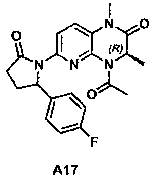 | (3*R*)-4-acetyl-6-(2-(4-fluorophenyl) -5-oxopyrrolidin-1-yl)-1,3-dimethyl-3,4-dihydropyrido[2,3-b]pyrazin-2(1 H)-one | **A17-P1:** LCMS: *m*/*z* 397.0 (M+H); ¹H NMR (CDCl₃, 400 MHz) 8.42 (d, *J* = 8.8 Hz, 1H), 7.41 (d, *J* = 8.8 Hz, 1H), 7.14 - 7.08 (m, 2H), 6.99 (t, *J* = 8.4 Hz, 2H), 5.79 (d, *J* = 6.6 Hz, 1H), 5.43 (q, *J* = 7.2 Hz, 1H), 3.33 (s, 3H), 2.80 - 2.69 (m, 1H), 2.68 - 2.55 (m, 2H), 2.04 - 1.94 (m, 1H), 1.52 (s, 3H), 1.16 (d, *J* = 7.2 Hz, 3H). |
| | | | **A17-P2:** LCMS: *m*/*z* 397.0 (M+H); ¹H NMR (CDCl₃, 400 MHz) 8.13 (d, *J* = 8.8 Hz, 1H), 7.36 (d, *J* = 8.8 Hz, 1H), 7.15 - 7.09 (m, 2H), 6.93 (t, *J* = 8.4 Hz, 2H), 5.58 - 5.52 (m, 1H), 5.38 (q, *J* = 7.2 Hz, 1H), 3.31 (s, 3H), 2.92 - 2.81 (m, 1H), 2.79 - 2.60 (m, 2H), 2.38 (s, 3H), 2.11 - 2.00 (m, 1H), 0.89 (d, *J* = 7.2 Hz, 3H). |

### Example A18: Synthesis of 4-cyclopropyl-1,3-dimethyl-6-(2-oxo-5-phenylimidazolin-1-yl)-3,4-dihydropyrido[2,3-b]pyrazin-2(1H)-one (A18)

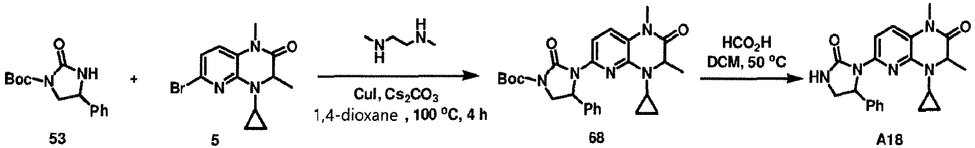

### Step 1. Synthesis of Tert-butyl 3-(4-cyclopropyl-1,3-dimethyl-2-oxo-1,2,3,4-tetrahydropyrid o[2,3-b]pyrazin-6-yl)-2-oxo-4-phenylimidazolidine-1-carboxylate (68)

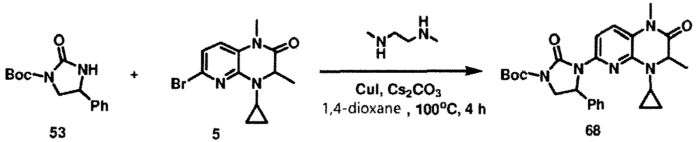

Into a dry 100 mL round-bottom flask, Intermediate **53** (0.5 g, 1.9 mmol), 1,4-dioxane (30mL), Intermediate **5** (0.56 g, 1.9 mmol), N,N'-dimethylethylenediamine (0.334 g, 3.8 mmol), cesium carbonate (1.235 g, 3.8 mmol) and copper iodide (0.361 g, 1.9 mmol) were added. The mixture was heated to 100°C under stirring for 4 hours. After the completion of the reaction, the mixture was concentrated under reduced pressure. The obtained residue was purified by column chromatography (ethyl acetate : petroleum ether = 1:1) to yield tert-butyl 3-(4-cyclopropyl-1,3-dimethyl-2-oxo-1,2,3,4-tetrahydropyrido[2,3-b]pyrazin-6-yl)-2-oxo-4-phenylimidazolidine-1-carboxylate **(68)** (0.7 g, white solid) yield: 77%. LCMS:m/z 477.7 (M+H).

### Step 2. Synthesis of 4-cyclopropyl-1,3-dimethyl-6-(2-oxo-5-phenylimidazolidin-1-yl)-3,4-dih ydropyrido[2,3-b]pyrazin-2(1H)-one (A18)

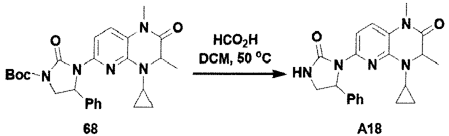

Into a dry 100 mL round-bottom flask, Intermediate **68** (0.7 g, 1.4 mmol), DCM (5mL) and anhydrous formic acid (5mL) were added. The mixture was stirred at 50°C for 2 hours. After the completion of the reaction, the mixture was cooled to RT, adjusted with saturated aqueous sodium carbonate solution to pH 10, and extracted with ethyl acetate (20mL×3). The combined organic phase was dried over anhydrous Na₂SO₄ and filtered. The filtrate was concentrated under reduced pressure to yield 4-cyclopropyl-1,3-dimethyl-6-(2-oxo-5-phenylimidazolidin-1-yl)-3,4-dihydro pyrido[2,3-b]pyrazin-2(1H)-one **(A18)** (crude 0.47 g), wherein 0.17 g was purified and separated by chiral high-performance preparative chromatography to yield compound **A18-P1** (24.4 mg, white solid), **A18-P2** (19.2 mg, white solid), **A18-P3** (22.1 mg, white solid) and **A18-P4** (8.9 mg, white solid), yield: 85%.

Chiral separation conditions (SFC): column: OD-H, eluent: 70% CO₂ + 30% MeOH, flow rate: 12.5ml/min, to yield **A18-P1** and **A18-P4** respectively; column: AD-H, eluent: 70% CO₂ + 30% EtOH (0.2% DEA), flow rate: 12.5ml/min, to yield **A18-P2** and **A18-P3** respectively.
**A18-P1:** LCMS:m/*z* 377.9 (M+H); ¹H NMR (DMSO-*d*₆, 400mHz): δ7.53 (d, *J* = 8.8 Hz, 1H), 7.32 - 7.22 (m, 5H), 7.21 - 7.15 (m, 2H), 5.65 (dd, *J* = 9.2, 4.4 Hz, 1H), 3.95 - 3.86 (m, 2H), 3.15 (s, 3H), 3.10 (dd, *J* = 9.2, 4.4 Hz, 1H), 2.01 - 1.93 (m, 1H), 1.01 - 0.92 (m, 1H), 0.85 (d, *J* = 6.8 Hz, 3H), 0.62 - 0.55 (m, 1H), 0.50 - 0.41 (m, 2H).
**A18-P2:** LCMS:m/*z* 377.9 (M+H); ¹H NMR (DMSO-*d*₆, 400mHz): δ7.53 (d, *J* = 8.8 Hz, 1H), 7.32 - 7.22 (m, 5H), 7.21 - 7.15 (m, 2H), 5.65 (dd, *J* = 9.2, 4.4 Hz, 1H), 3.95 - 3.86 (m, 2H), 3.15 (s, 3H), 3.10 (dd, *J* = 9.2, 4.4 Hz, 1H), 2.01 - 1.93 (m, 1H), 1.01 - 0.92 (m, 1H), 0.85 (d, *J* = 6.8 Hz, 3H), 0.62 - 0.55 (m, 1H), 0.50 - 0.41 (m, 2H).
**A18-P3:** LCMS:m/*z* 377.9 (M+H); ¹H NMR (DMSO-*d*₆, 400mHz): δ7.60 (d, *J* = 8.4 Hz, 1H), 7.33 - 7.26 (m, 3H), 7.23 - 7.16 (m, 3H), 7.09 (s, 1H), 5.80 (dd, *J* = 9.2, 4.4 Hz, 1H), 3.97 - 3.87 (m, 2H), 3.16 (s, 3H), 3.03 (dd, *J* = 9.2, 4.4 Hz, 1H), 2.45 - 2.38 (m, 1H), 1.09 (d, *J* = 6.8 Hz, 3H), 0.52 - 0.43 (m, 1H), 0.38 - 0.27(m, 2H), -0.40 - -0.48 (m, 1H).
**A18-P4:** LCMS:m/*z* 377.9 (M+H); ¹H NMR (DMSO-*d*₆, 400mHz): δ7.60 (d, *J=* 8.4 Hz, 1H), 7.33 - 7.26 (m, 3H), 7.23 - 7.16 (m, 3H), 7.09 (s, 1H), 5.80 (dd, *J* = 9.2, 4.4 Hz, 1H), 3.97 - 3.87 (m, 2H), 3.16 (s, 3H), 3.03 (dd, *J=* 9.2, 4.4 Hz, 1H), 2.45 - 2.38 (m, 1H), 1.09 (d, *J* = 6.8 Hz, 3H), 0.52 - 0.43 (m, 1H), 0.38 - 0.27(m, 2H), -0.40 - -0.48 (m, 1H).

### Example A19: Synthesis of 4-cyclopropyl-1,3-dimethyl-6-(3-methyl-2-oxo-5-phenylimidazoli din-1-yl)-3,4-dihydropyrido[2,3-b]pyrazin-2(1H)-one (A19)

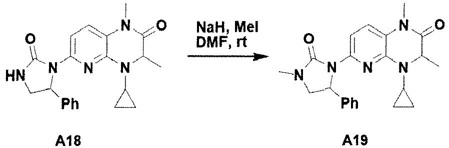

Into a dry 100 mL three-necked flask, **A18** (0.3 g, 0.79 mmol) and DMF (10mL) were added. Then sodium hydride (0.063 g, 1.5 mmol) and methyl iodide (0.338 g, 2.3 mmol) was added slowly at 0°C. The mixture was warmed to RT and stirred for 4 hours. After the completion of the reaction, the reaction mixture was quenched by saturated aqueous ammonium chloride solution, and extracted with ethyl acetate (30mL×3). The organic phases were combined, washed with saturated brine (30mL×2), dried over anhydrous Na₂SO₄ and filtered. The filtrate was concentrated under reduced pressure. The obtained residue was purified and separated by chiral high performance preparative chromatography to yield compound **A19-P1** (55.3 mg, white solid), **A19-P2** (41.9 mg, white solid), **A19-P3** (23 mg, white solid) and **A19-P4** (25.6 mg, white solid), yield: 46.7%.

Chiral separation conditions (SFC): column: OD-H, eluent: 70% CO₂ + 30% IPA (0.2% DEA), flow rate: 12.5ml/min, to yiedl **A19-P1** and **A19-P4** respectively; column: AD-H, eluent: 70% CO₂ + 30% EtOH (0.2% DEA), flow rate: 12.5ml/min, to yield **A19-P2** and **A19-P3** respectively.
**A19-P1:** LCMS:m/*z* 392.0 (M+H); ¹H NMR (DMSO-*d*₆, 400mHz): δ7.55 (d, *J* = 8.8 Hz, 1H), 7.34 - 7.25 (m, 5H), 7.23 - 7.17 (m, 1H), 5.61 (dd, *J* = 9.6, 4.4 Hz, 1H), 3.97 - 3.87 (m, 2H), 3.20 (dd, *J=* 8.8, 4.4 Hz, 1H), 3.15 (s, 3H), 2.81 (s, 3H), 2.02 - 1.94 (m, 1H), 1.02 - 0.94 (m, 1H), 0.85 (d, *J* = 6.8 Hz, 3H), 0.63 - 0.55 (m, 1H), 0.53 - 0.45 (m, 1H), 0.42 - 0.37 (m, 1H).
**A19-P2:** LCMS:m/*z* 391.9 (M+H); ¹H NMR (DMSO-*d*₆, 400mHz): δ7.55 (d, *J* = 8.8 Hz, 1H), 7.34 - 7.25 (m, 5H), 7.23 - 7.17 (m, 1H), 5.61 (dd, *J* = 9.6, 4.4 Hz, 1H), 3.97 - 3.87 (m, 2H), 3.20 (dd, *J* = 8.8, 4.4 Hz, 1H), 3.15 (s, 3H), 2.81 (s, 3H), 2.02 - 1.94 (m, 1H), 1.02 - 0.94 (m, 1H), 0.85 (d, *J* = 6.8 Hz, 3H), 0.63 - 0.55 (m, 1H), 0.53 - 0.45 (m, 1H), 0.42 - 0.37 (m, 1H).
**A19-P3:** LCMS:m/*z* 392.0 (M+H); ¹H NMR (DMSO-*d*₆, 400mHz): δ7.62 (d, *J* = 8.4 Hz, 1H), 7.33 - 7.28 (m, 3H), 7.23 - 7.18 (m, 3H), 5.76 (dd, *J* = 8.8, 3.2 Hz, 1H), 3.99 - 3.94 (m, 1H), 3.89 (t, *J=* 8.8 Hz, 1H), 3.17 (s, 3H), 3.16 - 3.13 (m, 1H), 2.77 (s, 3H), 2.45 - 2.38 (m, 1H), 1.09 (d, *J=* 6.8 Hz, 3H), 0.52 - 0.45 (m, 1H), 0.37 - 0.27 (m, 2H), -0.39 - -0.48 (m, 1H).
**A19-P4:** LCMS:m/z 392.0 (M+H); ¹H NMR (DMSO-*d*₆, 400mHz): δ7.62 (d, *J* = 8.4 Hz, 1H), 7.33 - 7.28 (m, 3H), 7.23 - 7.18 (m, 3H), 5.76 (dd, *J=* 8.8, 3.2 Hz, 1H), 3.99 - 3.94 (m, 1H), 3.89 (t, *J=* 8.8 Hz, 1H), 3.17 (s, 3H), 3.16 - 3.13 (m, 1H), 2.77 (s, 3H), 2.45 - 2.38 (m, 1H), 1.09 (d, *J=* 6.8 Hz, 3H), 0.52 - 0.45 (m, 1H), 0.37 - 0.27 (m, 2H), -0.39 - -0.48 (m, 1H).

### Example A20: Synthesis of (3R)-1,3-dimethyl-6-(2-oxo-5-phenylpyrrolidin-1-yl)-3,4-dihydro pyrido[2,3-b]pyrazin-2(1H)-one (A20)

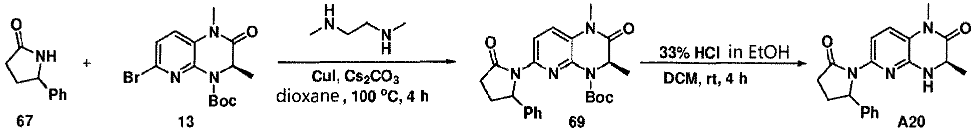

### Step 1. Synthesis of tert-butyl (3R)-1,3-dimethyl-2-oxo-6-(2-oxo-5-phenylpyrrolidin-1-yl)-2, 3-dihydropyrido[2,3-b]pyrazine-4(1H)-carboxylate (69)

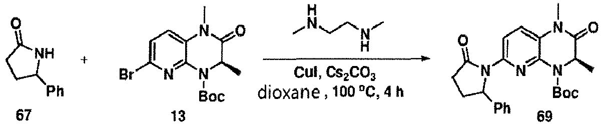

Into a dry 100 mL round-bottom flask, Intermediate **13** (188 mg, 0.53 mmol), compound 67 (85 mg, 0.53 mmol), N,N'-dimethylethylenediamine (93 mg, 1.06 mmol), Copper iodide (101 mg, 0.53 mmol), cesium carbonate (345 mg, 1.06 mmol) and 1,4-dioxane (5mL) were sequentially added at RT. The mixture was purged with nitrogen gas for three times, and heated to 100°C under stirring for 4 hours. After the completion of reaction as monitored on TLC plate, the mixture was cooled to RT and filtered. The filtrate was concentrated under reduced pressure. The obtained residue was purified by column chromatography (methanol:dichloromethane = 1:20) to yield tert-butyl (3*R*)-1,3-dimethyl-2-oxo-6-(2-oxo-5-phenylpyrrolidin-1-yl)-2,3-dihydropyrido[2,3-b] pyrazine-4(1H)-carboxylate **(69)** (140 mg, yellow solid), yield: 60%. LCMS:*m*/*z* 437.0 (M+H).

### Step 2. Synthesis of (3R)-1,3-dimethyl-6-(2-oxo-5-phenylpyrrolidin-1-yl)-3,4-dihydropyrido [2,3-b]pyrazin-2(1H)-one (A20)

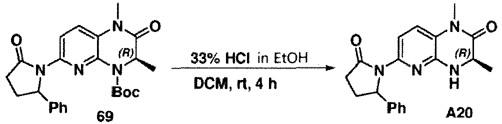

Into a dry 100 mL round-bottom flask, Intermediate **69** (140 mg, 0.32 mmol) and dichloromethane (4mL) were sequentially added at RT, followed by addition of 33% solution of hydrochloric acid in EtOH (2mL). The mixture was stirred for 4 hours. After the completion of reaction as monitored on TLC plate, the obtained residue was concentrated under reduced pressure to yield (3*R*)-1,3-dimethyl-6-(2-oxo-5-phenylpyrrolidin-1-yl)-3,4-dihydropyrido[2,3-b]pyrazin-2(1H)-one **A20** (108mg, yellow solid), yield: 100%. LCMS:*m*/*z* 337.0 (M+H).

Chiral separation conditions (SFC): column: OJ-H, eluent: 70% CO₂ +30% MeOH, flow rate: 12.5 mL /min; the common HPLC separation conditions: column: Gemini-C18 150×21.2 mm, 5 um; mobile phase: ACN-H₂O (0.5%FA), gradient: 30-35.
**A20-P1:** LCMS:*m*/*z* 337.0 (M+H); ¹H NMR (CDCl₃, 400mHz) 7.36-7.28 (m, 3H), 7.25 - 7.19 (m, 3H), 7.05 (d, *J=* 8.0 Hz, 1H), 5.70 (s, 1H), 4.19 (q, *J=* 6.4 Hz, 1H), 3.27 (s, 3H), 2.82-2.72 (m, 1H), 2.69-2.60 (m, 2H), 2.05-1.97 (m, 1H), 1.47 (d, *J* = 6.6 Hz, 3H).
**A20-P2:** LCMS:*m*/*z* 337.0 (M+H); ¹H NMR (CDCl₃, 400mHz) 7.67 (d, *J* = 8.4 Hz, 1H), 7.32 - 7.27 (m, 2H), 7.20 (t, *J=* 8.8 Hz, 3H), 7.07 (d, *J=* 8.4 Hz, 1H), 5.72 (d, *J* = 4.8 Hz, 1H), 4.46 (brs, 1H), 4.06 (q, *J* = 6.4 Hz, 1H), 3.26 (s, 3H), 2.84 - 2.70 (m, 1H), 2.67 - 2.52 (m, 2H), 2.05 - 1.94m, 1H), 1.36 (d, *J* = 6.6 Hz, 3H).

### Example A21: Synthesis of 6-(2-(4-fluorophenyl)-5-oxopyrrolidin-1-yl)-1,3-dimethyl-1H-pyr ido[2,3-B][1,4]oxazin-2(3H)-one (A21)

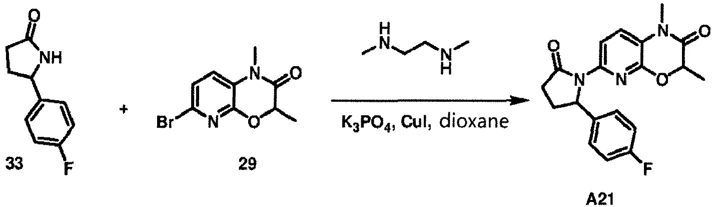

Into a dry 50 mL round-bottom flask, Intermediate **33** (100 mg, 0.56 mmol), Intermediate **29** (143 mg, 0.56 mmol), N,N'-dimethylethylenediamine (98mg, 1.12 mmol), Copper iodide (67 mg, 0.56 mmol), potassium phosphate (237mg,1.12 mmol) and 1,4-dioxane (10mL) were sequentially added at RT. The mixture was purged with nitrogen gas for three times, and heated to 100°C under stirring for 16 hours. The mixture was cooled to RT and filtered. The filtrate was concentrated under reduced pressure. The obtained residue was purified by column chromatography (petroleum ether:ethyl acetate = 2:1) to yield 6-(2-(4-fluorophenyl)-5-oxopyrrolidin-1-yl)-1,3-dimethyl-1*H-*pyrido[2,3-B][1,4]oxazin-2(3H)-one **(A21)** (220 mg, white solid). LCMS: *m*/*z* 355.8 (M+H).

Chiral separation conditions (SFC): column: OD-H, eluent: 70% CO₂ + 30% IPA (DEA), flow rate: 12.5 mL /min, to yield **A21-P1, A21-P2, A21-P3** and **A21-P4** repectively.
**A21-P1:** LCMS: *m*/*z* 355.9 (M+H); ¹H NMR (CD₃OD, 400 MHz) δ 7.85 (d, *J* = 8.4 Hz, 1H), 7.53 (d, *J=* 8.4 Hz, 1H), 7.29 (dd, *J=* 8.4, 5.4 Hz, 2H), 7.01 (t, *J* = 8.6 Hz, 2H), 5.76 (dd, *J=* 8.0, 4.0 Hz, 1H), 4.81 (q, *J=* 6.8 Hz, 1H), 3.31 (s, 3H), 2.79-2.84 (m, 1H), 2.71 -2.59 (m, 2H), 1.96-2.03 (m, 1H), 1.51 (d, *J* = 6.8 Hz, 3H).
**A21-P2:** LCMS: *m*/*z* 355.9 (M+H); ¹H NMR (CD₃OD, 400 MHz) δ7.82 (d, *J=* 8.4 Hz, 1H), 7.52 (d, *J* = 8.4 Hz, 1H), 7.28 (dd, *J =* 8.4, 5.4 Hz, 2H), 7.01 (t, *J* = 8.6 Hz, 2H), 5.73 (dd, *J* = 8.0, 4.6 Hz, 1H), 4.81 (q, *J* = 6.8 Hz, 1H), 3.29 (s, 3H), 2.88-2.74 (m, 1H), 2.72-2.58 (m, 2H), 2.07-1.91 (m, 1H), 1.45 (d, *J* = 6.8 Hz, 3H).
**A21-P3:** LCMS: *m*/*z* 355.9 (M+H); ¹H NMR (CD₃OD, 400 MHz)δ7.85 (d, *J* = 8.4 Hz, 1H), 7.51 (d, *J* = 8.4 Hz, 1H), 7.28 (dd, *J* = 8.2, 5.4 Hz, 2H), 7.01 (t, *J* = 8.6 Hz, 2H), 5.75 (dd, *J* = 8.0, 4.0 Hz, 1H), 4.79 (q, *J* = 6.4 Hz, 1H), 3.30 (s, 3H), 2.88-2.74 (m, 1H), 2.71-2.58 (m, 2H), 2.04-1.91 (m, 1H), 1.50 (d, *J* = 6.8 Hz, 3H).
**A21-P4:** LCMS: *m*/*z* 355.9 (M+H); ¹H NMR (CD₃OD, 400 MHz) δ7.82 (d, *J=* 8.4 Hz, 1H), 7.52 (d, *J=* 8.4 Hz, 1H), 7.28 (dd, *J=* 8.4, 5.4 Hz, 2H), 7.01 (t, *J=* 8.6 Hz, 2H), 5.73 (dd, *J=* 8.0, 4.6 Hz, 1H), 4.81 (q, *J=* 6.8 Hz, 1H), 3.30 (s, 3H), 2.90-2.73 (m, 1H), 2.74-2.58 (m, 2H), 2.09-1.92 (m, 1H), 1.45 (d, *J* = 6.8 Hz, 3H).

### Example A22: Synthesis of 4-cyclopropyl-6-(2-(4-fluorophenyl)-5-oxopyrrolidin-1-yl)-1,3-di methyl-3,4-dihydropyrido[2,3-b]pyrazin-2(1H)-one (A22)

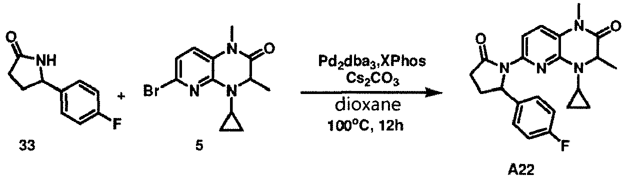

Into a dry 100 mL round-bottom flask, Intermediate **33** (0.08 g, 0.496 mmol), 1,4-dioxane (30mL), Intermediate **5** (0.144 g, 0.496 mmol), cesium carbonate (0.24 g, 0.744 mmol), Pd₂dba₃ (0.044 g, 0.0496 mmol) and X-Phos (0.044 g, 0.0992 mmol) were sequentially added. The mixture was purged with argon gas for three times, warmed to 100°C and stirred for 12 hours. After the completion of the reaction, the mixture was filtered, and the filtrate was concentrated under reduced pressure. The resulting crude was purified by column chromatography (ethyl acetate : petroleum ether = 2:1), to yield 4-cyclopropyl-6-(2-(4-fluorophenyl)-5-oxopyrrolidin-1-yl)-1,3-dimethyl-3,4-dihydro pyrido[2,3-b]pyrazin-2(1H)-one **(A22).** The racemate was purified and separated by chiral high performance preparative chromatography to yield **A22-P1** (6.4 mg, white solid), **A22-P2** (10.7 mg, pale yellow solid), **A22-P3** (6.2 mg, white solid) and **A22-P4** (18 mg, white solid). yield: 21%.

Chiral separation conditions (SFC): column: OJ-H, eluent: 70% CO₂ + 30% EtOH (0.2% DEA), flow rate: 12.5 mL/min, to yield **A22-P1** and **A22-P4** respectively; column: OD-H, eluent: 70% CO₂ + 30% MeOH (0.2% DEA), flow rate: 12.5 mL/min, to yiedl **A22-P2** and **A22-P3** respectively.
**A22-P1:** LCMS: *m*/*z* 394.7 (M+H); ¹H NMR (DMSO-*d₆*, 400 MHz): δ7.63 (d, *J=* 8.4 Hz, 1H), 7.33 - 7.23 (m, 3H), 7.07 (t, *J* = 8.8 Hz, 2H), 5.72 - 5.66 (m, 1H), 3.97 (q, *J* = 6.4 Hz, 1H), 3.16 (s, 3H), 2.76 - 2.68 (m, 1H), 2.63 - 2.54 (m, 2H), 2.04 - 1.97 (m, 1H), 1.88 - 1.79 (m, 1H), 0.99 - 0.86 (m, 1H), 0.87 (d, *J=* 6.8 Hz, 3H), 0.64 - 0.56 (m, 1H), 0.53 - 0.45 (m, 1H), 0.43 - 0.36 (m, 1H).
**A22-P2:** LCMS: *m*/*z* 394.9 (M+H); ¹H NMR (DMSO-*d*₆, 400 MHz): δ7.68 (d, *J=* 8.4 Hz, 1H), 7.35 (d, *J* = 8.4 Hz, 1H), 7.21 - 7.17 (m, 2H), 7.11 (t, *J* = 8.8 Hz, 2H), 5.89-5.85 (m, 1H), 4.00 (q, *J* = 6.8 Hz, 1H), 3.18 (s, 3H), 2.69 - 2.54 (m, 3H), 2.48 - 2.42 (m, 1H), 1.85 - 1.76 (m, 1H), 1.10 (d, *J* = 6.8 Hz, 3H), 0.57 - 0.42 (m, 2H), 0.42 - 0.34 (m, 1H), -0.30 - -0.38 (m, 1H).
**A22-P3:** LCMS: *m*/*z* 394.7 (M+H); ¹H NMR (DMSO-*d*₆, 300 MHz): δ7.68 (d, *J* = 8.4 Hz, 1H), 7.35 (d, *J* = 8.4 Hz, 1H), 7.21 - 7.17 (m, 2H), 7.11 (t, *J* = 8.8 Hz, 2H), 5.89 - 5.85 (m, 1H), 4.00 (q, *J* = 6.8 Hz, 1H), 3.18 (s, 3H), 2.69 - 2.54 (m, 3H), 2.48 - 2.42 (m, 1H), 1.85 - 1.76 (m, 1H), 1.10 (d, *J* = 6.8 Hz, 3H), 0.57 - 0.42 (m, 2H), 0.42 - 0.34 (m, 1H), -0.30 - -0.38 (m, 1H).
**A22-P4:** LCMS: *m*/*z* 394.8 (M+H); ¹H NMR (DMSO-*d*₆, 300 MHz): δ7.63 (d, *J=* 8.4 Hz, 1H), 7.33 - 7.23 (m, 3H), 7.07 (t, *J* = 8.8 Hz, 2H), 5.72 - 5.66 (m, 1H), 3.97 (q, *J* = 6.4 Hz, 1H), 3.16 (s, 3H), 2.76 - 2.68 (m, 1H), 2.63 - 2.54 (m, 2H), 2.04 - 1.97 (m, 1H), 1.88 - 1.79 (m, 1H), 0.99 - 0.86 (m, 1H), 0.87 (d, *J* = 6.8 Hz, 3H), 0.64 - 0.56 (m, 1H), 0.53 - 0.45 (m, 1H), 0.43 - 0.36 (m, 1H).

Analogous to Example **A22,** the following examples were synthesized according to general synthetic procedure B:

| Ex. | Structure | Name | Analysis Data |
|---|---|---|---|
| A23 | 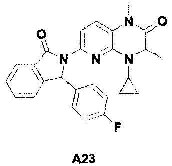 | 4-cyclopropyl-6-(1-(4-fluorophenyl) -3-oxoisoindolin -2-yl)-1,3-dimethyl-3,4-dihydropyrid o[2,3-b]pyrazin-2(1 H)-one | **A23P1:** LCMS: *m*/*z* 442.8 (M+H); ¹H NMR (DMSO-*d*₆, 400 MHz): δ7.86 (t, *J=* 8.2 Hz, 2H), 7.65 (t, *J=* 7.4 Hz, 1H), 7.57 (t, *J* = 7.4 Hz, 1H), 7.42 - 7.36 (m, 2H), 7.33 (dd, *J* = 8.4, 5.2 Hz, 2H), 7.08 (t, *J* = 8.8 Hz, 2H), 6.67 (s, 1H), 4.05 - 4.00 (m, 1H), 3.19 (s, 3H), 2.26 - 2.17 (m, 1H), 1.24 - 1.15 (m, 1H), 0.92 (d, *J* = 6.8 Hz, 3H), 0.75 - 0.66 (m, 1H), 0.60 - 0.53 (m, 1H), 0.51 -0.44 (m, 1H). |
| | | | **A23P2:** LCMS: *m*/*z* 442.8 (M+H); ¹H NMR (DMSO-*d*₆, 400 MHz): δ7.86 (t, *J* = 8.2 Hz, 2H), 7.65 (t, *J=* 7.4 Hz, 1H), 7.57 (t, *J* = 7.4 Hz, 1H), 7.42 - 7.36 (m, 2H), 7.33 (dd, *J* = 8.4, 5.2 Hz, 2H), 7.08 (t, *J* = 8.8 Hz, 2H), 6.67 (s, 1H), 4.05 - 4.00 (m, 1H), 3.19 (s, 3H), 2.26 - 2.17 (m, 1H), 1.24 - 1.15 (m, 1H), 0.92 (d, *J=* 6.8 Hz, 3H), 0.75 - 0.66 (m, 1H), 0.60 - 0.53 (m, 1H), 0.51 - 0.44 (m, 1H). |
| | | | **A23P3:** LCMS: *m*/*z* 442.7 (M+H); ¹H NMR (DMSO-*d₆*, 400 MHz): δ7.84 (d*, J* = 7.6 Hz, 1H), 7.77 (d*, J* = 8.4 Hz, 1H), 7.63 (t, *J* = 7.4 Hz, 1H), 7.54 (t, *J* = 7.4 Hz, 1H), 7.47 - 7.36 (m, 4H), 7.11 (t, *J =* 8.8 Hz, 2H), 6.79 (s, 1H), 4.07 - 4.01 (m, 1H), 3.20 (s, 3H), 2.62 - 2.54 (m, 1H), 1.14 (d, *J =* 6.8 Hz, 3H), 0.90 - 0.81 (m, 1H), 0.67 - 0.58 (m, 1H), 0.50 - 0.44 (m, 1H), -0.11 - -0.19 (m, 1H). |
| | | | **A23P4:** LCMS: *m*/*z* 442.7 (M+H); ¹H NMR (DMSO-*d₆*, 400 MHz): δ7.84 (d, *J* = 7.6 Hz, 1H), 7.77 (d*, J* = 8.4 Hz, 1H), 7.63 (t, *J* = 7.4 Hz, 1H), 7.54 (t, *J=* 7.4 Hz, 1H), 7.47 - 7.36 (m, 4H), 7.11 (t, *J* = 8.8 Hz, 2H), 6.79 (s, 1H), 4.07 - 4.01 (m, 1H), 3.20 (s, 3H), 2.62 - 2.54 (m, 1H), 1.14 (d, *J* = 6.8 Hz, 3H), 0.90 - 0.81 (m, 1H), 0.67 - 0.58 (m, 1H), 0.50 - 0.44 (m, 1H), -0.11 - -0.19 (m, 1H). |
| A24 | 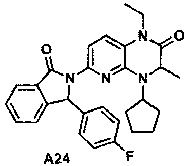 | 4-cyclopentyl-1-ethyl-6-(1-(4-fluorophenyl) -3-oxoisoindolin -2-yl)-3-methyl-3,4-dihydropyrid o[2,3-b]pyrazin-2(1 H)-one | **A24,** the racemate: LCMS: *m*/*z* 485.2 (M+H) |

Analogously to the synthesis of Example **A1**, the following examples was synthesized according to general synthetic procedure A:

| Ex. | Structure | Name | Analysis Data |
|---|---|---|---|
| A25 | 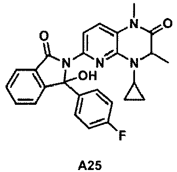 | 4-cyclopropyl-6-(1-(4-fluorophenyl)-1-hydroxy-3-oxoisoindolin-2-yl)-1,3-dimethyl-3,4-dihydropyrido[2,3 -b]pyrazin-2(1H)-one | **A25** (the racemate ): LCMS: *m*/*z* 458.6 (M+H); ¹H NMR (DMSO-*d₆*, 400 MHz): δ 7.87 (t, *J* = 6.6 Hz, 1H), 7.71 - 7.37 (m, 8H), 7.16 - 7.08 (m, 2H), 4.10 - 4.02 (m, 1H), 3.20 (d, *J* = 2.4 Hz, 3H), 2.23 - 2.16 (m, 0.5H), 1.17 (d, *J* = 6.8 Hz, 1H), 0.93 (d, *J* = 6.8 Hz, 2H), 0.81 - 0.70 (m, 1H), 0.68 - 0.60 (m, 1H), 0.57 - 0.49 (m, 1H), 0.45 - 0.39 (m, 1H), -0.14 - -0.22 (m, 0.5H). |

### Intermediate 70: Synthesis of 6-bromo-1,3-dimethyl-4-(oxetan-3-yi)-3,4-dihydropyrido[2,3-b]pyrazin-2(1H)-one (70)

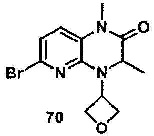

Intermediate **70** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from oxetan-3-amine.

### Intermediate 71: Synthesis of 6-bromo-4-(2-hydroxyethyl)-1,3-dimethyl-3,4-dihydropyrido [2,3-b]pyrazin-2(1H)-one (71)

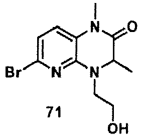

Intermediate **71** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from 2-aminoethan-1-ol.

### Intermediate 72: Synthesis of 4-benzyl-6-bromo-1,3-dimethyl-3,4-dihvdropyrido[2,3-b]pyra zin-2(1H)-one (72)

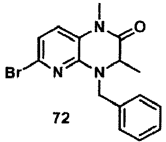

Intermediate **72** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from benzylamine.

### Intermediate 73: Synthesis of 6-bromo-4-cyclopentyl-1-ethyl-3-methyl-3,4-dihydropyrido[2, 3-b]pyrazin-2(1H)-one (73)

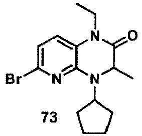

Intermediate **73** was prepared analogously to the synthetic procedure of Intermediate **7** 6-bromo-4-cyclopentyl-1,3-dimethyl-3,4-dihydropyrido[2,3-b]pyrazin-2(1H)-one **(7),** starting from ethyl iodide.

### Intermediate 76: Synthesis of 3-(pyridin-3-yl)isoindolin-1-one (76)

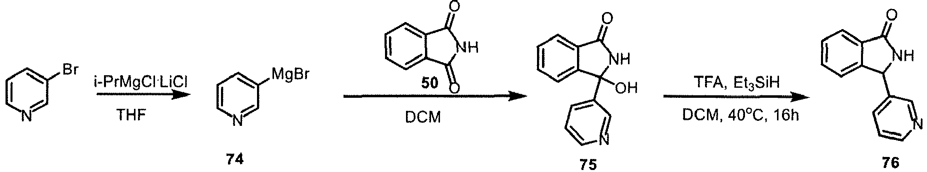

### Step 1. Synthesis of 3-pyridine magnesium bromide (74)

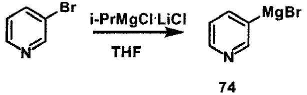

Into a dry 500 mL three-necked flask, 3-bromopyridine (10 g, 63.29 mmol) and dry tetrahydrofuran (50mL) were added under ice-water bath. The mixture was purged with nitrogen gas for three times, and the solution of isopropyl magnesium chloride lithium chloride complex in tetrahydrofuran (1.3 M, 97 mL, 126.58 mmol) was added. The mixture was slowly warmed to RT, and stirred for 5 hours. The solution was directly used in the next reaction (0.42M).

### Step 2. Synthesis of 3-hydroxy-3-(pyridin-3-yl)isoindolin-1-one (75)

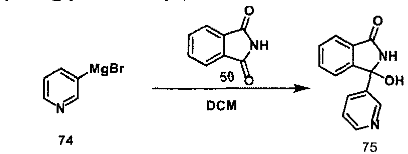

Into a dry 500 mL three-necked flask, phthalamide **50** (3 g, 21.1 mmol) and dry dichloromethane (150mL) were added under ice-water bath. The mixture was purged with nitrogen gas for three times, and the solution of **74** in tetrahydrofuran (0.42M, 150 mL, 63.29 mmol) was added. The mixture was slowly warmed to RT, and stirred for 16 hours. After the completion of the reaction, water was added (200mL). The insoluble substance was precipitated and filtered. The filter cake was purified by column chromatography (dichloromethane:methanol = 10:1) to yield 3-hydroxy-3-(pyridin-3-yl)isoindolin-1-one **75** (2.02 g, brown solid), yield: 42%. **LCMS:** *m*/*z* 226.9 (M+H).

### Step 3. Synthesis of 3-(pyridin-3-yl)isoindolin-1-one (76)

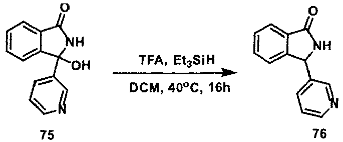

Into a dry 250 mL three-necked flask, **75** (1.56 g, 6.9 mmol), dichloromethane (20mL), trifluoroacetic acid (5mL) and triethylsilane (2.4 g, 20.7 mmol) were sequentially added at RT. The mixture was purged with nitrogen gas for three times, heated to 40°C under stirring for 16 hours, and concentrated under reduced pressure. The obtained residue was purified by column chromatography (dichloromethane:methanol = 30:1) to yield 3-(pyridin-3-yl)isoindolin-1-one **76** (800 mg, white solid), yield: 55%. **LCMS:** *m*/*z* 210.9 (M+H).

### Intermediate 79: Synthesis of 7-bromo-1-cyclopropyl-2,4-dimethyl-1,2-dihydropyrido[4,3-b] pyrazin-3(4H)-one (79)

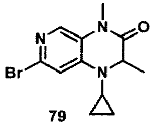

Intermediate **79** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from 3-amino-4,6-dibromopyridine **(78)** as follows:

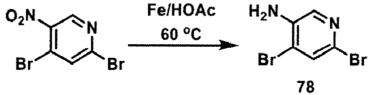

Into a dry 100 mL round-bottom flask, 2,4-dibromo-5-nitropyridine (500 mg, 1.78 mmol), acetic acid (12mL) and iron powder (500 mg, 8.93 mmol) were sequentially added. The mixture was heated to 60°C under stirring for 3 hours. The mixture was cooled to RT and filtered. The filter cake was washed with acetic acid (5mL×3), and the filtrate was concentrated under reduced pressure. The obtained residue was purified by column chromatography (petroleum ether:ethyl acetate = 2:1) to yield 3-amino-4,6-dibromopyridine **78** (350 mg, light yellow solid), yield: 78%. **LCMS:** *m*/*z* 252.7 (M+H).

### Intermediate 80: Synthesis of 6-bromo-4-cyclopropyl-1-ethyl-3-methyl-3,4-dihydropyrido[3, 2-b]pyrazin-2(1H)-one (80)

Intermediate **80** was prepared analogously to the synthetic procedure of Intermediate **5** 6-bromo-4-cyclopropyl-1,3-dimethyl-3,4-dihydropyrido[2,3-b]pyrazin-2(1H)-one **(5),** and ethyl iodide was used as starting material in final step.

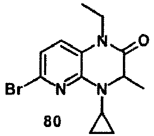

### Intermediate 82: Synthesis of 3-benzylisoindolin-1-one (82)

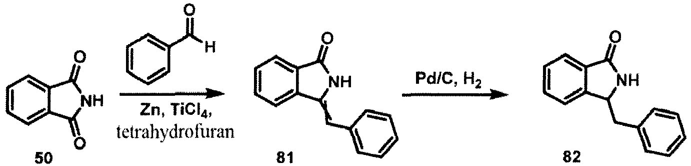

### Step 1. Synthesis of 3-benzylideneisoindolin-1-one (81)

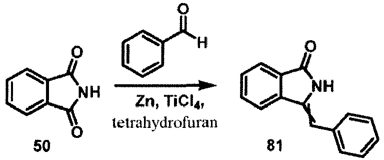

Into a dry 50 mL three-necked flask, phthalamide **50** (1.47 g, 10 mmol), benzaldehyde (4.48 g, 30 mmol), zinc powder (5.1 g, 80 mmol) and 80 mL tetrahydrofuran were sequentially added. Under the protection of nitrogen gas, anhydrous titanium tetrachloride (7.56 g, 40 mmol) was added in portions at 0°C. The mixture was then stirred at 50°C for 4 hours. After the completion of reaction detected by TLC, 50 mL water was added slowly to the reaction mixture. The mixture was extracted with ethyl acetate (100mL×3). The organic phases were combined, washed with saturated brine (100mL×3), dried over anhydrous Na₂SO₄ and filtered. The filtrate was concentrated under reduced pressure to yield the crude, which was finally purified by column chromatography (petroleum ether:ethyl acetate = 30%∼60%) to yield E,Z mixed 3-benzylideneisoindolin-1-one (81) 480 mg, yield: 20%.

### Step 2. Synthesis of 3-benzylisoindolin-1-one (82)

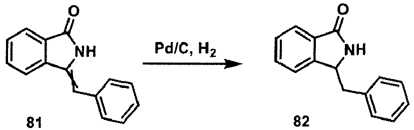

Into a dry 50 mL three-necked flask, E,Z mixed 3-benzylideneisoindolin-1-one **81** (480 mg, 2.2 mmol), Pd/C (59 mg, 0.05 mmol) and 5 mL methanol were sequentially added. The reaction was carried out at 50°C overnight under the protection of H2. After the completion of the reaction, the mixture was filtered and the filter cake was washed with a large amount of methanol. The filtrate was spin-dried to yield 3-benzylisoindolin-1-one **82,** 400 mg. yield: 80%. ¹HNMR (CDCl₃, 400MHz): δ 6.75-7.35 (m,9H), 4.78 (t,1H), 3.19 (d,1H), 2.13 (d,1H).

Analogously to the synthesis of Example **A1**, the following examples were synthesized according to general synthetic procedure A, wherein Cs₂CO₃ was replaced with K₂CO₃:

| Ex. | Structure | Name | Analysis Data |
|---|---|---|---|
| A26 | 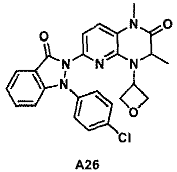 | 6-(1-(4-chlorophenyl)-3-oxo-1,3-dihydro-2H-indazol-2-yl)-1,3-dimethyl-4-(oxetan-3-yl)-3,4-dihydropyrido[ 2,3-b]pyrazin-2(1 H)-one | **A26-P1:** LCMS: *m*/*z* 475.9 (M+H); ¹H NMR (CDCl₃, 400 MHz) 7.96 (d, *J* = 7.6 Hz, 1H), 7.60 - 7.52 (m, 2H), 7.40-7.33 (m, 2H), 7.31 - 7.27 (m, 2H), 7.26 - 7.21 (m, 3H), 4.81 (t, *J* = 6.6 Hz, 1H), 4.71-4.63 (m, 2H), 4.46 - 4.37 (m, 1H), 4.34 (t, *J* = 6.8 Hz, 1H), 4.05 (q, *J =* 6.8 Hz, 1H), 3.34 (s, 3H), 1.01 (d, *J* = 6.8 Hz, 3H). |
| | | | **A26-P2:** LCMS: *m*/*z* 475.9 (M+H); ¹H NMR (CDCl₃, 400 MHz) 7.96 (d, *J* = 7.7 Hz, 1H), 7.60 - 7.52 (m, 2H), 7.38-7.34 (m, 2H), 7.31 - 7.27 (m, 2H), 7.26 - 7.20 (m, 3H), 4.81 (t, *J* = 6.6 Hz, 1H), 4.70 - 4.64 (m, 2H), 4.46 - 4.38 (m, 1H), 4.34 (t, *J* = 6.8 Hz, 1H), 4.05 (q, *J* = 6.8 Hz, 1H), 3.34 (s, 3H), 1.01 (d, *J* = 6.8 Hz, 3H). |
| A27 | 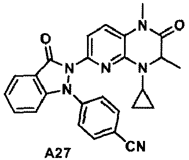 | 6-(1-(4-cyanophenyl)-3-oxo-1,3-dihydro-2H-indazol-2-yl)-4-cyclopropyl-1,3-dimethyl-3,4-dihydropyrido[ 2,3-b]pyrazin-2(1 H)-one | **A27-P1:** LCMS: *m*/*z* 450.9 (M+H); ¹H NMR (CDCl₃, 400 MHz): δ8.01 (d*, J* = 7.6 Hz, 1H), 7.71 (t, *J* = 7.6 Hz, 3H), 7.62 (m, 1H), 7.47 (d, *J* = 8.8 Hz, 2H), 7.39 (d, *J* = 8.0 Hz, 1H), 7.34 (t, *J* = 7.6 Hz, 1H), 7.23 (d*, J* = 8.4 Hz, 1H),4.13 (q, *J* = 6.4 Hz, 1H), 3.33 (s, 3H), 2.27 (m, 1H), 1.17 (d, *J* = 6.8 Hz, 3H), 0.66 - 0.60 (m, 2H), 0.46 - 0.40 (m, 1H),-0.02 - -0.07(m, 1H). |
| | | | **A27-P2:** LCMS: *m*/*z* 450.9 (M+H); ¹H NMR (CDCl₃, 400 MHz): δ8.01 (d, *J* = 7.6 Hz, 1H), 7.71 (t, *J* = 7.6 Hz, 3H), 7.62 (m, 1H), 7.47 (d, *J* = 8.8 Hz, 2H), 7.39 (d*, J* = 8.0 Hz, 1H), 7.34 (t, *J* = 7.6 Hz, 1H), 7.23 (d, *J* = 8.4 Hz, 1H),4.13 (q, *J* = 6.4 Hz, 1H), 3.33 (s, 3H), 2.27 (m, 1H), 1.17 (d, *J* = 6.8 Hz, 3H), 0.66 - 0.60 (m, 2H), 0.46 - 0.40 (m, 1H), - 0.02 - -0.07(m, 1H). |
| | | | **A27-P3:** LCMS: *m*/*z* 450.9 (M+H); ¹H NMR (CDCl₃, 400 MHz): δ7.94 (d, *J* = 8.8 Hz, 2H), 7.86-7.81 (m, 3H), 7.59 (d, *J* = 8.0 Hz, 1H), 7.54 (t, *J* = 8.4 Hz, 1H), 7.25 (d, *J* = 7.6 Hz, 1H), 7.18 (d, *J* = 8.0 Hz, 1H), 6.66 (d, *J* = 8.4 Hz, 1H),4.16 (q, *J* = 6.4 Hz, 1H), 3.35 (s, 3H), 2.35 (m, 1H), 1.28 (d, *J* = 6.8 Hz, 3H), 0.55 - 0.48 (m, 1H), 0.44 - 0.37 (m, 1H), 0.27 - 0.17(m, 2H). |
| | | | **A27-P4:** LCMS: *m*/*z* 450.9 (M+H); ¹H NMR (CDCl₃, 400 MHz): δ7.94 (d, *J=* 8.8 Hz, 2H), 7.86-7.81 (m, 3H), 7.59 (d, *J* = 8.0 Hz, 1H), 7.54 (t, *J* = 8.4 Hz, 1H), 7.25 (d, *J* = 7.6 Hz, 1H), 7.18 (d, *J* = 8.0 Hz, 1H), 6.66 (d, *J* = 8.4 Hz, 1H),4.16 (q, *J =* 6.4 Hz, 1H), 3.35 (s, 3H), 2.35 (m, 1H), 1.28 (d, *J* = 6.8 Hz, 3H), 0.55 - 0.48 (m, 1H), 0.44 - 0.37 (m, 1H), 0.27 - 0.17(m, 2H). |
| A28 | 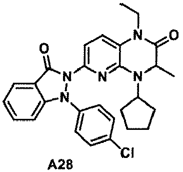 | 6-(1-(4-chlorophenyl)-3-oxo-1,3-dihydro-2H-indazol-2-yl)-4-cyclopentyl-1-ethyl-3-methyl-3,4-dihydropyridin e[2,3-b]pyrazin-2(1 H)-one | **A28,** the racemate : LCMS: *m*/*z* 502.2 (M+H). |
| A29 | 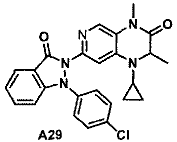 | 7-(1-(4-chlorophenyl)-3-oxo-1H-indazol-2(3H)-yl)-1-cyclopropyl-2,4-dimethyl-1,2-dihydropyrido[ 3,4-b]pyrazin-3(4H)-one | **A29-P1: LCMS:** *m*/*z* 459.7 (M+H); **¹H NMR (CD₃OD, 400 MHz**)δ7.81 (d, *J* = 8.4 Hz, 1H), 7.74 (dd, *J* = 6.8, 2.0 Hz, 2H), 7.67 (s, 1H), 7.59-7.53 (m, 4H), 7.25 (d, *J* = 7.0 Hz, 1H), 6.98 (s, 1H), 4.21 (d, *J* = 6.8 Hz, 1H), 3.36 (d, *J* = 3.4 Hz, 3H), 2.64 (d, *J* = 3.2 Hz, 1H), 1.35-1.31 (m, 3H), 1.06-0.98 (m, 1H), 0.93-0.88 (m, 1H), 0.78-0.71 (m, 1H), 0.66-0.54 (m, 1H). |
| | | | **A29-P2: LCMS:** *m*/*z* 459.8 (M+H); **¹H NMR** (**CD₃OD, 400 MHz)** δ7.83 (d, *J* = 8.8 Hz, 1H), 7.76 (d, *J* = 8.6 Hz, 2H), 7.68 (s, 1H), 7.57 (dd, *J* = 15.0, 8.4 Hz, 4H), 7.25 (t, *J* = 7.6 Hz, 1H), 6.99 (s, 1H), 4.21 (t, *J* = 6.8 Hz, 1H), 3.37 (s, 3H), 2.69-2.61 (m, 1H), 1.34 (d, *J=* 6.8 Hz, 3H), 1.07-0.99 (m, 1H), 0.95-0.88 (m, 1H), 0.80-0.72 (m, 1H), 0.66-0.57 (m, 1H). |
| | | | **A29-P3: LCMS:** *m*/*z* 459.8 (M+H); **¹H NMR (CD₃OD, 400 MHz)**δ7.96 (d, *J=* 8.0 Hz, 1H), 7.91 (s, 1H), 7.69 (t, *J* = 7.8 Hz, 1H), 7.54 (s, 1H), 7.44 (s, 4H), 7.38 (t, *J=* 7.6 Hz, 1H), 7.32 (d, *J =* 8.4 Hz, 1H), 4.21 (q, *J =* 6.8 Hz, 1H), 3.34 (s, 3H), 2.73-2.64 (m, 1H), 1.30 (d, *J =* 6.8 Hz, 3H), 1.19-1.11 (m, 1H), 0.98-0.89 (m, 1H), 0.79-0.70 (m, 1H), 0.57-0.49 (m, 1H). |
| | | | **A29-P4: LCMS:** *m*/*z* 459.8 (M+H); **¹H NMR (CD₃OD, 400 MHz)** δ7.96 (d, *J* = 8.0 Hz, 1H), 7.91 (s, 1H), 7.69 (t, *J* = 7.8 Hz, 1H), 7.54 (s, 1H), 7.44 (s, 4H), 7.38 (t, *J* = 7.6 Hz, 1H), 7.32 (d, *J* = 8.4 Hz, 1H), 4.21 (q, *J* = 6.8 Hz, 1H), 3.34 (d*, J* = 2.0 Hz, 3H), 2.73-2.64 (m, 1H), 1.30 (d, *J* = 7.0 Hz, 3H), 1.16-1.12 (m, 1H), 0.98-0.89 (m, 1H), 0.76-0.72 (m, 1H), 0.55-0.51 (m, 1H). |

Analogously to the synthesis of Example **A1**, the following examples were synthesized according to general synthetic procedure A, wherein Cs₂CO₃ was replaced with K₃PO₄:

| Ex. | Structure | Name | Analysis Data |
|---|---|---|---|
| A30 | 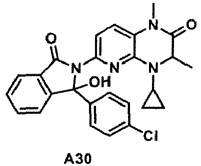 | 6-(1-(4-chlorophenyl)-1-hydroxy-3-oxoisoindolin-2-yl)-4-cyclopropyl-1,3-dimethyl-3,4-dihydropyrido[3,2-b]pyrazin-2(H)-one | **A30** (racemate): LCMS: *m*/*z* 474.8 (M+H); ¹H NMR (CD₃OD, 400 MHz)δ7.92 (t, *J* = 7.6 Hz, 2H), 7.81 (d, *J* = 8.4 Hz, 1H), 7.74 (d, *J* = 8.4 Hz, 1H), 7.69-7.62 (m, 2H), 7.62-7.58 (m, 1H), 7.58-7.52 (m, 2H), 7.51-7.46(m, 3H), 744-741 (m, 2H), 7.31 (dd, *J* = 8.7, 2.2 Hz, 3H), 4.19-4.11 (m, 2H), 3.30 (s, 5H), 2.60-2.55 (m, 1H), 2.36-2.31 (m, 1H), 2.01 (s, 1H), 1.33-1.20 (m, 3H), 1.13-1.07 (m, 1H), 1.00 (d, *J* = 9.6 Hz, 3H), 0.97-0.83 (m, 2H), 0.82-0.74 (m, 1H), 0.67 (dt, *J* = 10.4, 4.8 Hz, 1H), 0.61-0.49 (m, 2H), 0.04-0.06 (m, 1H). |
| A31 | 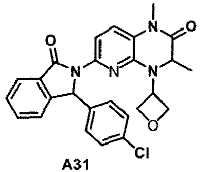 | 6-(1-(4-chlorophenyl)-3-oxoisoindolin-2-yl)-1,3-dimethyl-4-(oxacyclebutan-3-yl)-3,4-dihydropyrido[3,2-B]pyrazin-2(1H)-one | **A31-P1:** LCMS: *m*/*z* 474.7 (M+H); ¹H NMR (CD₃OD, 400 MHz)δ7.92 (d, *J* = 8.4 Hz, 2H), 7.64 (t, *J* = 7.4 Hz, 1H), 7.57 (t, *J* = 7.4 Hz, 1H), 7.44 (dd, *J* = 7.4, 5.2 Hz, 2H), 7.33 (brs, 4H), 6.56 (s, 1H), 5.16 (t, *J* = 6.8 Hz, 1H), 4.79 (d, *J* = 7.0 Hz, 2H), 4.61 (t, *J* = 6.6 Hz, 1H), 4.52-4.43 (m, 1H), 4.05 (q, *J* = 6.8 Hz, 1H), 3.35 (s, 3H), 0.84 (d, J = 6.8 Hz, 3H). |
| | | | **A31-P2:** LCMS: *m*/*z* 474.8 (M+H); ¹H NMR (CD₃OD, 400 MHz) δ7.92 (d, *J* = 8.4 Hz, 2H), 7.64 (s, 1H), 7.57 (s, 1H), 7.45 (dd, *J* = 8.0, 3.4 Hz, 2H), 7.33 (s, 4H), 6.56 (s, 1H), 5.16 (t, *J* = 6.8 Hz, 1H), 4.79 (d, *J =* 7.0 Hz, 2H), 4.61 (t, *J =* 6.6 Hz, 1H), 4.53-4.44 (m, 1H), 4.05 (q, *J=* 6.8 Hz, 1H), 3.35 (s, 3H), 0.84 (d, *J=* 6.8 Hz, 3H). |
| | | | **A31-P3:** LCMS: *m*/*z* 474.8 (M+H); ¹H NMR (CD₃OD, 400 MHz)δ7.90 (d, *J* = 7.6 Hz, 1H), 7.82 (d, *J* = 8.6 Hz, 1H), 7.64 (t, *J* = 7.4 Hz, 1H), 7.56 (t, *J* = 7.4 Hz, 1H), 7.49 (t, *J* = 8.8 Hz, 2H), 7.36-7.29 (m, 4H), 6.67 (s, 1H), 4.88-4.81 (m, 2H), 4.80-4.72 (m, 2H), 4.34 (t, *J* = 6.0 Hz, 1H), 4.17 (q, *J* = 6.8 Hz, 1H), 3.35 (d, *J* = 1.0 Hz, 3H), 1.12 (d, *J* = 6.8 Hz, 3H). |
| | | | **A31-P4:** LCMS: *m*/*z* 474.7 (M+H); ¹H NMR (CD₃OD, 400 MHz) δ7.90 (d, *J=* 7.6 Hz, 1H), 7.82 (d, *J* = 8.6 Hz, 1H), 7.67-7.59 (m, 1H), 7.55 (t, *J* = 7.4 Hz, 1H), 7.51-7.43 (m, 2H), 7.31 (dd, *J* = 5.6, 4.0 Hz, 4H), 6.65 (d, *J* = 4.2 Hz, 1H), 4.87-4.69 (m, 4H), 4.33 (t, *J* = 5.6 Hz, 1H), 4.21-4.11 (m, 1H), 3.33 (t, *J* = 2.4 Hz, 3H), 1.14-1.06 (m, 3H). |
| A32 | 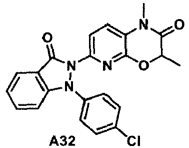 | 6-(1-(4-chlorophenyl)-3-oxo-1,3-dihydro-2H-indazol-2-yl)-1,3-dimethyl-1H-pyrido[2,3-B][1,4]oxazin-2(3H)-one | **A32-P1**: LCMS: *m*/*z* 420.8 (M+H); ¹H NMR (CD₃OD, 400 MHz): δ7.83 (d, *J* = 8.8 Hz, 1H), 7.76 (t, *J* = 2.4 Hz, 1H), 7.74 (t, *J* = 2.0 Hz, 1H), 7.70 (d, *J* = 8.4 Hz, 1H), 7.60-7.53 (m, 4H), 7.26 (t, *J* = 8.0 Hz, 1H), 7.03 (d, *J* = 7.6 Hz, 1H), 5.36 (t, *J=* 4.8 Hz, 1H), 3.39 (s, 3H), 1.55 (d, *J* = 6.8 Hz, 3H). |
| | | | **A32-P2:** LCMS: *m*/*z* 420.8 (M+H); ¹H NMR (CD₃OD, 400 MHz): δ7.83 (d, *J* = 8.8 Hz, 1H), 7.76 (t, *J =* 2.4 Hz, 1H), 7.74 (t, *J* = 2.0 Hz, 1H), 7.70 (d, *J* = 8.4 Hz, 1H), 7.60-7.53 (m, 4H), 7.26 (t, *J* = 8.0 Hz, 1H), 7.03 (d, *J* = 7.6 Hz, 1H), 5.36 (t, *J* = 4.8 Hz, 1H), 3.39 (s, 3H), 1.55 (d, *J=* 6.8 Hz, 3H). |
| | | | **A32-P3:** LCMS: *m*/*z* 420.8 (M+H); ¹H NMR (DMSO, 400 MHz): δ7.89 (d, *J* = 7.6 Hz, 1H), 7.73-7.66 (m, 3H), 7.48 (s, 4H), 7.34 (m, 2H), 4.95 (q, *J=* 6.8 Hz, 1H), 3.25 (s, 3H), 1.42 (d, *J* = 6.8 Hz, 3H). |
| | | | **A32-P4:** LCMS: *m*/*z* 420.8 (M+H); ¹H NMR (DMSO, 400 MHz): δ7.89 (d*, J* = 7.6 Hz, 1H), 7.73-7.66 (m, 3H), 7.48 (s, 4H), 7.34 (m, 2H), 4.95 (q, *J* = 6.8 Hz, 1H), 3.25 (s, 3H), 1.42 (d, *J* = 6.8 Hz, 3H). |
| A33 | 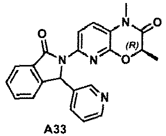 | (*R*)-1,3-dimethyl-6-(1-oxo-3-(pyridin-3-yl)isoindolin-2-yl)-1H-pyrido[2,3-B][1,4]oxazin-2(3H)-one | **A33-P1:** LCMS: *m*/*z* 387.1; ¹H NMR (CD₃OD, 400 MHz): δ8.23 (d, *J* = 8.8 Hz, 1H), 8.14 (s, 1H), 8.00 (d, *J* = 7.6 Hz, 1H), 7.72-7.59 (m, 5H), 7.44 (d, *J* = 7.2 Hz, 1H), 6.80 (s, 1H), 4.82 (q, *J* = 5.6 Hz, 1H), 1.54 (d, *J* = 6.8 Hz, 3H). |
| | | | **A33-P2:** LCMS: *m*/*z* 387.1; ¹H NMR (CD₃OD, 400 MHz): δ8.23 (d, *J* = 8.4 Hz, 1H), 8.18 (m, 1H), 8.00 (d*, J* = 7.2 Hz, 1H), 7.72-7.59 (m, 5H), 7.45 (d, *J* = 7.6 Hz, 1H), 6.79 (s, 1H), 1.47 (d, *J=* 6.8 Hz, 3H). |

Analogously to Example **A22,** the following examples were synthesized according to general synthetic procedure B:

| Ex. | Structure | Name | Analysis Data |
|---|---|---|---|
| A34 | 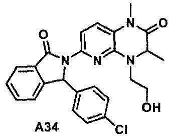 | 6-(1-(4-chlorophenyl)-3-oxoisoindolin-2-yl)-4-(2-hydroxyethyl)-1,3-dimethyl-3,4-dihydropyrido[2, 3-b]pyrazin-2(1 H)-one | **A34,** the racemate, LCMS: *m*/*z* 463.1 (M+H). |
| A35 | 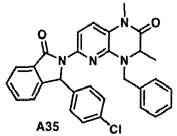 | 4-benzyl-6-(1-(4-chlorophenyl)-3-oxoisoindolin-2-yl)-1,3-dimethyl-3,4-dihydropyrido[2, 3-b]pyrazin-2(1 H)-one | **A35,** the racemate, LCMS: *m*/*z* 509.1(M+1). |
| A36 | 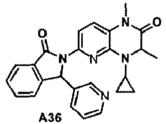 | 4-cyclopropyl-1,3-dimethyl-6-(1-oxo-3-(pyridin-3-yl)isoindolin-2-yl)-3,4-dihydropyrido[3, 2-b]pyrazin-2-(1 H)-one | **A36-P1:** LCMS: *m*/*z* 425.9 (M+H); ¹H NMR (CD₃OD, 400 MHz)δ8.73 (s, 1H), 8.42 (d, *J* = 4.2 Hz, 1H), 7.97 (t, *J* = 7.6 Hz, 2H), 7.66 (td, *J=* 7.6, 1.2 Hz, 1H), 7.63-7.58 (m, 2H), 7.44 (d, *J* = 7.6 Hz, 1H), 7.37 (d, *J* = 8.4 Hz, 1H), 7.32 (dd, *J* = 8.0, 5.0 Hz, 1H), 6.75 (s, 1H), 4.12 (q, *J* = 6.8 Hz, 1H), 3.30 (s, 3H), 2.30 (dt, *J* = 10.4, 3.4 Hz, 1H), 1.28-1.22 (m, 1H), 1.00 (d, *J* = 7.0 Hz, 3H), 0.85-0.76 (m, 1H), 0.66-0.55 (m, 2H). |
| | | | **A36-P2:** LCMS: *m*/*z* 425.9 (M+H); ¹H NMR (CD₃OD, 400 MHz) δ8.74 (s, 1H), 8.42 (d, *J* = 4.8 Hz, 1H), 7.98 (t, *J* = 7.6 Hz, 2H), 7.67 (t, *J* = 7.6 Hz, 1H), 7.63-7.60 (m, 2H), 7.44 (d, *J* = 7.6 Hz, 1H), 7.38 (d, *J* = 8.4 Hz, 1H), 7.33 (dd, *J* = 8.0, 5.0 Hz, 1H), 6.75 (s, 1H), 4.12 (q, *J* = 6.8 Hz, 1H), 3.31 (s, 3H), 2.35-2.27 (m, 1H), 1.29-1.22 (m, 1H), 1.00 (d, *J=* 6.8 Hz, 3H), 0.84-0.78 (m, 1H), 0.67-0.55 (m, 2H). |
| | | | **A36-P3:** LCMS: *m*/*z* 425.8 (M+H); ¹H NMR (CD₃OD, 300 MHz)δ8.74 (s, 1H), 8.41 (s, 1H), 7.90 (t, *J* = 6.6 Hz, 2H), 7.75-7.43 (m, 4H), 7.42-7.18 (m, 2H), 6.86 (s, 1H), 4.10 (d, *J* = 6.4 Hz, 1H), 3.26 (s, 3H), 2.65 (s, 1H), 1.27 (s, 1H), 1.18 (d, *J* = 5.4 Hz, 3H), 0.78-0.69 (m, 2H), 0.44 (d, *J* = 4.3 Hz, 1H), -0.12 (brs, 1H). |
| | | | **A36-P4:** LCMS: *m*/*z* 425.8 (M+H); ¹H NMR (CD₃OD, 300 MHz) δ8.74 (s, 1H), 8.40 (s, 1H), 7.89 (d, *J* = 8.0 Hz, 2H), 7.65-7.48 (m, 3H), 7.43-7.21 (m, 2H), 6.86 (s, 1H), 4.20-3.99 (m, 1H), 3.26 (s, 3H), 2.65 (d, *J* = 3.0 Hz, 1H), 1.35-1.08 (m, 4H), 0.77-0.69 (m, 2H), 0.44 (s, 1H), -0.11 (brs, 1H). |
| A37 | 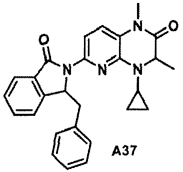 | 4-cyclopropyl-6-(1-benzyl-3-oxoisoindolin-2-yl)-1,3-dimethyl-3,4-dihydropyrido[2, 3-b]pyrazin-2(1 H)-one | **A37,** the racemate : LCMS: *m*/*z* 439.5(M+1). |
| A38 | 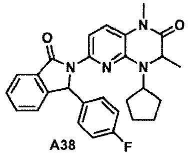 | 4-cyclopentyl-6-(1-(4-fluorophenyl)-3-oxoisoindolin-2-yl)-1,3-dimethyl-3,4-dihydropyrido[2, 3-b]pyrazin-2(1 H)-one | **A38,** the racemate, LCMS: *m*/*z* 471.1 (M+1). |
| | | | **A38P1**: LCMS: *m*/*z* 471.1 (M+1); ¹H NMR (DMSO, 400 MHz) 7.89 (d, *J* = 8.0 Hz, 1H), 7.83 (d, *J* = 8.0 Hz, 1H), 7.62 (t, *J* = 8.0 Hz, 1H), 7.53 (t, *J* = 8.0 Hz, 1H), 7.39-7.31 (m, 4H), 7.09 (m, *J* = 7.2 Hz, 2H), 6.62 (s, 1H), 4.15 (m, 2H), 3.50-3.21 (m, 3H), 2.06-1.90 (m, 2H), 1.98-1.87 (m, 2H), 1.48-1.23 (m, 2H), 1.02-0.98 (m, 2H), 0.78 (m, 3H). |
| | | | **A38P2:** LCMS: *m*/*z* 471.1 (M+1); ¹H NMR (DMSO, 400 MHz) 7.88 (d, *J* = 8.0 Hz, 1H), 7.83 (d, *J* = 8.0 Hz, 1H), 7.60 (t, *J =* 8.0 Hz, 1H), 7.53 (t, *J* = 8.0 Hz, 1H), 7.39-7.31 (m, 4H), 7.09 (m, *J* = 7.2 Hz, 2H), 6.62 (s, 1H), 4.15 (q, *J* = 6.8 Hz, 1H), 4.00(s, 1H),3.50-3.21 (m, 3H), 2.06-1.90 (m, 1H), 1.75-1.51 (m, 3H), 1.48-1.40 (m, 2H), 1.23-1.20 (m, 2H), 1.06 (m, 3H). |
| | | | **A38P3:** LCMS: *m*/*z* 471.1 (M+1); ¹H NMR (DMSO, 400 MHz) 7.88 (d, *J* = 8.0 Hz, 1H), 7.83 (d, *J* = 8.0 Hz, 1H), 7.62 (t, *J* = 8.0 Hz, 1H), 7.53 (t, *J* = 8.0 Hz, 1H), 7.39-7.31 (m, 4H), 7.09 (m, *J* = 7.2 Hz, 2H), 6.62 (s, 1H), 4.16 (m, 2H), 3.50-3.21 (m, 3H), 2.06-1.90 (m, 2H), 1.75-1.51 (m, 2H), 1.48-1.40 (m, 2H), 1.35-1.24 (m, 2H), 0.82(m, 3H). |
| | | | **A38P4:** LCMS: *m*/*z* 471.1 (M+1); ¹1H NMR (DMSO, 400 MHz) 7.86(d, *J* = 8.0 Hz, 1H), 7.83 (d, *J* = 8.0 Hz, 1H), 7.62 (t, *J* = 8.0 Hz, 1H), 7.53 (t, *J* = 8.0 Hz, 1H), 7.39-7.31 (m, 4H), 7.09 (m, *J* = 7.2 Hz, 2H), 6.62 (s, 1H), 4.15 (q, *J* = 6.8 Hz, 1H), 4.08 (s, 1H), 3.29-3.21 (m, 3H), 2.06-1.90 (m, 2H), 1.75-1.51 (m, 2H), 1.48-1.40 (m, 2H), 1.35-1.24 (m, 2H), 1.06 (m, 3H). |

Analogously to Example **A22,** the following examples were synthesized according to general synthetic procedure B, wherein Cs₂CO₃ was replaced with K₃PO₄:

| | | | |
|---|---|---|---|
| A39 | 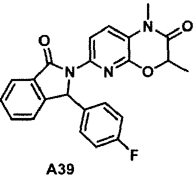 | 6-(1-(4-fluorophenyl)-3-oxoisoindolin- 2-yl)-1,3-dimethyl-1 H-pyrido[2,3-b][1,4]oxazin-2(3H)-one | **A39-P1:** LCMS: *m*/*z* 403.7 (M+H); ¹H NMR (CDCl₃, 400 MHz): δ8.12 (d*, J* = 8.4 Hz, 1H), 7.99 (d*, J* = 6.8 Hz, 1H), 7.61 - 7.50 (m, 2H), 7.33 - 7.23 (m, 4H), 6.99 - 6.93 (m, 2H), 6.60 (s, 1H), 4.86 (q, *J* = 6.8 Hz, 1H), 3.33 (s, 3H), 1.62 (d, *J* = 6.8 Hz, 3H). |
| | | | **A39-P2:** LCMS: *m*/*z* 403.9 (M+H); ¹H NMR (CDCl₃, 400 MHz): δ8.10 (d, *J* = 8.0 Hz, 1H), 7.97 (d, *J* = 4 Hz, 1H), 7.58 - 7.49 (m, 2H), 7.30 - 7.21 (m, 4H), 6.96 - 6.89 (m, 2H), 6.57 (s, 1H), 4.80 (q, *J* = 6.8 Hz, 1H), 3.30 (s, 3H), 1.56 (d, *J* = 4.0 Hz, 3H). |
| | | | **A39-P3:** LCMS: *m*/*z* 403.9 (M+H); ¹H NMR (CDCl₃, 400 MHz): δ8.10 (d, *J* = 8.0 Hz, 1H), 7.97 (d, *J* = 4 Hz, 1H), 7.58 - 7.49 (m, 2H), 7.30 - 7.21 (m, 4H), 6.96 - 6.89 (m, 2H), 6.57 (s, 1H), 4.80 (q, *J* = 6.8 Hz, 1H), 3.30 (s, 3H), 1.56 (d, *J* = 4.0 Hz, 3H). |
| | | | **A39-P4:** LCMS: *m*/*z* 403.7 (M+H); ¹H NMR (CDCl₃, 400 MHz): δ8.12 (d, *J* = 8.4 Hz, 1H), 7.99 (d, *J* = 6.8 Hz, 1H), 7.61 - 7.50 (m, 2H), 7.33 - 7.23 (m, 4H), 6.99-6.93 (m, 2H), 6.60 (s, 1H), 4.86 (q, *J* = 6.8 Hz, 1H), 3.33 (s, 3H), 1.62 (d, *J* = 6.8 Hz, 3H). |
| A40 | 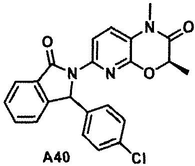 | (*3R*)-6-(1-(4-chlorophenyl)-3-oxoisoindolin-2-yl)-1,3-dimethyl-1 H-pyrido[2,3-B][1,4]oxazin-2(3H)-one | **A40-P1:** LCMS: *m*/*z* 419.8 (M+H); ¹H NMR (CD₃OD, 400 MHz): δ 8.07 (d, *J=* 8.4 Hz, 1H), 7.94 (d, *J=* 7.2 Hz, 1H), 7.65 (td, *J =* 7.2, 1.2 Hz, 2H), 7.59 (t, *J* = 2.8 Hz, 1H), 7.57 (t, *J* = 3.6 Hz, 1H), 7.36 (d, *J* = 7.6 Hz, 1H), 7.28 (s, 4H), 6.62 (s, 1H), 4.86 (q, *J =* 6.8 Hz, 1H), 1.55 (d, *J=* 6.8 Hz, 3H). |
| | | | **A40-P2:** LCMS: *m*/*z* 419.7 (M+H); ¹H NMR (CD₃OD, 400 MHz): δ 8.06 (d, *J =* 8.4 Hz, 1H), 7.94 (d, *J =* 7.2 Hz, 1H), 7.66 (td, *J* = 7.2, 0.8 Hz, 1H), 7.59 (t, *J* = 7.2 Hz, 2H), 7.37 (d, *J* = 6.8 Hz, 1H), 7.27 (s, 4H), 6.61 (s, 1H), 1.50 (d, *J =* 6.8 Hz, 3H). |

Analogously to the synthesis of Example **A1,** the following examples were synthesized according to general synthetic procedure A:

| | | | |
|---|---|---|---|
| A41 | 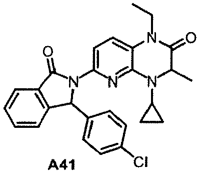 | 6-(1-(4-chlorophenyl)-3-oxoisoindolin-2-yl)-4-cyclopropyl-1-ethyl-3-methyl-3,4-dihydropyrido[3,2 -b]pyrazin-2( 1H)-one | **A41-P1:** LCMS: *m*/*z* 473.1; ¹H NMR (CD₃OD, 400 MHz):δ7.94 (s, 1H), 7.91 (s, 1H), 7.68-7.60 (m, 1H), 7.57 (t, *J* = 7.4 Hz, 1H), 7.44-7.36 (m, 2H), 7.34-7.23 (m, 4H), 6.68 (s, 1H), 4.09 (q, *J* = 7.0 Hz, 1H), 4.03-3.82 (m, 2H), 2.38-2.27 (m, 1H), 1.31 (s, 1H), 1.24-1.17 (m, 4H), 1.00 (d, *J* = 6.8 Hz, 3H), 0.82-0.75 (m, 1H), 0.61-0.55 (m, 2H). |
| | | | **A41-P2:** LCMS: *m*/*z* 473.1; ¹H NMR (CD₃OD, 400 MHz): δ7.94 (s, 1H), 7.91 (s, 1H), 7.63 (t, *J* = 7.4 Hz, 1H), 7.57 (t, *J* = 7.4 Hz, 1H), 7.39 (dd, *J* = 8.4, 3.4 Hz, 2H), 7.33-7.24 (m, 4H), 6.67 (s, 1H), 4.09 (q, *J=* 6.8 Hz, 1H), 4.02-3.95 (m, 1H), 3.92-3.85 (m, 1H), 2.38-2.28 (m, 1H), 1.31 (s, 1H), 1.24-1.16 (m, 4H), 1.00 (d, *J* = 6.8 Hz, 3H), 0.82-0.75 (m, 1H), 0.64-0.52 (m, 2H). |
| | | | **A41-P3:** LCMS: *m*/*z* 473.1 (M+H); ¹H NMR (CD₃OD, 400 MHz): δ7.91 (d, *J* = 7.6 Hz, 1H), 7.83 (d, *J* = 8.6 Hz, 1H), 7.63 (dd, *J* = 10.6,4.4 Hz, 1H), 7.56 (t, J = 7.4 Hz, 1H), 7.45 (dd, *J* = 14.0, 8.0 Hz, 2H), 7.35-7.24 (m, 4H), 6.79 (s, 1H), 4.11 (q, *J* = 6.8 Hz, 1H), 3.98 (dt, *J* = 14.2, 7.0 Hz, 1H), 3.84 (dd, *J* = 14.2, 7.0 Hz, 1H), 2.65 (dt, J = 10.4, 3.6 Hz, 1H), 1.31 (s, 1H), 1.22-1.18 (m, 6H), 0.91-0.82 (m, 1H), 0.76-0.71 (m, 1H), 0.52-0.44 (m, 1H), 0.06- -0.03 (m, 1H). |
| | | | **A41-P4:** LCMS: *m*/*z* 473.1; ¹H NMR (CD₃OD, 400 MHz): δ 7.91 (d, *J* = 7.6 Hz, 1H), 7.83 (d, *J* = 8.6 Hz, 1H), 7.63 (t, *J* = 7.6 Hz, 1H), 7.55 (t, *J* = 7.6 Hz, 1H), 7.45 (dd, *J* = 14.2, 8.0 Hz, 2H), 7.37-7.23 (m, 4H), 6.79 (s, 1H), 4.11 (q, *J* = 6.8 Hz, 1H), 3.98 (dt, *J* = 14.2, 7.2 Hz, 1H), 3.84 (dd, *J* = 14.0, 7.0 Hz, 1H), 2.70-2.60 (m, 1H), 1.31 (s, 1H), 1.22-1.18 (m, 6H), 0.89-0.84 (m, 1H), 0.76-0.67 (m, 1H), 0.49-0.45 (m, 1H), 0.05- -0.01 (m, 1H). |

### Intermediate 83: Synthesis of 6-bromo-1-deuteromethyl-3-methyl-4-cyclopropyl-3,4-dihydr opyrido[2,3-b]pyrazin-2(1H)-one (83)

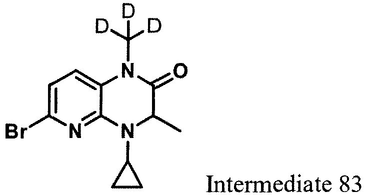

Intermediate **83** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from deuteromethyl iodide.

### Intermediate 84: Synthesis of 6-bromo-1-fluoromethyl-3-methyl-4-cyclopropyl-3,4-dihydro pyrido[2,3-b]pyrazin-2(1H)-one (84)

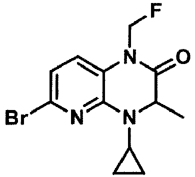

Intermediate **84** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from fluorochloromethane.

### Intermediate 91: Synthesis of 6-bromo-4-cyclopropyl-7-fluoro-1,3-dimethyl-3,4-dihydropyri do[2,3-b]pyrazin-2(1H)-one (91)

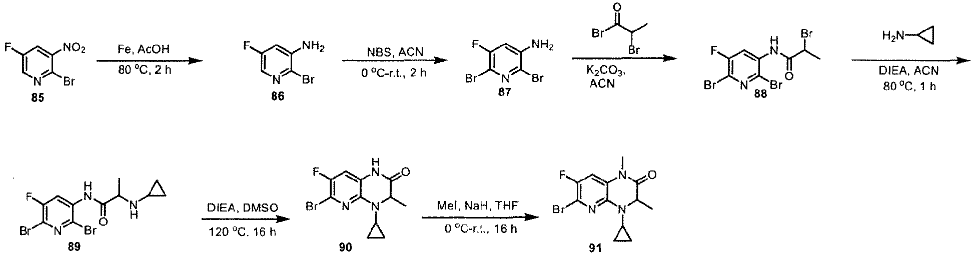

### Step 1: Synthesis of 2-bromo-5-fluoropyridin-3-amine (86)

Into a dry 100 mL round-bottom flask, compound **85** (1.0 g, 4.52 mmol), iron powder (1 g) and acetic acid (50mL) were sequentially added at RT. The mixture was heated to 80°C under stirring for 2 hours. After the completion of the reaction as monitored by LCMS, the mixture was concentrated under reduced pressure. Saturated aqueous sodium bicarbonate solution (100mL) was added to the residue, and the mixture was extracted with ethyl acetate (80mL×3). The combined organic phase was dried over anhydrous Na₂SO₄ and filtered. The filtrate was concentrated under reduced pressure. The obtained residue was purified by silica gel column chromatography (ethyl acetate : petroleum ether = 1:10) to yield 2-bromo-5-fluoropyridin-3-amine **86** (800 mg, white solid), yield: 92%. **LCMS:** *m*/*z* 190.9/192.9 (M+H).

### Step 2: Synthesis of 2,6-dibromo-5-fluoropyridin-3-amine (87)

Into a dry 100 mL round-bottom flask, compound **86** (800 mg, 4.19 mmol), acetonitrile (30mL) and *N*-bromosuccinimide (745 mg, 4.19 mmol) were sequentially added under ice bath. The mixture was stirred at 0°C for 2 hours. After the completion of the reaction as monitored by LCMS, the mixture was concentrated under reduced pressure, diluted with water (100mL), and extracted with ethyl acetate (80mL×3). The combined organic phase was dried over anhydrous Na₂SO₄ and filtered. The filtrate was concentrated under reduced pressure to yield 2,6-dibromo-5-fluoropyridin-3-amine **87** (1.2 g crude, pale yellow solid). **LCMS:** *m*/*z* 270.7 (M+H).

### Step 3: Synthesis of 2-bromo-N-(2,6-dibromo-5-fluoropyridin-3-yl)propanamide (88)

Into a dry 100 mL round-bottom flask, compound **87** (1.2 g, 4.4 mmol), acetonitrile (50mL), compound 2-bromopropionyl bromide (1.15 g, 5.3 mmol) and potassium carbonate (1.2 g, 8.69 mmol) were sequentially added under ice bath. The mixture was warmed to RT under stirring for 1 hour. After the completion of the reaction as monitored by TLC, the mixture was concentrated under reduced pressure, diluted with water (100mL), and extracted with ethyl acetate (80mL×3). The combined organic phase was dried over anhydrous Na₂SO₄ and filtered. The filtrate was concentrated under reduced pressure to yield 2-bromo-*N*-(2,6-dibromo-5-fluoropyridin-3-yl) propanamide **88** (1.8 g crude, yellow solid). **LCMS:** *m*/*z* 404.5/406.5 (M+H).

### Step 4: Synthesis of 2-(cyclopropylamino)-N-(2,6-dibromo-5-fluoropyridin-3-yl)propanamide (89)

Into a dry 50 mL sealed tube, compound **88** (1.8 g, 0.004 mol), acetonitrile (10mL), cyclopropylamine (1.0 g, 0.017 mol) and N,N-diisopropylethylamine (1.15 g, 0.009 mol) were sequentially added at RT. The mixture was warmed to 80°C under stirring for 1 hour. After the completion of the reaction as monitored by LCMS, the mixture was concentrated under reduced pressure. The obtained residue was purified by silica gel column chromatography (ethyl acetate : petroleum ether = 1:10) to yield 2-(cyclopropylamino)-N-(2,6-dibromo-5-fluoropyridin-3-yl) propanamide **89** (1.3 g, white solid), yield: 77%. **LCMS:** *m*/*z* 381.7 (M+H).

### Step 5: Synthesis of 6-bromo-4-cyclopropyl-7-fluoro-3-methyl-3,4-dihydropyrido[2,3-b]pyr azin-2(1H)-one (90)

Into a dry 100 mL round-bottom flask, compound **89** (1.0 g, 0.0026 mol), N,N-diisopropylethylamine (1.0 g, 0.0079mol) and dimethyl sulfoxide (30mL) were sequentially added at RT. The mixture was warmed to 120°C under stirring for 16 hours. After the completion of the reaction as monitored by LCMS, the mixture was diluted with water (100mL), and extracted with ethyl acetate (80mL×3). The organic phases were combined, washed with saturated brine (200mL×3), dried over anhydrous Na₂SO₄ and filtered. The filtrate was concentrated under reduced pressure. The obtained residue was purified by silica gel column chromatography (ethyl acetate : petroleum ether = 1:5) to yield 6-bromo-4-cyclopropyl-7-fluoro-3-methyl-3,4-dihydropyrido [2,3-b]pyrazin-2(1H)-one **90** (560 mg, white solid), yield: 71%. **LCMS:** *m*/*z* 299.8/301.8 (M+H).

### Step 6: Synthesis of 6-bromo-4-cyclopropyl-7-fluoro-1,3-dimethyl-3,4-dihydropyrido[2,3-b] pyrazin-2(1H)-one (91)

Into a dry 100 mL round-bottom flask, compound **90** (300 mg, 1.0 mmol), tetrahydrofuran (20mL) and sodium hydride (160 mg, 3.9 mmol) were sequentially added under ice bath. The mixture was stirred for 30 minutes under ice bath. Methyl iodide (284 mg, 2 mmol) was added, and the mixture was warmed to RT under stirring for 16 hours. After the completion of the reaction as monitored by LCMS, the mixture was quenched by addition of ice water (50mL), and extracted with ethyl acetate (50mL×3). The combined organic phase was dried over anhydrous Na₂SO₄ and filtered. The filtrate was concentrated under reduced pressure. The obtained residue was purified by silica gel column chromatography (ethyl acetate : petroleum ether = 1:7) to yield 6-bromo-4-cyclopropyl-7-fluoro-1,3-dimethyl-3,4-dihydropyrido[2,3-b]pyrazin-2(1H)-one **91** (200 mg, white solid), yield: 63.7%. **LCMS:** *m*/*z* 313.9/315.9 (M+H).

### Intermediate 92: Synthesis of 6-bromo-4-cyclopropyl-1-trifluoroethyl-3-methyl-3,4-dihydro pyrido[2,3-b]pyrazin-2(1H)-one (92)

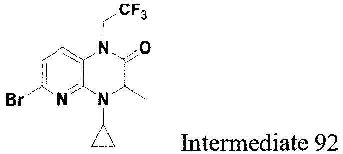

Intermediate **92** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from trifluoromethyl iodide.

### Intermediate 93: Synthesis of 6-bromo-4-(1-methylcyclopropyl)-1,3-dimethyl-3,4-dihydropy rido[2,3-b]pyrazin-2(1H)-one (93)

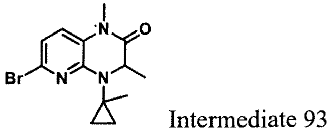

Intermediate **93** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from methylcyclopropylamine.

### Intermediate 94: Synthesis of 6-bromo-4-cyclopropyl-1-fluoroethyl-3-methyl-3,4-dihydropy rido[2,3-b]pyrazin-2(1H)-one (94)

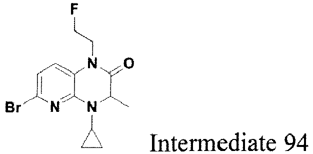

Intermediate **94** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from 1-fluoroethyl iodide.

### Intermediate 95: 6-bromo-4-cyclopropyl-1-(2,2-difluoroethyl)-3-methyl-3,4-dihydropyrido[2, 3-b]pyrazin-2(1H)-one (95)

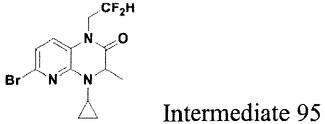

Intermediate **95** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from difluoroethyl iodide.

### Intermediate 96: 6-bromo-4-(2,2,2-trifluoroethyl-1,3-dimethyl-3,4-dihydropyrido[2,3-b]pyra zin-2(1H)-one (96)

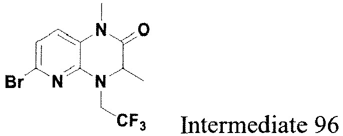

Intermediate **96** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from trifluoroethylamine.

### Intermediate 97: 6-bromo-4-(2-fluoroethyl)-1,3-dimethyl-3,4-dihydropyrido[2,3-b]pyrazin-2 (1H)-one (97)

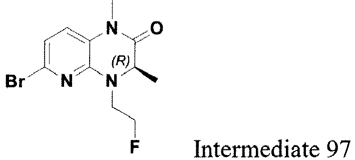

Intermediate **97** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from 2-fluoroethylamine.

### Intermediate 98: 6-bromo-4-isopropyl-1,3-dimethyl-3,4-dihydropyrido[2,3-b]pyrazin-2(1H)-one (98)

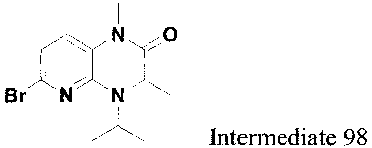

Intermediate **98** was prepared analogously to the synthetic procedure of Intermediate **5** 6-bromo-4-cyclopropyl-1,3-dimethyl-3,4-dihydropyrido[2,3-b]pyrazin-2(1H)-one **(5),** starting from isopropylamine.

### Intermediate 105: Synthesis of (R)-2-bromo-5,7,8-trimethyl-7,8-dihydropyrimidin-6(5H)-one (105)

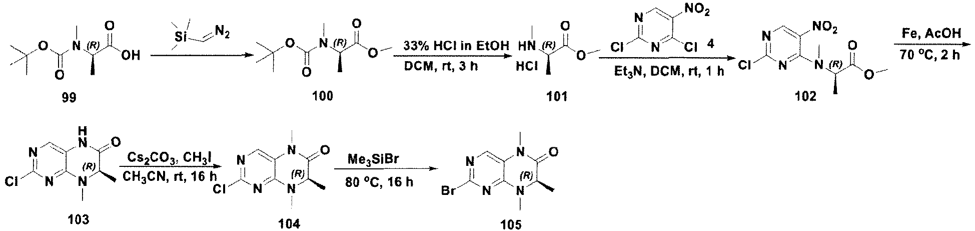

### Step 1: Synthesis of Methyl N-(tert-butoxycarbonyl)-N-methyl-D-alanine ester (100)

Into a dry 250 mL three-necked flask, compound **99** (5 g, 24.63 mmol), benzene (75mL) and anhydrous methanol (50mL) were sequentially added at RT, and (diazomethyl)trimethylsilane (2 M solution in n-hexane, 17.3mL) was slowly added dropwise. The mixture was stirred at RT for 1 hour. The reaction mixture was concentrated under reduced pressure to yield methyl N-(tert-butoxycarbonyl)-N-methyl-D-alanine ester **100** (light yellow oil, 5.3 g), which was directly used in the next reaction. **LCMS:** *m*/*z* 240.1 (M+Na).

### Step 2: Synthesis of Methyl N-methyl-D-alanine ester hydrochloride (101)

Into a dry 100 mL single-necked flask, compound **100** (5.3 g, 24.63 mmol) and dichloromethane (40mL) were sequentially added at RT, and 33% hydrochloric acid alcohol solution (20mL) was added. The mixture was stirred at RT for 3 hours. The reaction mixture was concentrated under reduced pressure to yield methyl N-methyl-D-alanine ester hydrochloride **101** (yellow oil, 3.7 g), which was directly used in the next reaction. **LCMS:** *m*/*z* 118.2 (M+H).

### Step 3: Synthesis of Methyl N-(2-chloro-5-nitropyrimidin-4-yl)-N-methyl-D-alanine ester (102)

Into a dry 100 mL single-necked flask, compound **101** (3.56 g, 23.1 mmol), triethylamine (3.12 g, 30.8 mmol) and dichloromethane (60mL) were sequentially added at RT, followed by addition of compound 2,4-dichloro-5-nitropyrimidine (3.0 g, 15.4 mmol). The mixture was stirred at RT for 1 hour. After the completion of reaction as monitored on TLC plate, the mixture was cooled to RT, 20 mL water was added, and the mixture was extracted with ethyl acetate (30mL×2). The combined organic phase was dried over anhydrous Na₂SO₄ and filtered. The filtrate was concentrated under reduced pressure. The obtained residue was purified by column chromatography (ethyl acetate : petroleum ether = 1:10) to yield methyl N-(2-chloro-5-nitropyrimidin-4-yl)-N-methyl- D-alanine ester **102** (1.7 g, yellow oil), yield: 40%. **LCMS:** *m*/*z* 275.0 (M+H).

### Step 4: Synthesis of (R)-2-chloro-7,8-dimethyl-7,8-dihydropyrimidin-6(5H)-one (103)

Into a dry 100 mL single-necked flask, compound **102** (1.65 g, 6 mmol) and acetic acid (20mL) were sequentially added at RT, and iron powder for reduction (1.68 g, 30 mmol) was added. The mixture was heated to 70°C, stirred for 2 hours, and filtered. The filter cake was washed with methanol, and the filtrate was concentrated under reduced pressure to yield (*R*)-2-chloro-7,8-dimethyl-7,8-dihydropyrimidin-6(5H)-one **103** (yellow oil, 980 mg), which was directly used in the next reaction. **LCMS:** *m*/*z* 213.0 (M+H).

### Step 5: Synthesis of (R)-2-chloro-5,7,8-trimethyl-7,8-dihydropyrimidin-6(5H)-one (104)

Into a dry 100 mL round-bottom flask, compound **103** (500 mg, 2.34 mmol) and acetonitrile (10mL) were added at RT, and cesium carbonate (1.5 g, 4.68 mmol) and methyl iodide (665 mg, 4.68 mmol) were added. The mixture was stirred at RT for 16 hours. After the completion of reaction as monitored on TLC plate, the mixture was filtered, and the filtrate was concentrated under reduced pressure. The obtained residue was purified by column chromatography (dichloromethane:methanol = 20:1) to yield (*R*)-2-chloro-5,7,8-trimethyl-7,8-dihydropyrimidin -6(5H)-one **104** (450 mg, white solid), yield: 84%. **LCMS:** *m*/*z* 226.8 (M+H).

### Step 6: Synthesis of (R)-2-bromo-5,7,8-trimethyl-7,8-dihydropyrimidin-6(5H)-one (105)

Into a dry 75 mL glass sealed tube, compound **104** (430 mg, 1.89 mmol) and acetonitrile (15mL) were added at RT, and trimethylbromosilane (1.45 g, 9.45 mmol) was added. The mixture was heated to 80°C, and stirred for 16 hours. After the completion of reaction as monitored on TLC plate, the mixture was concentrated under reduced pressure. Ethyl acetate and water were added to the residue. The phases were separated, and the aqueous phase was extracted with ethyl acetate (20mL×2). The organic phases were combined, and concentrated under reduced pressure. The obtained residue was purified by column chromatography (dichloromethane:methanol = 20:1) to yield (*R*)-2-bromo-5,7,8-trimethyl-7,8-dihydropyrimidin-6(5H)-one **105** (380 mg, yellow solid), yield: 74%. **LCMS:** *m*/*z* 271.0, 273.0 (M+H).

### Intermediate 112: Synthesis of 7-(ethylsulfinyl)-3-(pyridin-3-yl)isoindolin-1-one (112)

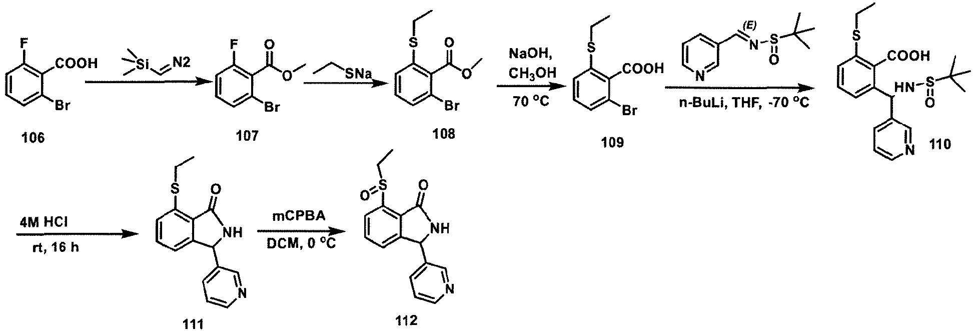

### Step 1: Synthesis of Methyl 2-bromo-6-fluorobenzoate (107)

Into a dry 250 mL three-necked flask, compound **106** (16 g, 73 mmol), benzene (50mL) and anhydrous methanol (30mL) were sequentially added at RT. The mixture was cooled to 0°C, and then (diazomethyl)trimethylsilane (2M solution in n-hexane, 54.7mL) was slowly added dropwise. The mixture was purged with nitrogen gas once, and stirred at 0°C for 1 hour. The mixture was concentrated under reduced pressure. The obtained residue was purified by column chromatography (ethyl acetate : petroleum ether = 1:10) to yield methyl 2-bromo-6-fluorobenzoate **107** (colorless oil, 16.1 g), yield: 95%.

### Step 2: Synthesis of Methyl 2-bromo-6-(ethylthio)benzoate (108)

Into a dry 250 mL single-necked flask, compound **107** (5.0 g, 21.46 mmol) and tetrahydrofuran (70mL) were sequentially added at RT, followed by addition of sodium ethanethiolate (2.16 g, 25.75 mmol). The mixture was heated to reflux under stirring for 16 hours. After the completion of reaction as monitored on TLC plate, the mixture was concentrated under reduced pressure. To the obtained residue was added 30 mL water, and the mixture was extracted with ethyl acetate (50mL×3). The organic phases were combined, dried over anhydrous Na₂SO₄ and filtered. The filtrate was concentrated under reduced pressure. The obtained residue was purified by column chromatography (ethyl acetate : petroleum ether = 1:20) to yield methyl 2-bromo-6-(ethylthio)benzoate **108** (4.6 g, colorless oil), yield: 78%. **LCMS:** *m*/*z* 296.8, 298.7 (M+Na); RT = 1.499 min (2.5 min).

### Step 3: Synthesis of 2-bromo-6-(ethylthio)benzoic acid (109)

Into a dry 250 mL single-necked flask, compound **108** (4.6 g, 16.72 mmol), methanol (30mL) and 4N aqueous sodium hydroxide solution (30mL) were sequentially added at RT. The mixture was heated to 70°C under stirring for 16 hours. After the completion of reaction as monitored on TLC plate, the organic solvent was concentrated under reduced pressure. The residue was adjusted to pH 3-4, and extracted with ethyl acetate (50mL×3). The organic phases were combined, dried over anhydrous Na₂SO₄ and filtered. The filtrate was concentrated under reduced pressure to yield 2-bromo-6-(ethylthio)benzoic acid **109** (4.1 g, yellow solid), yield: 94%. **LCMS:** *m*/*z* 258.8, 260.8 (M-H); RT = 1.271 min (2.5 min).

### Step 4: Synthesis of 2-(((tert-butylsulfinyl)amino)(pyridin-3-yl)methyl)-6-(ethylthio)benzoic acid (110)

Into a dry 100 mL three-necked flask, compound **109** (4.1 g, 15.7 mmol) and anhydrous tetrahydrofuran (50mL) were sequentially added at RT. The mixture was cooled to -70°C, and then n-butyllithium (2.4 M solution in n-hexane, 14.4 mL, 34.54 mmol) was slowly added dropwise. The mixture was stirred at -70°C for 1 hour, and compound 5 (3.62 g, 17.26 mmol) was added. The mixture was stirred at -70°C for another 2 hours. After the completion of reaction as monitored on TLC plate, saturated aqueous ammonium chloride solution (20mL) was added, and the mixture was extracted with ethyl acetate (50mL×2). The aqueous phases was combined, freeze-dried to yield 2-(((tert-butylsulfinyl)amino)(pyridin-3-yl)methyl)-6-(ethylthio)benzoic acid **110** (crude 12 g, containing inorganic salts, yellow solid), which was directly used in the next reaction. **LCMS:** *m*/*z* 392.8 (M+H); RT = 1.088 min and 1.120 (2.5 min).

### Step 5: Synthesis of 7-(ethylthio)-3-(pyridin-3-yl)isoindolin-1-one (111)

Into a dry 250 mL round-bottom flask, compound **110** (12 g, crude) and 4 M solution of chlorine hydride in methanol (100mL) were added at RT. The mixture was stirred at RT for 16 hours. After the completion of reaction as monitored on TLC plate, the mixture was concentrated under reduced pressure. The obtained residue was adjusted to pH 8-9 by adding saturated aqueous sodium bicarbonate solution, and then was extracted with ethyl acetate (30mL×2). The organic phases were combined, dried over anhydrous Na₂SO₄, and filtered. The filtrate was concentrated under reduced pressure, washed with ethyl acetate and filtered. The filter cake was dried to yield 7-(ethylthio)-3-(pyridin-3-yl)isoindolin-1-one **111** (507 mg, white solid), yield over two steps: 11%. **LCMS:** *m*/*z* 271.1 (M+H); RT = 1.187 min (2.5 min).

### Step 6: Synthesis of 7-(ethylsulfinyl)-3-(pyridin-3-yl)isoindolin-1-one (112)

Into a dry 100 mL single-necked flask, compound **111** (300 mg, 1.11 mmol) and dichloromethane (20mL) were added at RT. The mixture was cooled to 0°C, and m-chloroperoxybenzoic acid (364 mg, 2.1 mmol) was added. The mixture was stirred for 2 hours. After the completion of reaction as monitored on TLC plate, saturated aqueous sodium bicarbonate solution (20mL) was added, and the mixture was extracted with dichloromethane (30mL×2). The organic phases were combined, and concentrated under reduced pressure. The obtained residue was purified by preparative high performance liquid chromatography (mobile phase: acetonitrile and water) to yield 7-(ethylsulfinyl)-3-(pyridin-3-yl)isoindolin-1-one **112** (70 mg, white solid), yield: 22%. **LCMS:** *m*/*z* 286.9 (M+H); RT = 0.761 min and 0.811min (2.5 min).

### Intermediate 116: Synthesis of 3-(6-fluoropyridin-3-yl)isoindolin-1-one (116)

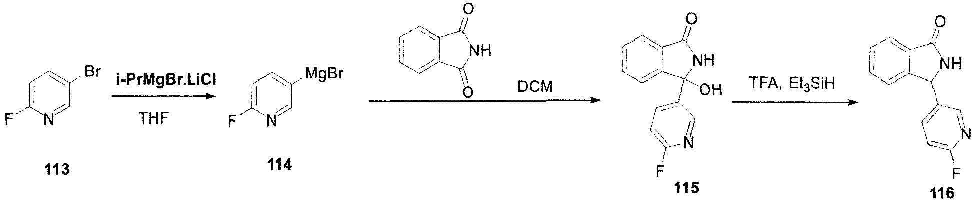

### Step 1: Synthesis of 6-fluoro-3-pyridine magnesium bromide (114)

Into a dry 500 mL three-necked flask, **113** (6 g, 34.09 mmol) and dry tetrahydrofuran (10mL) were added under ice-water bath. The mixture was purged with nitrogen gas for three times. The solution of isopropylmagnesium bromide-lithium chloride complex in tetrahydrofuran (1.3M, 39 mL, 51.13 mmol) was added. The mixture was heated to 50°C under stirring for 4 hours. The resultant solution of 6-fluoro-3-pyridine magnesium bromide **114** was directly used in the next reaction (0.7M).

### Step 2: Synthesis of 3-hydroxy-3-(6-fluoropyridin-3-yl)isoindolin-1-one (115)

Into a dry 500 mL three-necked flask, phthalimide (1.67 g, 11.36 mmol) and dry dichloromethane (50mL) were added under ice-water bath. The mixture was purged with nitrogen gas for three times, and the solution of **114** in tetrahydrofuran obtained in the previous step (0.7M, 50 mL, 34.09 mmol) was added. The mixture warmed to RT under stirring for 16 hours. After the reaction was completed, water (100mL) was added. The insoluble matter was precipitated, and filtered. The filter cake was purified by column chromatography (dichloromethane:methanol = 50:1) to yield 3-hydroxy-3-(6-fluoropyridin-3-yl)isoindolin-1-one **115** (11 g, purity: 25%, brown oil). **LCMS:** *m*/*z* 244.0, 244.9 (M+H); RT = 1.222 min (2.5 min).

### Step 3: Synthesis of 3-(6-fluoropyridin-3-yl)isoindolin-1-one (116)

Into a dry 250 mL three-necked flask, **115** (10 g, purity: 25%, 10.24 mmol), dichloromethane (50mL), trifluoroacetic acid (10mL) and triethylsilane (3.56 g, 30.73 mmol) were sequentially added at RT. The mixture was purged with nitrogen gas for three times, and was heated to 40°C under stirring for 16 hours. After the completion of the reaction, the mixture was adjusted to pH 10-11 with 5N NaOH solution, and extracted with dichloromethane (50mL×3). The combined organic phase was dried over anhydrous Na₂SO₄ and filtered. The filtrate was concentrated under reduced pressure. The obtained residue was purified by column chromatography (dichloromethane:methanol = 50:1) to yield 3-(6-fluoropyridin-3-yl)isoindolin-1-one **116** (1.68 g, light brown solid), yield: 64%. **LCMS:** *m*/*z* 228.1, 228.9 (M+H); RT = 1.264 min (2.5 min).

### Intermediate 124: Synthesis of 1-(6-methylpyridin-3-yl)-1,2-dihvdro-3H-indazol-3-one (124)

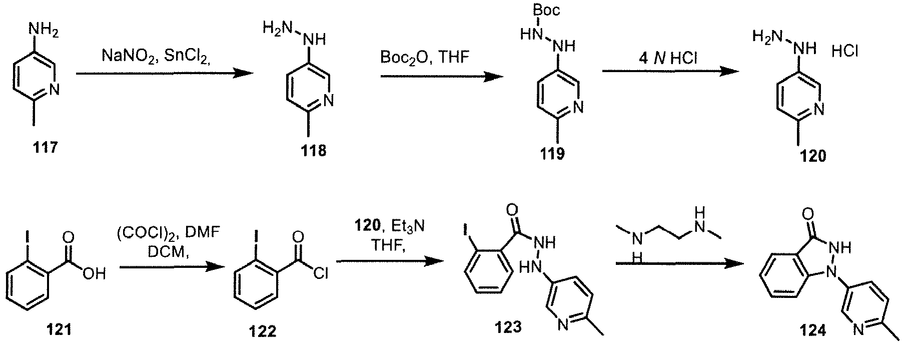

### Step 1: Synthesis of 5-hydrazino-2-methylpyridine (118)

Into a dry 100 mL round-bottom flask, compound **117** (3 g, 27.52 mmol) and concentrated hydrochloric acid (20mL) were sequentially added at RT. The mixture was cooled to 0°C. Sodium nitrite (1.96 g, 28.35 mmol) was added, and the mixture was stirred for 0.5 hours. The solution of stannous chloride dihydrate (12.44 g, 55.04 mmol) in concentrated hydrochloric acid (10mL) was added dropwise at 0°C. After the addition, the mixture was warmed to RT, and stirred for 2 hours. After the completion of reaction, the mixture was adjusted to pH 8∼9 with 5N solution of sodium hydroxide, and extracted with ethyl acetate (50mL×4). The combined organic phase was dried over anhydrous Na₂SO₄ and filtered. The filtrate was concentrated under reduced pressure to yield 5-hydrazino-2-methylpyridine **118** (1.0 g, brown liquid), crude.

### Step 2: Synthesis of Tert-butyl 2-(6-methylpyridin-3-yl)hydrazine-1-carboxylate (119)

Into a dry 100 mL round-bottom flask, compound **118** (1 g, 8.13 mmol), tetrahydrofuran (40mL) and di-tert-butyl dicarbonate (1 g, 8.13 mmol) were sequentially added at RT. The mixture was stirred at RT for 16 hours. After the completion of reaction, the mixture was concentrated under reduced pressure. The obtained residue was purified by column chromatography (dichloromethane:methanol = 10:1) to yield tert-butyl 2-(6-methylpyridin-3-yl)hydrazine-1- carboxylate **119** (1.4 g, yellow liquid), yield over two steps: 23%. **LCMS:** *m*/*z* 223.1, 224.2 (M+H); RT = 0.847 min (2.5 min).

### Step 3: Synthesis of 5-hydrazino-2-methylpyridine hydrochloride (120)

Into a dry 100 mL round-bottom flask, compound **119** (1.4 g, 6.25 mmol) and 4 N hydrogen chloride in methanol (20mL) were sequentially added at RT. The mixture was stirred at RT for 16 hours. After the completion of reaction, the mixture was concentrated under reduced pressure to yield 5-hydrazino-2-methylpyridine hydrochloride **120** (1 g, brown solid), yield: 99%. **LCMS:** *m*/*z* 123.1, 124.2 (M+H); RT = 0.180 min (2.5 min).

### Step 4: Synthesis of 2-indobenzoyl chloride (122)

Into a dry 100 mL round-bottom flask, compound **121** (775 mg, 3.13 mmol), anhydrous dichloromethane (40mL), oxalyl chloride (2.0 g, 15.65 mmol) and N,N'-dimethylformamide (0.2mL) were sequentially added at RT. The mixture was stirred at RT for 2 hours, and concentrated under reduced pressure to yield 2-indobenzoyl chloride **122** (1.2 g, white solid), yield: 99%. **LCMS:** *m*/*z* 262.0, 262.9 (M+H); RT = 1.443 min (2.5 min).

### Step 5: Synthesis of 2-indo-N'-(6-methylpyridin-3-yl)benzohydrazide (123)

Into a dry 100 mL round-bottom flask, compound **122** (775 mg, 3.13 mmol), anhydrous tetrahydrofuran (40mL), compound **120** (0.5 g, 3.13 mmol) and triethylamine (948 mg, 9.39 mmol) were sequentially added at RT. The mixture was stirred at RT for 16 hours, and concentrated under reduced pressure. The obtained residue was purified by HPLC to yield 2-indo-N'-(6-methylpyridin-3-yl)benzohydrazide **123** (185 mg, brown liquid), yield: 17%. **LCMS:** *m*/*z* 353.0, 354.0 (M+H); RT = 0.950 min (2.5 min).

### Step 6: Synthesis of 1-(6-methylpyridin-3-yl)-1,2-dihydro-3H-indazol-3-one (124)

Into a dry 50 mL round-bottom flask, compound **123** (185 mg, 0.522 mmol), dimethyl sulfoxide (4mL), N,N'-dimethylethylenediamine (93 mg, 1.044 mmol), potassium carbonate (144 mg, 1.044 mmol) and copper iodide (100 mg, 0.522 mmol) were sequentially added at RT. The mixture was purged with argon gas for three times, stirred at RT for 16 hours, and concentrated under reduced pressure. The obtained residue was purified by HPLC to yield 1-(6-methylpyridin-3-yl)-1,2-dihydro-3H-indazol-3-one **124** (50 mg, yellow liquid), yield: 42%. **LCMS:** *m*/*z* 225.1, 226.1 (M+H); RT = 1.056 min (2.5 min).

Analogously to the synthesis of Example **A1,** the following examples were synthesized according to general synthetic procedure A, with replaceing Cs₂CO₃ with potassium phosphate:

| | | | |
|---|---|---|---|
| A42 | 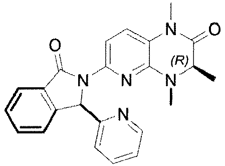 | (3*R*)-1,3,4-trimethyl-6-(1-oxo-3-(pyridin-2-yl)isoindolin-2-yl)-3,4-dihydropyrido[2,3-b]pyrazin-2-(1 H)-one (A42) | **A42-P1** |
| | | | **LCMS:** m/z 399.1, 400.0 (M+H); RT = 1.458 min (2.5 min). |
| | | | **¹H NMR (CD₃OD, 400 MHz)** δ8.48 (d, J = 4.4 Hz, 1H), 7.99 (d, J = 8.4 Hz, 1H), 7.91 (d, J = 7.6 Hz, 1H), 7.74 (t, J = 7.6 Hz, 1H), 7.57-7.53 (m, 2H), 7.47 (d, J = 7.2 Hz, 1H), 7.36-7.28 (m, 3H), 6.63 (s, 1H), 4.02 (q, J = 6.8 Hz, 1H), 3.32 (s, 3H), 2.63 (s, 3H), 0.98 (d, J = 6.8 Hz, 3H). |
| | | | **A42-P2** |
| | | | **LCMS:** m/z 399.1, 400.0 (M+H); RT = 1.508 min (2.5 min). |
| | | | **¹H NMR (CD₃OD, 400 MHz)** δ8.48 (d, J = 4.4 Hz,1H), 8.02 (d, J = 8.4 Hz, 1H), 7.93 (d, J = 7.6 Hz, 1H), 7.73 (t, J = 7.6 Hz, 1H), 7.63-7.53 (m, 2H), 7.48 (d, J = 7.2 Hz, 1H), 7.36-7.28 (m, 3H), 6.71 (s, 1H), 4.08 (q, J = 6.8 Hz, 1H), 3.32 (s, 3H), 2.67 (s, 3H), 1.20 (d, J = 6.8 Hz, 3H). |
| | 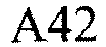 | | |
| A43 | 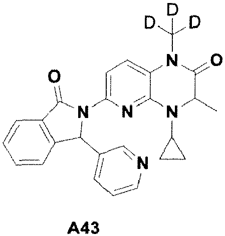 | 4-cyclopropyl-3-methyl-1-(trideuteromethyl)-6-(1-oxo-3-(pyridin-3-yl)isoindolin-2-yl)-3,4-dihydropyrido[2,3-B]pyrazin-2(1 H)-one (A43) | **A43-P1** |
| | | | **LCMS:** *m*/*z* 428.2, 428.9(M+H); RT = 1.413 min (2.5 min). |
| | | | **¹H NMR (CD₃OD, 400 MHz)**δ8.79 (s, 1H), 8.43 (s, 1H), 7.97 (t, *J* = 7.6 Hz, 3H), 7.64 (dt, *J =* 24.6, 7.0 Hz, 4H), 7.44 (d, *J* = 7.6 Hz, 1H), 7.37 (d, *J* = 8.4 Hz, 2H), 6.75 (s, 1H), 4.12 (q, *J* = 6.8 Hz, 1H), 2.37 - 2.25 (m, 1H), 1.28-1.23 (m, 1H), 1.00 (d, *J* = 6.8 Hz, 3H), 0.81-0.77 (m, 1H), 0.74-0.51 (m, 2H). |
| | | | **A43-P2** |
| | | | **LCMS:** *m*/*z* 428.2, 428.9(M+H); RT = 1.414 min (2.5 min). |
| | | | **¹H NMR (CD₃OD, 400 MHz)**δ8.76 (s, 1H), 8.53 (s, 1H), 8.13 - 7.91 (m, 2H), 7.91 - 7.74 (m, 2H), 7.66 (dt, *J* = 14.6, 6.8 Hz, 2H), 7.47-7.42 (m, 2H), 6.77 (s, 1H), 4.21 (q, *J* = 6.8 Hz, 1H), 3.32 (s, 3H), 2.73 - 2.61 (m, 1H), 1.27 (d, *J* = 6.8 Hz, 3H), 1.20 - 1.08 (m, 1H), 1.00 - 0.88 (m, 1H), 0.78-0.72 (m, 1H), 0.58-0.52 (m, 1H). |
| | | | **A43-P3** |
| | | | **LCMS:** *m*/*z* 428.2, 428.9(M+H); RT = 1.398 min (2.5 min). |
| | | | **¹H NMR (CD₃OD, 400 MHz)** δ 8.75 (s, 2H), 7.93 (dd, *J=* 14.6, 8.0 Hz, 2H), 7.69 (d, *J =* 9.8 Hz, 1H), 7.65 (d, *J =* 7.4 Hz, 1H), 7.59 (t, *J* = 7.4 Hz, 1H), 7.51 (d, *J* = 7.6 Hz, 1H), 7.42 (d, *J* = 8.0 Hz, 1H), 6.91 (s, 1H), 4.14 (q, *J* = 6.8 Hz, 1H), 2.69 (s, 1H), 1.22 (d, *J=* 6.8 Hz, 1H), 0.84-0.79 (m, 1H), 0.76-0.69 (m, 1H), 0.51-0.44 (m, 1H), 0.04 - -0.12 (m, 1H). |
| | | | **A43-P4** |
| | | | **LCMS:** *m*/*z* 428.2, 428.9(M+H); RT = 1.398 min (2.5 min). |
| | | | **¹H NMR (CD₃OD, 400 MHz)** δ 8.76 (s, 1H), 8.44 (s, 1H), 7.93 (dd, *J* = 14.4, 8.0 Hz, 2H), 7.66 (t, *J* = 9.6 Hz, 2H), 7.59 *(t, J=* 7.4 Hz, 1H), 7.51 (d, *J* = 7.6 Hz, 1H), 7.41 (d, *J* = 8.4 Hz, 1H), 7.39 - 7.31 (m, 1H), 6.90 (s, 1H), 4.14 (q, *J* = 6.8 Hz, 1H), 2.73 - 2.62 (m, 1H), 1.22 (d, *J* = 6.8 Hz, 3H), 0.84-0.79 (m, 1H), 0.77 - 0.68 (m, 1H), 0.52 - 0.43 (m, 1H), -0.02 - -0.12 (m, 1H). |
| A44 | 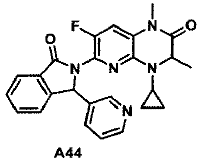 | 4-cyclopropyl-7-fluoro-1,3-dimethyl-6-(1-oxo-3-(pyridin-3-yl)isoindolin-2-yl)-3,4-dihydropyrido[2,3-b]pyrazin-2(1 H)-one (A44) | **A44-P1-1** |
| | | | **LCMS:** *m*/*z* 444.1 (M+H); RT = 1.44 min (2.5 min). |
| | | | **¹H NMR (400 MHz, CD₃OD)** δ 8.58 (s, 1H), 8.44 (d, *J=* 4.4 Hz, 1H), 7.97 (d, *J* = 7.6 Hz, 1H), 7.71-7.60(m, 3H), 7.43-7.33 (m, 2H), 7.30 (d, *J* = 10.4 Hz, 1H), 6.64 (s, 1H), 4.19 (q, *J* = 6.4 Hz, 1H), 3.25 (s, 3H), 2.68-2.62 (m, 1H), 1.25 (d, *J=* 6.8 Hz, 3H),1.22-1.13 (m, 1H), 0.83-0.75(m, 1H), 0.61-0.53(m, 1H), 0.42-0.33(m, 1H) |
| | | | **A44-P1-2** |
| | | | **LCMS:** *m*/*z* 444.0 (M+H); RT = 1.45 min (2.5 min). |
| | | | **1H NMR (400 MHz, DMSO-*d*₆)** δ 8.59 (s, 1H), 8.46 (d, *J* = 4.8 Hz, 1H), 7.90 (d, *J* = 8.0 Hz, 1H), 7.70-7.54(m, 3H), 7.46-7.40 (m, 2H), 7.36-7.31 (m, 1H), 6.65 (s, 1H), 4.12 (q, *J* = 6.8 Hz, 1H), 3.18 (s, 3H), 2.62-2.57 (m, 1H), 1.159(d, *J* = 6.8 Hz, 3H),0.87-0.80 (m, 1H), 0.72-0.65(m, 1H), 0.61-0.54(m, 1H), 0.21-0.15(m, 1H) |
| | | | **A44-P2-1** |
| | | | **LCMS:** *m*/*z* 444.0 (M+H); RT = 1.45 min (2.5 min). |
| | | | **1H NMR (400 MHz, CD₃OD)** δ 8.57 (s, 1H), 8.44 (d, *J* = 4.4 Hz, 1H), 7.97 (d, *J* = 7.6 Hz, 1H), 7.72-7.61(m, 3H), 7.42-7.34 (m, 2H), 7.30 (d, *J* = 10.0 Hz, 1H), 6.61 (s, 1H), 4.17 (q, *J* = 7.2 Hz, 1H), 3.27 (s, 3H), 2.70-2.63 (m, 1H), 1.15 (d, *J* = 6.8 Hz, 3H),1.03-0.95(m, 1H), 0.82-0.73(m, 1H), 0.59-0.52(m, 1H), 0.47-0.41(m, 1H) |
| | | | **A44-P2-2** |
| | | | **LCMS:** *m*/*z* 444.1 (M+H); RT = 1.45 min (2.5 min). |
| | | | **¹H NMR (400 MHz, CD₃OD)** δ 8.57 (s, 1H), 8.44 (d, *J* = 4.4 Hz, 1H), 7.97 (d, *J* = 7.6 Hz, 1H), 7.72-7.61(m, 3H), 7.42-7.34 (m, 2H), 7.30 (d, *J* = 10.4 Hz, 1H), 6.61 (s, 1H), 4.17 (q, *J* = 6.4 Hz, 1H), 3.27 (s, 3H), 2.70-2.63 (m, 1H), 1.15 (d, *J* = 6.8 Hz, 3H),1.03-0.95(m, 1H), 0.82-0.73(m, 1H), 0.59-0.52(m, 1H), 0.47-0.41(m, 1H) |
| A45 | 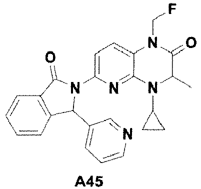 | 4-cyclopropyl-1-(fluoromethy 1)-3-methyl-6-(1-oxo-3-(pyridin-3-yl)isoindolin-2-yl)-3,4-dihydropyrido[2,3-b]pyrazin-2(1 H)-one (A45) | **A45-P1** |
| | | | **LCMS:** *m*/*z* 443.2, 443.8(M+H); RT = 1.383 min (2.5 min). |
| | | | **¹H NMR (CD₃OD, 400 MHz)** δ 8.76 (s, 1H), 8.44 (s, 1H), 7.99 (dd, *J* = 18.4, 8.0 Hz, 2H), 7.72 - 7.57 (m, 3H), 7.46 (dd, *J* = 17.6, 7.8 Hz, 2H), 7.37 - 7.26 (m, 1H), 6.75 (s, 1H), 6.28 (dd, *J* = 51.4, 8.6 Hz, 1H), 5.58 (dd, *J* = 53.8, 8.4 Hz, 1H), 4.17 (q, *J* = 6.6 Hz, 1H), 2.30 (s, 1H), 1.29 - 1.20 (m, 1H), 1.01 (d, *J* = 6.8 Hz, 3H), 0.86 - 0.75 (m, 1H), 0.69 - 0.52 (m, 2H). |
| | | | **A45-P2** |
| | | | **LCMS:** *m*/*z* 443.2, 443.8(M+H); RT = 1.381 min (2.5 min). |
| | | | **¹H NMR (CD₃OD, 400 MHz)** δ 8.76 (s, 1H), 8.44 (s, 1H), 7.99 (dd, *J* = 19.0, 7.8 Hz, 2H), 7.76 - 7.57 (m, 3H), 7.47 (dd, *J =* 18.8, 8.0 Hz, 2H), 7.40 - 7.27 (m, 1H), 6.75 (s, 1H), 6.27 (dd, *J* = 51.3, 8.4 Hz, 1H), 5.58 (dd, *J* = 54.0, 8.6 Hz, 1H), 4.17 (q, *J* = 6.6 Hz, 1H), 2.30 (s, 1H), 1.29 - 1.21 (m, 1H), 1.00 (d, *J* = 6.9 Hz, 3H), 0.81-3-0.78 (m, 1H), 0.72 - 0.50 (m, 2H). |
| | | | **A45-P3** |
| | | | **LCMS:** *m*/z 443.2, 443.8(M+H); RT = 1.386 min (2.5 min). |
| | | | **¹H NMR (CD₃OD, 400 MHz)** δ 8.77 (s, 1H), 8.44 (s, 1H), 7.96 (dd, *J* = 14.0, 8.0 Hz, 2H), 7.66 (t, *J* = 7.8 Hz, 2H), 7.62 - 7.43 (m, 3H), 7.34 (s, 1H), 6.90 (s, 1H), 6.26 (dd, *J* = 51.2, 8.4 Hz, 1H), 5.56 (dd, *J* = 54.0, 8.4 Hz, 1H), 4.19 (dd, *J* = 13.8, 6.8 Hz, 1H), 2.69 (d, J = 3.4 Hz, 1H), 1.24 (d, *J* = 6.8 Hz, 3H), 0.87-0.67 (m, 2H), 0.51-0.44 (m, 1H), -0.01 - -0.14 (m, 1H). |
| | | | **A45-P4** |
| | | | **LCMS:** *m*/*z* 443.2, 443.8(M+H); RT = 1.386 min (2.5 min). |
| | | | **¹H NMR (CD₃OD, 400 MHz)**δ 8.76 (s, 1H), 8.43 (d, *J* = 4.8 Hz, 1H), 7.97 (dd, *J* = 13.8, 8.0 Hz, 2H), 7.66 (t, *J =* 7.6 Hz, 2H), 7.62 - 7.45 (m, 2H), 7.41 - 7.28 (m, 1H), 6.90 (s, 1H), 6.27 (dd, *J=* 51.4, 8.4 Hz, 1H), 5.56 (dd, *J* = 54.2, 8.4 Hz, 1H), 4.20 (q, *J* = 6.8 Hz, 1H), 2.70 (s, 1H), 1.25 (d, *J* = 6.8 Hz, 2H), 0.87 - 0.68 (m, 2H), 0.49 (dt, *J* = 9.8, 4.6 Hz, 1H), -0.06 (dt, *J* = 13.8 4.8 Hz, 1H). |
| A46 | 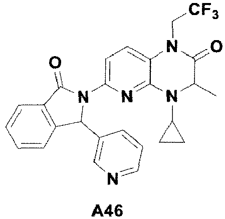 | 4-cyclopropyl-1-(2,2,2-trifluoroethyl)-3-methyl-6-(1-oxo-3-(pyridin-3-yl)isoindolin-2-yl)-3,4-dihydropyrido[2,3-b]pyrazin-2(1 H)-one (A46) | **A46-P1** |
| | | | LCMS: m/z 493.2, 493.7(M+H); RT = 1.433 min (2.5 min). |
| | | | **¹H** NMR **(CD₃OD, 400** MHz) δ 8.74 (s, 1H), 8.43 (d, *J* = 4.8 Hz, 1H), 8.01 (d, *J* = 8.6 Hz, 1H), 7.97 (d, *J* = 7.6 Hz, 1H), 7.67 (t, *J =* 7.4 Hz, 1H), 7.61 (t, *J* = 7.2 Hz, 2H), 7.51 (d, *J* = 8.8 Hz, 1H), 7.45 (d, *J =* 7.6 Hz, 1H), 7.34 (dd, *J* = 7.8, 4.8 Hz, 1H), 6.75 (s, 1H), 4.71 (ddd, *J* = 24.8, 16.0, 7.8 Hz, 2H), 4.20 (q, *J* = 6.8 Hz, 1H), 2.29 (d, *J* = 3.4 Hz, 1H), 1.30 - 1.20 (m, 1H), 1.00 (d, *J* = 6.8 Hz, 3H), 0.87 - 0.77 (m, 1H), 0.71 - 0.52 (m, 2H). |
| | | | **A46-P2** |
| | | | **LCMS:** *m*/*z* 493.2, 493.7(M+H); RT = 1.433 min (2.5 min). |
| | | | **¹H NMR (CD₃OD, 400 MHz)** δ 8.74 (s, 1H), 8.43 (d, *J* = 4.8 Hz, 1H), 8.01 (d, *J =* 8.6 Hz, 1H), 7.96 (d, *J* = 7.4 Hz, 1H), 7.67 (t, *J=* 7.5 Hz, 1H), 7.60 (t, *J =* 7.4 Hz, 2H), 7.51 (d, *J=* 8.6 Hz, 1H), 7.45 (d, *J* = 7.6 Hz, 1H), 7.33 (dd, *J* = 7.8, 5.0 Hz, 1H), 6.75 (s, 1H), 4.82 - 4.63 (m, 2H), 4.19 (q, *J =* 6.8 Hz, 1H), 2.34 - 2.25 (m, 1H), 1.29 - 1.22 (m, 1H), 1.00 (d, *J* = 6.8 Hz, 3H), 0.84-0.78 (m, 1H), 0.71 - 0.55 (m, 2H). |
| | | | **A46-P3** |
| | | | **LCMS:** *m*/*z* 493.2, 493.7(M+H); RT = 1.435 min (2.5 min). |
| | | | **¹H NMR (CD₃OD, 400 MHz)** δ 8.76 (s, 1H), 8.44 (d, *J* = 4.6 Hz, 1H), 8.02 - 7.91 (m, 2H), 7.67 (dd, *J* = 13.6, 7.2 Hz, 2H), 7.62 - 7.49 (m, 3H), 7.39 - 7.30 (m, 1H), 6.90 (s, 1H), 4.81-4.75 (m, 1H), 4.68 - 4.52 (m, 1H), 4.21 (q, *J* = 6.6 Hz, 1H), 2.71 (s, 1H), 1.24 (d, *J* = 6.6 Hz, 3H), 0.84 - 0.68 (m, 2H), 0.55 - 0.43 (m, 1H), -0.04 - -0.09 (m, 1H). |
| | | | **A46-P4** |
| | | | **LCMS:** *m*/*z* 493.2, 493.8(M+H); RT = 1.434 min (2.5 min). |
| | | | **¹H NMR (CD₃OD, 400 MHz)** δ 8.76 (s, 1H), 8.44 (d, *J* = 4.8 Hz, 1H), 7.95 (dd, *J=* 7.8, 5.4 Hz, 2H), 7.68 (dd, *J* = 13.4, 7.8 Hz, 2H), 7.63 - 7.48 (m, 3H), 7.40 - 7.32 (m, 1H), 6.90 (s, 1H), 4.84 - 4.73 (m, 1H), 4.68-4.57 (m, 1H), 4.22 (dd, *J* = 13.4, 6.8 Hz, 1H), 2.77 - 2.61 (m, 1H), 1.24 (d, *J* = 6.8 Hz, 3H), 0.85 - 0.67 (m, 2H), 0.52-0.45 (m, 1H), -0.01 - -0.10 (m, 1H). |
| A47 | | 1,3-dimethyl-4-(1-methylcyclopropyl)-6-(1-oxo-3-(pyridin-3-yl)isoindolin-2-yl)-3,4-dihydropyrido[2,3-b]pyrazin-2(1 H)-one (A47) | **A47-P1** |
| | | | **LCMS:** *m*/*z* 439.2, 439.9(M+H); RT = 1.462 min (2.5 min). |
| | | | **¹H NMR (CD₃OD, 400 MHz)** δ 8.79 (s, 1H), 8.45 (s, 1H), 8.02 (d, *J =* 8.6 Hz, 1H), 7.94 (d, *J* = 7.8 Hz, 1H), 7.67 (dd, *J =* 19.4, 7.8 Hz, 2H), 7.57 (dd, *J =* 17.8, 7.6 Hz, 1H), 7.43 (d, *J* = 8.6 Hz, 1H), 7.39 - 7.34 (m, 1H), 4.20 (q, *J* = 6.6 Hz, 1H), 3.30 (s, 2H), 1.54 (s, 2H), 1.28 (d, *J =* 6.8 Hz, 2H), 0.60 (t, *J=* 8.1 Hz, 1H), 0.35 (dd, *J* = 14.2, 7.2 Hz, 1H), 0.06-0.01 (m, 1H). |
| | 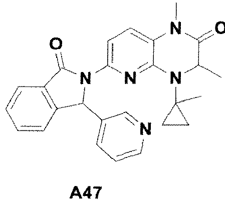 | | **A47-P2** |
| | | | **LCMS:** *m*/*z* 439.2, 439.9(M+H); RT = 1.452 min (2.5 min). |
| | | | **¹H NMR (CD₃OD, 400 MHz)**δ8.80 (s, 1H), 8.43 (s, 1H), 8.00 (d, *J =* 8.6 Hz, 1H), 7.93 (d, *J =* 7.6 Hz, 1H), 7.75 (d, *J* = 8.0 Hz, 1H), 7.66 (t, *J* = 7.4 Hz, 1H), 7.56 (dd, *J* = 17.8, 7.6 Hz, 2H), 7.42 (d, *J* = 8.6 Hz, 1H), 7.38 (d, *J* = 4.6 Hz, 1H), 6.78 (s, 1H), 4.20 (q, *J* = 6.9 Hz, 1H), 3.31 (s, 2H), 1.15 (d, *J* = 6.8 Hz, 2H), 1.06 (s, 2H), 1.04-0.98 (m, 1H), 0.88 - 0.78 (m, 1H), 0.65 (t, *J* = 8.1 Hz, 2H). |
| | | | **A47-P3** |
| | | | **LCMS:** *m*/*z* 439.2, 439.9(M+H); RT = 1.442 min (2.5 min). |
| | | | **¹H NMR (CD₃OD, 400 MHz)** δ 8.79 (s, 1H), 8.42 (d, *J* = 4.6 Hz, 1H), 8.00 (d, *J* = 8.6 Hz, 1H), 7.93 (d, *J* = 7.6 Hz, 1H), 7.75 (d, *J =* 8.0 Hz, 1H), 7.66 (t, *J=* 7.6 Hz, 1H), 7.56 (dd, *J =* 17.8, 7.6 Hz, 2H), 7.42 (d, *J =* 8.6 Hz, 1H), 7.37 (dd, *J =* 7.8, 5.0 Hz, 1H), 6.78 (s, 1H), 4.20 (q, *J* = 6.7 Hz, 1H), 3.31 (s, 3H), 1.15 (d, *J* = 6.8 Hz, 3H), 1.06 (s, 3H), 1.04 - 0.98 (m, 1H), 0.89 - 0.79 (m, 1H), 0.65 (t, *J* = 8.0 Hz, 2H). |
| | | | **A47-P4** |
| | | | **LCMS:** *m*/*z* 439.2, 439.8(M+H); RT = 1.355 min (2.5 min). |
| | | | **¹H NMR (CD₃OD, 400 MHz)** δ 8.79 (s, 1H), 8.45 (d, *J* = 4.0 Hz, 1H), 8.02 (d, *J* = 8.6 Hz, 1H), 7.94 (d, *J =* 7.8 Hz, 1H), 7.76 - 7.63 (m, 2H), 7.57 (dd, *J =* 17.4, 7.6 Hz, 2H), 7.43 (d, *J* = 8.6 Hz, 1H), 7.37 (dd, *J=* 7.8, 5.0 Hz, 1H), 6.83 (s, 1H), 4.20 (q, *J* = 6.8 Hz, 1H), 3.29 (s, 2H), 1.54 (s, 3H), 1.28 (d, *J* = 6.8 Hz, 3H), 0.60 (t, J = 8.2 Hz, 2H), 0.35 (dd, *J* = 13.6, 6.4 Hz, 1H), 0.09 - -0.03 (m, 1H). |
| A48 | 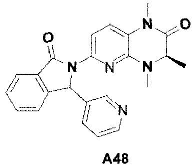 | (3*R*)-1,3,4-trimethyl-6-(1-oxo-3-(pyridin-3-yl)isoindolin-2-yl)-3,4-dihydropyrido[2,3-b]pyrazin-2-(1 H)-one (A48) | **A48-P1** |
| | | | **LCMS:** m/z 399.1, 399.9(M+H); RT = 1.277 min (2.5 min). |
| | | | **¹H NMR (CD₃OD, 400 MHz)** δ 8.71 (s, 1H), 8.43 (d, J = 4.0 Hz, 1H), 7.95 (d, J = 7.6 Hz, 1H), 7.90 (d, J = 8.4 Hz, 1H), 7.70-7.55 (m, 3H), 7.41 (d, J = 7.6 Hz, 1H), 7.34 (dd, J = 8.2, 3.0 Hz, 2H), 6.76 (s, 1H), 4.11 (q, J = 6.8 Hz, 1H), 3.31 (s, 3H), 2.83 (s, 3H), 1.22 (d, J = 6.8 Hz, 3H). |
| | | | **A48-P2** |
| | | | **LCMS:** m/z 399.1, 399.9(M+H); RT = 1.268 min (2.5 min). |
| | | | **¹H NMR (CD₃OD, 400 MHz)** δ 8.73 (s, 1H), 8.44 (s, 1H), 7.95 (d, J = 7.4 Hz, 1H), 7.86 (d, J = 8.4 Hz, 1H), 7.62 (td, J = 15.0, 7.4 Hz, 3H), 7.41 (d, J = 7.6 Hz, 1H), 7.38-7.28 (m, 2H), 6.74 (s, 1H), 4.10 (q, J = 6.8 Hz, 1H), 3.32 (s, 3H), 2.87 (s, 3H), 1.03 (d, J = 6.8 Hz, 3H). |
| A49 | 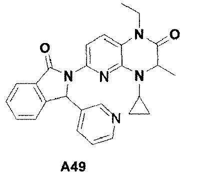 | 4-cyclopropyl-1-ethyl-3-methyl-6-(1-oxo-3-(pyridin-3-yl)isoindolin-2-yl)-3,4-dihydropyrido[2,3-b]pyrazin-2-(1H)-one (A49) | **A49-P1** |
| | | | **LCMS:** *m*/*z* 439.2, 439.9(M+H); RT = 1.459 min (2.5 min). |
| | | | **¹H NMR (CD₃OD, 400 MHz)**δ8.74 (s, 1H), 8.43 (d, *J =* 4.8 Hz, 1H), 7.97 (t, *J* = 7.6 Hz, 2H), 7.72-7.57 (m, 3H), 7.43 (dd, *J* = 14.6, 8.0 Hz, 2H), 7.37-7.30 (m, 1H), 6.75 (s, 1H), 4.09 (q, *J* = 7.0 Hz, 1H), 4.04-3.81 (m, 3H), 2.35-2.23 (m, 1H), 1.29-1.23 (m, 1H), 1.19 (t, *J* = 7.0 Hz, 3H), 0.98 (d, *J* = 6.8 Hz, 3H), 0.86-0.75 (m, 1H), 0.73-0.48 (m, 3H). |
| | | | **A49-P2** |
| | | | LCMS: m/z 439.2, 439.9(M+H); RT = 1.457 min (2.5 min). |
| | | | **¹H** NMR **(CD₃OD,** 400 **MHz)**δ8.73 (s, 1H), 8.42 (d, *J=* 4.8 Hz, 1H), 7.97 (t, *J* = 7.4 Hz, 2H), 7.73-7.55 (m, 3H), 7.43 (dd, *J* = 14.0, 8.0 Hz, 2H), 7.33 (dd, *J* = 8.0, 5.0 Hz, 1H), 6.75 (s, 1H), 4.09 (q, *J* = 6.8 Hz, 1H), 4.03-3.80 (m, 2H), 2.36-2.23 (m, 1H), 1.28-1.23 (m, 1H), 1.19 (t, *J* = 7.0 Hz, 3H), 0.98 (d, *J* = 6.8 Hz, 3H), 0.86-0.75 (m, 1H), 0.69-0.51 (m, 2H). |
| | | | **A49-P3** |
| | | | **LCMS:** *m*/*z* 439.2, 439.9(M+H); RT = 1.366 min (2.5 min). |
| | | | **¹H NMR (CD₃OD, 400 MHz)** δ 8.75 (s, 1H), 8.44 (d, *J* = 4.6 Hz, 1H), 7.93 (t, *J=* 8.8 Hz, 2H), 7.67 (dd, *J* = 14.0, 7.2 Hz, 2H), 7.59 (t, *J =* 7.4 Hz, 1H), 7.51 (d, *J =* 7.6 Hz, 1H), 7.46 (d, *J =* 8.4 Hz, 1H), 7.35 (dd, *J* = 7.8, 4.98 Hz, 1H), 6.89 (s, 1H), 4.11 (q, *J* = 6.8 Hz, 1H), 3.99 (dt, *J* = 14.2, 7.0 Hz, 1H), 3.92-3.80 (m, 1H), 2.73-2.63 (m, 1H), 1.22-1.18 (m, 6H), 0.84-0.70 (m, 2H), 0.49-0.45 (m, 1H), -0.03 - -0.09 (m, 1H). |
| | | | **A49-P4** |
| | | | **LCMS:** *m*/*z* 439.2, 439.8(M+H); RT = 1.366 min (2.5 min). |
| | | | **¹H NMR (CD₃OD, 400 MHz)** δ 8.76 (s, 1H), 8.44 (d, *J* = 4.8 Hz, 1H), 7.93 (t, *J=* 8.6 Hz, 2H), 7.67 (dd, *J* = 14.0, 7.4 Hz, 2H), 7.59 (t, *J* = 7.4 Hz, 1H), 7.51 (d, *J* = 7.6 Hz, 1H), 7.46 (d, *J* = 8.4 Hz, 1H), 7.35 (dd, *J =* 8.0, 4.8 Hz, 1H), 6.90 (s, 1H), 4.11 (q, *J* = 6.8 Hz, 1H), 3.99 (dt, *J* = 14.0, 6.8 Hz, 1H), 3.92-3.80 (m, 1H), 2.71-2.65 (m, 1H), 1.22-1.18 (m, 6H), 0.86 - 0.65 (m, 3H), 0.54 - 0.36 (m, 1H), -0.04- -0.11 (m, 1H). |
| A50 | 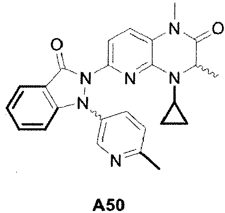 | 6-(1-(6-methyl-pyridin-3-yl)-3-oxo-1,3-dihydro-2H-indazol-2-yl)-4-cyclopropyl-1-methyl-3-methyl-3,4-dihydropyridine[2,3-b]pyrazin-2(1 H)-one (A50) | **A50-P1** |
| | | | **LCMS:** m/z 440.2, 440.8 (M+H); RT = 1.416 min (2.5 min). |
| | | | **¹H NMR (CD₃OD, 400 MHz):** δ8.86 (s, 1H), 8.16 (dd, J = 8.4 Hz, 2.8 Hz, 1H), 7.83 (d, J = 8.4 Hz, 1H), 7.59-7.45 (m, 4H), 7.26 (t, J = 8.0 Hz, 1H), 6.70 (d, J = 8.4 Hz, 1H), 4.13 (q, J = 6.4 Hz, 1H), 3.37 (s, 3H), 2.65 (s, 3H), 2.32-2.28 (m, 1H), 1.24 (d, J = 6.8 Hz, 3H), 0.54-0.48 (m, 1H), 0.43-0.37 (m, 2H), 0.18-0.12 (m, 1H). |
| | | | **A50-P2** |
| | | | **LCMS:** m/z 440.2, 441.0 (M+H); RT = 1.608 min (2.5 min). |
| | | | **¹H NMR (CD₃OD, 400 MHz):** δ8.97 (s, 1H), 8.44 (dd, J = 8.4 Hz, 2.4 Hz, 1H), 7.91 (d, J = 8.8 Hz, 1H), 7.73 (d, J = 8.8 Hz, 1H), 7.61 (m, 1H), 7.52 (d, J = 8.0 Hz, 1H), 7.47 (d, J = 8.4 Hz, 1H), 7.29 (t, J = 7.6 Hz, 1H), 6.71 (d, J = 8.4 Hz, 1H), 4.13 (q, J = 6.4 Hz, 1H), 3.37 (s, 3H), 2.65 (s, 3H), 2.32-2.28 (m, 1H), 1.24 (d, J = 6.8 Hz, 3H), 0.54-0.48 (m, 1H), 0.43-0.37 (m, 1H), 0.18-0.12 (m, 2H). |
| | | | **A50-P3** |
| | | | **LCMS:** m/z 440.2, 440.8 (M+H); RT = 1.350 min (2.5 min). |
| | | | **¹H NMR (CDCl₃, 400 MHz):** δ 8.74 (d, J = 2.4 Hz, 1H), 8.00 (d, J = 7.6 Hz, 2.4 Hz, 1H), 7.59 (m, 1H), 7.45 (s, 1H),7.32 (d, J = 7.6 Hz, 1H), 7.25 (d, J = 8.4 Hz, 1H), 7.20 (d, J = 8.4 Hz, 1H), 7.16 (d, J = 9.2 Hz, 1H), 4.13 (q, J = 6.8 Hz, 1H), 3.31 (s, 3H), 2.59 (s, 3H), 2.40 (m, 1H), 1.17 (d, J = 6.8 Hz, 3H), 0.81-0.76 (m, 1H), 0.71-0.66 (m, 1H), 0.47-0.40 (m, 1H), 0.13-0.07 (m, 1H). |
| | | | **A50-P4** |
| | | | **LCMS:** m/z 440.2, 440.8 (M+H); RT = 1.348 min (2.5 min). |
| | | | **¹H NMR (CDCl₃, 400 MHz):** δ8.74 (d, J = 2.4 Hz, 1H), 8.00 (d, J = 7.6 Hz, 2.4 Hz, 1H), 7.59 (m, 1H), 7.45 (s, 1H),7.32 (d, J = 7.6 Hz, 1H), 7.25 (d, J = 8.4 Hz, 1H), 7.20 (d, J = 8.4 Hz, 1H), 7.16 (d, J = 9.2 Hz, 1H), 4.13 (q, J = 6.8 Hz, 1H), 3.31 (s, 3H), 2.59 (s, 3H), 2.40 (m, 1H), 1.17 (d, J = 6.8 Hz, 3H), 0.81-0.76 (m, 1H), 0.71-0.66 (m, 1H), 0.47-0.40 (m, 1H), 0.13-0.07 (m, 1H). |
| A51 | 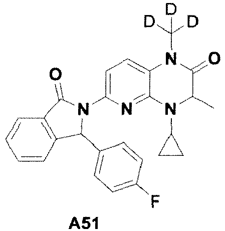 | 4-cyclopropyl-6-(1-(4-)-3-oxoisoindoli n-2-yl)-3-methyl-1-(trideuteromethyl)-3,4-dihydropyrido[2,3-B]pyrazin2(1H)-one (A51) | **A51-P1** |
| | | | **LCMS:** *m*/*z* 445.2, 445.8(M+H); RT = 1.680 min (2.5 min). |
| | | | fluorophenyl **¹H NMR (CD₃OD, 400 MHz)** δ 7.91 (dd, *J=* 10.8, 8.0 Hz, 1H), 7.63 (t, *J=* 7.4 Hz, 1H), 7.56 (t, *J* = 7.4 Hz, 1H), 7.41-7.26 (m, 2H), 7.00 (t, *J=* 8.6 Hz, 1H), 6.68 (s, 1H), 4.12 (q, *J=* 6.8 Hz, 1H), 2.40-2.36 (m, 1H), 1.34-1.21 (m, 1H), 1.02 (d, *J* = 6.8 Hz, 1H), 0.79-0.76 (m, 1H), 0.63-0.54 (m, 1H). |
| | | | **A51-P2** |
| | | | **LCMS:** *m*/*z* 445.2, 445.9(M+H); RT = 1.681 min (2.5 min). |
| | | | **¹H NMR (CD₃OD, 400 MHz**)δ7.91 (dd, *J* = 12.4, 8.0 Hz, 1H), 7.63 (t, *J* = 7.6 Hz, 0H), 7.57 (t, *J* = 7.6 Hz, 1H), 7.40-7.30 (m, 2H), 7.01 (t, *J* = 8.6 Hz, 1H), 6.69 (s, 1H), 4.12 (q, *J* = 6.8 Hz, 1H), 2.46-2.34 (m, 1H), 1.26-1.17 (m, 1H), 1.02 (d, *J=* 6.8 Hz, 1H), 0.85-0.75 (m, 1H), 0.65-0.51 (m, 1H). |
| | | | **A51-P3** |
| | | | **LCMS:** *m*/*z* 445.2, 445.9(M+H); RT = 1.657 min (2.5 min). |
| | | | **¹H NMR (CD₃OD, 400 MHz)** δ 7.91 (d, *J =* 7.6 Hz, 1H), 7.78 (d, *J=* 8.4 Hz, 1H), 7.62 (t, *J=* 7.4 Hz, 1H), 7.55 (t, *J* = 7.4 Hz, 1H), 7.45 (d, *J* = 7.4 Hz, 1H), 7.42-7.30 (m, 3H), 7.01 (t, *J* = 8.6 Hz, 2H), 6.80 (s, 1H), 4.14 (q, *J* = 6.8 Hz, 1H), 2.73-2.62 (m, 1H), 1.22 (d, *J* = 6.8 Hz, 3H), 0.99-0.86 (m, 1H), 0.80-0.68 (m, 1H), 0.59-0.42 (m, 1H), 0.09-0.02 (m, 1H). |
| | | | **A51-P4** |
| | | | **LCMS:** *m*/*z* 445.2, 445.8(M+H); RT = 1.658 min (2.5 min). |
| | | | **¹H NMR (CD₃OD, 400 MHz)** δ 7.91 (d, *J* = 7.4 Hz, 1H), 7.78 (d, *J* = 8.4 Hz, 1H), 7.63 (t, *J* = 7.4 Hz, 1H), 7.55 (t, *J* = 7.4 Hz, 1H), 7.45 (d, *J* = 7.4 Hz, 1H), 7.42-7.32 (m, 3H), 7.02 (t, *J* = 8.2 Hz, 2H), 6.80 (s, 1H), 4.14 (q, *J* = 6.8 Hz, 1H), 2.72-2.63 (m, 1H), 2.68 (s, 1H), 1.22 (d, *J* = 6.8 Hz, 3H), 0.97-0.90 (m, 1H), 0.80-0.65 (m, 1H), 0.54-0.42 (m, 1H), 0.13-0.03 (m, 1H). |
| A52 | | 4-cyclopropyl-1-(2-fluoroethyl)-3-methyl-6-(1-oxo-3-(pyridin-3-yl)isoindolin-2-yl)-3,4-dihydropyrido[2,3-b]pyrazin-2(1H)-one (A52) | **A52-P1** |
| | | | **LCMS:** m/z 458.1 (M+H); RT = 1.486 min (2.5 min). |
| | | | **¹H NMR (400 MHz, CDCl₃)** δ 8.80 (s, 1H), 8.46 (s, 1H), 8.03 - 7.94 (m, 2H), 7.55 (dd, J = 16.8, 7.6 Hz, 2H), 7.36 - 7.27 (m, 3H), 7.16 - 7.09 (m, 1H), 6.59 (s, 1H), 4.74 - 4.68 (m, 1H), 4.62 - 4.57 (m, 1H), 4.33 - 4.19 (m, 1H), 4.10 (q, J = 6.8 Hz, 1H), 4.02 - 3.90 (m, 1H), 2.32 - 2.24 (m, 1H), 1.15 - 1.07 (m, 1H), 1.03 (d, J = 6.8 Hz, 3H), 0.80 - 0.71 (m, 1H), 0.69 - 0.61 (m, 1H), 0.59 - 0.51 (m, 1H). |
| | 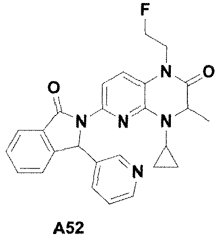 | | **A52-P2** |
| | | | **LCMS:** m/z 458.1 (M+H); RT = 1.485 min (2.5 min). |
| | | | **¹H NMR (400 MHz, CDCl₃)** δ 8.82 (s, 1H), 8.47 (s, 1H), 8.04 - 7.92 (m, 2H), 7.60 - 7.50 (m, 2H), 7.36 (d, J = 7.4 Hz, 1H), 7.32 - 7.27 (m, 2H), 7.20-7.12 (m, 1H), 6.60 (s, 1H), 4.73 - 4.69 (m, 1H), 4.62 - 4.57 (m, 1H), 4.33 - 4.19 (m, 1H), 4.10 (q, J = 6.8 Hz, 1H), 4.02 - 3.90 (m, 1H), 2.33 - 2.25 (m, 1H), 1.14 - 1.08 (m, 1H), 1.04 (d, J = 6.8 Hz, 3H), 0.80 - 0.72 (m, 1H), 0.71 - 0.62 (m, 1H), 0.59 - 0.51 (m, 1H). |
| | | | **A52-P3** |
| | | | **LCMS:** m/z 458.1 (M+H); RT = 1.478 min (2.5 min). |
| | | | **¹H NMR (400 MHz, CDCl₃)** δ 8.78 (s, 1H), 8.49 (s, 1H), 7.99 - 7.91 (m, 2H), 7.58 - 7.48 (m, 2H), 7.43 (d, J = 8.0 Hz, 1H), 7.36 - 7.31 (m, 2H), 7.21 - 7.13 (m, 1H), 6.72 (s, 1H), 4.75 - 4.69 (m, 1H), 4.64 - 4.57 (m, 1H), 4.32 - 4.20 (m, 1H), 4.12 (q, J = 6.8 Hz, 1H), 4.01 - 3.89 (m, 1H), 2.61 - 2.54 (m, 1H), 1.22 (d, J = 6.8 Hz, 3H), 0.75 - 0.64 (m, 2H), 0.47 - 0.40 (m, 1H), 0.09 - 0.02 (m, 1H). |
| | | | **A52-P4** |
| | | | **LCMS:** m/z 458.1 (M+H); RT = 1.478 min (2.5 min). |
| | | | **¹H NMR (400 MHz, CDCl₃)** δ 8.80 (s, 1H), 8.50 (s, 1H), 8.00 - 7.93 (m, 2H), 7.60 - 7.49 (m, 2H), 7.45 (d, J = 7.8 Hz, 1H), 7.39 - 7.30 (m, 2H), 7.22 - 7.16 (m, 1H), 6.74 (s, 1H), 4.78 - 4.70 (m, 1H), 4.67 - 4.59 (m, 1H), 4.33 - 4.20 (m, 1H), 4.14 (q, J = 6.8 Hz, 1H), 4.04 - 3.93 (m, 1H), 2.61 - 2.56 (m, 1H), 1.24 (d, J = 6.8 Hz, 3H), 0.77 - 0.64 (m, 2H), 0.50 - 0.40 (m, 1H), 0.11 - 0.03 (m, 1H). |
| A53 | 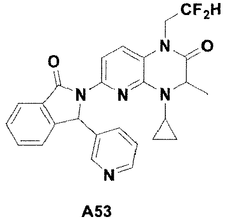 | 4-cyclopropyl-1-(2,2-difluoroethyl)-3-methyl-6-(1-oxo-3-(pyridin-3-yl)isoindolin-2-yl)-3,4-dihydropyrido[2,3-b]pyrazin-2(1 H)-one (A53) | **A53-P1** |
| | | | **LCMS:** m/z 475.6 (M+H); RT = 1.505 min (2.5 min). |
| | | | **¹H NMR (DMSO-d6, 400 MHz):** δ8.70 (s, 1H), 8.41 (d, J = 4.0 Hz, 1H), 7.90 (dd, J = 8.0, 4.0 Hz, 2H), 7.67 (t, J = 8.0 Hz, 1H), 7.60 (d, J = 8.0 Hz, 1H), 7.55 (d, J = 8.0 Hz, 1H), 7.46 (d, J = 4.0 Hz, 1H), 7.29 - 7.21 (m, 1H), 6.70 (s, 1H), 6.23 (t, J = 56 Hz, 1H), 4.44 - 4.20 (m, 3H), 4.12 - 4.04 (m, 1H), 2.12 - 2.05 (m, 1H), 1.26 - 1.18 (m, 1H), 0.88 (d, *J* = 8.0 Hz, 3H), 0.74 - 0.65 (m, 1H), 0.62 - 0.57 (m, 1H), 0.52 - 0.45 (m, 1H). |
| | | | **A53-P2** |
| | | | **LCMS:** m/z 475.6 (M+H); RT = 1.509 min (2.5 min). |
| | | | **¹H NMR (DMSO-d6, 400 MHz):** δ8.70 (s, 1H), 8.41 (d, J = 4.0 Hz, 1H), 7.90 (dd, J = 8.0, 4.0 Hz, 2H), 7.67 (t, J = 8.0 Hz, 1H), 7.60 (d, J = 8.0 Hz, 1H), 7.55 (d, J = 8.0 Hz, 1H), 7.46 (d, J = 4.0 Hz, 1H), 7.29 - 7.21 (m, 1H), 6.70 (s, 1H), 6.23 (t, J = 56 Hz, 1H), 4.44 - 4.20 (m, 3H), 4.12 - 4.04 (m, 1H), 2.12 - 2.05 (m, 1H), 1.26 - 1.18 (m, 1H), 0.88 (d, J = 8.0 Hz, 3H), 0.74 - 0.65 (m, 1H), 0.62 - 0.57 (m, 1H), 0.52 - 0.45 (m, 1H). |
| | | | **A53-P3** |
| | | | **LCMS:** m/z 475.6 (M+H); RT = 1.490 min (2.5 min). |
| | | | **¹H NMR (DMSO-d6, 400 MHz):** δ8.75 (s, 1H), 8.43 (d, J = 4.0 Hz, 1H), 7.90 - 7.84 (m, 2H), 7.69 - 7.59 (m, 3H), 7.59 - 7.50 (m, 2H), 7.31 - 7.26 (m, 1H), 6.85 (s, 1H), 6.25 (t, J = 56 Hz, 1H), 4.42 - 4.33 (m, 1H), 4.30 - 4.19 (m, 1H), 4.13 - 4.06 (m, 1H), 2.63 - 2.57 (m, 1H), 1.15 (d, J = 8.0 Hz, 3H), 0.77 - 0.69 (m, 1H), 0.68 - 0.60 (m, 1H), 0.52 - 0.44 (m, 1H), -0.21 - -0.29(m, 1H). |
| | | | **A53-P4** |
| | | | **LCMS:** m/z 475.6 (M+H); RT = 1.493 min (2.5 min). |
| | | | **¹H NMR (DMSO-d6, 400 MHz):** δ8.75 (s, 1H), 8.43 (d, J = 4.0 Hz, 1H), 7.90 - 7.84 (m, 2H), 7.69 - 7.59 (m, 3H), 7.59 - 7.50 (m, 2H), 7.31 - 7.26 (m, 1H), 6.85 (s, 1H), 6.25 (t, J = 56 Hz, 1H), 4.42 - 4.33 (m, 1H), 4.30 - 4.19 (m, 1H), 4.13 - 4.06 (m, 1H), 2.63 - 2.57 (m, 1H), 1.15 (d, J = 8.0 Hz, 3H), 0.77 - 0.69 (m, 1H), 0.68 - 0.60 (m, 1H), 0.52 - 0.44 (m, 1H), -0.21 - -0.29(m, 1H). |
| A54 | 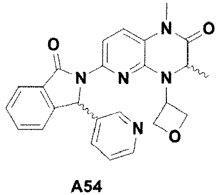 | 1,3-dimethyl-4-(oxetan-3-yl)-6-( 1-oxo-3-(pyridin-3-yl)isoindolin-2-yl)-3,4-dihydropyrido[2,3-b]pyrazin-2(1 H)-one (A54) | **A54-P1** |
| | | | **LCMS:** m/z 442.1 (M+H); RT = 1.342 min (2.5 min). |
| | | | **¹H NMR (400 MHz, CDCl₃)** δ 8.78 (s, 1H), 8.49 (s, 1H), 7.97 (t, J = 7.4 Hz, 2H), 7.62 - 7.49 (m, 2H), 7.41 (d, J = 8.0 Hz, 1H), 7.31 (d, J = 7.4 Hz, 1H), 7.18 (d, J = 8.4 Hz, 2H), 6.43 (s, 1H), 5.05 (t, J = 6.6 Hz, 1H), 4.81 - 4.72 (m, 2H), 4.63 (t, J = 6.6 Hz, 1H), 4.51 - 4.42 (m, 1H), 3.98 (d, J = 6.9 Hz, 1H), 3.32 (s, 3H), 0.86 (d, J = 6.8 Hz, 3H). |
| | | | **A54-P2** |
| | | | **LCMS:** m/z 442.1 (M+H); RT = 1.339 min (2.5 min). |
| | | | **¹H NMR (400 MHz, CDCl₃)** δ 8.78 (s, 1H), 8.49 (d, J = 4.6 Hz, 1H), 7.97 (t, J = 7.2 Hz, 2H), 7.61 - 7.50 (m, 2H), 7.40 (d, J = 8.0 Hz, 1H), 7.31 (d, J = 7.4 Hz, 1H), 7.20 - 7.15 (m, 2H), 6.43 (s, 1H), 5.05 (t, J = 6.6 Hz, 1H), 4.81-4.71 (m, 2H), 4.63 (t, J = 6.6 Hz, 1H), 4.51 - 4.43 (m, 1H), 3.98 (q, J = 6.8 Hz, 1H), 3.32 (s, 3H), 0.86 (d, J = 6.8 Hz, 3H). |
| | | | **A54-P3** |
| | | | **LCMS:** m/z 442.1 (M+H); RT = 1.335 min (2.5 min). |
| | | | **¹H NMR (400 MHz, CDCl₃)** δ 8.66 (s, 1H), 8.49 (d, J = 4.4 Hz, 1H), 7.96 (d, J = 7.4 Hz, 1H), 7.86 (d, J = 8.4 Hz, 1H), 7.59 - 7.48 (m, 3H), 7.36 (d, J = 7.6 Hz, 1H), 7.24 - 7.18 (m, 2H), 6.59 (s, 1H), 4.81 - 4.72 (m, 2H), 4.72 - 4.65 (m, 2H), 4.51 - 4.46 (m, 1H), 4.12 (q, J = 6.8 Hz, 1H), 3.32 (s, 3H), 1.14 (d, J = 6.8 Hz, 3H). |
| | | | **A54-P4** |
| | | | **LCMS:** m/z 442.1 (M+H); RT = 1.339 min (2.5 min). |
| | | | **¹H NMR (400 MHz, CDCl₃)** δ 8.65 (s, 1H), 8.50 (s, 1H), 7.96 (d, J = 7.4 Hz, 1H), 7.87 (d, J = 8.8 Hz, 1H), 7.61 - 7.48 (m, 3H), 7.36 (d, J = 7.6 Hz, 1H), 7.25 - 7.20 (m, 2H), 6.64 - 6.56 (m, 1H), 4.83 - 4.73 (m, 2H), 4.72 - 4.61 (m, 2H), 4.54 - 4.47 (m, 1H), 4.12 (q, J = 6.8 Hz, 1H), 3.32 (s, 3H), 1.14 (d, J = 6.8 Hz, 3H). |
| A55 | 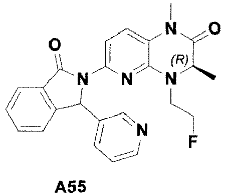 | 4-(2-fluoroethyl)-1,3-dimethyl-6-(1-oxo-3-(pyridin-3-yl)isoindolin-2-yl)-3,4-dihydropyrido[2,3-b]pyrazin-2(1H)-one (A55) | **A55-P1** |
| | | | **LCMS:** *m*/*z* 431.8 (M+H); RT = 1.235 min (2.5 min). |
| | | | **¹H NMR (400 MHz, CDCl₃)** δ 8.74 (s, 1H), 8.51 (s, 1H), 8.01 - 7.94 (m, 2H), 7.59 - 7.49 (m, 2H), 7.44 (d, *J* = 7.8 Hz, 1H), 7.29 (s, 1H), 7.22 (s, 1H), 7.14 (d, *J* = 8.4 Hz, 1H), 6.42 (s, 1H), 4.62 - 4.56 (m, 1H), 4.52 - 4.44 (m, 1H), 4.16 (q, *J* = 6.4 Hz, 1H), 3.86 - 3.70 (m, 1H), 3.31 (s, 3H), 3.27 - 3.14 (m, 1H), 1.11 (d, *J* = 6.4 Hz, 3H). |
| | | | **A55-P2** |
| | | | **LCMS:** *m*/*z* 431.9 (M+H); RT = 1.235 min (2.5 min). |
| | | | **¹H NMR (400 MHz, CDCl₃)** δ 8.72 (s, 1H), 8.51 (s, 1H), 8.05 (d, *J* = 8.4 Hz, 1H), 7.96 (d, *J* = 7.2 Hz, 1H), 7.59 - 7.49 (m, 2H), 7.40 (d, *J* = 7.8 Hz, 1H), 7.29 (d, *J* = 7.6 Hz, 1H), 7.22 - 7.11 (m, 2H), 6.47 (s, 1H), 4.35 - 4.28 (m, 0.5H), 4.23 - 4.14 (m, 1.5H), 4.05 - 3.86 (m, 2H), 3.30 (s, 3H), 3.27 - 3.15 (m, 1H), 1.26 (d, *J* = 6.8 Hz, 3H). |
| A56 | 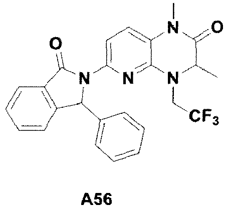 | 1,3-dimethyl-6-(1-oxo-3-phenylisoindolin-2-yl)-4-(2,2,2-trifluoroethyl)-3,4-dihydropyrido[2,3-b]pyrazin-2(1 H)-one (A56) | **A56-P1-1** |
| | | | **LCMS:** m/z 466.8 (M+H); RT = 1.519 min (2.5 min). |
| | | | **¹H NMR (DMSO-d6, 400 MHz):** δ8.00 (d, J = 8.0 Hz, 1H), 7.84 (d, J = 4.0 Hz, 1H), 7.61 (t, J = 8.0 Hz, 1H), 7.53 (t, J = 8.0 Hz, 2H), 7.36 (d, J = 8.0 Hz, 1H), 7.27 - 7.21 (m, 4H), 7.19 - 7.14 (m, 1H), 6.70 (s, 1H), 4.82 - 4.76 (m, 1H), 4.12 - 4.06 (m, 1H), 3.83 - 3.76 (m, 1H), 3.23 (s, 3H), 1.12 (d, J = 8.0 Hz, 3H). |
| | | | **A56-P1-2** |
| | | | **LCMS:** m/z 466.8 (M+H); RT = 1.517 min (2.5 min). |
| | | | **¹H NMR (DMSO-d6, 400 MHz):** δ8.00 (d, J = 8.0 Hz, 1H), 7.84 (d, J = 4.0 Hz, 1H), 7.61 (t, J = 8.0 Hz, 1H), 7.53 (t, J = 8.0 Hz, 2H), 7.36 (d, J = 8.0 Hz, 1H), 7.27 - 7.21 (m, 4H), 7.19 - 7.14 (m, 1H), 6.70 (s, 1H), 4.82 - 4.76 (m, 1H), 4.12 - 4.06 (m, 1H), 3.83 - 3.76 (m, 1H), 3.23 (s, 3H), 1.12 (d, J = 8.0 Hz, 3H). |
| | | | **A56-P2-1** |
| | | | **LCMS:** m/z 467.1 (M+H); RT = 1.685 min (2.5 min). |
| | | | **¹H NMR (DMSO-d6, 400 MHz):** δ 7.96 (d, J = 8.0 Hz, 1H), 7.86 (d, J = 8.0 Hz, 1H), 7.62 (t, J = 4.0 Hz, 1H), 7.54 (t, J = 4.0 Hz, 1H), 7.49 (d, J = 8.0 Hz, 1H), 7.41 (d, J = 8.0 Hz, 1H), 7.33 - 7.26 (m, 4H), 7.25 - 7.19 (m, 1H), 6.51 (s, 1H), 4.41 - 4.31 (m, 1H), 4.11 - 4.03 (m, 1H), 3.77 - 3.67 (m, 1H), 3.23 (s, 3H), 0.89 (d, J = 8.0 Hz, 3H). |
| | | | **A56-P2-2** |
| | | | **LCMS:** m/z 467.1 (M+H); RT = 1.693 min (2.5 min). |
| | | | **¹H NMR (DMSO-d6, 400 MHz):** δ 7.96 (d, J = 8.0 Hz, 1H), 7.86 (d, J = 8.0 Hz, 1H), 7.62 (t, J = 4.0 Hz, 1H), 7.54 (t, J = 4.0 Hz, 1H), 7.49 (d, J = 8.0 Hz, 1H), 7.41 (d, J = 8.0 Hz, 1H), 7.33 - 7.26 (m, 4H), 7.25 - 7.19 (m, 1H), 6.51 (s, 1H), 4.41 - 4.31 (m, 1H), 4.11 - 4.03 (m, 1H), 3.77 - 3.67 (m, 1H), 3.23 (s, 3H), 0.89 (d, J = 8.0 Hz, 3H). |
| A57 | 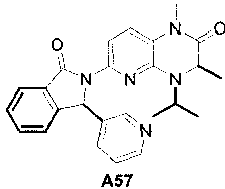 | 4-isopropyl-1,3-dimethyl-6-(1-oxo-3-(pyridin-3-yl)isoindolin-2-yl)-3,4-dihydropyrido[2,3-b]pyrazin-2(1 H)-one (A57) | **A57-P1** (7.05 mg, white solid) |
| | | | **LCMS:** m/z 427.2, 427.7 (M+H); RT = 1.335 min (2.5 min). |
| | | | **¹H NMR (CD₃OD, 400 MHz)** δ8.72 (s,1H), 8.44 (d, J = 4.4 Hz, 1H), 8.01 (d, J = 8.4 Hz, 1H), 7.95 (d, J = 7.2 Hz, 1H), 7.67-7.57 (m, 3H), 7.45-7.33 (m, 3H), 6.68 (s, 1H), 4.26 (q, J = 6.8 Hz, 1H), 4.03 (m, 1H), 3.31 (s, 3H), 1.35 (d, J = 6.8 Hz, 3H), 1.18 (d, J = 6.8 Hz, 3H), 1.03 (d, J = 6.8 Hz, 3H). |
| | | | **A57-P2** (2.57 mg, white solid) |
| | | | **LCMS:** m/z 427.2, 427.7 (M+H); RT = 1.319 min (2.5 min). |
| | | | **¹H NMR (CD₃OD, 400 MHz)** δ8.78 (s,1H), 8.46 (s, 1H), 7.95 (d, J = 7.6 Hz, 1H), 7.93 (d, J = 8.4 Hz, 1H), 7.72 (d, J = 8.0 Hz, 1H), 7.66 (t, J = 7.6 Hz, 1H), 7.59 (t, J = 7.6 Hz, 1H), 7.45 (d, J = 7.6 Hz, 1H), 7.40 (d, J = 8.4 Hz, 1H), 6.75 (s, 1H), 4.71 (m, 1H), 4.26 (q, J = 6.8 Hz, 1H), 1.25 (d, J = 6.8 Hz, 3H), 1.04 (d, J = 6.8 Hz, 3H), 0.99 (d, J = 6.8 Hz, 3H). |
| | | | **A57-P3** (1.76 mg, white solid) |
| | | | **LCMS:** m/z 427.2, 427.8 (M+H); RT = 1.338 min (2.5 min). |
| | | | **¹H NMR (CD₃OD, 400 MHz)** δ8.73 (s,1H), 8.44 (s, 1H), 8.01 (d, J = 8.4 Hz, 1H), 7.95 (d, J = 7.6 Hz, 1H), 7.67-7.57 (m, 3H), 7.45-7.35 (m, 3H) ,6.69 (s, 1H), 4.25 (q, J = 6.8 Hz, 1H), 4.04 (m, 1H), 3.31 (s, 3H), 1.35 (d, J = 6.8 Hz, 3H), 1.18 (d, J = 6.8 Hz, 3H), 1.03 (d, J = 6.8 Hz, 3H). |
| | | | **A57-P4** (4.52 mg, white solid) |
| | | | **LCMS:** m/z 427.2, 427.8 (M+H); RT = 1.321 min (2.5 min). |
| | | | **¹H NMR (CD₃OD, 400 MHz)** δ8.78 (s,1H), 8.46 (s, 1H), 7.95 (d, J = 7.6 Hz, 1H), 7.93 (d, J = 8.4 Hz, 1H), 7.72 (d, J = 8.0 Hz, 1H), 7.66 (t, J = 7.6 Hz, 1H), 7.59 (t, J = 7.6 Hz, 1H), 7.45 (d, J = 7.6 Hz, 1H), 7.40 (d, J = 8.4 Hz, 1H), 6.75 (s, 1H), 4.71 (m, 1H), 4.26 (q, J = 6.8 Hz, 1H), 1.25 (d, J = 6.8 Hz, 3H), 1.04 (d, J = 6.8 Hz, 3H), 0.99 (d, J = 6.8 Hz, 3H). |

### Intermediate 127: Synthesis of 3-(pyridin-4-yl)isoindolin-1-one (127)

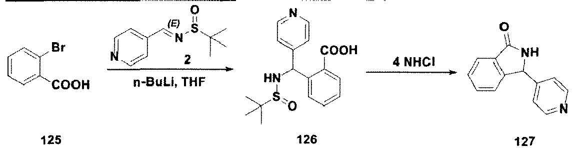

### Step 1: Synthesis of 2-(((tert-butylsulfinyl)amino)(pyridin4-yl)methyl)benzoic acid (126)

Into a dry 100 mL three-necked flask, compound **125** (1 g, 4.97 mmol) and anhydrous tetrahydrofuran (40mL) were sequentially added. The mixture was purged with argon gas for three times, and cooled to -60°C using dry ice bath. N-butyl lithium (4.5 mL, 2.4 M solution in hexane) was slowly added dropwise. The reaction system was kept at -60°C under stirring for 1 hour. The solution of compound **2** (1.04 g, 4.97 mmol) in anhydrous tetrahydrofuran was slowly added dropwise. The mixture was warmed to RT under stirring for 1 hour. After the completion of the reaction as monitored by LCMS, the reaction mixture was poured into water, and extracted with ethyl acetate (50mL×2). The aqueous phase was concentrated under reduced pressure to yield the crude2-(((tert-butylsulfinyl)amino)(pyridin-4-yl)methyl)benzoic acid **126** (2 g, white solid), yield: 121 %. **LCMS:** *m*/*z* 332.8 (M+H); RT = 1.164 min (2.5 min).

### Step 2: Synthesis of 3-(pyridin-4-yl)isoindolin-1-one (127)

Into a dry 100 mL round-bottom flask, compound **126** (2 g, 6.02 mmol) and a solution of chlorine hydride in methanol (4 mol/L, 20mL) were added. The mixture was stirred at RT for 12 hours. After the completion of the reaction, the mixture was concentrated under reduced pressure. To the residue was added water 20 mL. The mixture was adjusted to pH 8 with saturated sodium bicarbonate solution, and extracted with ethyl acetate (30mL×3). The combined organic phase was dried over anhydrous Na₂SO₄ and filtered. The filtrate was concentrated under reduced pressure. The obtained residue was purified by column chromatography (dichloromethane: methanol = 10: 1) to yield 3-(pyridin-4-yl)isoindolin-1-one **127** (0.24 g, white solid), yield: 19 %. **LCMS:** *m*/*z* 211.1 (M+H); RT = 0.928 min (2.5 min).

The following Example **A58** and **A59** were synthesized analogously to the synthesis of Example **A1.**

| | | | |
|---|---|---|---|
| A58 | 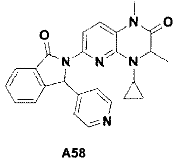 | 4-cyclopropyl-1,3-dimethyl-6-(1-oxo-3-(pyridin-4-yl)isoindolin-2-yl)-3,4-diliydropyrido[2,3-b]pyrazin-2(1H)-one **(A58)** | **A58-P1** |
| | | | **LCMS:** *m*/*z* 425.7 (M+H); RT = 1.328 min (2.5 min). **¹H NMR (DMSO-d6, 400 MHz):** δ8.43 (d, *J* = 4.0 Hz, 2H), 7.96 (d, *J* = 8.0 Hz, 1H), 7.87 (d, *J* = 8.0 Hz, 1H), 7.65 (t, *J* = 8.0 Hz, 1H), 7.57 (t, *J* = 8.0 Hz, 1H), 7.48 (d, *J* = 8.0 Hz, 1H), 7.40 (d, *J* = 8.0 Hz, 1H), 7.35 (d, *J =* 4.0 Hz, 2H), 6.64 (s, 1H), 4.04 - 3.95 (m, 1H), 3.19 (s, 3H), 1.98 - 1.92 (m, 1H), 1.19 - 1.12 (m, 1H), 0.87 (d, *J* = 8.0 Hz, 3H), 0.69 - 0.61 (m, 1H), 0.57 - 0.49 (m, 1H), 0.48 - 0.40 (m, 1H). |
| | | | **A58-P2** |
| | | | **LCMS:** *m*/*z* 425.7 (M+H); RT = 1.309 min (2.5 min). **¹H NMR (DMSO-*d₆*, 400 MHz):** δ8.47 (d, *J=* 4.0 Hz, 2H), 7.92 (d, *J=* 8.0 Hz, 1H), 7.85 (d, *J=* 8.0 Hz, 1H), 7.67 - 7.62 (m, 1H), 7.59 - 7.53 (m, 2H), 7.45 (d, *J* = 8.0 Hz, 1H), 7.40 (d, *J=* 8.0 Hz, 2H), 6.80 (s, 1H), 4.06 - 3.99 (m, 1H), 3.20 (s, 3H), 2.47 - 2.43 (m, 1H), 1.13 (d, *J* = 8.0 Hz, 3H), 0.65 - 0.56 (m, 2H), 0.46 - 0.40 (m, 1H), -0.31 - -0.38 (m, 1H). |
| | | | **A58-P3** |
| | | | **LCMS:** *m*/*z* 425.7 (M+H); RT = 1.316 min (2.5 min). **¹H NMR (DMSO-*d₆*, 400 MHz):** δ8.47 (d, *J* = 4.0 Hz, 2H), 7.92 (d, *J* = 8.0 Hz, 1H), 7.85 (d, *J* = 8.0 Hz, 1H), 7.67 - 7.62 (m, 1H), 7.59 - 7.53 (m, 2H), 7.45 (d, *J* = 8.0 Hz, 1H), 7.40 (d, *J* = 8.0 Hz, 2H), 6.80 (s, 1H), 4.06 - 3.99 (m, 1H), 3.20 (s, 3H), 2.47 - 2.43 (m, 1H), 1.13 (d, *J* = 8.0 Hz, 3H), 0.65 - 0.56 (m, 2H), 0.46 - 0.40 (m, 1H), -0.31 - -0.38 (m, 1H). |
| | | | **A58-P4** |
| | | | **LCMS:** *m*/*z* 425.6 (M+H); RT = 1.330 min (2.5 min). **¹H NMR (DMSO-*d₆*, 400 MHz):** δ8.43 (d, *J* = 4.0 Hz, 2H), 7.96 (d, *J* = 8.0 Hz, 1H), 7.87 (d, *J* = 8.0 Hz, 1H), 7.65 (t, *J* = 8.0 Hz, 1H), 7.57 (t, *J=* 8.0 Hz, 1H), 7.48 (d, *J* = 8.0 Hz, 1H), 7.40 (d, *J* = 8.0 Hz, 1H), 7.35 (d, *J* = 4.0 Hz, 2H), 6.64 (s, 1H), 4.04 - 3.95 (m, 1H), 3.19 (s, 3H), 1.98 - 1.92 (m, 1H), 1.19 - 1.12 (m, 1H), 0.87 (d, *J* = 8.0 Hz, 3H), 0.69 - 0.61 (m, 1H), 0.57 - 0.49 (m, 1H), 0.48 - 0.40 (m, 1H). |
| A59 | 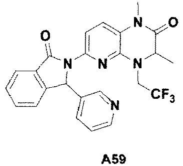 | 1,3-dimethyl-6-(1-oxo-3-(pyridin-3-yl)isoindolin-2-yl)-4-(2,2,2-trifluoroethyl)-3,4-dihydropyrido[2,3-b]pyrazin-2(1H)-one **(A59)** | **A59-P1** |
| | | | **LCMS:** *m*/*z* 468.1 (M+H); RT = 1.445 min (2.5 min). **¹H NMR (DMSO-*d₆*, 400 MHz):** δ8.72 (s, 1H), 8.46 (d, *J =* 4.0 Hz, 1H), 7.96 (d, *J* = 12.0 Hz, 1H), 7.90 (d, *J* = 8.0 Hz, 1H), 7.67 (t, *J* = 8.0 Hz, 1H), 7.62 - 7.56 (m, 2H), 7.53 (d, *J* = 8.0 Hz, 1H), 7.48 (d, *J* = 8.0 Hz, 1H), 7.32 - 7.27 (m, 1H), 6.67 (s, 1H), 4.46 - 4.36 (m, 1H), 4.13 - 4.06 (m, 1H), 3.86 - 3.77 (m, 1H), 3.26 (s, 3H), 0.92 (d, *J* = 8.0 Hz, 3H). |
| | | | **A59-P2** |
| | | | **LCMS:** *m*/*z* 468.1 (M+H); RT = 1.391 min (2.5 min). **¹H NMR (DMSO-*d₆*, 400 MHz):** δ8.63 (s, 1H), 8.40 (d, *J* = 4.0 Hz, 1H), 8.01 (d, *J* = 8.0 Hz, 1H), 7.87 (d, *J* = 8.0 Hz, 1H), 7.66 - 7.53 (m, 4H), 7.43 (d, *J* = 8.0 Hz, 1H), 7.25 - 7.21 (m, 1H), 6.82 (s, 1H), 4.82 - 4.75 (m, 1H), 4.13 - 4.08 (m, 1H), 3.84 - 3.78 (m, 1H), 3.24 (s, 3H), 1.12 (d, *J* = 8.0 Hz, 3H). |
| | | | **A59-P3** |
| | | | **LCMS:** *m*/*z* 468.1 (M+H); RT = 1.428 min (2.5 min). **¹H NMR (DMSO-*d₆*, 400 MHz):** δ8.72 (s, 1H), 8.46 (d, *J* = 4.0 Hz, 1H), 7.96 (d, *J* = 12.0 Hz, 1H), 7.90 (d, *J* = 8.0 Hz, 1H), 7.67 (t, *J* = 8.0 Hz, 1H), 7.62 - 7.56 (m, 2H), 7.53 (d, *J* = 8.0 Hz, 1H), 7.48 (d, *J* = 8.0 Hz, 1H), 7.32 - 7.27 (m, 1H), 6.67 (s, 1H), 4.46 - 4.36 (m, 1H), 4.13 - 4.06 (m, 1H), 3.86 - 3.77 (m, 1H), 3.26 (s, 3H), 0.92 (d, *J* = 8.0 Hz, 3H). |
| | | | **A59-P4** |
| | | | **LCMS:** *m*/*z* 468.1 (M+H); RT = 1.387 min (2.5 min). **¹H NMR (DMSO-*d₆*, 400 MHz):** δ8.63 (s, 1H), 8.40 (d, *J* = 4.0 Hz, 1H), 8.01 (d, *J* = 8.0 Hz, 1H), 7.87 (d, *J* = 8.0 Hz, 1H), 7.66 - 7.53 (m, 4H), 7.43 (d, *J* = 8.0 Hz, 1H), 7.25 - 7.21 (m, 1H), 6.82 (s, 1H), 4.82 - 4.75 (m, 1H), 4.13 - 4.08 (m, 1H), 3.84 - 3.78 (m, 1H), 3.24 (s, 3H), 1.12 (d, *J* = 8.0 Hz, 3H). |

### Intermediate 134: Synthesis of 7-(methylsulfinyl)-3-(pyridin-3-yl)isoindolin-1-one (134)

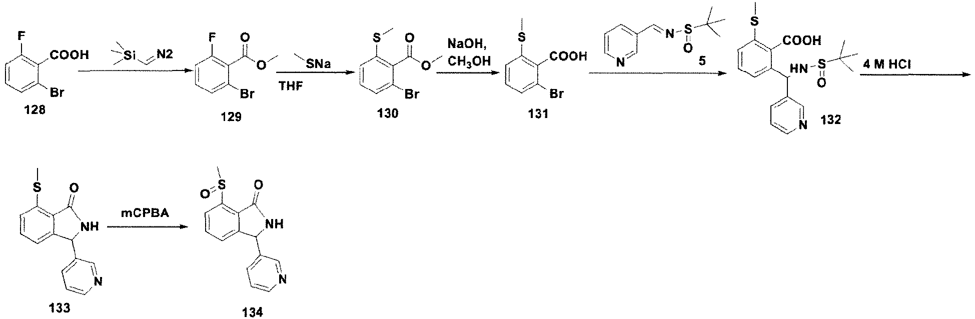

### Step 1: Synthesis of Methyl 2-bromo-6-fluorobenzoate (129)

Into a dry 250 mL three-necked flask, compound **128** (16 g, 73 mmol), benzene (50mL) and anhydrous methanol (30mL) were sequentially added at RT. The mixture cooled to 0°C. (Diazomethyl)trimethylsilane(2 M solution in n-hexane, 54.7mL) was slowly added dropwise. The mixture was stirred at 0°C for 1 hour, and concentrated under reduced pressure. The obtained residue was purified by column chromatography (ethyl acetate : petroleum ether = 1:10) to yield methyl 2-bromo-6-fluorobenzoate **129** (colorless oil, 16.1 g), yield: 95%.

### Step 2: Synthesis of Methyl 2-bromo-6-(methylthio)benzoate (130)

Into a dry 250 mL single-necked flask, compound **129** (6.0 g, 25.75 mmol) and tetrahydrofuran (60mL) were sequentially added at RT, followed by addition of sodium thiomethoxide (1.98 g, 28.32 mmol). The mixture was heated to reflux under stirring for 4 hours. After the completion of reaction as monitored on TLC plate, the mixture was concentrated under reduced pressure. 30 mL water was added to the residue, and the mixture was extracted with ethyl acetate (50mL×3). The combined organic phase was dried over anhydrous Na₂SO₄ and filtered. The filtrate was concentrated under reduced pressure. The obtained residue was purified by column chromatography (ethyl acetate : petroleum ether = 1:25) to yield methyl 2-bromo-6-(methylthio)benzoate **130** (1.87 g, colorless oil), yield: 29%.

### Step 3: Synthesis of 2-bromo-6-(methylthio)benzoic acid (131)

Into a dry 250 mL single-necked flask, compound **130** (1.87 g, 7.16 mmol), methanol (30mL) and 4N aqueous sodium hydroxide solution (30mL) were sequentially added at RT. The mixture was heated to 70°C under stirring for 16 hours. After the completion of reaction as monitored on TLC plate, the mixture was concentrated under reduced pressure, adjusted to pH 3-4 with 1 N hydrochloric acid, and extracted with ethyl acetate (30mL×2). The combined organic phase was dried over anhydrous Na₂SO₄ and filtered. The filtrate was concentrated under reduced pressure to yield 2-bromo-6-(methylthio)benzoic acid **131** (1.2 g, white solid), yield: 68%. **LCMS:** *m*/*z* 245.0, 247.0 (M-H); RT = 1.195 min (2.5 min).

### Step 4: Synthesis of 2-(((t-butylsulfinyl)amino)(pyridin-3-yl)methyl)-6-(methylthio)benzoic acid (132)

Into a dry 100 mL three-necked flask, compound **131** (1.0 g, 4.05 mmol) and anhydrous tetrahydrofuran (20mL) were sequentially added at RT. The mixture was cooled to -70°C, and n-butyllithium (2.4M solution in n-hexane, 3.7 mL, 8.91 mmol) was slowly added dropwise. The mixture was stirred at -70°C for 1 hour, and compound 3-t-butylsulfinyliminopyridine (850 mg, 4.05 mmol) was added. The mixture was stirred at -70°C for another 2 hours. After the completion of reaction as monitored on TLC plate, the saturated aqueous ammonium chloride solution (20mL) was added, and the mixture was extracted with ethyl acetate (30mL×2). The aqueous phases were combined, and lyophilized to yield 2-(((t-butylsulfinyl)amino)(pyridin-3-yl)methyl)-6-(methylthio) benzoic acid **132** (crude 4 g, containing inorganic salts, yellow solid), which was directly used in the next reaction. **LCMS:** *m*/*z* 378.7 (M+H); RT = 1.143 min and 1.176 min (2.5 min).

### Step 5: Synthesis of 7-(methylthio)-3-(pyridin-3-yl)isoindolin-1-one (133)

Into a dry 100 mL round-bottom flask, compound **132** (3.9 g, crude) and 4 M hydrogen chloride methanolic solution (40mL) were added at RT. The mixture was stirred for 16 hours. After the completion of reaction as monitored on TLC plate, the mixture was concentrated under reduced pressure. The residue was adjusted to pH 8-9 with saturated aqueous sodium bicarbonate solution, and extracted with ethyl acetate (30mL×2). The combined organic phase was dried over anhydrous Na₂SO₄ and filtered. The filtrate was concentrated under reduced pressure. The obtained residue was purified by column chromatography (dichloromethane:methanol = 20:1) to yield 7-(methylthio)-3-(pyridin-3-yl)isoindolin-1-one **133** (480 mg, yellow solid), yield over two steps: 39%. **LCMS:** *m*/*z* 256.9 (M+H); RT = 0.982 min (2.5 min).

### Step 6: Synthesis of 7-(methylsulfinyl)-3-(pyridin-3-yl)isoindolin-1-one (134)

Into a dry 100 mL single-necked flask, compound **133** (300 mg, 1.17 mmol) and dichloromethane (15mL) were added at RT. The mixture was cooled to 0°C, and m-chloroperoxybenzoic acid (384 mg, 2.22 mmol) was added. The mixture was stirred for 2 hours. After the completion of reaction as monitored on TLC plate, saturated aqueous sodium bicarbonate solution (20mL) was added, and the mixture was extracted with dichloromethane (20mL×2). The organic phases were combined and concentrated under reduced pressure to yield 7-(methylsulfinyl)-3-(pyridin-3-yl)isoindolin-1-one **134** (180 mg, white solid), yield: 56%. **LCMS:** *m*/*z* 272.8 (M+H); RT = 0.425 and 0.452 min (2.5 min).

### Example A60: Synthesis of 4-cyclopropyl-1,3-dimethyl-6-(4-(methylsulfinyl)-3-oxo-1-(pyridi n-3-yl)isoindolin-2-yl)-3,4-dihydropyrido[2,3-b]pyrazin-2(1H)-one (A60)

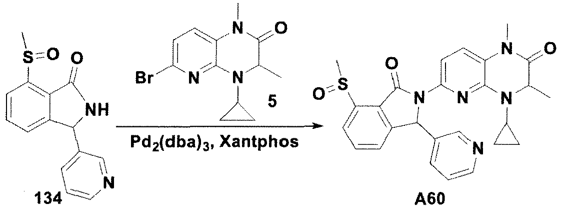

Into a dry 500 mL single-necked flask, compound **134** (6g×4 batches, 23.4 mmol×4 batches), compound **5** (6.92 g×4 batches, 23.4 mmol×4 batches), Tris(dibenzylideneacetone)dipalladium (2.1 g×4 batches, 2.3 mmol×4 batches), 4,5-bisdiphenylphosphine-9,9-dimethylxanthene (2.7 g×4 batches, 4.6 mmol×4 batches), cesium carbonate (15.2g×4 batches, 46.8 mmol×4 batches) and 1,4-dioxane (200mL×4 batches) were sequentially added at RT. Under protection of nitrogen gas, the mixture was heated to 90°C under stirring for 16 hours. The mixture was cooled to RT and filtered. The filtrate was concentrated under reduced pressure. The obtained residue was purified by column chromatography (dichloromethane:methanol = 50:1) to yield 4-cyclopropyl-1,3-dimethyl- 6-(4-(methylsulfinyl)-3-oxo-1-(pyridin-3-yl)isoindolin-2-yl)-3,4-dihydropyrido[2,3-b]pyrazin-2(1H)-one **A60** (24.8 g, yellow solid), yield 56%, and 28.9 g impure product (purity about 70%). For detailed HNMR data, see the lists.

### Example A61, A62 and A63:

### Synthesis of (3R)-1,3,4-trimethyl-6-(4-(methylthio)-3-oxo-1-(pyridin-3-yl)isoindolin-2-yl)-3,4-dihydropyrido[2,3-b]pyrazin-2(1H)-one (A61),

### Synthesis of (3R)-1,3,4-trimethyl-6-(4-(methanesulfonyl)-3-oxo-1-(pyridin-3-yl)isoindolin-2-y l)-3,4-dihydropyrido[2,3-b]pyrazin-2(1H)-one (A62),

### Synthesis of (3R)-1,3,4-trimethyl-6-(4-(methylsulfinyl)-3-oxo-1-(pyridin-3-yl)isoindolin-2-yl)-3,4-dihydropyrido[2,3-b]pyrazin-2(1H)-one (A63)

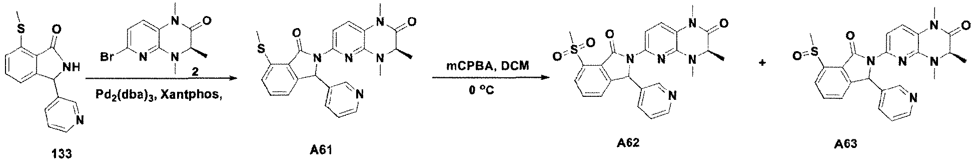

### Step 1: Synthesis of (3R)-1,3,4-trimethyl-6-(4-(methylthio)-3-oxo-1-(pyridin-3-yl)isoindolin-2-yl)-3,4-dihydropyrido[2,3-b]pyrazin-2(1H)-one (A61)

Into a dry 100 mL single-necked flask, compound **133** (300 mg, 1.17 mmol), compound **2** (316 mg, 1.17 mmol), Tris(dibenzylideneacetone)dipalladium (165 mg, 0.18 mmol), 4,5-bisdiphenylphosphine-9,9-dimethylxanthene (208 mg, 0.36 mmol), cesium carbonate (761 mg, 2.34 mmol) and 1,4-dioxane (20mL) were sequentially added at RT. Under protection of nitrogen gas, the mixture was heated to 100°C under stirring for 16 hours. The mixture was cooled to RT, and filtered. The filtrate was concentrated under reduced pressure. The obtained residue was purified by column chromatography (dichloromethane:methanol = 20:1) to yield (3*R*)-1,3,4-trimethyl-6-(4-(methylthio)-3-carbonyl-1-(pyridin-3-yl)isoindolin-2-yl)-3,4-dihydropyrido[2,3-b]pyrazin-2(1H)-one **A61** (450 mg, yellow solid), yield 86%. **LCMS:** *m*/*z* 445.8(M+H); RT = 1.278 min.

### Step 2:

### Synthesis of (3R)-1,3,4-trimethyl-6-(4-(methanesulfonyl)-3-oxo-1-(pyridin-3-yl)isoindolin-2-y l)-3,4-dihydropyrido[2,3-b]pyrazin-2(1H)-one (A62);

### Synthesis of (3R)-1,3,4-trimethyl-6-(4-(methylsulfinyl)-3-oxo-1-(pyridin-3-yl)isoindolin-2-yl)-3,4-dihydropyrido[2,3-b]pyrazin-2(1H)-one (A63)

Into a dry 100 mL single-necked flask, compound **A61** (450 mg, 1.0 mmol) and dichloromethane (20mL) were added at RT. The mixture was cooled to 0°C, followed by addition of m-chloroperoxybenzoic acid (432 mg, 2.5 mmol). The mixture was stirred at 0°C for 2 hours. After the completion of reaction as monitored on TLC plate, saturated aqueous sodium bicarbonate solution (20mL) was added, and the mixture was extracted with dichloromethane (20mL×2). The organic phase was dried over anhydrous Na₂SO₄, and concentrated under reduced pressure. The obtained residue was purified by column chromatography (dichloromethane:methanol = 20:1) to yield (3R)-1,3,4-trimethyl-6-(4-(methanesulfonyl)-3-oxo-1-(pyridin-3-yl)isoindolin-2-yl)-3,4-dihydropyrido[2,3-b]pyrazin-2(1H)-one **(A62)** (20 mg, yellow solid) and (3*R*)-1,3,4-trimethyl-6-(4-(methylsulfinyl)-3-oxo-1-(pyridin-3-yl)isoindolin-2-yl)-3,4-dihydropyrido[2,3-b]pyrazin-2(1H)-one **(A63)** (190 mg, yellow solid)
**LCMS:** *m*/*z* 477.7 (M+H); RT = 1.152 min (2.5 min). **[A62]**
**LCMS:** *m*/*z* 461.8(M+H); RT = 1.110 min (2.5 min). **[A63]**

### Step 3: Resolution of (3R)-1,3,4-trimethyl-6-(4-(methanesulfonyl)-3-oxo-1-(pyridin-3-yl)isoi ndolin-2-yl)-3,4-dihydropyrido[2,3-b]pyrazin-2(1H)-one (A62)

Chiral resolution conditions (SFC):
chiral column: AD-H
mobile phase: 70% carbon dioxide + 30% isopropanol (0.2% diethylamine)
flow rate: 40 g/min

### Step 4: Resolution of (3R)-1,3,4-trimethyl-6-(4-(methylsulfinyl)-3-oxo-1-(pyridin-3-yl)isoind olin-2-yl)-3,4-dihydropyrido[2,3-b]pyrazin-2(1H)-one (A63)

Chiral resolution conditions (SFC):
chiral column: AD-H
mobile phase: 70% carbon dioxide + 30% isopropanol (0.2% diethylamine)
flow rate: 40 g/min

The data of compounds A60 to A63 are as follows, and the following Examples A64-66 were synthesized according to examples A60, A60, A62, A63.

| | | | |
|---|---|---|---|
| A60 | 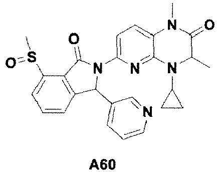 | 4-cyclopropyl-1,3-dimethyl-6-(4-(methylsulfinyl)-3-carbonyl-1-(pyridin-3-yl)isoindolin-2-yl)-3,4-dihydropyrido[2,3-b]pyrazin-2(1H)-one (A60) | **A60-P1** |
| | | | **LCMS:** *m*/*z* 488.0 (M+H); RT = 1.267 min (2.5 min). **¹H NMR (400 MHz, CDCl₃)** δ 8.18 (d, *J* = 7.8 Hz, 1H), 7.78 (dd, *J* = 16.8, 8.0 Hz, 2H), 7.45 (d, *J* = 7.6 Hz, 2H), 7.30 - 7.26 (m, 3H), 7.14 (d, *J* = 8.4 Hz, 1H), 6.84 (s, 1H), 4.14 (q, *J* = 6.8 Hz, 1H), 3.28 (s, 3H), 2.99 (s, 3H), 2.61 - 2.55 (m, 1H), 1.22 (d, *J* = 6.8 Hz, 3H), 0.82 - 0.75 (m, 1H), 0.74 - 0.67 (m, 1H), 0.50 - 0.42 (m, 1H), 0.11 - 0.04 (m, 1H). |
| | | | **A60-P2** |
| | | | **LCMS:** *m*/*z* 488.1 (M+H); RT = 1.268 min (2.5 min). **¹H NMR (400 MHz, CDCl₃)** δ 8.18 (d, *J* = 7.8 Hz, 1H), 7.78 (dd, *J* = 16.8, 8.0 Hz, 2H), 7.45 (d, *J* = 7.6 Hz, 2H), 7.29 - 7.26 (m, 3H), 7.14 (d, *J* = 8.4 Hz, 1H), 6.85 (s, 1H), 4.14 (q, *J* = 6.8 Hz, 1H), 3.28 (s, 3H), 2.99 (s, 3H), 2.62 - 2.55 (m, 1H), 1.22 (d, *J* = 6.8 Hz, 3H), 0.85 - 0.66 (m, 2H), 0.51 - 0.42 (m, 1H), 0.13 - 0.05 (m, 1H). |
| | | | **A60-P3** |
| | | | **LCMS:** *m*/*z* 488.1 (M+H); RT = 1.250 min (2.5 min). **¹H NMR (400 MHz, CDCl₃)**δ 8.87 (s, 1H), 8.62 (s, 1H), 8.19 (d, *J* = 7.2 Hz, 1H), 7.91 (d, *J* = 8.0 Hz, 1H), 7.84 - 7.76 (m, 2H), 7.54 - 7.44 (m, 2H), 7.15 (d, *J* = 8.4 Hz, 1H), 6.88 (s, 1H), 4.12 (q, *J* = 6.8 Hz, 1H), 3.29 (s, 3H), 3.09 (s, 3H), 2.58 - 2.52 (m, 1H), 1.22 (d, *J* = 6.8 Hz, 3H), 0.72 - 0.64 (m, 1H), 0.60 - 0.51 (s, 1H), 0.49 - 0.41 (m, 1H), -0.05 - -0.09 (m, 1H). |
| | | | **A60-P4** |
| | | | **LCMS:** *m*/*z* 488.1 (M+H); RT = 1.258 min (2.5 min). **¹H NMR (400 MHz, CDCl₃)** δ 8.78 (s, 1H), 8.57 (s, 1H), 8.18 (d, *J* = 7.8 Hz, 1H), 7.89 (d, *J* = 8.4 Hz, 1H), 7.80 (t, *J* = 7.6 Hz, 1H), 7.63 (s, 1H), 7.49 (d, *J* = 7.8 Hz, 1H), 7.38 - 7.31 (m, 1H), 7.14 (d, *J* = 8.6 Hz, 1H), 6.85 (s, 1H), 4.13 (q, *J* = 6.8 Hz, 1H), 3.28 (s, 3H), 3.08 (s, 3H), 2.59 - 2.52 (m, 1H), 1.22 (d, *J* = 6.8 Hz, 3H), 0.72 - 0.65 (m, 1H), 0.65 - 0.56 (m, 1H), 0.50 - 0.41 (m, 1H), -0.02 - -0.09 (m, 1H). |
| | | | **A60-P5** |
| | | | **LCMS:** *m*/*z* 487.8 (M+H); RT = 1.173 min (2.5 min). **¹H NMR (400 MHz, CDCl₃)** δ 8.20 (d, *J* = 7.6 Hz, 1H), 7.85 - 7.77 (m, 2H), 7.40 - 7.35 (m, 2H), 7.29 - 7.26 (m, 3H), 7.10 (d, *J* = 8.8 Hz, 1H), 6.72 (s, 1H), 4.13 (q, *J* = 6.8 Hz, 1H), 3.27 (s, 3H), 3.01 (s, 3H), 2.39 - 2.32 (m, 1H), 1.15 - 1.09 (m, 1H), 1.05 (d, *J* = 6.6 Hz, 3H), 0.82 - 0.74 (m, 1H), 0.66 - 0.60 (m, 1H), 0.59 - 0.53 (s, 1H). |
| | | | **A60-P6** |
| | | | **LCMS:** *m*/*z* 487.8 (M+H); RT = 1.174 min (2.5 min). **¹H NMR (400 MHz, CDCl₃)** δ 8.20 (d, *J* = 7.8 Hz, 1H), 7.85 - 7.77 (m, 2H), 7.38 (d, *J* = 7.6 Hz, 2H), 7.29 - 7.26 (m, 3H), 7.09 (d, *J* = 8.4 Hz, 1H), 6.72 (s, 1H), 4.13 (q, *J* = 6.8 Hz, 1H), 3.27 (s, 3H), 3.01 (s, 3H), 2.40 - 2.32 (m, 1H), 1.16 - 1.10 (m, 1H), 1.06 (d, *J* = 6.6 Hz, 3H), 0.83 - 0.76 (m, 1H), 0.67 - 0.60 (m, 1H), 0.59 - 0.53 (s, 1H). |
| | | | **A60-P7** |
| | | | **LCMS:** *m*/*z* 488.2 (M+H); RT = 1.279 min (2.5 min). **¹H NMR (400 MHz, CDCl₃)** δ 8.77 (s, 1H), 8.51 (d, *J* = 4.8 Hz, 1H), 8.19 (d, *J* = 7.8 Hz, 1H), 7.91 (d, *J* = 8.4 Hz, 1H), 7.79 (t, *J* = 8.0 Hz, 1H), 7.42 (t, *J* = 7.4 Hz, 2H), 7.24 - 7.18 (m, 1H), 7.08 (d, *J* = 8.6 Hz, 1H), 6.68 (s, 1H), 4.12 (q, *J* = 6.6 Hz, 1H), 3.27 (s, 3H), 3.11 (s, 3H), 2.28 - 2.21 (m, 1H), 1.05 (d, *J* = 6.8 Hz, 3H), 0.82 - 0.70 (m, 2H), 0.65 - 0.59 (m, 1H), 0.58 - 0.53 (m, 1H). |
| | | | **A60-P8** |
| | | | **LCMS:** *m*/*z* 488.0 (M+H); RT = 1.277 min (2.5 min). **¹H NMR (400 MHz, CDCl₃)** δ 8.77 (s, 1H), 8.50 (d, *J* = 4.8 Hz, 1H), 8.19 (d, *J* = 7.6 Hz, 1H), 7.91 (d, *J* = 8.4 Hz, 1H), 7.79 (t, *J* = 8.0 Hz, 1H), 7.41 (d, *J* = 7.2 Hz, 2H), 7.23 - 7.18 (m, 1H), 7.08 (d, *J* = 8.6 Hz, 1H), 6.68 (s, 1H), 4.12 (q, *J* = 6.8 Hz, 1H), 3.27 (s, 3H), 3.11 (s, 3H), 2.28 - 2.21 (m, 1H), 1.05 (d, *J* = 6.8 Hz, 3H), 0.88 - 0.71 (m, 2H), 0.66 - 0.59 (m, 1H), 0.58 - 0.51 (m, 1H). |
| A61 | 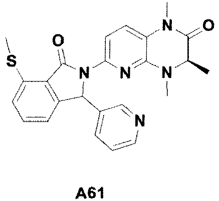 | (3*R*)-1,3,4-trimethyl-6-(4-(methylthio)-3-oxo-1-(pyridin-3-yl)isoindolin-2-yl)-3,4-dihydropyrido[2,3-b]pyrazin-2(1H)-one (A62), the racemate | **A61** |
| | | | **LCMS:** *m*/*z* 456.54.0 (M+H); |
| | | | **the racemate** |
| A62 | | (3*R*)-1,3,4-trimethyl-6-(4-(methanesulfonyl)-3-oxo-1-(pyridin-3-yl)isoindolin-2-yl)-3,4-dihydropyrido[2,3-b]pyrazin-2(1 H)-one (A62) | **A62-P1** |
| | | | **LCMS:** *m*/*z* 477.8 (M+H); RT = 1.154 min (2.5 min). **¹H NMR (400 MHz, CDCl₃)**δ 8.89 (s, 1H), 8.55 (s, 1H), 8.27 (d, *J* = 7.8 Hz, 1H), 7.87 (d, *J* = 8.4 Hz, 1H), 7.75 (t, *J* = 7.6 Hz, 1H), 7.55 (d, *J* = 7.8 Hz, 1H), 7.46 (d, *J* = 7.6 Hz, 1H), 7.26 - 7.20 (m, 1H), 7.07 (d, *J* = 8.4 Hz, 1H), 6.64 (s, 1H), 4.07 (q, *J* = 6.8 Hz, 1H), 3.64 (s, 3H), 3.30 (s, 3H), 2.84 (s, 3H), 1.10 (d, *J* = 6.8 Hz, 3H). |
| | 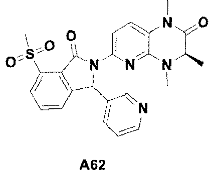 | | **A62-P2** |
| | | | **LCMS:** *m*/*z* 477.7 (M+H); RT = 1.156 min (2.5 min). **¹H NMR (400 MHz, CDCl₃)**δ 8.90 (s, 1H), 8.57 (s, 1H), 8.29 (d, *J* = 7.6 Hz, 1H), 7.95 (d, *J* = 8.4 Hz, 1H), 7.76 (t, *J* = 7.6 Hz, 1H), 7.59 (d, *J* = 7.6 Hz, 1H), 7.51 (d, *J* = 7.8 Hz, 1H), 7.31 - 7.29 (m, 1H),7.10 (d, *J=* 8.4 Hz, 1H), 6.70 (s, 1H), 4.09 (q, *J* = 6.8 Hz, 1H), 3.66 (s, 3H), 3.31 (d, *J=* 2.7 Hz, 3H), 2.81 (s, 3H), 1.26 (d, *J* = 6.8 Hz, 3H). |
| A63 | 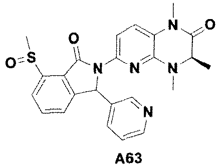 | (3*R*)-1,3,4-trimethyl-6-(4-(methylsulfinyl)-3-oxo-1-(pyridin-3-yl)isoindolin-2-yl)-3,4-dihydropyrido[2,3-b]pyrazin-2(1H)-one (A63) | **A63-P1** |
| | | | **LCMS:** *m*/*z* 461.8 (M+H); RT = 1.114 min (2.5 min). **¹H NMR (400 MHz, CDCl₃)** δ8.76 (s, 1H), 8.54 (s, 1H), 8.18 (d, *J* = 8.0 Hz, 1H), 7.87 (d, *J* = 8.4 Hz, 1H), 7.78 (t, *J* = 7.6 Hz, 1H), 7.51 (d, *J* = 7.6 Hz, 1H), 7.39 (d, *J=* 7.6 Hz, 1H), 7.29 - 7.27 (m, 1H), 7.07 (d, *J* = 8.4 Hz, 1H), 6.66 (s, 1H), 4.06 (q, *J=* 6.8 Hz, 1H), 3.29 (s, 3H), 3.09 (s, 3H), 2.74 (s, 3H), 1.24 (d, *J* = 6.8 Hz, 3H). |
| | | | **A63-P2** |
| | | | **LCMS:** *m*/*z* 461.8 (M+H); RT = 1.110 min (2.5 min). **¹H NMR (400 MHz, CDCl₃)** δ8.76 (s, 1H), 8.54 (s, 1H), 8.17 (d, *J* = 7.6 Hz, 1H), 7.82 - 7.76 (m, 2H), 7.46 (d, *J* = 7.6 Hz, 1H), 7.38 (d, *J* = 7.6 Hz, 1H), 7.25 - 7.22 (m, 1H), 7.05 (d, *J* = 8.4 Hz, 1H), 6.61 (s, 1H), 4.06 (q, *J* = 6.8 Hz, 1H), 3.30 (s, 3H), 3.10 (s, 3H), 2.78 (s, 3H), 1.10 (d, *J* = 6.8 Hz, 3H). |
| | | | **A63-P3** |
| | | | **LCMS:** *m*/*z* 461.8 (M+H); RT = 1.103 min (2.5 min). **¹H NMR (400 MHz, CDCl₃)** δ 8.86 (s, 1H), 8.55 (s, 1H), 8.20 (d, *J* = 7.6 Hz, 1H), 7.80 (t, *J* = 7.6 Hz, 1H), 7.73 (d, *J* = 8.4 Hz, 1H), 7.48 (d, *J* = 8.4 Hz, 1H), 7.37 (d, *J* = 7.6 Hz, 1H), 7.31 - 7.27 (m, 1H), 7.07 (d, *J=* 8.4 Hz, 1H), 6.69 (s, 1H), 4.07 (q,*J* = 6.8 Hz, 1H), 3.29 (s, 3H), 3.01 (s, 3H), 2.87 (s, 3H), 1.11 (d, *J* = 6.8 Hz, 3H). |
| | | | **A63-P4** |
| | | | **LCMS:** *m*/*z* 461.8 (M+H); RT = 1.109 min (2.5 min). **¹H NMR (400 MHz, CDCl₃)** δ 8.90 (s, 1H), 8.59 (s, 1H), 8.23 (d, *J* = 8.0 Hz, 1H), 7.86 - 7.78 (m, 2H), 7.60 (d, *J* = 7.4 Hz, 1H), 7.40 (d, *J* = 7.6 Hz, 2H), 7.10 (d, *J* = 8.4 Hz, 1H), 6.75 (s, 1H), 4.10 (q,*J* = 6.8 Hz, 1H), 3.31 (s, 3H), 3.03 (s, 3H), 2.83 (s, 3H), 1.26 (d, *J* = 6.8 Hz, 3H). |
| A64 | 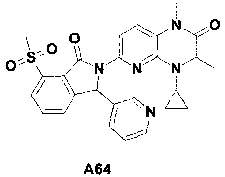 | 4-cyclopropyl-1,3-dimethyl-6-(4-(methanesulfonyl)-3-oxo-1-(pyridin-3-yl)isoindolin-2-yl)-3,4-dihydropyrido[2,3-b]pyrazin-2(1 H)-one (A64) | **A64-P1** |
| | | | **LCMS:** *m*/*z* 503.7 (M+H); RT = 1.211 min (2.5 min). **¹H NMR (400 MHz, CDCl₃)** δ8.89 (s, 1H), 8.53 (s, 1H), 8.29 (d, *J* = 8.0 Hz, 1H), 7.97 (d, *J* = 8.4 Hz, 1H), 7.76 (t, *J* = 7.6 Hz, 1H), 7.57 (d, *J* = 7.6 Hz, 1H), 7.47 (d, *J* = 7.8 Hz, 1H), 7.26 - 7.24 (m, 1H), 7.10 (d, *J* = 8.4 Hz, 1H), 6.70 (s, 1H), 4.14 (q, *J* = 6.8 Hz, 1H), 3.64 (s, 3H), 3.27 (s, 3H), 2.39 - 2.34 (m, 1H), 1.16 - 1.09 (m, 1H), 1.07 (d, *J* = 7.2 Hz, 3H), 0.85 - 0.77 (m, 1H), 0.68 - 0.60 (m, 1H), 0.60 - 0.53 (m, 1H). |
| | | | **A64-P2** |
| | | | **LCMS:** *m*/*z* 503.7 (M+H); RT = 1.208 min (2.5 min). **¹H NMR (400 MHz, CDCl₃)** δ 8.89 (s, 1H), 8.54 (s, 1H), 8.30 (d, *J* = 7.6 Hz, 1H), 7.99 (d, *J* = 8.4 Hz, 1H), 7.78 (t, *J* = 7.6 Hz, 1H), 7.59 (d, *J* = 7.6 Hz, 1H), 7.45 (d, *J* = 7.6 Hz, 1H), 7.26 - 7.20 (m, 1H), 7.12 (d, *J* = 8.4 Hz, 1H), 6.71 (s, 1H), 4.15 (q, *J* = 6.8 Hz, 1H), 3.66 (s, 3H), 3.29 (s, 3H), 2.39 - 2.33 (m, 1H), 1.16 - 1.11 (m, 1H), 1.08 (d, *J* = 6.8 Hz, 3H), 0.85 - 0.77 (m, 1H), 0.68 - 0.55 (m, 2H). |
| | | | **A64-P3** |
| | | | **LCMS:** *m*/*z* 503.7 (M+H); RT = 1.200 min (2.5 min). **¹H NMR (400 MHz, CDCl₃)** δ9.30 (d, *J* = 1.6 Hz, 1H), 8.68 (d, *J* = 4.4 Hz, 1H), 8.30 (d, *J* = 7.6 Hz, 1H), 7.99 (d, *J* = 8.4 Hz, 1H), 7.98 - 7.94(M, 1H),7.81 (t, *J* = 7.8 Hz, 1H), 7.72 - 7.62 (m, 2H), 7.18 (d, *J* = 8.4 Hz, 1H), 6.97 (s, 1H), 4.15 (q, *J* = 6.8 Hz, 1H), 3.63 (s, 3H), 3.29 (s, 3H), 2.63 - 2.52 (m, 1H), 1.22 (d, *J* = 6.8 Hz, 3H), 0.77 - 0.69 (m, 1H), 0.65 - 0.57 (m, 1H), 0.52 - 0.45 (m, 1H), -0.06 - -0.15 (m, 1H). |
| | | | **A64-P4** |
| | | | **LCMS:** *m*/*z* 503.8 (M+H); RT = 1.198 min (2.5 min). **¹H NMR (400 MHz, CDCl₃)** δ 8.84 (s, 1H), 8.54 (s, 1H), 8.26 (d, *J* = 7.6 Hz, 1H), 7.90 (d, *J* = 8.4 Hz, 1H), 7.75 (t, *J* = 7.6 Hz, 1H), 7.64 (d, *J* = 7.6 Hz, 1H), 7.48 (d, *J* = 7.6 Hz, 1H), 7.25 - 7.20 (m, 1H), 7.14 (d, *J* = 8.4 Hz, 1H), 6.82 (s, 1H), 4.14 (q, *J* = 6.8 Hz, 1H), 3.63 (s, 3H), 3.28 (s, 3H), 2.64 - 2.54 (m, 1H), 1.22 (d, *J* = 6.8 Hz, 3H), 0.80 - 0.66 (m, 2H), 0.51 - 0.43 (m, 1H), 0.11 - 0.04 (m, 1H). |
| A65 | 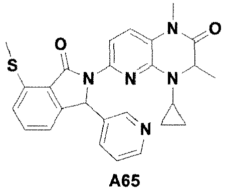 | 4-cyclopropyl-1,3-dimethyl-6-(4-(methylthio)-3-oxo-1-(pyridin-3-yl)isoindolin-2-yl)-3,4-dihydropyrido[2,3-b]pyrazin-2(1H)-one (A64) | **A65-P1** |
| | | | **LCMS:** *m*/*z* 471.8 (M+H); RT = 1.359 min (2.5 min). **¹H NMR (400 MHz, CDCl₃)** δ 8.82 (s, 1H), 8.47 (s, 1H), 8.02 (d, *J* = 8.4 Hz, 1H), 7.47 (t, *J* = 7.6 Hz, 1H), 7.42 (d, *J* = 8.0 Hz, 1H), 7.23 (d,*J* = 8.0 Hz, 1H), 7.21 - 7.15 (m, 1H), 7.09 (d, *J* = 8.4 Hz, 1H), 6.97 (d, *J* = 7.2 Hz, 1H), 6.55 (s, 1H), 4.10 (q, *J* = 6.8 Hz, 1H), 3.27 (s, 3H), 2.57 (s, 3H), 2.30 - 2.23 (m, 1H), 1.14 - 1.06 (m, 1H), 1.03 (d, *J* = 7.2 Hz, 3H), 0.80 - 0.72 (m, 1H), 0.66 - 0.59 (m, 1H), 0.59 - 0.50 (m, 1H). |
| | | | **A65-P2** |
| | | | **LCMS:** *m*/*z* 471.8 (M+H); RT = 1.358 min (2.5 min). **¹H NMR (400 MHz, CDCl₃)** δ 8.83 (s, 1H), 8.49 (s, 1H), 8.04 (d, *J* = 8.4 Hz, 1H), 7.49 (t, *J* = 7.6 Hz, 1H), 7.42 (d, *J* = 7.6 Hz, 1H), 7.25 (d, *J* = 8.0 Hz, 1H), 7.22 - 7.16 (m, 1H), 7.11 (d, *J* = 8.4 Hz, 1H), 6.99 (d, *J* = 7.6 Hz, 1H), 6.56 (s, 1H), 4.12 (q, *J* = 6.8 Hz, 1H), 3.29 (s, 3H), 2.59 (s, 3H), 2.34 - 2.26 (m, 1H), 1.15 - 1.08 (m, 1H), 1.05 (d, *J* = 6.8 Hz, 3H), 0.82 - 0.74 (m, 1H), 0.69 - 0.60 (m, 1H), 0.59 - 0.52 (m, 1H). |
| | | | **A65-P3** |
| | | | **LCMS:** *m*/*z* 471.8 (M+H); RT = 1.343 min (2.5 min). **¹H NMR (400 MHz, CDCl₃)** δ8.79 (s, 1H), 8.49 (s, 1H), 7.98 (d, *J* = 8.4 Hz, 1H), 7.47 (dd, *J* = 16.0, 8.2 Hz, 2H), 7.21 (d, *J* = 7.6 Hz, 2H), 7.14 (d, *J* = 8.4 Hz, 1H), 7.04 (d, *J* = 7.6 Hz, 1H), 6.68 (s, 1H), 4.11 (q, *J* = 6.8 Hz, 1H), 3.27 (s, 3H), 2.55 (s, 3H), 2.59 - 2.52(m, 1H), 1.21 (d, *J* = 7.2 Hz, 3H), 0.70 - 0.61 (m, 2H), 0.45 - 0.40 (m, 1H), 0- -0.06 (m, 1H). |
| | | | **A65-P4** |
| | | | **LCMS:** *m*/*z* 471.8 (M+H); RT = 1.343 min (2.5 min). **¹H NMR (400 MHz, CDCl₃)** δ8.81 (s, 1H), 8.52 (s, 1H), 7.97 (d, *J* = 7.4 Hz, 1H), 7.70 - 7.60 (m, 1H), 7.48 (t, *J* = 7.6 Hz, 1H), 7.38 - 7.30 (m, 1H), 7.22 (d, *J* = 8.0 Hz, 1H), 7.15 (d, *J* = 8.4 Hz, 1H), 7.05 (d, *J* = 7.6 Hz, 1H), 6.71 (s, 1H), 4.12 (q, *J* = 6.8 Hz, 1H), 3.28 (s, 3H), 2.60 - 2.54(m, 1H), 2.55 (s, 3H), 1.22 (d, *J* = 6.8 Hz, 3H), 0.72 - 0.60 (m, 2H), 0.48 - 0.41 (m, 1H), 0- -0.06 |
| A66 | 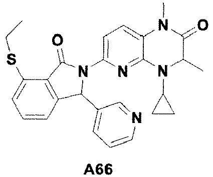 | 4-cyclopropyl-6-(4-(ethylthio)-3-oxo-1-(pyridin-3-yl)isoindolin-2-yl)-1,3-dimethyl-3,4-dihydropyrido[2,3-b]pyrazin-2(1 H)-one (A66) | **A66-P1** |
| | | | **LCMS:** *m*/*z* 485.8 (M+H); RT = 1.400 min (2.5 min). **¹H NMR (400 MHz, CDCl₃)** δ8.82 (s, 1H), 8.47 (s, 1H), 8.03 (d, *J* = 8.8 Hz, 1H), 7.44 (t, *J* = 7.6 Hz, 2H), 7.28 (s, 1H), 7.23 - 7.15 (m, 1H), 7.09 (d, *J=* 8.4 Hz, 1H), 6.96 (d, *J* = 7.6 Hz, 1H), 6.54 (s, 1H), 4.10 (q, *J* = 6.8 Hz, 1H), 3.27 (s, 3H), 3.09 (q, *J* = 7.4 Hz, 2H), 2.30 - 2.24 (m, 1H), 1.48 (t, *J* = 7.4 Hz, 3H), 1.13 - 1.07 (m, 1H), 1.03 (d, *J* = 6.8 Hz, 3H), 0.79 - 0.73 (m, 1H), 0.67 - 0.51 (m, 2H). |
| | | | **A66-P2** |
| | | | **LCMS:** *m*/*z* 485.8 (M+H); RT = 1.400 min (2.5 min). **¹H NMR (400 MHz, CDCl₃)** δ8.80 (s, 1H), 8.47 (s, 1H), 8.03 (d, *J* = 8.4 Hz, 1H), 7.43 (dd, *J* = 15.8, 8.2 Hz, 2H), 7.27 (s, 1H), 7.20 - 7.14 (m, 1H), 7.09 (d, *J* = 8.4 Hz, 1H), 6.96 (d, *J* = 7.6 Hz, 1H), 6.53 (s, 1H), 4.10 (q, *J* = 6.8 Hz, 1H), 3.27 (s, 3H), 3.09 (q, *J* = 7.4 Hz, 2H), 2.30 - 2.22 (m, 1H), 1.48 (t, *J* = 7.4 Hz, 3H), 1.14 - 1.06 (m, 1H), 1.03 (d, *J* = 6.8 Hz, 3H), 0.79 - 0.71 (m, 1H), 0.66 - 0.59 (m, 1H), 0.58 - 0.49 (m, 1H). |
| | | | **A66-P3** |
| | | | **LCMS:** *m*/*z* 485.8 (M+H); RT = 1.383 min (2.5 min). **¹H NMR (400 MHz, CDCl₃)** δ8.80 (s, 1H), 8.51 (s, 1H), 8.01 (d, *J* = 8.4 Hz, 1H), 7.51 - 7.41 (m, 2H), 7.26 (d, *J* = 8.0 Hz, 1H), 7.24 - 7.18 (m, 1H), 7.16 (d, *J* = 8.4 Hz, 1H), 7.05 (d, *J* = 7.6 Hz, 1H), 6.69 (s, 1H), 4.13 (q, *J* = 6.8 Hz, 1H), 3.29 (s, 3H), 3.09 (q, *J* = 7.4 Hz, 2H), 2.60 - 2.55 (m, 1H), 1.48 (t,*J* = 7.4 Hz, 3H), 1.23 (d, *J* = 6.8 Hz, 3H), 0.71 - 0.61 (m, 2H), 0.47 - 0.41 (m, 1H), -0.01 - -0.04 (m, 1H). |
| | | | **A66-P4** |
| | | | **LCMS:** *m*/*z* 485.7 (M+H); RT = 1.383 min (2.5 min). **¹H NMR (400 MHz, CDCl₃)** δ 8.78 (s, 1H), 8.49 (s, 1H), 7.99 (d, *J* = 8.4 Hz, 1H), 7.49 - 7.39 (m, 2H), 7.24 (d, *J* = 8.0 Hz, 1H), 7.21-7.16 (m, 1H), 7.14 (d, *J* = 8.4 Hz, 1H), 7.03 (d, *J* = 7.6 Hz, 1H), 6.67 (s, 1H), 4.11 (q, *J* = 6.8 Hz, 1H), 3.27 (s, 3H), 3.07 (q, *J* = 7.4 Hz, 2H), 2.58 - 2.52 (m, 1H), 1.46 (t, *J* = 7.4 Hz, 3H), 1.21 (d, *J* = 6.8 Hz, 3H), 0.68-0.62 (m, 2H), 0.45-0.39 (m, 1H), -0.01 - -0.07 (m, 1H). |

### Intermediate 138: Synthesis of 3-(5-fluoropyridin-2-yl)isoindolin-1-one (138)

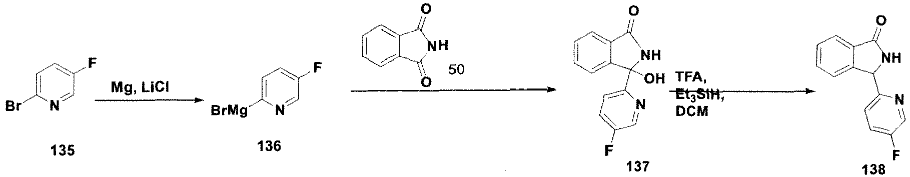

### Step 1: Synthesis of (5-fluoropyridin-2-yl)magnesium bromide (136)

Into a dry 250 mL three-necked flask, magnesium (1.7 g, 71 mmol), lithium chloride (1.5 g, 35.5 mmol) and dry tetrahydrofuran (50mL) were added under ice-water bath. The mixture was purged with nitrogen gas for three times, and a solution of diisopropylaluminum hydride (DIBAL) in tetrahydrofuran (1.0M, 0.3 mL, 0.3 mmol) was added at 0°C. The mixture was stirred at 0°C for 2 hours. compound **135** (5 g, 28.4 mmol) was added, and the mixture was stirred for another 2 hours. The resulting solution of (5-fluoropyridin-2-yl)magnesium bromide **136** was directly used in the next reaction.

### Step 2: Synthesis of 3-(5-fluoropyridin-2-yl)-3-hydroxyisoindolin-1-one (137)

Into a dry 250 mL three-necked flask, compound **50** (1.04 g, 7.1 mmol) and dry dichloromethane (30mL) were added. The mixture was purged with nitrogen gas for three times. The solution of compound **136** in tetrahydrofuran (50 mL, 28.4 mmol) was added, and the mixture was stirred at RT for 16 hours. After the completion of the reaction, the mixture was concentrated under reduced pressure to yield 3-(5-fluoropyridin-2-yl)-3-hydroxyisoindolin-1-one **137** (10 g, crude containing salt, brown solid). **LCMS:** *m*/*z* 244.8 (M+H); RT = 1.213 min (2.5 min).

### Step 3: Synthesis of 3-(5-fluoropyridin-2-yl)isoindolin-1-one (138)

Into a dry 250 mL three-necked flask, compound **137** (10 g, 7.1 mmol), dichloromethane (50mL), trifluoroacetic acid (20mL) and triethylsilane (20mL) were sequentially added at RT. The mixture was heated to 40°C under stirring for 16 hours. After the completion of the reaction, the mixture was adjusted with 5N NaOH solution to pH 8-9, and extracted with dichloromethane (50mL×3). The combined organic phase was dried over anhydrous Na₂SO₄ and filtered. The filtrate was concentrated under reduced pressure. The obtained residue was purified by column chromatography (dichloromethane:methanol = 20:1) to yield 3-(5-fluoropyridin-2-yl) isoindolin-1-one **138,** yield: 55%. **LCMS:** *m*/*z* 228.9 (M+H); RT = 1.265 min (2.5 min).

### Intermediate 142: Synthesis of 3-(pyridin-2-yl)isoindolin-1-one (142)

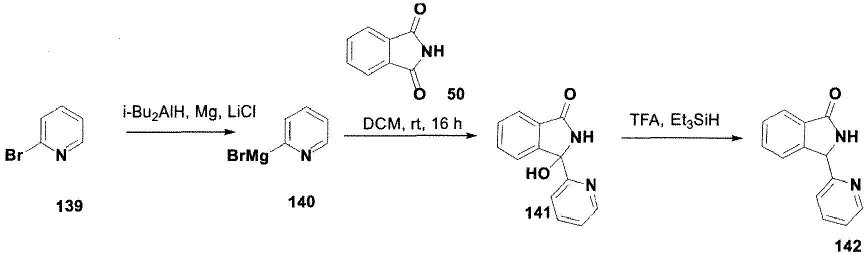

### Step 1: Synthesis of 2-pyridinylmagnesium bromide (140)

Into a dry 250 mL three-necked flask, magnesium (1.9 g, 79.13 mmol), lithium chloride (1.68 g, 39.56 mmol) and dry tetrahydrofuran (80mL) were added under ice-water bath. The mixture was purged with nitrogen gas for three times. A solution of diisopropylaluminum hydride in tetrahydrofuran (1.0M, 0.32 mL, 0.32 mmol) was added at 0°C. The mixture was stirred for 10 minutes. Then 139 1 (5 g, 31.65 mmol) was added at 0°C, and the mixture was stirred at 0°C for 2 hours. The resulting solution of 2-pyridinylmagnesium bromide **140** was directly used in the next reaction.

### Step 2: Synthesis of 3-hydroxy-3-(pyridin-2-yl)isoindolin-1-one (141)

Into a dry 500 mL three-necked flask, phthalimide (1.2 g, 7.91 mmol) and dry dichloromethane (50mL) were added under ice-water bath. The mixture was purged with nitrogen gas for three times. A solution of **140** in tetrahydrofuran (0.4M, 80 mL, 31.65 mmol) was added. The mixture was stirred at RT for 16 hours. After the completion of the reaction, the mixture was concentrated under reduced pressure to yield 3-hydroxy-3-(pyridin-2-yl)isoindolin-1-one **141** (10 g, crude containing the salt, brown oil). **LCMS:** *m*/*z* 226.0, 226.9 (M+H); RT = 1.103 min (2.5 min).

### Step 3: Synthesis of 3-(pyridin-2-yl)isoindolin-1-one (142)

Into a dry 250 mL three-necked flask, **141** (10 g, 7.91 mmol), dichloromethane (60mL), trifluoroacetic acid (10mL) and triethylsilane (10mL) were sequentially added at RT. The mixture was purged with nitrogen gas for three times, and then was heated to 40°C under stirring for 16 hours. After the completion of the reaction, the mixture was adjusted with 5N NaOH solution to pH 10-11, and extracted with dichloromethane (50mL×3). The combined organic phase was dried over anhydrous Na₂SO₄ and filtered. The filtrate was concentrated under reduced pressure. The obtained residue was purified by column chromatography (dichloromethane:methanol = 20:1) to yield 3-(pyridin-2-yl)isoindolin-1-one **142** (1 g, light brown solid), yield: 60%. **LCMS:** *m*/*z* 210.1, 210.9 (M+H); RT = 1.153 min (2.5 min).

According to the synthesis method of Al, the following examples were synthesized.

| | | | |
|---|---|---|---|
| A67 | 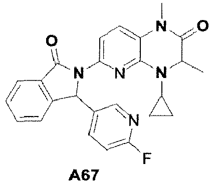 | 4-cyclopropyl-1,3-dimethyl-6-(1-oxo-3-(6-fluoropyridin-3-yl)isoindolin-2-yl)-3,4-dihydropyrido[3,2-b]pyrazin-2-(1H)-one (A67) | **A67-P1** |
| | | | **LCMS:** *m*/*z* 443.2, 443.9 (M+H); RT = 1.576 min (2.5 min). |
| | | | **¹H NMR (CD₃OD, 400 MHz)** δ 8.42 (s, 1H), 7.95 (t, *J* = 7.2 Hz, 2H), 7.73-7.56 (m, 3H), 7.44 (d, *J* = 7.4 Hz, 1H), 7.36 (d, *J* = 8.0 Hz, 1H), 6.95 (d, *J* = 8.4 Hz, 1H), 6.76 (s, 1H), 4.14 (q, *J* = 6.8 Hz, 1H), 3.30 (s, 3H), 2.46-2.27 (m, 1H), 1.29-1.23 (m, 1H), 1.05 (d, *J* = 6.8 Hz, 3H), 0.83-0.79 (m, 1H), 0.67-0.59 (m, 2H). |
| | | | **A67-P2** |
| | | | **LCMS:** *m*/*z* 443.2, 443.8 (M+H); RT = 1.592 min (2.5 min). |
| | | | **¹H NMR (CD₃OD, 400 MHz)** δ 8.42 (s, 1H), 7.95 (t, *J* = 7.4 Hz, 1H), 7.72-7.55 (m, 2H), 7.44 (d, *J* = 7.6 Hz, 1H), 7.37 (d, *J* = 8.4 Hz, 1H), 6.95 (d, *J* = 8.4 Hz, 1H), 6.76 (s, 1H), 4.14 (q, *J* = 6.8 Hz, 1H), 3.30 (s, 1H), 2.39 (s, 1H), 1.29-1.22 (m, 1H), 0.85-0.81 (m, 1H), 0.65-0.57 (m, 1H). |
| | | | **A67-P3** |
| | | | **LCMS:** *m*/*z* 443.2,443.9 (M+H); RT = 1.555 min (2.5 min). **¹H NMR (CD₃OD, 400 MHz)** δ 8.43 (s, 1H), 7.94 (d, *J* = 7.6 Hz, 1H), 7.87 (d, *J =* 8.4 Hz, 1H), 7.74 (t, *J* = 7.0 Hz, 1H), 7.67 (t, *J* = 7.4 Hz, 1H), 7.59 (t, *J* = 7.4 Hz, 1H), 7.50 (d, *J* = 7.6 Hz, 1H), 7.42 (d, *J* = 8.4 Hz, 1H), 6.97 (d, *J* = 8.4 Hz, 1H), 6.89 (s, 1H), 4.18-4.11 (m, 1H), 3.30 (s, 3H), 2.71 (s, 1H), 1.22 (d, *J* = 6.9 Hz, 4H), 0.95 - 0.83 (m, 1H), 0.9-0.74 (m, 1H), 0.53-0.50 (m, 1H), 0.06- -0.01 (m, 1H). |
| | | | **A67-P4** |
| | | | **LCMS:** *m*/*z* 443.2, 443.9 (M+H); RT = 1.556 min (2.5 min). |
| | | | **¹H NMR (CD₃OD, 400 MHz)** δ 8.43 (s, 1H), 7.94 (d, *J*= 7.6 Hz, 1H), 7.87 (d, *J* = 8.4 Hz, 1H), 7.75 (dd, *J* = 11.0, 5.0 Hz, 1H), 7.67 (t, *J* = 7.6 Hz, 1H), 7.59 (t, *J* = 7.6 Hz, 1H), 7.50 (d, *J* = 7.6 Hz, 1H), 7.42 (d, *J* = 8.4 Hz, 1H), 6.97 (d, *J* = 8.2 Hz, 1H), 6.90 (s, 1H), 4.15 (q, *J=* 6.8 Hz, 1H), 3.31 (s, 3H), 2.77-2.63 (m, 1H), 1.23 (d, *J* = 6.8 Hz, 3H), 0.95 - 0.81 (m, 1H), 0.81 - 0.70 (m, 1H), 0.58 - 0.43 (m, 1H), 0.06- -0.002 (m, 1H). |
| A68 | 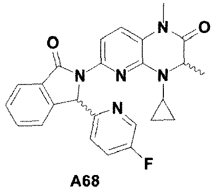 | 4-cyclopropyl-6-(1-(5-fluoropyridin-2-yl)-3-oxoisoindolin -2-yl)-1,3-dimethyl-3,4-dihydropyrido [2,3-b]pyrazin-2(1 H)-one (A68) | **A68-P1** |
| | | | **LCMS:** *m*/*z* 444.1 (M+H); RT = 1.644 min (2.5 min). **¹H NMR (400 MHz, CDCl₃)** δ 8.41 (d, *J* = 2.4 Hz, 1H), 8.14 (d, *J* = 8.4 Hz, 1H), 7.95 (d, *J* = 7.4 Hz, 1H), 7.58 - 7.44 (m, 3H), 7.23 - 7.17 (m, 1H), 7.15 (d, *J* = 8.5 Hz, 1H), 7.04 (dd, *J* = 8.6, 4.0 Hz, 1H), 6.68 (s, 1H), 4.08 (q, *J* = 6.8 Hz, 1H), 3.29 (s, 3H), 1.86 - 1.75 (m, 1H), 1.09 - 1.05 (m, 1H), 1.03 (d, *J=* 8.0 Hz, 3H), 0.67 - 0.58 (m, 1H), 0.53 - 0.42 (m, 2H). |
| | | | **A68-P2** |
| | | | **LCMS:** *m*/*z* 444.1 (M+H); RT = 1.643 min (2.5 min). **¹H NMR (400 MHz, CDCl₃)** δ 8.41 (s, 1H), 8.14 (d, *J* = 8.6 Hz, 1H), 7.95 (d, *J* = 7.4 Hz, 1H), 7.59 - 7.47 (m, 3H), 7.24 - 7.13 (m, 2H), 7.09 - 7.03 (m, 1H), 6.70 (s, 1H), 4.08 (q, *J* = 6.8 Hz, 1H), 3.29 (s, 3H), 1.84 - 1.77 (m, 1H), 1.10 - 1.05(m, 1H), 1.03 (d, *J* = 6.8 Hz, 3H), 0.67 - 0.60 (m, 1H), 0.57 - 0.43 (m, 2H). |
| | | | **A68-P3** |
| | | | **LCMS:** *m*/*z* 444.1 (M+H); RT = 1.647 min (2.5 min). **¹H NMR (400 MHz, CDCl₃)** δ 8.51 (s, 1H), 8.16 (d, *J* = 8.4 Hz, 1H), 7.92 (d, *J* = 7.4 Hz, 1H), 7.69 (d, *J* = 7.4 Hz, 1H), 7.54 (t, *J* = 7.6 Hz, 1H), 7.48 (t, *J* = 7.4 Hz, 1H), 7.23 - 7.17 (m, 2H), 6.96 (dd, *J=* 8.4, 4.0 Hz, 1H), 6.86 (s, 1H), 4.11 (q, *J=* 6.8 Hz, 1H), 3.30 (s, 3H), 2.53 - 2.46 (m, 1H), 1.22 (d, *J* = 6.8 Hz, 3H), 0.61 - 0.54 (m, 1H), 0.49 - 0.40 (d, *J=* 6.7 Hz, 1H), 0.38 - 0.31 (d, *J=* 4.8 Hz, 1H), -0.35 - -0.43 (s, 1H). |
| | | | **A68-P4** |
| | | | **LCMS:** *m*/*z* 444.1 (M+H); RT = 1.648 min (2.5 min). **¹H NMR (400 MHz, CDCl₃)** δ 8.51 (d, *J* = 2.4 Hz, 1H), 8.16 (d, *J* = 8.6 Hz, 1H), 7.92 (d, *J* = 7.6 Hz, 1H), 7.69 (d, *J* = 7.6 Hz, 1H), 7.54 (t, *J* = 7.4 Hz, 1H), 7.48 (t, *J* = 7.4 Hz, 1H), 7.22 - 7.15 (m, 2H), 6.95 (dd, *J=* 8.8, 4.0 Hz, 1H), 6.85 (s, 1H), 4.11 (q, *J* = 6.8 Hz, 1H), 3.30 (s, 3H), 2.54 - 2.44 (m, 1H), 1.22 (d, *J=* 6.8 Hz, 3H), 0.62 - 0.53 (m, 1H), 0.47 - 0.39 (m, 1H), 0.38 - 0.30 (m, 1H), -0.37- -0.43 (m, 1H). |
| A69 | 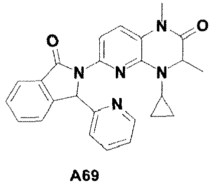 | 4-cyclopropyl-1,3-dimethyl-6-(1-oxo-3-(pyridin-2-yl)isoindolin-2-yl)-3,4-dihydropyrido [2,3-b]pyrazin-2(1 H)-one (A69) | **A69-P1** |
| | | | **LCMS:** *m*/*z* 425.2, 425.7 (M+H); RT = 1.550 min (2.5 min) |
| | | | **¹H NMR (CD₃OD, 400 MHz)** δ 8.45 (d, *J* = 5.2 Hz, 1H), 8.12 (d, *J* = 8.4 Hz, 1H), 7.94 (d, *J* = 7.2 Hz, 1H), 7.28 (t, *J* = 7.6 Hz, 1H), 7.61-7.53 (m, 3H), 7.39 (t, *J* = 8.4 Hz, 2H), 7.28 (t, *J* = 6.0 Hz, 1H), 6.72 (s, 1H), 4.07 (q, *J* = 6.8 Hz, 1H), 3.31 (s, 3H), 1.94 (m, 1H), 1.18 (m, 1H), 0.95 (d, *J* = 6.8 Hz, 3H), 0.70 (m, 1H), 0.52 (m, 2H). |
| | | | **A69-P2** |
| | | | **LCMS:** *m*/*z* 425.2, 426.1 (M+H); RT = 1.399 min (2.5 min). |
| | | | **¹H NMR (CD₃OD, 400 MHz)** 8.45 (d, *J* = 5.2 Hz, 1H), 8.12 (d, *J* = 8.4 Hz, 1H), 7.94 (d, *J* = 7.2 Hz, 1H), 7.28 (t, *J* = 7.6 Hz, 1H), 7.61-7.53 (m, 3H), 7.39 (t, *J* = 8.4 Hz, 2H), 7.28 (t, *J* = 6.0 Hz, 1H), 6.72 (s, 1H), 4.07 (q, *J* = 6.8 Hz, 1H), 3.31 (s, 3H), 1.94 (m, 1H), 1.18 (m, 1H), 0.95 (d, *J* = 6.8 Hz, 3H), 0.70 (m, 1H), 0.52 (m, 2H). |
| | | | **A69-P3** |
| | | | **LCMS:** *m*/*z* 425.2, 426.1 (M+H); RT = 1.395 min (2.5 min). |
| | | | **¹H NMR (CD₃OD, 400 MHz)** δ8.58 (d, *J* = 4.4 Hz, 1H), 8.11 (d, *J* = 8.0 Hz, 1H), 7.92 (d, *J* = 7.6 Hz, 1H), 7.71-7.61 (m, 3H), 7.56 (t, *J* = 7.2 Hz, 1H), 7.45 (d, *J* = 8.8 Hz, 1H), 7.30 (t, *J* = 6.0 Hz, 1H), 7.22 (d, *J* = 8.0 Hz, 1H), 6.87 (s, 1H), 4.11 (q, *J* = 6.8 Hz, 1H), 2.60 (m, 1H), 1.22 (d, *J* = 6.8 Hz, 3H), 0.65 (m, 1H), 0.56 (m, 1H), 0.38 (m, 1H), -0.36 (m, 1H). |
| | | | **A69-P4** |
| | | | **LCMS:** *m*/*z* 425.2, 425.7 (M+H); RT = 1.556 min (2.5 min). |
| | | | **¹H NMR (CD₃OD, 400 MHz)** δδ8.58 (d, *J* = 4.4 Hz, 1H), 8.11 (d,*J* = 8.0Hz, 1H), 7.92 (d, *J* = 7.6 Hz, 1H), 7.71-7.61 (m, 3H), 7.56 (t, *J* = 7.2 Hz, 1H), 7.45 (d, *J* = 8.8 Hz, 1H), 7.30 (t, *J* = 6.0 Hz, 1H), 7.22 (d, *J* = 8.0 Hz, 1H), 6.87 (s, 1H), 4.11 (q, *J* = 6.8 Hz, 1H), 2.60 (m, 1H), 1.22 (d, *J* = 6.8 Hz, 3H), 0.65 (m, 1H), 0.56 (m, 1H), 0.38 (m, 1H), - 0.36 (m, 1H). |

The following examples were prepared according to Example A1, using K₃PO₄ instead of Cs₂CO₃.

| | | | |
|---|---|---|---|
| A70 | 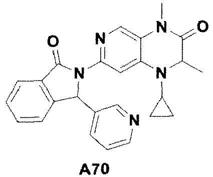 | 1-cyclopropyl-2,4-dimethyl-7-(1-oxo-3-(pyridin-3-yl)isoindolin-2-yl)-1,4-dihydropyrido [3,4-b]pyrazin-3(2H)-one (A70) | **A70-P1** |
| | | | **LCMS:** *m*/*z* 425.2, 425.8(M+H); RT = 1.231 min (2.5 min). **¹H NMR (CD₃OD, 400 MHz)**δ 8.65 (s, 1H), 8.42 (d, *J* = 4.8 Hz, 1H), 8.21 (s, 1H), 7.97 (d, *J* = 7.6 Hz, 1H), 7.83 (d, *J* = 4.2 Hz, 1H), 7.71 - 7.54 (m, 3H), 7.40 (d, *J* = 7.0 Hz, 1H), 7.34 (dd, *J* = 7.6, 5.0 Hz, 1H), 6.74 (d, *J* = 7.6 Hz, 1H), 4.18 (q, *J* = 6.6 Hz, 1H), 3.33 (s, 3H), 2.67 - 2.60 (m, 1H), 1.23-1.16 (m, 4H), 0.98 - 0.87 (m, 1H), 0.81 - 0.64 (m, 2H). |
| | | | **A70-P2** |
| | | | **LCMS:** *m*/*z* 425.2, 425.9(M+H); RT = 1.229 min (2.5 min). **¹H NMR (CD₃OD, 400 MHz)**δ8.76 (s, 1H), 8.53 (s, 1H), 8.13 - 7.91 (m, 2H), 7.91 - 7.74 (m, 2H), 7.66 (dt, *J* = 14.6, 6.8 Hz, 2H), 7.47-7.42 (m, 2H), 6.77 (s, 1H), 4.21 (q, *J* = 6.8 Hz, 1H), 3.32 (s, 3H), 2.73 - 2.61 (m, 1H), 1.27 (d, *J* = 6.8 Hz, 3H), 1.20 - 1.08 (m, 1H), 1.00 - 0.88 (m, 1H), 0.78-0.72 (m, 1H), 0.58-0.52 (m, 1H). |
| | | | **A70-P3** |
| | | | **LCMS:** *m*/*z* 425.2, 425.8(M+H); RT = 1.247 min (2.5 min). **¹H NMR (CD₃OD, 400 MHz)** δ 8.65 (s, 1H), 8.42 (d, *J* = 4.2 Hz, 1H), 8.23 (s, 1H), 7.97 (d, *J* = 7.6 Hz, 1H), 7.84 (s, 1H), 7.72 - 7.55 (m, 3H), 7.40 (d, *J* = 7.5 Hz, 1H), 7.36 - 7.30 (m, 1H), 6.75 (s, 1H), 4.17 (q, *J* = 6.8 Hz, 1H), 3.30 (s, 3H), 2.74 - 2.66 (m, 1H), 1.29 (d, *J* = 6.8 Hz, 3H), 1.19 - 1.08 (m, 1H), 0.98 - 0.88 (m, 1H), 0.77 - 0.65 (m, 1H), 0.60 - 0.47 (m, 1H). |
| | | | **A70-P4** |
| | | | **LCMS:** *m*/*z* 425.2, 425.9(M+H); RT = 1.247 min (2.5 min). **¹H NMR (CD₃OD, 400 MHz)** δ 9.17 (s, 1H), 8.81 (s, 1H), 8.48 (d, *J* = 8.1 Hz, 1H), 8.06 (d, *J* = 7.6 Hz, 1H), 7.97 (s, 1H), 7.85 (s, 1H), 7.78 (t, *J* = 7.6 Hz, 1H), 7.71 (t, *J* = 7.6 Hz, 1H), 7.61 - 7.49 (m, 2H), 6.97 (s, 1H), 4.30 (q, *J* = 6.8 Hz, 1H), 3.34 (s, 3H), 2.74 - 2.64 (m, 1H), 1.37 (d, *J* = 6.8 Hz, 3H), 1.30 - 1.22 (m, 1H), 1.06 - 0.96 (m, 1H), 0.89-0.83 (m, 1H), 0.72-0.66 (m, 1H). |
| A71 | 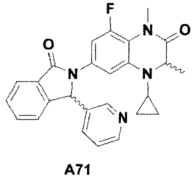 | 4-cyclopropyl-8-fluoro-1,3-dimethyl-6-(1-oxo-3-(pyridin-3-yl)isoindolin-2-yl)-3,4-dihydroquino xalin-2(1H)-one (A71) | **A71-P1** |
| | | | **LCMS:** *m*/*z* 443.1 (M+H); RT = 1.504 min (2.5 min). **¹H NMR (400 MHz, CDCl₃)** δ 8.70 (s, 1H), 8.57 (s, 1H), 7.99 (d, *J* = 7.6 Hz, 1H), 7.61 - 7.52 (m, 2H), 7.41 (d, *J* = 7.6 Hz, 1H), 7.26 - 7.19 (m, 3H), 6.89 (d, *J* = 14.4 Hz, 1H), 6.05 (s, 1H), 4.03 (q, *J* = 6.8 Hz, 1H), 3.36 (d, *J* = 6.4 Hz, 3H), 2.35 - 2.25 (m, 1H), 1.08 (d, *J* = 6.8 Hz, 3H), 0.96 - 0.88 (m, 1H), 0.81 - 0.73 (m, 1H), 0.65 - 0.56 (m, 1H), 0.51 - 0.43 (m, 1H). |
| | | | **A71-P2** |
| | | | **LCMS:** *m*/*z* 443.0 (M+H); RT = 1.504 min (2.5 min). **¹H NMR (400 MHz, CDCl₃)** δ 8.69 (s, 1H), 8.56 (s, 1H), 7.99 (d, *J* **=** 7.6 Hz, 1H), 7.60 - 7.52 (m, 2H), 7.40 (d, *J* = 7.9 Hz, 1H), 7.26 - 7.22 (m, 3H), 6.89 (dd, *J* = 14.4, 2.2 Hz, 1H), 6.05 (s, 1H), 4.02 (q, *J* = 6.8 Hz, 1H), 3.36 (d, *J* = 6.4 Hz, 3H), 2.33 - 2.25 (m, 1H), 1.08 (d, *J* = 6.8 Hz, 3H), 0.95 - 0.87 (m, 1H), 0.81 - 0.73 (m, 1H), 0.65 - 0.57 (m, 1H), 0.51 - 0.43 (m, 1H). |
| | | | **A71-P3** |
| | | | **LCMS:** *m*/*z* 443.1 (M+H); RT = 1.528 min (2.5 min). **¹H NMR (400 MHz, CDCl₃)** δ 8.81 (s, 1H), 8.59 (s, 1H), 8.00 (d, *J* = 7.0 Hz, 1H), 7.66 - 7.40 (m, 3H), 7.29 - 7.23 (m, 3H), 7.01 (s, 1H), 6.17 (s, 1H), 4.03 (q, *J* = 6.8 Hz, 1H), 3.37 (d, *J* = 6.4 Hz, 3H), 2.35-2.28 (m, 1H), 1.10 (d, *J* = 6.8 Hz, 3H), 0.91 - 0.82 (m, 1H), 0.80 - 0.73 (m, 1H), 0.63 - 0.55 (m, 1H), 0.36 - 0.28 (m, 1H). |
| | | | **A71-P4** |
| | | | **LCMS:** *m*/*z* 443.1 (M+H); RT = 1.529 min (2.5 min). **¹H NMR (400 MHz, CDCl₃)** δ 8.73 (s, 1H), 8.57 (s, 1H), 7.99 (d, *J* = 7.6 Hz, 1H), 7.60 - 7.52 (m, 2H), 7.41 (d, *J* = 7.8 Hz, 1H), 7.31 - 7.24 (m, 3H), 6.99 (s, 1H), 6.11 (s, 1H), 4.03 (q, *J* = 6.8 Hz, 1H), 3.37 (d, *J* = 6.4 Hz, 3H), 2.35 - 2.26 (m, 1H), 1.10 (d, *J* = 6.8 Hz, 3H), 0.88 - 0.80 (m, 1H), 0.79 - 0.72 (m, 1H), 0.62 - 0.54 (m, 1H), 0.36 - 0.25 (m, 1H). |

### Example A72: Synthesis of 5-(4-cyclopropyl-1,3-dimethyl-2-oxo-1,2,3,4-tetrahydropyrido[2, 3-b]pyrazin-6-yl)-4-(pyridin-3-yl)4,5-dihydro-6H-thieno[2,3-c]pyrrol-6-one (A72)

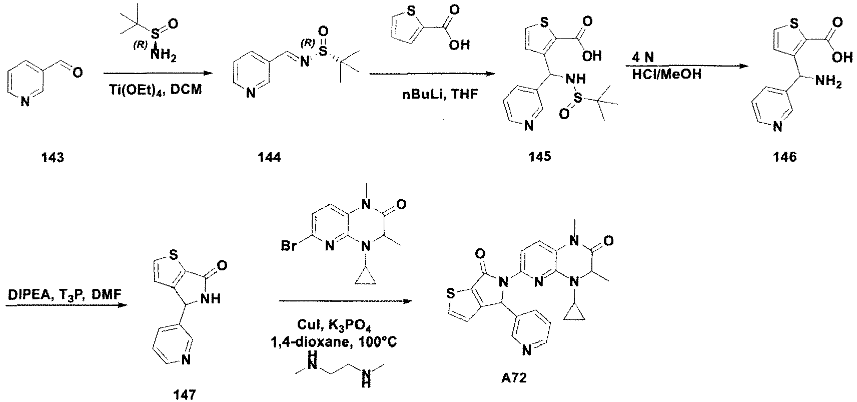

### Step 1. Synthesis of (E)-2-methyl-N-(pyridin-3-ylmethylene)propane-2-sulfinamide (144)

Into a dry 500 mL three-neck reaction flask, compound **143** (16 g, 149.5 mmol), anhydrous dichloromethane (300mL) and *R*-t-butylsulfinamide (18.1 g, 149.5 mmol) were added. The mixture was cooled to 0°C using ice salt bath. The mixture was purged with argon gas for three times, and tetraethyl titanate (136 g, 598.1 mmol) was added in portions. After the addition, the mixture was warmed to 40 °C under stirring for 12 hours. After the completion of the reaction, the reaction mixture was poured into water, precipitating a large amount of white solid. The reaction was filtered, and the filtrate was extracted with dichloromethane (500mL×2). The organic phases were combined, washed with saturated brine (300mL×2), dried over anhydrous Na₂SO₄ and filtered. The filtrate was concentrated under reduced pressure to yield (*E*)-2-methyl-*N*-(pyridin-3-ylmethylene)propane-2-sulfinamide **144** (24 g, yellow liquid), yield: 76 %. **LCMS:** *m*/*z* 210.9 (M+H); RT = 1.299 min (2.5 min).

### Step 2: Synthesis of 3-(((tert-butylsulfinyl)amino)(pyridin-3-yl)methyl)thiophene-2-carboxylic acid (145)

Into a dry 100 mL round-bottom flask, compound **3** (3.2 g, 25 mmol) and anhydrous tetrahydrofuran (50mL) were sequentially added. The mixture was purged with argon gas for three times and cooled to -60°C using dry ice bath. Then n-butyllithium (50 mL, 2.4 M solution in hexane) was slowly added dropwise. The reaction system was kept at -60°C under stirring for 3 hours. After the mixture was cooled to -78°C, a solution of compound **144** (5.25 g, 25 mmol) in anhydrous tetrahydrofuran (40mL) was slowly added dropwise. After addition, the mixture was spontaneously warmed to RT, and was stirred for 2 hours. After the completion of the reaction, the reaction mixture was poured into aqueous ammonium chloride solution, concentrated under reduced pressure, and lyophilized to yield crude 3-(((tert-butylsulfinyl)amino)(pyridin-3-yl)methyl)thiophene-2-carboxylic acid **145** (4 g, yellow liquid), yield: 47.6 %. **LCMS:** *m*/*z* 339.0 (M+H); RT = 1.16 min and 1.19 min (2.5 min).

### Step 3: Synthesis of 3-(amino(pyridin-3-yl)methyl)thiophene-2-carboxylic acid (146)

Into a dry 100 mL round-bottom flask, compound **145** (4 g, 11.8 mmol), anhydrous methanol (10mL) and 4M HCl in methanol (20mL) were sequentially added. The mixture was stirred at RT for 4 hours. After the completion of the reaction, the mixture was concentrated under reduced pressure to yield the crude 3-(amino(pyridin-3-yl)methyl)thiophene-2-carboxylic acid **146** (4 g, yellow oil), yield: 148 %. **LCMS:** *m*/*z* 234.8 (M+H); RT = 0.451 min (2.5 min).

### Step 4: Synthesis of 4-(pyridin-3-yl)-4,5-dihydro-6H-thieno[2,3-c]pyrrol-6-one (147)

Into a dry 100 mL round-bottom flask, compound **146** (2.5 g, 10.6 mmol), N,N-dimethylformamide (30mL), N,N-diisopropylethylamine (4.12 g, 32 mmol) and 1-propylphosphoric anhydride (50 % solution in ethyl acetate, 20.3 g, 32 mmol) were sequentially added. After addition, the mixture was stirred at 40°C for 6 hours. After the completion of the reaction, the mixture was diluted with water, and extracted with ethyl acetate (50mL×2). The organic phases were combined, washed with saturated brine (50mL×2), dried over anhydrous Na₂SO₄ and filtered. The filtrate was concentrated under reduced pressure. The obtained residue was purified by column chromatography (ethyl acetate) to yield 4-(pyridin-3-yl)-4,5-dihydro-6H-thieno[2,3-c]pyrrol-6-one **147** (0.4 g, yellow solid), yield: 17.4 %. **LCMS:** *m*/*z* 216.9 (M+H); RT = 0.889 min (2.5 min).

### Step 5: Synthesis of 5-(4-cyclopropyl-1,3-dimethyl-2-oxo-1,2,3,4-tetrahydropyrido[2,3-b]pyr azin-6-yl)-4-(pyridin-3-yl)-4,5-dihydro-6H-thieno[2,3-c]pyrrol-6-one (A72)

Into a dry 100 mL round-bottom flask, compound **147** (0.2 g, 0.92 mmol), 1,4-dioxane (20mL), compound **5** (0.27 g, 0.92 mmol), potassium phosphate (0.39 g, 1.84 mmol), copper iodide (0.17 g, 0.92 mmol) and N,N'-dimethylethylenediamine (0.16 g, 1.84 mmol) were sequentially added. The mixture was purged with argon gas for three times, and warmed to 100°C under stirring for 4 hours. After the completion of the reaction, the mixture was filtered, and the filtrate was concentrated under reduced pressure. The obtained crude was purified by column chromatography (petroleum ether:ethyl acetate = 1:1) to yield 5-(4-cyclopropyl-1,3-dimethyl-2-oxo-1,2,3,4-tetrahydropyrido[2,3-b]pyrazin-6-yl)-4-(pyridin-3-yl)-4,5-dihydro-6H-thieno[2,3-c]pyrrol-6-one **A72.** The racemate was purified and separated by chiral high performance preparative chromatography to yield compounds **A72-P1** (2.2 mg, yellow solid), **A72-P2** (14.5 mg, yellow solid), **A72-P3** (7.7 mg, yellow solid) and **A72-P4** (13.8 mg, yellow solid), yield: 9.6 %.
Chiral separation conditions are as follows (SFC) (**A72-P1** and **A72-P4):**
   column: AD-H, eluent: 70% CO₂ + 30% MeOH, flow rate: 12.5 mL /min
Chiral separation conditions are as follows (SFC) **(A72-P2** and **A72-P3):**
   column: AD-H, eluent: 70% CO₂ + 30% IPA (0.2% DEA), flow rate: 12.5 mL /min

### A72-P1

**LCMS:** *m*/*z* 431.6 (M+H); RT = 1.409 min (2.5 min).

**¹H NMR (DMSO-*d₆*, 400 MHz):** δ8.67 (s, 1H), 8.42 (d, *J* = 4.0 Hz, 1H), 8.09 (d, *J* = 4.0 Hz, 1H), 7.73 (d, *J* = 8.0 Hz, 1H), 7.58 (d, *J=* 8.0 Hz, 1H), 7.36 (d, *J* = 8.0 Hz, 1H), 7.28 - 7.25 (m, 1H), 7.14 (d, *J* = 4.0 Hz, 1H), 6.66 (s, 1H), 4.04 - 3.99 (m, 1H), 3.18 (s, 3H), 2.14 - 2.08 (m, 1H), 1.18 - 1.13 (m, 1H), 0.89 (d, *J* = 8.0 Hz, 3H), 0.71 - 0.66 (m, 1H), 0.58 - 0.53 (m, 1H), 0.51 - 0.46 (m, 1H).

### A72-P2

**LCMS:** *m*/*z* 431.7 (M+H); RT = 1.403 min (2.5 min).

**¹H NMR (DMSO-*d₆*, 400 MHz):** δ8.67 (s, 1H), 8.42 (d, *J* = 4.0 Hz, 1H), 8.09 (d, *J* = 4.0 Hz, 1H), 7.73 (d, *J=* 8.0 Hz, 1H), 7.58 (d, *J* = 8.0 Hz, 1H), 7.36 (d, *J* = 8.0 Hz, 1H), 7.28 - 7.25 (m, 1H), 7.14 (d, *J* = 4.0 Hz, 1H), 6.66 (s, 1H), 4.04 - 3.99 (m, 1H), 3.18 (s, 3H), 2.14 - 2.08 (m, 1H), 1.18 - 1.13 (m, 1H), 0.89 (d, *J* = 8.0 Hz, 3H), 0.71 - 0.66 (m, 1H), 0.58 - 0.53 (m, 1H), 0.51 - 0.46 (m, 1H).

### A72-P3

**LCMS:** *m*/*z* 431.8 (M+H); RT = 1.308 min (2.5 min).

**¹H NMR (DMSO-*d₆*, 400 MHz):** δ8.70 (s, 1H), 8.44 (d, *J* = 4.0 Hz, 1H), 8.08 (d, *J* = 4.0 Hz, 1H), 7.73 (d, *J* = 8.0 Hz, 1H), 7.64 (d, *J=* 8.0 Hz, 1H), 7.42 (d, *J* = 8.0 Hz, 1H), 7.34 - 7.27 (m, 1H), 7.22 (d, *J* = 4.0 Hz, 1H), 6.83 (s, 1H), 4.05 - 4.00 (m, 1H), 3.20 (s, 3H), 2.60 - 2.56 (m, 1H), 1.13 (d, *J* = 4.0 Hz, 3H), 0.62 - 0.56 (m, 2H), 0.46 - 0.40 (m, 1H), -0.31 - -0.37 (m, 1H).

### A72-P4

**LCMS:** *m*/*z* 431.8 (M+H); RT = 1.320 min (2.5 min).

**¹H NMR (DMSO-*d₆*, 400 MHz):** δ8.70 (s, 1H), 8.44 (d, *J=* 4.0 Hz, 1H), 8.08 (d, *J=* 4.0 Hz, 1H), 7.73 (d, *J* = 8.0 Hz, 1H), 7.64 (d, *J* = 8.0 Hz, 1H), 7.42 (d, *J* = 8.0 Hz, 1H), 7.34 - 7.27 (m, 1H), 7.22 (d, *J=* 4.0 Hz, 1H), 6.83 (s, 1H), 4.05 - 4.00 (m, 1H), 3.20 (s, 3H), 2.60 - 2.56 (m, 1H), 1.13 (d, *J* = 4.0 Hz, 3H), 0.62 - 0.56 (m, 2H), 0.46 - 0.40 (m, 1H), -0.31 - -0.37 (m, 1H).

### Example A73: Synthesis of 4-cyclopropyl-6-(4-(ethylsulfinyl)-3-oxo-1-(pyridin-3-yl)isoindoli n-2-yl)-1,3-dimethyl-3,4-dihydropyrido[2,3-b]pyrazin-2(1H)-one (A73)

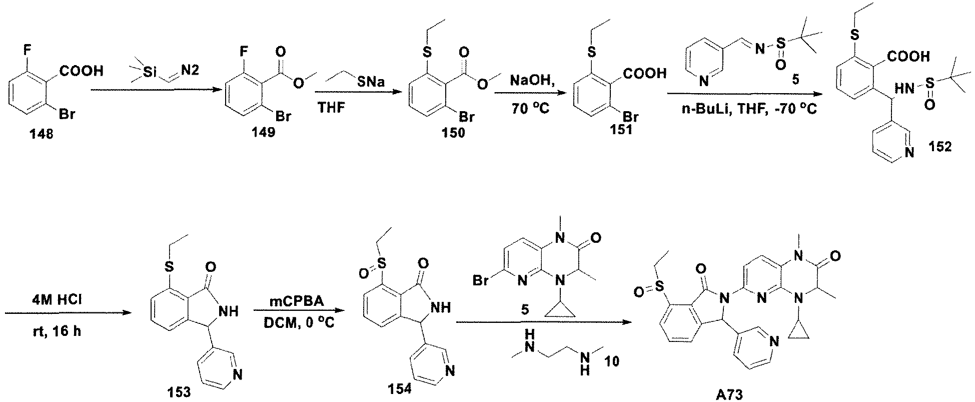

### Step 1: Synthesis of Methyl 2-bromo-6-fluorobenzoate (149)

Into a dry 250 mL three-necked flask, compound **148** (16 g, 73 mmol), benzene (50mL) and anhydrous methanol (30mL) were sequentially added at RT. The mixture cooled to 0°C. (Diazomethyl)trimethylsilane (2M solution in n-hexane, 54.7mL) was slowly added dropwise. The mixture was purged with nitrogen gas once, stirred at 0°C for 1 hour, and concentrated under reduced pressure. The obtained residue was purified by column chromatography (ethyl acetate : petroleum ether = 1:10) to yield methyl 2-bromo-6-fluorobenzoate **149** (colorless oil, 16.1 g), yield: 95%.

### Step 2: Synthesis of Methyl 2-bromo-6-(ethylthio)benzoate (150)

Into a dry 250 mL single-necked flask, compound **149** (5.0 g, 21.46 mmol) and tetrahydrofuran (70mL) were sequentially added at RT, followed by addition of sodium ethanethiolate (2.16 g, 25.75 mmol). The mixture was heated to reflux under stirring for 16 hours. After the completion of reaction as monitored on TLC plate, the mixture was concentrated under reduced pressure. To the obtained residue was added 30 mL water, and the mixture was extracted with ethyl acetate (50mL×3). The organic phases were combined, dried over anhydrous Na₂SO₄ and filtered. The filtrate was concentrated under reduced pressure. The obtained residue was purified by column chromatography (ethyl acetate : petroleum ether = 1:20) to yield methyl 2-bromo-6-(ethylthio)benzoate **150** (4.6 g, colorless oil), yield: 78%. **LCMS:** *m*/*z* 296.8, 298.7 (M+Na); RT = 1.499 min (2.5 min).

### Step 3: Synthesis of 2-bromo-6-(ethylthio)benzoic acid (151)

Into a dry 250 mL single-necked flask, compound **150** (4.6 g, 16.72 mmol), methanol (30mL) and 4N aqueous sodium hydroxide solution (30mL) were sequentially added at RT. The mixture was heated to 70°C under stirring for 16 hours. After the completion of reaction as monitored on TLC plate, the organic solvent was concentrated under reduced pressure. The residue was adjusted to pH 3-4, and was extracted with ethyl acetate (50mL×3). The organic phases were combined, dried over anhydrous Na₂SO₄ and filtered. The filtrate was concentrated under reduced pressure to yield 2-bromo-6-(ethylthio)benzoic acid **151** (4.1 g, yellow solid), yield: 94%. **LCMS:** *m*/*z* 258.8, 260.8 (M-H); RT = 1.271 min (2.5 min)

### Step 4: Synthesis of 2-(((tert-butylsulfinyl)amino)(pyridin-3-yl)methyl)-6-(ethylthio)benzoic acid (152)

Into a dry 100 mL three-necked flask, compound **151** (4.1 g, 15.7 mmol) and anhydrous tetrahydrofuran (50mL) were sequentially added at RT. The mixture was cooled to -70°C, and n-butyllithium (2.4 M solution in n-hexane, 14.4 mL, 34.54 mmol) was slowly added dropwise. The mixture was stirred at -70°C for 1 hour. compound **5** (3.62 g, 17.26 mmol) was added, and the mixture was stirred at -70°C for another 2 hours. After the completion of reaction as monitored on TLC plate, saturated aqueous ammonium chloride solution (20mL) was added, and the mixture was extracted with ethyl acetate (50mL×2). The aqueous phases was combined, and lyophilized to yield 2-(((tert-butylsulfinyl)amino)(pyridin-3-yl)methyl)-6-(ethylthio)benzoic acid **152** (crude 12 g, containing inorganic salt, yellow solid), which was directly used in the next reaction. **LCMS:** *m*/*z* 392.8 (M+H); RT = 1.088 min and 1.120 (2.5 min).

### Step 5: Synthesis of 7-(ethylthio)-3-(pyridin-3-yl)isoindolin-1-one (153)

Into a dry 250 mL round-bottom flask, compound **152** (12 g, crude) and 4 M solution of chlorine hydride in methanol (100mL) were added at RT. The mixture was stirred at RT for 16 hours. After the completion of reaction as monitored on TLC plate, the mixture was concentrated under reduced pressure. The obtained residue was adjusted to pH8-9 with saturated aqueous sodium bicarbonate solution, and was extracted with ethyl acetate (30mL×2). The organic phases were combined, dried over anhydrous Na₂SO₄ and filtered. The filtrate was concentrated under reduced pressure, washed with ethyl acetate and filtered. The filter cake was dried to yield 7-(ethylthio)-3-(pyridin-3-yl)isoindolin-1-one **153** (507 mg, white solid), yield over two steps: 11%. **LCMS:** *m*/*z* 271.1 (M+H); RT = 1.187 min (2.5 min).

### Step 6: Synthesis of 7-(ethylsulfinyl)-3-(pyridin-3-yl)isoindolin-1-one (154)

Into a dry 100 mL single-necked flask, compound **153** (300 mg, 1.11 mmol) and dichloromethane (20mL) were added at RT. The mixture was cooled to 0°C. M-chloroperoxybenzoic acid (364 mg, 2.1 mmol) was added. The mixture was stirred for 2 hours. After the completion of reaction as monitored on TLC plate, saturated aqueous sodium bicarbonate solution (20mL) was added, and the mixture was extracted with dichloromethane (30mL×2). The organic phases were combined, and concentrated under reduced pressure. The obtained residue was purified by HPLC (mobile phase acetonitrile and water) to yield 7-(ethylsulfinyl)-3-(pyridin-3-yl)isoindolin-1-one **154** (70 mg, white solid), yield: 22%. **LCMS:** *m*/*z* 286.9 (M+H); RT = 0.761 min and 0.811min (2.5 min).

### Step 7: Synthesis of 4-cyclopropyl-6-(4-(ethylsulfinyl)-3-oxo-1-(pyridin-3-yl)isoindolin-2-yl)-1,3-dimethyl-3,4-dihydropyrido[2,3-b]pyrazin-2(1H)-one (A73)

Into a dry 50 mL single-necked flask, compound **154** (70 mg, 0.17 mmol), compound **5** (50 mg, 0.17 mmol), N,N-dimethylethylenediamine (30 mg, 0.34 mmol), copper iodide (32 mg, 0.17 mmol), potassium phosphate (108 mg, 0.51 mmol) and 1,4-dioxane (8mL) were sequentially added at RT. The mixture was purged with nitrogen gas for three times, and heated to 100°C under stirring for 5 hours. The mixture was cooled to RT, and filtered. The filtrate was concentrated under reduced pressure. The obtained residue was purified by column chromatography (dichloromethane:methanol = 20:1) to yield 4-cyclopropyl-6-(4-(ethylsulfinyl)-3-oxo-1-(pyridin-3-yl)isoindolin-2-yl)-1,3-dimethyl-3,4-dihydropyrido[2,3-b]pyrazin-2(1H)-one **A73** (40 mg, yellow solid). **LCMS:** *m*/*z* 501.7 (M+H); RT = 1.201 min and 1.213 min (2.5 min).

### Step 8: Resolution of 4-cyclopropyl-6-(4-(ethylsulfinyl)-3-oxo-1-(pyridin-3-yl)isoindolin-2-y l)-1,3-dimethyl-3,4-dihydropyrido[2,3-b]pyrazin-2(1H)-one (A73)

Chiral resolution conditions (SFC):
**A73-P1** and **A73-P2**
   chiral column: AD-H
   mobile phase: 70% carbon dioxide + 30% isopropanol (0.2% diethylamine)
   flow rate: 40 g/min
**A73-P3** and **A73-P4**
   chiral column: IB
   mobile phase: 70% carbon dioxide + 30% ethanol (0.2% diethylamine)
   flow rate: 40 g/min
**A73-P5, A73-P6, A73-P7** and **A73-P8**
   chiral column: AD-H
   mobile phase: 70% carbon dioxide + 30% isopropanol (0.2% diethylamine)
   flow rate: 40 g/min
**A73-P1: LCMS:** *m*/*z* 501.8 (M+H); RT = 1.213 min (2.5 min).

**¹H NMR (400 MHz, CDCl₃)** δ8.11 (d, *J* = 7.2 Hz, 1H), 7.89 (d, *J* = 8.4 Hz, 1H), 7.78 (t, *J* = 6.8 Hz, 1H), 7.70 - 7.60 (m, 1H), 7.49 - 7.44 (m, 1H), 7.26 - 7.23 (m, 3H), 7.13 (d, *J* = 8.4 Hz, 1H), 6.86 (s, 1H), 4.13 (q, *J* = 6.8 Hz, 1H), 3.47 - 3.40 (m, 1H), 3.29 (s, 3H), 3.11 - 3.06 (m, 1H), 2.59 - 2.52 (m, 1H), 1.34 (t, *J=* 7.2 Hz, 3H), 1.22 (d, *J=* 6.8 Hz, 3H), 0.72 - 0.57 (m, 2H), 0.48 - 0.41 (m, 1H), -0.02 - -0.08 (m, 1H).

**A73-P2: LCMS:** *m*/*z* 501.8 (M+H); RT = 1.214 min (2.5 min).

**¹H NMR (400 MHz, CDCl₃)** δ 8.12 (s, 1H), 7.80 - 7.72 (m, 2H), 7.48 - 7.38 (m, 2H), 7.26 - 7.25 (m, 3H), 7.14 (d, *J* = 8.2 Hz, 1H), 6.87 (s, 1H), 4.14 (q, *J* = 6.8 Hz, 1H), 3.38 - 3.32 (m, 1H), 3.28 (s, 3H), 3.10 - 3.06 (m, 1H), 2.62 -2.56(m, 1H), 1.25 (t, *J* = 7.2 Hz, 3H), 1.22 (d, *J* = 6.4 Hz, 3H), 0.91 - 0.82 (m, 2H), 0.75 - 0.69 (m, 1H), 0.51 - 0.44 (m, 1H).

**A73-P3: LCMS:** *m*/*z* 501.8 (M+H); RT = 1.213 min (2.5 min).

**¹H NMR (400 MHz, CDCl₃)** δ 8.75 (s, 1H), 8.58 (s, 1H), 8.14 (d, *J* = 7.8 Hz, 1H), 7.91 (d, *J* = 8.4 Hz, 1H), 7.79 (t, *J* = 7.8 Hz, 1H), 7.53 - 7.43 (m, 2H), 7.15 (d, *J* = 8.4 Hz, 1H), 7.06 - 6.98 (m, 1H), 6.88 (s, 1H), 4.12 (q, *J* = 6.8 Hz, 1H), 3.49 - 3.42 (m, 1H), 3.29 (s, 3H), 3.12 - 3.05 (m, 1H), 2.58 - 2.52 (m, 1H), 1.35 (t, *J* = 7.4 Hz, 3H), 1.22 (d, *J* = 6.4 Hz, 3H), 0.72 - 0.65 (m, 1H), 0.60 - 0.52 (m, 1H), 0.50 - 0.43 (m, 1H), -0.03 -0.11 (m, 1H).

**A73-P4: LCMS:** *m*/*z* 501.8 (M+H); RT = 1.213 min (2.5 min).

**¹H NMR (400 MHz, CDCl₃)** δ 8.84 (s, 1H), 8.53 (s, 1H), 8.12 (d, *J* = 7.6 Hz, 1H), 7.80 - 7.73 (m, 2H), 7.57 - 7.37 (m, 3H), 7.14 (d, *J* = 8.4 Hz, 1H), 6.85 (s, 1H), 4.14 (q, *J* = 6.8 Hz, 1H), 3.38 - 3.32 (m, 1H), 3.28 (s, 3H), 3.03 - 2.96 (m, 1H), 2.61 - 2.55 (m, 1H), 1.26 (t, *J* = 6.4 Hz, 3H), 1.22 (d, *J* = 6.4 Hz, 3H), 0.82 - 0.67 (m, 2H), 0.50 - 0.43 (m, 1H), 0.10 - 0.05 (m, 1H).

**A73-P5: LCMS:** *m*/*z* 502.1 (M+H); RT = 1.324 min (2.5 min).

**¹H NMR (400 MHz, CDCl₃) δ** 8.12 (d, *J* = 7.6 Hz, 1H), 7.91 (d, *J* = 8.4 Hz, 1H), 7.77 (t, *J* = 7.6 Hz, 1H), 7.47 - 7.30 (m, 2H), 7.26 - 7.22 (m, 3H), 7.08 (d, *J* = 8.4 Hz, 1H), 6.68 (s, 1H), 4.12 (q, *J* = 6.8 Hz, 1H), 3.50 - 3.42 (m, 1H), 3.27 (s, 3H), 3.17 - 3.09 (m, 1H), 2.29 - 2.20 (m, 1H), 1.36 (t, *J* = 7.4 Hz, 3H), 1.13 - 1.09 (m, 1H), 1.05 (d, *J* = 6.8 Hz, 3H), 0.80 - 0.73 (m, 1H), 0.66 - 0.60 (m, 1H), 0.59 - 0.52 (m, 1H).

**A73-P6: LCMS:** *m*/*z* 502.2 (M+H); RT = 1.327 min (2.5 min).

**¹H NMR (400 MHz, CDCl₃)** δ 8.12 (d, *J* = 7.6 Hz, 1H), 7.91 (d, *J* = 8.4 Hz, 1H), 7.76 (t, *J* = 7.6 Hz, 2H), 7.44 - 7.37 (m, 2H), 7.26 - 7.25 (m, 2H), 7.08 (d, *J* = 8.4 Hz, 1H), 6.68 (s, 1H), 4.12 (q, *J* = 6.8 Hz, 1H), 3.49 - 3.41 (m, 1H), 3.27 (s, 3H), 3.16 - 3.08 (m, 1H), 2.28 - 2.22 (m, 1H), 1.36 (t, *J* = 7.4 Hz, 3H), 1.12 - 1.08 (m, 1H), 1.05 (d, *J* = 6.8 Hz, 3H), 0.79 - 0.73 (m, 1H), 0.66 - 0.59 (m, 1H), 0.58 - 0.52 (m, 1H).

**A73-P7: LCMS:** *m*/*z* 502.1 (M+H); RT = 1.312 min (2.5 min).

**¹H NMR (400 MHz, CDCl₃)** δ 9.43 (s, 1H), 8.71 (d, *J* = 5.0 Hz, 1H), 8.21 (d, *J* = 7.8 Hz, 1H), 7.96 - 7.83 (m, 2H), 7.81 (d, *J* = 8.4 Hz, 1H), 7.77 - 7.70 (m, 1H), 7.47 (d, *J* = 7.0 Hz, 1H), 7.12 (d, *J* = 8.4 Hz, 1H), 6.89 (s, 1H), 4.22 (q, *J* = 6.8 Hz, 1H), 3.47 - 3.39 (m, 1H), 3.28 (s, 3H), 3.14 - 3.05 (m, 1H), 2.38 - 2.32 (m, 1H), 1.33 (t, *J* = 7.2 Hz, 3H), 1.25 - 1.20 (m, 1H), 1.11 (d, *J* = 6.8 Hz, 3H), 0.91 - 0.83 (m, 1H), 0.69 - 0.56 (m, 2H).

**A73-P8: LCMS:** *m*/*z* 502.2 (M+H); RT = 1.325 min (2.5 min).

**¹H NMR (400 MHz, CDCl₃)** δ 8.13 (d, *J* = 7.6 Hz, 1H), 7.83 (d, *J* = 8.4 Hz, 1H), 7.77 (t, *J* = 7.6 Hz, 1H), 7.37 (d, *J* = 7.4 Hz, 2H), 7.26 - 7.23 (m, 3H), 7.09 (d, *J* = 8.4 Hz, 1H), 6.71 (s, 1H), 4.13 (q, *J* = 6.8 Hz, 1H), 3.41 - 3.33 (m, 1H), 3.27 (s, 3H), 3.05 - 2.97 (m, 1H), 2.40 - 2.32 (m, 1H), 1.29 (t, *J* = 7.2 Hz, 3H), 1.15 - 1.10 (m, 1H), 1.05 (d, *J* = 6.8 Hz, 3H), 0.83 - 0.74 (m, 1H), 0.68 - 0.52 (m, 2H).

### Intermediate 157 and 157a: Synthesis of 6-fluoro-3-(pyridin-3-yl)isoindolin-1-one (157) and 4-fluoro-3-(pyridin-3-yl)isoindolin-1-one (157a)

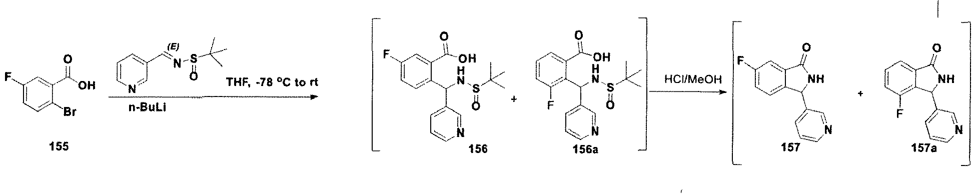

### Step 1: 2-(((t-butylsulfinyl)amino)(pyridin-3-yl)methyl)-5-fluorobenzoic acid (156) and 2-(((t-butylsulfinyl)amino)(pyridin-3-yl)methyl)-3-fluorobenzoic acid (156a)

Into a dry 100 mL round-bottom flask, compound **155** (500 mg, 2.29 mmol), compound **2** (482 mg, 2.29 mmol) and dry tetrahydrofuran (15mL) were sequentially added at RT. The mixture was cooled to -78°C, and a solution of n-butyllithium in tetrahydrofuran (2.4M, 2.1 mL, 5.03 mmol) was added dropwise. After the addition, the mixture was spontaneously warmed to RT under stirring for 16 hours. The reaction mixture was poured into water (50mL), and extracted with ethyl acetate (30mL×3). The organic phases were combined, washed with saturated brine (20mL×3), dried over anhydrous Na₂SO₄ and filtered. The filtrate was concentrated under reduced pressure to yield the mixed product 2-(((t-butylsulfinyl)amino)(pyridin-3-yl)methyl)-5-fluorobenzoic acid **(156)** and 2-(((t-butylsulfinyl)amino)(pyridin-3-yl)methyl)-3-fluorobenzoic acid **(156a)** (770 mg, yellow solid), yield: 96%. **LCMS:** *m*/*z* 350.1, 350.9(M+H); RT = 1.180 min (2.5 min).

### Step 2: 6-fluoro-3-(pyridin-3-yl)isoindolin-1-one (157) and 4-fluoro-3-(pyridin-3-yl)isoindolin-1-one (157a)

Into a dry 50 mL round-bottom flask, the mixture of compounds **156** and **156a** (770 mg, 2.20 mmol) and a solution of chloro hydride in methanol (4.0M, 15mL) were sequentially added at RT. The mixture was stirred at RT for 16 hours, and concentrated under reduced pressure. The obtained residue was purified by column chromatography (dichloromethane:methanol = 20:1) to yield the mixed product 6-fluoro-3-(pyridin-3-yl)isoindolin-1-one **(157)** and 4-fluoro-3-(pyridin-3-yl)isoindolin-1-one **(157a)** (130 mg, light brown solid), yield: 25%. **LCMS:** *m*/*z* 228.0, 229.0 (M+H); RT = 1.057 min (2.5 min).¹H NMR showed that the obtained product was the mixture of **157** and **157a** (in a ratio of about 3:2)

The following examples were synthesized according to Example A1.

| | | | |
|---|---|---|---|
| A74 | 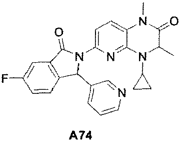 | 4-cyclopropyl-6-(5-fluoro-3-oxo-1-(pyridin-3-yl)isoindolin-2-yl)-1,3-dimethyl-3,4-dihydropyrido[2,3 -b]pyrazin-2( 1H)-one (A74) | **A74-P1** |
| | | | **LCMS:** *m*/*z* 443.2, 443.7(M+H); RT = 1.358 min (2.5 min). |
| | | | **¹H NMR (CD₃OD, 400 MHz)** δ 8.75 (s, 1H), 8.43 (s, 1H), 7.97 (d, *J* = 8.6 Hz, 1H), 7.70 - 7.58 (m, 2H), 7.51 - 7.26 (m, 4H), 6.74 (s, 1H), 4.12 (dd, *J* = 13.6, 6.8 Hz, 1H), 3.30 (s, 3H), 2.36 - 2.23 (m, 1H), 1.29 - 1.19 (m, 1H), 1.00 (d, *J* = 6.8 Hz, 3H), 0.84-0.77 (m, 1H), 0.69 - 0.53 (m, 2H). |
| | | | **A74-P2** |
| | | | **LCMS:** *m*/*z* 443.2, 443.7(M+H); RT = 1.351 min (2.5 min). |
| | | | **¹H NMR (CD₃OD, 400 MHz)** δ 8.74 (s, 1H), 8.43 (s, 1H), 7.96 (d, *J* = 8.4 Hz, 1H), 7.70 - 7.56 (m, 2H), 7.54 - 7.28 (m, 4H), 6.73 (s, 1H), 4.12 (q, *J=* 6.8 Hz, 1H), 3.30 (s, 3H), 2.31 (s, 1H), 1.30 - 1.17 (m, 2H), 0.99 (d, *J* = 6.8 Hz, 3H), 0.86 - 0.75 (m, 1H), 0.67 - 0.51 (m, 2H). |
| | | | **A74-P3** |
| | | | **LCMS:** *m*/*z* 443.2, 443.7(M+H); RT = 1.343 min (2.5 min). |
| | | | **¹H NMR (CD₃OD, 400 MHz)** δ 8.82 (s, 1H), 8.51 (s, 1H), 7.96 (d, *J* = 8.4 Hz, 1H), 7.75 (d, *J* = 8.2 Hz, 1H), 7.69 (d, J = 7.8 Hz, 1H), 7.63 - 7.55 (m, 1H), 7.49 (d, *J* = 8.4 Hz, 2H), 7.42 (d, J = 5.8 Hz, 1H), 6.95 (s, 1H), 4.26 - 4.14 (m, 1H), 3.37 (s, 3H), 2.82 - 2.68 (m, 1H), 1.29 (d, *J* = 6.7 Hz, 3H), 0.92-0.85 (m, 1H), 0.84 - 0.74 (m, 1H), 0.61 - 0.46 (m, 1H), 0.06 - -0.06 (m, 1H). |
| | | | **A74-P4** |
| | | | **LCMS:** *m*/*z* 443.2, 443.7(M+H); RT = 1.340 min (2.5 min). |
| | | | **¹H NMR (CD₃OD, 400 MHz)**δ 8.83 (s, 1H), 8.51 (s, 1H), 7.96 (d, *J* = 8.4 Hz, 1H), 7.75 (d, *J* = 7.8 Hz, 1H), 7.69 (d, J = 7.6 Hz, 1H), 7.60 (dd, *J* = 8.4, 4.8 Hz, 1H), 7.49 (d, *J* = 8.4 Hz, 2H), 7.45 - 7.38 (m, 1H), 6.95 (s, 1H), 4.20 (q, *J* = 6.8 Hz, 1H), 3.37 (s, 3H), 2.80 - 2.63 (m, 1H), 1.29 (d, *J* = 6.9 Hz, 3H), 0.93 - 0.85 (m, 1H), 0.83-0.76 (m, 1H), 0.60 - 0.45 (m, 1H), 0.05 - -0.09 (m, 1H). |
| A75 | 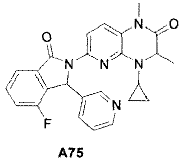 | 4-cyclopropyl-6-(7-fluoro-3-oxo-1-(pyridin-3-yl)isoindolin-2-yl)-1,3-dimethyl-3,4-dihydropyrido[2,3 -b]pyrazin-2(1H)-one (A75) | **A75-P1** |
| | | | **LCMS:** *m*/*z* 443.2, 443.7(M+H); RT = 1.361 min (2.5 min). |
| | | | **¹H NMR (CD₃OD, 400 MHz)** δ 8.73 (s, 1H), 8.43 (s, 1H), 7.95 (d, *J* = 8.4 Hz, 1H), 7.82 (d, *J* = 7.4 Hz, 1H), 7.66 (dd, J = 15.2, 7.6 Hz, 2H), 7.44 - 7.27 (m, 3H), 6.88 (s, 4H), 4.13 (q, *J* = 6.6 Hz, 1H), 3.29 (s, 3H), 2.45 - 2.33 (m, 1H), 1.30 - 1.22 (m, 1H), 1.00 (d, *J* = 6.8 Hz, 3H), 0.88 - 0.78 (m, 1H), 0.72 - 0.52 (m, 2H). |
| | | | **A75-P2** |
| | | | **LCMS:** *m*/*z* 443.2, 443.7(M+H); RT = 1.361 min (2.5 min). |
| | | | **¹H NMR (CD₃OD, 400 MHz)** δ 8.73 (s, 1H), 8.44 (s, 1H), 7.95 (d, *J* = 8.6 Hz, 1H), 7.82 (d, *J* = 7.6 Hz, 1H), 7.67 (dd, *J* = 16.4, 8.2 Hz, 2H), 7.37 (dd, *J* = 17.8, 9.2 Hz, 3H), 6.88 (s, 1H), 4.19 - 4.06 (m, 1H), 3.30 (s, 3H), 2.44 - 2.34 (m, 1H), 1.30 - 1.23 (m, 1H), 1.00 (d, *J* = 6.8 Hz, 3H), 0.87 - 0.77 (m, 1H), 0.70 - 0.51 (m, 2H). |
| | | | **A75-P3** |
| | | | **LCMS:** *m*/*z* 443.2, 443.7(M+H); RT = 1.349 min (2.5 min). |
| | | | **¹H NMR (CD₃OD, 400 MHz)** δ 8.75 (s, 1H), 8.43 (s, 1H), 7.86 (d, *J* = 8.4 Hz, 1H), 7.81 (d, *J* = 7.6 Hz, 1H), 7.73 - 7.59 (m, 2H), 7.42 - 7.28 (m, 3H), 7.05 (s, 1H), 4.15 (q, *J* = 6.6 Hz, 1H), 3.30 (s, 3H), 2.80 - 2.67 (m, 1H), 1.23 (d, *J* = 6.8 Hz, 3H), 1.01 - 0.86 (m, 1H), 0.84 - 0.70 (m, 1H), 0.60 - 0.46 (m, 1H), 0.13 - -0.03 (m, 1H). |
| | | | **A75-P4** |
| | | | **LCMS:** *m*/*z* 443.2, 443.7(M+H); RT = 1.350 min (2.5 min). |
| | | | **¹H NMR (CD₃OD, 400 MHz)** δ 8.76 (s, 1H), 8.44 (s, 1H), 7.86 (d, *J* = 8.4 Hz, 1H), 7.81 (d, *J* = 7.6 Hz, 1H), 7.73 - 7.59 (m, 2H), 7.45 - 7.30 (m, 3H), 7.05 (s, 1H), 4.19 - 4.01 (m, 1H), 3.31 - 3.22 (m, 3H), 2.81 - 2.69 (m, 1H), 1.22 (s, 3H), 0.95-0.90 (m, 2H), 0.83 - 0.66 (m, 1H), 0.57 - 0.43 (m, 1H), 0.09 - -0.10 (m, 1H). |
| A76 | 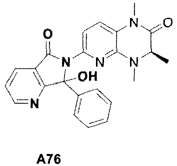 | 1,3,4-trimethyl-6-(1-oxo-3-hydroxy-3-phenyl)isodihydro 1-N-indol-2-yl)-3,4-dihydropyrido[2,3 -b]pyrazin-2(1H)-one (A76) | **A76-P1** |
| | | | **LCMS:** m/z 416.1 (M+H); RT = 1.469 min (2.5 min). **¹H NMR (DMSO-d6, 400 MHz):** 9.07 (s, 1H), 8.78 (d, J = 8.0 Hz, 1H), 7.55 (d, J = 8.0 Hz, 1H), 7.49 - 7.44 (m, 3H), 7.40 - 7.35 (m, 2H), 7.34 - 7.30 (m, 2H), 7.28 - 7.20 (m, 1H), 4.09 - 4.02 (m, 1H), 3.21 (d, J = 4.0 Hz, 3H), 2.42 (s, 1.5H), 2.36 (s, 1.5H), 1.10 (d, J = 8.0 Hz, 1.5H), 0.97 (d, J = 8.0 Hz, 1.5H). |
| | | | **A76-P2** |
| | | | **LCMS:** m/z 415.7 (M+H); RT = 1.417 min (2.5 min). **¹H NMR (DMSO-d6, 400 MHz):** 9.07 (s, 1H), 8.78 (d, J = 8.0 Hz, 1H), 7.55 (d, J = 8.0 Hz, 1H), 7.49 - 7.44 (m, 3H), 7.40 - 7.35 (m, 2H), 7.34 - 7.30 (m, 2H), 7.28 - 7.20 (m, 1H), 4.09 - 4.02 (m, 1H), 3.21 (d, J = 4.0 Hz, 3H), 2.42 (s, 1.5H), 2.36 (s, 1.5H), 1.10 (d, J = 8.0 Hz, 1.5H), 0.97 (d, J = 8.0 Hz, 1.5H). |

### Example A78: Synthesis of (4-cyclopropyl-6-(1-(4-fluorophenyl)-5-(4-methylpiperazine-1-ca rbonyl)-3-oxoisoindolin-2-yl)-1,3-dimethyl-3,4-dihydropyrido[2,3-b]pyrazin-2(1H)-one (A78)

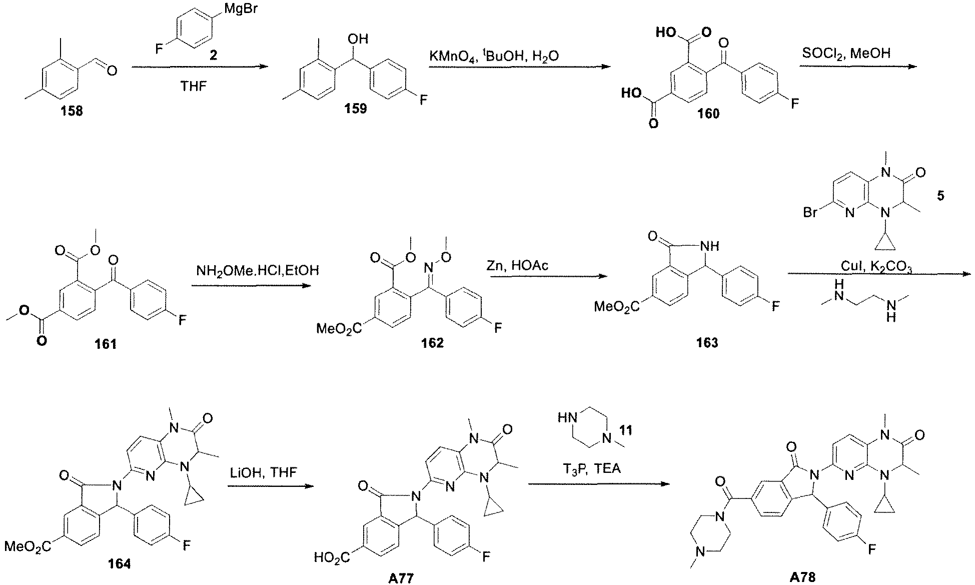

### Step 1: (2,4-dimethylphenyl)(4-fluorophenyl)methanol (159)

Into a dry 100 mL three-necked flask, compound **158** (4 g, 29.8 mmol) and dry tetrahydrofuran (100mL) were sequentially added at RT, followed by addition of the solution of compound 2 in tetrahydrofuran (1.0M, 33 mL, 32.79 mmol). The mixture was purged with nitrogen gas for three times, and stirred at RT for 16 hours. After the completion of the reaction, the mixture was quenched by addition of methanol (20mL), and concentrated under reduced pressure. The obtained residue was purified by column chromatography (ethyl acetate : petroleum ether = 10:1) to yield (2,4-dimethylphenyl)(4-fluorophenyl)methanol **159** (3.24 g, yellow liquid), yield: 47%. **LCMS:** *m*/*z* 230.1, 213.0 (M-17); RT = 1.659 min (2.5 min).

### Step 2: 4-(4-fluorobenzoyl)isophthalic acid (160)

Into a dry 100 mL round-bottom flask, compound **159** (3.24 g, 14.1 mmol), KMnO₄ (15.58 g, 98.6 mmol), t-butyl alcohol (80mL) and water (80mL) were sequentially added at RT. The mixture was heated to 80°C under stirring for 16 hours. The mixture was filtered, and the filtrate was concentrated under reduced pressure. To the residue was added water (50mL). The mixture was adjusted to pH 3-4 with IN aqueous hydrochloric acid, precipitating white solid. The mixture was filtered, and the filter cake was dried to yield 4-(4-fluorobenzoyl)isophthalic acid **160** (2.9 g, white liquid), yield: 71%. **LCMS:** *m*/*z* 288.0, 289.0 (M+H); RT = 1.325 min (2.5 min).

### Step 3: Dimethyl 4-(4-fluorobenzoyl)isophthalate (161)

Into a dry 100 mL round-bottom flask, compound **160** (2.9 g, 10.06 mmol) and methanol (100mL) were sequentially added at RT, and thionyl chloride (3.59 g, 30.18 mmol) was added dropwise under ice bath. The mixture was stirred at RT for 16 hours, and concentrated under reduced pressure to yield dimethyl 4-(4-fluorobenzoyl)isophthalate **161** (2.6 g, yellow liquid), yield: 81%. **LCMS:** *m*/*z* 316.1, 317.0(M+H); RT = 1.652 min (2.5 min).

### Step 4: Dimethyl (E)-4-((4-fluorophenyl)(methoxyimino)methyl)isophthalate (162)

Into a dry 150 mL sealed tube, compound **161** (1.4 g, 4.42 mmol), methoxyamine hydrochloride (3.67 g, 44.2 mmol) and ethanol (20mL) were added. The mixture was heated to 100°C under stirring for 16 hours. After the completion of reaction, the mixture was concentrated under reduced pressure. To the residue was added 50 mL water, and the mixture was extracted with ethyl acetate (20mL×3). The combined organic phase was dried over anhydrous Na₂SO₄ and filtered. The filtrate was concentrated under reduced pressure to yield dimethyl (*E*)-4-((4-fluorophenyl)(methoxyimino)methyl)isophthalate **162** (1.2 g, yellow liquid), yield: 79%. **LCMS:** *m*/*z* 345.1, 346.0(M+H); RT = 1.649 min (2.5 min).

### Step 5: Methyl 1-(4-fluorophenyl)-3-oxoisoindoline-5-carboxylate (163)

Into a dry 100 mL round-bottom flask, compound **162** (1 g, 2.895 mmol), zinc powder (1.88 g, 28.95 mmol) and acetic acid (40mL) were sequentially added at RT. The mixture was heated to 90°C under stirring for 1 hour. The mixture was filtered, and the filtrate was concentrated under reduced pressure. The obtained residue was purified by column chromatography (ethyl acetate : petroleum ether = 1:1) to yield methyl 1-(4-fluorophenyl)-3-oxoisoindoline-5-carboxylate **163** (0.9 g, white solid), purity 91%. **LCMS:** *m*/*z* 285.0, 286.0 (M+H); RT = 1.431 min (2.5 min).

### Step 6: Methyl 2-(4-cyclopropyl-1,3-dimethyl-2-oxo-1,2,3,4-tetrahydropyrido[2,3-b]pyrazin-6-yl)-1-(4-fluorophenyl)-3-oxoisoindoline-5-carboxylate (164)

Into a dry 100 mL round-bottom flask, compound **163** (290 mg, 1.01 mmol), compound **5** (300 mg, 1.01 mmol), N,N-dimethylethylenediamine (178 mg, 2.02 mmol), copper iodide (193 mg, 1.01 mmol), potassium carbonate (279 mg, 2.02 mmol) and 1,4-dioxane (15mL) were sequentially added at RT. The mixture was purged with nitrogen gas for three times, and heated to 100°C under stirring for 16 hours. After the completion of reaction, the mixture was filtered, and the filtrate was concentrated under reduced pressure. 20 mL water was added to the residue, and the mixture was extracted with ethyl acetate (20mL×3). The combined organic phase was dried over anhydrous Na₂SO₄ and filtered. The filtrate was concentrated under reduced pressure to yield methyl 2-(4-cyclopropyl-1,3-dimethyl-2-oxo-1,2,3,4-tetrahydropyrido[2,3-b]pyrazin-6-yl)-1-(4-fluorophenyl)-3-oxoisoindoline-5-carboxylate **164** (300 mg, yellow solid), yield: 59%. **LCMS:** *m*/*z* 500.2, 501.0(M+H); RT = 1.650 min (2.5 min).

### Step 7: 2-(4-cyclopropyl-1,3-dimethyl-2-oxo-1,2,3,4-tetrahydropyrido[2,3-b]pyrazin-6-yl)-1-(4-fluorophenyl)-3-oxoisoindoline-5-carboxylic acid (A77)

Into a dry 100 mL round-bottom flask, compound **164** (300 mg, 0.6 mmol), aqueous lithium hydroxide solution (IN, 2.4mL, 2.4mmol) and tetrahydrofuran (6mL) were sequentially added at RT. The mixture was stirred at RT for 16 hours. After the completion of reaction, the mixture was concentrated under reduced pressure. 10 mL water was added to the residue. The mixture was adjusted to pH 3-4 with IN aqueous hydrochloric acid, and extracted with ethyl acetate (10mL×3). The combined organic phase was dried over anhydrous Na₂SO₄ and filtered. The filtrate was concentrated under reduced pressure to yield 2-(4-cyclopropyl-1,3-dimethyl-2-oxo-1,2,3,4-tetrahydropyrido[2,3-b]pyrazin-6-yl)-1-(4-fluorophenyl)-3-oxoisoindoline-5-carboxylic acid **A77** (200 mg, yellow solid), yield: 68%. **LCMS:** *m*/*z* 486.2, 487.0(M+H); RT = 1.510 min (2.5 min).

A portion of the resulting 2-(4-cyclopropyl-1,3-dimethyl-2-oxo-1,2,3,4-tetrahydropyrido[2,3-b]pyrazin-6-yl)-1-(4-fluorophenyl)-3-oxoisoindoline-5-carboxylic acid (A77) was resolved as follows:
Chiral separation conditions (SFC):
column: AD-H, eluent: 70% CO₂ + 30% EtOH (DEA), flow rate: 12.5 mL /min, to yield A77-P1, A77-P2, A77-P3 and A77-P4 respectively.
**A77-P1: LCMS:** *m*/*z* 486.2, 486.7(M+H); RT = 1.504 min (2.5 min).

**¹H NMR (CD₃OD, 400 MHz)** δ 8.55 (s, 1H), 8.28 (d, *J* = 7.8 Hz, 1H), 7.92 (d, *J* = 8.4 Hz, 1H), 7.50 (d, *J* = 7.8 Hz, 1H), 7.42 - 7.32 (m, 3H), 7.03 (t, *J* = 8.6 Hz, 2H), 6.77 (s, 1H), 4.13 (dd, *J* = 12.8, 6.0 Hz, 1H), 3.31 (s, 3H), 2.42-2.35 (m, 1H), 1.26 - 1.17 (m, 1H), 1.03 (d, *J* = 6.6 Hz, 3H), 0.82-0.80 (m, 1H), 0.64 - 0.49 (m, 1H).

**A77-P2: LCMS:** *m*/*z* 486.2, 487.1 (M+H); RT = 1.513 min (2.5 min).

**¹H NMR (CD₃OD, 400 MHz)** δ 8.54 (s, 1H), 8.27 (d, *J* = 8.2 Hz, 1H), 7.92 (d, *J* = 8.4 Hz, 1H), 7.50 (d, *J* = 7.6 Hz, 1H), 7.39 - 7.31 (m, 3H), 7.03 (t, *J* = 8.4 Hz, 2H), 6.77 (s, 1H), 4.13 (dd, *J* = 14.4, 7.4 Hz, 1H), 3.31 (s, 3H), 2.44 - 2.34 (m, 1H), 1.28 - 1.17 (m, 1H), 1.03 (d, *J* = 6.8 Hz, 3H), 0.85 - 0.76 (m, 1H), 0.65 - 0.52 (m, 1H).

**A77-P3: LCMS:** *m*/*z* 486.2, 486.7(M+H); RT = 1.499 min (2.5 min).

**¹H NMR (CD₃OD, 400 MHz)**δ 8.53 (s, 1H), 8.27 (d, *J* = 8.2 Hz, 1H), 7.81 (d, *J* = 8.4 Hz, 1H), 7.57 (d, *J* = 8.2 Hz, 1H), 7.42-7.37 (m, 3H), 7.04 (t, *J* = 8.6 Hz, 2H), 6.89 (s, 1H), 4.15 (dd, *J* = 13.4, 6.4 Hz, 1H), 3.31 (s, 3H), 2.73 - 2.64 (m, 1H), 1.23 (d, *J* = 6.8 Hz, 3H), 0.94-0.87 (m, 2H), 0.79 - 0.65 (m, 1H), 0.55 - 0.45 (m, 1H), 0.08-0.02 (m, 1H).

**A77-P4: LCMS:** *m*/*z* 486.2, 486.6(M+H); RT = 1.499 min (2.5 min).

**¹H NMR (CD₃OD, 400 MHz)** δ 8.53 (s, 1H), 8.27 (d, *J* = 8.0 Hz, 1H), 7.81 (d, *J* = 8.4 Hz, 1H), 7.56 (d, *J* = 8.2 Hz, 1H), 7.43-7.37 (m, 3H), 7.04 (t, *J* = 8.5 Hz, 2H), 6.89 (s, 1H), 4.15 (dd, *J* = 14.2, 7.2 Hz, 1H), 3.31 (s, 3H), 2.73 - 2.64 (m, 1H), 1.23 (d, *J* = 6.8 Hz, 3H), 0.97 - 0.85 (m, 1H), 0.78-0.70 (m, 1H), 0.54 - 0.45 (m, 1H), 0.10 - 0.01 (m, 1H).

### Step 8: (4-cyclopropyl-6-(1-(4-fluorophenyl)-5-(4-methylpiperazine-1-carbonyl)-3-oxoisoindo lin-2-yl)-1,3-dimethyl-3,4-dihydropyrido[2,3-b]pyrazin-2(1H)-one (A78)

Into a dry 100 mL round-bottom flask, compound **A77** (95 mg, 0.195 mmol), compound **11** (24 mg, 0.234 mmol), a solution of 1-propylphosphoric anhydride in ethyl acetate (50%, 0.5 mL, 0.29 mmol), triethylamine (59 mg, 0.585 mmol) and dichloromethane (10mL) were sequentially added at RT. The mixture was stirred at RT for 16 hours, and concentrated under reduced pressure. The obtained residue was purified by preparative chromatography (CAN-H₂O (0.1 %TFA) = 30-40) to yield (4-cyclopropyl-6-(1-(4-fluorophenyl)-5-(4-methylpiperazine-1-carbonyl)-3-oxoisoindolin-2 - yl)-1,3-dimethyl-3,4-dihydropyrido[2,3-b]pyrazin-2(1H)-one **A78** (30 mg, white solid). **LCMS:** *m*/*z* 568.2, 568.6 (M+H).

### Resolution of 4-cyclopropyl-6-(1-(4-fluorophenyl)-5-(4-methylpiperazine-1-carbonyl)-3-oxois oindolin-2-yl)-1,3-dimethyl-3,4-dihydropyrido[2,3-b]pyrazin-2(1H)-one (A78)

Chiral separation conditions (SFC):
column: IB-H, eluent: 70% CO₂ + 30% IPA(DEA), flow rate: 12.5 mL /min, to yield A78-P3 and A78-P4 respectively.
**A78-P3** (white solid, 0.91mg)

**LCMS:** *m*/*z* 568.2, 568.9(M+H); RT = 1.279 min (2.5 min).

**¹H NMR (CD₃OD, 400 MHz)** δ 7.95 (s, 1H), 7.82 (d, *J* = 8.6 Hz, 1H), 7.68 (d, *J* = 7.8 Hz, 1H), 7.57 (d, *J* = 7.8 Hz, 1H), 7.46 - 7.36 (m, 2H), 7.04 (t, *J* = 8.6 Hz, 1H), 6.88 (s, 1H), 4.15 (dd, *J* = 13.8, 7.2 Hz, 1H), 3.84-0.75 (m, 1H), 3.55 - 3.44 (m, 2H), 3.31 (s, 3H), 2.70-2.65 (m, 1H), 2.63 - 2.41 (m, 4H), 2.35 (s, 3H), 1.23 (d, *J=* 6.9 Hz, 3H), 0.94-0.87 (m, 1H), 0.80 - 0.67 (m, 1H), 0.53 - 0.41 (m, 1H), 0.12 - -0.06 (m, 1H).

**A78-P4** (white solid, 2.63mg)

**LCMS:** *m*/*z* 568.2, 568.7(M+H); RT = 1.278 min (2.5 min).

**¹H NMR (CD₃OD, 400 MHz)** δ 7.95 (s, 1H), 7.82 (d, *J* = 8.2 Hz, 1H), 7.69 (d, *J* = 8.4 Hz, 1H), 7.57 (d, *J* = 7.8 Hz, 1H), 7.43 - 7.35 (m, 3H), 7.04 (t, *J* = 8.6 Hz, 2H), 6.88 (s, 1H), 4.15 (dd, *J* = 11.8, 5.4 Hz, 1H), 3.95 - 3.74 (m, 2H), 3.58 - 3.45 (m, 2H), 3.31 (s, 3H), 2.76 - 2.48 (m, 5H), 2.39 (s, 3H), 1.23 (d, *J=* 6.8 Hz, 3H), 0.93-0.89 (m, 1H), 0.80 - 0.68 (m, 1H), 0.54 - 0.40 (m, 1H), 0.09 - -0.01 (m, 1H).

### Intermediate 170: Synthesis of Methyl 1-(4-fluorophenyl)-3-oxoisoindoline-4-carboxylate (170)

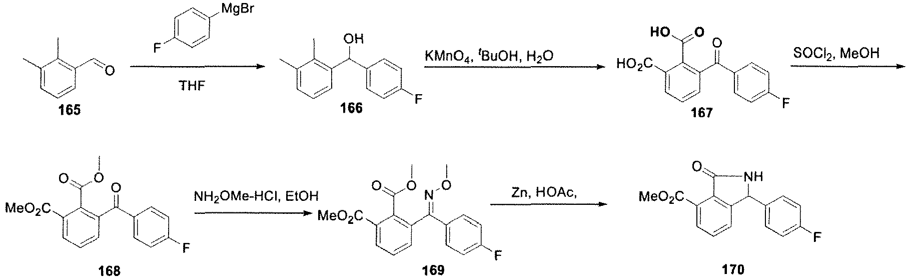

### Step 1: (2,3-dimethylphenyl)(4-fluorophenyl)methanol (166)

Into a dry 100 mL three-necked flask, compound **165** (0.5 g, 3.73 mmol) and dry tetrahydrofuran (10mL) were sequentially added at RT, followed by addition of the solution of tetrafluorophenyl magnesium bromide in tetrahydrofuran (1.0M, 7.5 mL, 7.46 mmol). The mixture was purged with nitrogen gas for three times, and stirred at RT for 16 hours. After the completion of reaction, the mixture was quenched with methanol (10mL), and concentrated under reduced pressure. The obtained residue was purified by column chromatography (ethyl acetate : petroleum ether = 5:1) to yield (2,3-dimethylphenyl)(4-fluorophenyl)methanol **166** (750 mg, colorless liquid), yield: 87%. **LCMS:** *m*/*z* 230.1, 213.0 (M-17); RT = 1.607 min (2.5 min).

### Step 2: 3-(4-fluorobenzoyl)phthalic acid (167)

Into a dry 100 mL round-bottom flask, compound **166** (0.75 g, 3.26 mmol), KMnO₄(3.6 g, 22.8 mmol), t-butyl alcohol (25mL) and water (25mL) were sequentially added at RT. The mixture was heated to 80°C under stirring for 16 hours, and then filtered. The filtrate was concentrated under reduced pressure. Water (50mL) was added to the residue. The mixture was adjusted to pH 3-4 with 6 N aqueous hydrochloric acid, and extracted with ethyl acetate (30mL×3). The combined organic phase was dried over anhydrous Na₂SO₄ and filtered. The filtrate was concentrated under reduced pressure to yield 3-(4-fluorobenzoyl)phthalic acid **167** (0.84 g, colorless liquid), purity: 90%. **LCMS:** *m*/*z* 288.0, 270.8 (M-18); RT = 1.318 min (2.5 min).

### Step 3: Dimethyl 3-(4-fluorobenzoyl)phthalate (168)

Into a dry 100 mL round-bottom flask, compound **167** (0.84 g, 2.91 mmol) and methanol (15mL) were sequentially added at RT, and thionyl chloride (2.06 g, 17.5 mmol) was added dropwise under ice bath. The mixture was stirred at RT for 16 hours, and concentrated under reduced pressure to yield dimethyl 3-(4-fluorobenzoyl)phthalate **168** (1.8 g, brown liquid), purity: 50%. **LCMS:** *m*/*z* 316.1, 284.8(M-32); RT = 1.544 min (2.5 min).

### Step 4: Dimethyl (E)-3-((4-fluorophenyl)(methoxyimino)methyl)phthalate (169)

Into a dry 100 mL sealed tube, compound **168** (0.6 g, 1.893 mmol), methoxyamine hydrochloride (1.57 g, 18.93 mmol) and ethanol (20mL) were added. The mixture was heated to 95°C under stirring for 16 hours. After the completion of reaction, the mixture was concentrated under reduced pressure, and 40 mL water was added to the residue. The mixture was extracted with ethyl acetate (20mL×3). The combined organic phase was dried over anhydrous Na₂SO₄ and filtered. The filtrate was concentrated under reduced pressure to yield dimethyl (*E*)-3-((4-fluorophenyl)(methoxyimino)methyl)phthalate **169** (0.65 g, yellow liquid), yield: 99%. **LCMS:** *m*/*z* 345.1, 346.0(M+H); RT = 1.607 min (2.5 min).

### Step 5: Methyl 1-(4-fluorophenyl)-3-oxoisoindoline-4-carboxylate (170)

Into a dry 100 mL round-bottom flask, compound **169** (0.65 g, 1.882 mmol), zinc powder (1.22 g, 18.82 mmol) and acetic acid (40mL) were sequentially added at RT. The mixture was heated to 90°C under stirring for 1 hour. The mixture was filtered, and the filtrate was concentrated under reduced pressure. The obtained residue was purified by column chromatography (ethyl acetate : petroleum ether = 1:1) to yield methyl 1-(4-fluorophenyl)-3-oxoisoindoline-4-carboxylate **170** (0.4 g, yellow solid), yield: 74%. **LCMS:** *m*/*z* 285.0, 286.0 (M+H); RT = 1.379 min (2.5 min).

### Intermediate 171: Synthesis of 3-(4-fluorophenyl)-7-(2-hydroxypropan-2-yl)isoindolin-1-one (171)

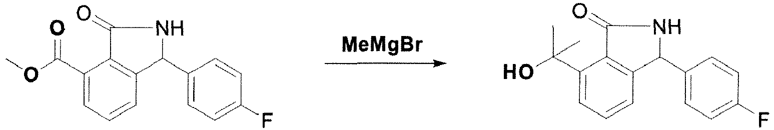

Into a dry 100 mL three-necked flask, **170** (500 mg, 1.754 mmol) and tetrahydrofuran (15mL) were sequentially added at RT, and the solution of methylmagnesium bromide in diethyl ether (3.0M, 1.5 mL, 4.386 mmol) was added dropwise under ice bath. The mixture was purged with nitrogen gas for three times, and was stirred at RT for 16 hours. After the completion of reaction, the mixture was quenched with methanol (15mL), and concentrated under reduced pressure. The obtained residue was purified by column chromatography (ethyl acetate : petroleum ether = 1:2) to yield 3-(4-fluorophenyl)-7-(2-hydroxypropan-2-yl)isoindolin-1-one **171** (180 mg, light brown solid), yield: 36%. **LCMS:** *m*/*z* 258.1, 258.8(M+H); RT = 1.382 min (2.5 min).

### Intermediate 176: Synthesis of Methyl 3-oxo-1-(pyridin-3-yl)isoindoline-4-carboxylate (176)

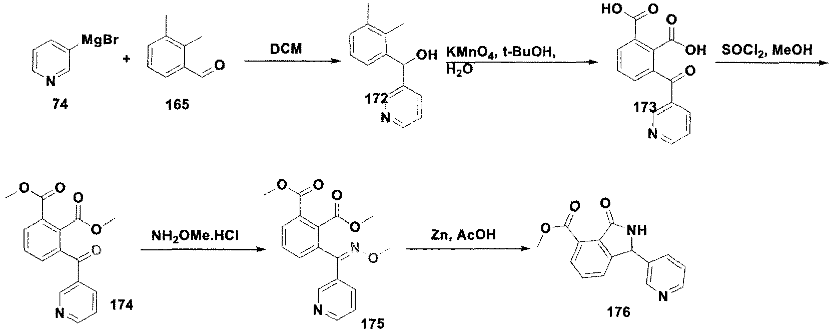

### Step 1: Synthesis of (2,3-dimethylphenyl)(pyridin-3-yl)methanol (172)

Into a dry 500 mL three-necked flask, compound **65** (2.12 g, 15.82 mmol) and dry dichloromethane (50mL) were added. The mixture was purged with nitrogen gas for three times, and the solution of compound **74** in tetrahydrofuran (150 mL, 63.29 mmol) was added. The mixture was stirred at RT for 16 hours, and filtered. The filtrate was concentrated under reduced pressure. The obtained residue was purified by column chromatography (dichloromethane:methanol = 40:1) to yield (2,3-dimethylphenyl)(pyridin-3-yl)methanol **172** (2.2 g, crude, yellow solid). **LCMS:** *m*/*z* 214.1 (M+H); RT = 1.143 min (2.5 min).

### Step 2: Synthesis of 3-nicotinoylphthalic acid (173)

Into a dry 250 mL single-necked flask, compound **172** (5.1 g, 23.9 mmol), t-butyl alcohol (50mL), water (50mL) and potassium permanganate (37.7 g, 239 mmol) were sequentially added at RT. The mixture was heated to 80°C under stirring for 16 hours, and then cooled to RT and filtered. The filter cake was washed with methanol (100mL), and the filtrate was concentrated under reduced pressure to yield 3-nicotinoylphthalic acid **173** (crude 6.4 g, yellow solid), which was directly used in the next reaction. **LCMS:** *m*/*z* 272.1 (M+H); RT = 1.045 min (2.5 min).

### Step 3: Synthesis of Dimethyl 3-nicotinoylphthalate (174)

Into a dry 250 mL single-necked flask, compound **173** (6.4 g, 23.6 mmol) and anhydrous methanol (50mL) were added, and sulfoxide chloride (10mL) was added. The mixture was heated to reflux under stirring for 2 hours, and then concentrated under reduced pressure. The obtained residue was purified by column chromatography (dichloromethane:methanol = 20:1) to yield dimethyl 3-nicotinoylphthalate **174** (1.6 g), yield over two steps: 22%. **LCMS:** *m*/*z* 299.9 (M+H); RT = 1.209 min (2.5 min).

### Step 4: Synthesis of Dimethyl (Z)-3-((methoxyimino)(pyridin-3-yl)methyl)phthalate (175)

Into a dry 125 mL glass sealed tube, compound **174** (1.6 g, 5.35 mmol), O-methylhydroxylamine hydrochloride (4.5 g, 53.5 mmol) and ethanol (40mL) were sequentially added at RT. The mixture was heated to 90°C under stirring for 16 hours. After the completion of reaction as monitored on TLC plate, the mixture was concentrated under reduced pressure. The residue was diluted with water, and extracted with dichloromethane (30mL×2). The combined organic phase was dried over anhydrous Na₂SO₄ and filtered. The filtrate was concentrated under reduced pressure. The obtained residue was purified by column chromatography (dichloromethane:methanol = 20:1) to yield dimethyl (Z)-3-((methoxyimino)(pyridin-3-yl)methyl)phthalate **175** (1.3 g, yellow oil), yield: 74%. **LCMS:** *m*/*z* 329.0 (M+H); RT = 1.372 min (2.5 min).

### Step 5: Synthesis of Methyl 3-oxo-1-(pyridin-3-yl)isoindoline-4-carboxylate (176)

Into a dry 100 mL single-necked flask, compound **175** (500 mg, 1.52 mmol), zinc powder (790 mg, 12.16 mmol) and acetic acid (8mL) were sequentially added at RT. The mixture was heated to 100°C under stirring for 2 hours. After the completion of reaction as monitored on TLC plate, water was added (20mL), and the mixture was extracted with dichloromethane (20mL×2). The organic phases were combined, dried over anhydrous Na₂SO₄ and filtered. The filtrate was concentrated under reduced pressure. The obtained residue was purified by column chromatography (dichloromethane:methanol = 10:1) to yield methyl 3-oxo-1-(pyridin-3-yl)isoindoline-4-carboxylate **176** (160 mg, white solid), yield: 39%. **LCMS:** *m*/*z* 268.8 (M+H); RT = 0.938 min (2.5 min).

### Intermediate 181: Synthesis of Methyl 3-carbonyl-1-phenylisoindoline-4-carboxylate (181)

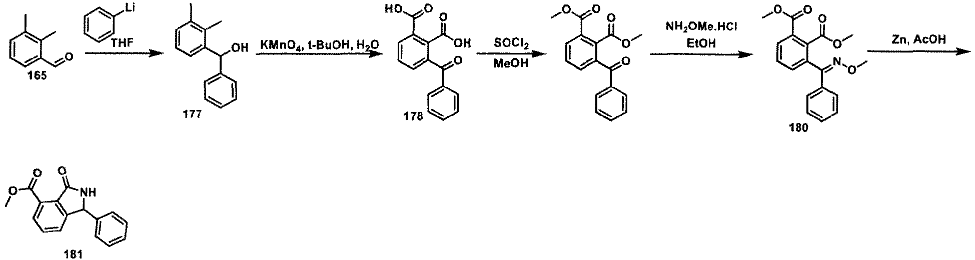

### Step 1. Synthesis of (2,3-dimethylphenyl)(phenyl)methanol (177)

Into a dry 250 mL three-necked flask, compound **165** (3.0 g, 22.4 mmol) and dry tetrahydrofuran (100mL) were added. The mixture was cooled to -78°C, and phenyllithium (2N solution in n-butyl ether, 13mL, 26mmol) was added dropwise. After the completion of addition dropwise, the mixture was stirred at -78°C for 1 hour. After the completion of reaction as monitored by LCMS, the mixture was quenched with ice water (100mL), and extracted with ethyl acetate (100mL^{∗}3). The organic phases were combined, dried, filtered, and concentrated. The obtained residue was purified by column chromatography (petroleum ether:ethyl acetate = 20:1) to yield (2,3-dimethylphenyl)(phenyl)methanol **177** (3.69 g, white solid). yield: 77.8%. **LCMS:** *m*/*z* 194.9(M-H₂O+H); RT = 1.581 min (2.5 min).

### Step 2. 3-benzoylphthalic acid (178)

Into a dry 250 mL single-necked flask, compound **177** (3.69 g, 0.017 mol), t-butyl alcohol (50mL), water (50mL) and potassium permanganate (27.5 g, 0.17 mol) were sequentially added at RT. The mixture was heated to 80°C under stirring for 18 hours. After the completion of reaction as monitored by LCMS, the mixture was cooled to RT and filtered. The filter cake was washed with methanol (100mL), and the filtrate was concentrated under reduced pressure to yield 3-benzoylphthalic acid **178** (crude 6.0 g, white solid), which was directly used in the next reaction. **LCMS:** *m*/*z* 268.9 (M-H); RT = 1.146 min (2.5 min).

### Step 3. Synthesis of Dimethyl 3-benzoylphthalate (179)

Into a dry 250 mL single-necked flask, compound **178** (5.0 g, crude), anhydrous methanol (50mL) and sulfoxide chloride (10mL) were added. The mixture was heated to 70°C under stirring for 18 hours. After the completion of reaction as monitored by LCMS, the mixture was concentrated under reduced pressure. The obtained residue was purified by column chromatography (petroleum ether:ethyl acetate = 5:1) to yield dimethyl 3-benzoylphthalate **179** (1.53 g, white solid). **LCMS:** *m*/*z* 320.7 (M+H); RT = 1.509 min (2.5 min).

### Step 4. Synthesis of Dimethyl (E)-3-((methoxyimino)(phenyl)methyl)phthalate (180)

Into a dry 125 mL glass sealed tube, compound **179** (1.9 g, 6.37 mmol), O-methylhydroxylamine hydrochloride (5.3 g, 63.8 mmol) and ethanol (30mL) were sequentially added at RT. The mixture was heated to 90°C under stirring for 18 hours. After the completion of reaction as monitored by LCMS, the mixture was concentrated under reduced pressure. The residue was dissolved by addition of ethyl acetate, and filtered. The filtrate was concentrated. The obtained residue was purified by column chromatography (petroleum ether:ethyl acetate = 5:1) to yield dimethyl (E)-3-((methoxyimino)(phenyl)methyl)phthalate **180** (2.3 g, colorless oil), **LCMS:** *m*/*z* 327.7 (M+H); RT = 1.590 min (2.5 min).

### Step 5. Synthesis of Methyl 3-oxo-1-phenylisoindoline-4-carboxylate (181)

Into a dry 100 mL single-necked flask, compound **180** (1.0 g, 3.058 mmol), zinc powder (1988 mg, 30.58 mmol) and acetic acid (50mL) were sequentially added at RT. The mixture was heated to 100°C under stirring for 6 hours. After the completion of reaction as monitored by LCMS, the mixture was filtered. The filtrate was concentrated, diluted with ethyl acetate, and adjusted to pH 7-8 with aqueous sodium bicarbonate solution. The organic phases were combined, and concentrated. The obtained residue was purified by column chromatography (petroleum ether:ethyl acetate = 1:1), to yield methyl 3-oxo-1-phenylisoindoline-4-carboxylate **181** (650 mg, pale yellow solid), yield: 79.6%. **LCMS:** *m*/*z* 267.9 (M+H); RT = 1.365 min (2.5 min).

The following examples were synthesized analogously to the synthesis scheme of Example A78.

| | | | |
|---|---|---|---|
| A79 | 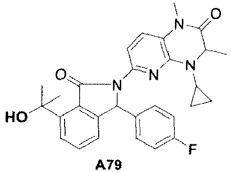 | 4-cyclopropyl-6-(1-(4-fluorophenyl)-4-(2-hydroxyprop-2-yl)-3-oxoisoindolin-2-yl)-1,3-dimethyl-3,4-dihydropyrido[2,3-b]pyrazin-2(1 H)-one (A79) | **A79-P1** |
| | | | **LCMS:** *m*/*z* 500.2, 500.7(M+H); RT = 1.666 min (2.5 min). |
| | | | **¹H NMR (CD₃OD, 400 MHz)** δ 7.75 (d, J = 8.4 Hz, 1H), 7.58 (t, *J=* 7.6 Hz, 1H), 7.53 (d, *J=* 7.6 Hz, 1H), 7.41 (d, *J* = 8.4 Hz, 1H), 7.38 - 7.31 (m, 3H), 7.03 (t, *J* = 8.6 Hz, 2H), 6.82 (s, 1H), 4.16 (q, *J* = 6.8 Hz, 1H), 3.30 (s, 3H), 2.78 - 2.64 (m, 1H), 1.75 (s, 3H), 1.70 (s, 3H), 1.24 (d, *J* = 6.8 Hz, 3H), 1.07 - 0.95 (m, 1H), 0.84 - 0.66 (m, 1H), 0.54-0.48 (m, 1H), 0.15-0.09 (m, 1H). |
| | | | **A79-P2** |
| | | | **LCMS:** *m*/*z* 500.2, 500.7(M+H); RT = 1.665 min (2.5 min). |
| | | | **¹H NMR (CD₃OD, 400 MHz) δ** 7.75 (d, *J* = 8.4 Hz, 1H), 7.58 (t, *J=* 7.6 Hz, 1H), 7.53 (d, *J=* 7.6 Hz, 1H), 7.41 (d, *J* = 8.4 Hz, 1H), 7.38 - 7.31 (m, 3H), 7.02 (t, *J=* 8.6 Hz, 2H), 6.82 (s, 1H), 4.16 (q, *J* = 6.8 Hz, 1H), 3.30 (s, 3H), 2.73-2.67 (m, 1H), 1.75 (s, 3H), 1.70 (s, 3H), 1.23 (d, *J=* 6.8 Hz, 3H), 1.07 - 0.96 (m, 1H), 0.83 - 0.68 (m, 1H), 0.59 - 0.46 (m, 1H), 0.15-0.09 (m, 1H). |
| | | | **A79-P3** |
| | | | **LCMS:** *m*/*z* 500.2, 500.7(M+H); RT = 1.684 min (2.5 min). |
| | | | **¹H NMR (CD₃OD, 400 MHz)** δ 7.89 (d, *J* = 8.4 Hz, 1H), 7.61 - 7.52 (m, 2H), 7.37 (d, *J* = 8.6 Hz, 1H), 7.31 (t, *J* = 9.2 Hz, 3H), 7.01 (t, *J* = 8.6 Hz, 2H), 6.72 (s, 1H), 4.14 (q, *J* = 6.8 Hz, 1H), 3.31 (s, 3H), 2.45-2.41 (m, 1H), 1.76 (s, 3H), 1.72 (s, 3H), 1.26-1.21 (m, 1H), 1.03 *(d, J=* 6.8 Hz, 3H), 0.87 - 0.74 (m, 1H), 0.65 - 0.54 (m, 1H). |
| | | | **A79-P4** |
| | | | **LCMS:** *m*/*z* 500.2, 500.7(M+H); RT = 1.683 min (2.5 min). |
| | | | **¹H NMR (CD₃OD, 400 MHz)** δ 1H NMR (400 MHz, MeOD) δ 7.89 (d, *J* = 8.5 Hz, 0H), 7.57 (dt, *J* = 14.4, 7.4 Hz, 1H), 7.37 (d, *J* = 8.5 Hz, 1H), 7.35 - 7.27 (m, 1H), 7.01 (t, *J=* 8.7 Hz, 1H), 6.72 (s, 0H), 4.14 (q, *J* = 6.9 Hz, 1H), 3.31 (s, 1H), 2.48 - 2.40 (m, 0H), 1.76 (s, 1H), 1.72 (s, 1H), 1.27 - 1.18 (m, 1H), 1.03 (d, *J* = 6.9 Hz, 1H), 0.81 (dd, *J* = 9.1, 4.7 Hz, 1H), 0.59 (td, *J* = 7.8, 4.1 Hz, 1H). |
| A80 | | Methyl 2-(4-cyclopropyl-1,3-dimethyl-2-oxo-1,2,3,4-tetrahydropyrido[2,3-b]pyrazin-6-yl)-1-(4-fluorophenyl) -3-oxoisoindoline-4-carboxylate **(A80)** | **A80-P1** |
| | | | **LCMS:** *m*/*z* 500.2, 500.7(M+H); RT = 1.623 min (2.5 min). |
| | | | **¹H NMR (CD₃OD, 400 MHz)** δ 7.87 (d, *J* = 8.6 Hz, 1H), 7.68 (q, *J* = 7.6 Hz, 2H), 7.52 (d, *J* = 6.8 Hz, 1H), 7.40 - 7.31 (m, 3H), 7.02 (t, *J* = 8.6 Hz, 2H), 6.71 (s, 1H), 4.12 (dd, *J=* 13.6, 6.6 Hz, 1H), 4.04 (s, 3H), 3.30 (s, 3H), 2.39 (ddd, *J* = 11.4, 7.6, 3.6 Hz, 1H), 1.27 - 1.19 (m, 1H), 1.02 (d, *J=* 6.8 Hz, 3H), 0.84 - 0.75 (m, 1H), 0.66 - 0.54 (m, 2H). |
| | 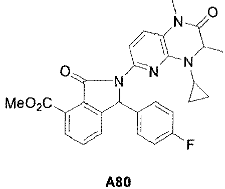 | | **A80-P2** |
| | | | **LCMS:** *m*/*z* 500.2, 500.6 (M+H); RT = 1.623 min (2.5 min). |
| | | | **¹H NMR (CD₃OD, 400 MHz)** δ 7.87 (d, *J* = 8.4 Hz, 1H), 7.72 - 7.60 (m, 2H), 7.52 (d, *J* = 6.6 Hz, 1H), 7.40 - 7.30 (m, 3H), 7.02 (t, *J* = 8.6 Hz, 2H), 6.71 (s, 1H), 4.12 (dd, *J* = 13.8, 6.8 Hz, 1H), 4.04 (s, 3H), 3.30 (s, 3H), 2.39 (ddd, *J* = 10.8, 7.2, 3.8 Hz, 1H), 1.28 - 1.18 (m, 1H), 1.02 (d, *J* = 6.8 Hz, 3H), 0.80-0.78 (m, 1H), 0.67 - 0.52 (m, 1H). |
| | | | **A80-P3** |
| | | | **LCMS:** *m*/*z* 500.2, 500.7 (M+H); RT = 1.609 min (2.5 min). |
| | | | **¹H NMR (CD₃OD, 400 MHz)** δ 7.76 (d, *J* = 8.4 Hz, 1H), 7.71 - 7.63 (m, 2H), 7.59 (d, *J* = 6.8 Hz, 1H), 7.39 (dd, *J* = 11.4, 5.8 Hz, 3H), 7.03 (t, *J* = 8.6 Hz, 2H), 6.83 (s, 1H), 4.15 (q, *J* = 6.8 Hz, 1H), 4.02 (s, 3H), 3.29 (s, 3H), 2.73 - 2.64 (m, 1H), 1.22 (d, *J* = 6.8 Hz, 3H), 0.97-0.89 (m, 1H), 0.79 - 0.69 (m, 1H), 0.53 - 0.43 (m, 1H), 0.10 - 0.01 (m, 1H). |
| | | | **A80-P4** |
| | | | **LCMS:** *m*/*z* 500.2, 500.7 (M+H); RT = 1.609 min (2.5 min). |
| | | | **¹H NMR (CD₃OD, 400 MHz)** δ 7.76 (d, *J* = 8.4 Hz, 1H), 7.71 - 7.62 (m, 2H), 7.59 (d, *J* = 7.2 Hz, 1H), 7.39 (dd, *J* = 11.2, 6.8 Hz, 3H), 7.03 (t, *J* = 8.6 Hz, 2H), 6.83 (s, 1H), 4.15 (q, *J* = 6.8 Hz, 1H), 4.03 (s, 3H), 3.29 (s, 3H), 2.73 - 2.62 (m, 1H), 1.22 (d, *J* = 6.8 Hz, 3H), 1.00 - 0.87 (m, 1H), 0.79 - 0.69 (m, 1H), 0.52-0.46 (m, 1H), 0.15 - 0.02 (m, 1H). |
| A81 | 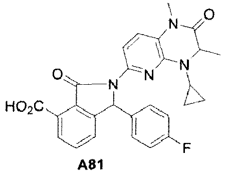 | 2-(4-cyclopropyl-1,3-dimethyl-2-oxo-1,2,3,4-tetrahydropyrido[2,3-b]pyrazin-6-yl)-1-(4-fluorophenyl) -3-oxoisoindoline-4-carboxylic acid (A81) | **A81-P1** |
| | | | **LCMS:** *m*/*z* 486.2, 486.7(M+H); RT = 1.609 min (2.5 min). |
| | | | **¹H NMR (CD₃OD, 400 MHz)** δ 8.33 (d, *J* = 7.6 Hz, 1H), 7.90 - 7.77 (m, 2H), 7.66 (d, *J* = 7.8 Hz, 1H), 7.43 - 7.35 (m, 3H), 7.04 (t, J = 8.8 Hz, 2H), 6.85 (s, 1H), 4.16 (dd, *J=* 13.8, 6.8 Hz, 1H), 3.32 (s, 3H), 2.49 - 2.42 (m, 1H), 1.29 - 1.21 (m, 1H), 1.05 (d, *J=* 6.8 Hz, 3H), 0.86 - 0.78 (m, 1H), 0.65 - 0.56 (m, 1H). |
| | | | **A81-P2** |
| | | | **LCMS:** *m*/*z* 486.2, 486.6 (M+H); RT = 1.609 min (2.5 min). |
| | | | **¹H NMR (CD₃OD, 400 MHz)** 8.34 (d, *J=* 7.8 Hz, 1H), 7.89 - 7.78 (m, 2H), 7.67 (d, *J* = 7.4 Hz, 1H), 7.44 - 7.35 (m, 3H), 7.04 (t, *J* = 8.8 Hz, 2H), 6.86 (s, 1H), 4.16 (dd, *J* = 13.8, 6.6 Hz, 1H), 3.32 (s, 3H), 2.51 - 2.43 (m, 1H), 1.30 - 1.22 (m, 1H), 1.05 (d, *J* = 6.8 Hz, 3H), 0.84-0.79 (m, 1H), 0.66 - 0.54 (m, 1H). |
| | | | **A81-P3** |
| | | | **LCMS:** *m*/*z* 486.2, 486.7 (M+H); RT = 1.609 min (2.5 min). |
| | | | **¹H NMR (CD₃OD, 400 MHz)** δ 8.32 (d, *J* = 7.8 Hz, 1H), 7.81 (t, *J* = 7.6 Hz, 1H), 7.73 (t, *J* = 8.6 Hz, 2H), 7.47 - 7.37 (m, 3H), 7.05 (t, *J* = 8.8 Hz, 2H), 6.96 (s, 1H), 4.18 (dd, *J* = 13.6, 6.8 Hz, 1H), 3.31 (s, 3H), 2.74-2.67 (m, 1H), 1.25 (d, *J* = 6.8 Hz, 3H), 1.07 - 0.97 (m, 1H), 0.82 - 0.72 (m, 1H), 0.55-0.49 (m, 1H), 0.19 - 0.09 (m, 1H). |
| | | | **A81-P4** |
| | | | **LCMS:** *m*/*z* 486.2, 486.7 (M+H); RT = 1.597 min (2.5 min). |
| | | | **¹H NMR (CD₃OD, 400 MHz)** δ 8.32 (d, *J* = 7.8 Hz, 1H), 7.81 (t, *J* = 7.6 Hz, 1H), 7.73 (t, *J* = 8.0 Hz, 2H), 7.47 - 7.39 (m, 3H), 7.05 (t, *J* = 8.8 Hz, 2H), 6.96 (s, 1H), 4.25 - 4.11 (m, 1H), 3.31 (s, 3H), 2.76 - 2.64 (m, 1H), 1.25 (d, *J* = 6.8 Hz, 2H), 1.09 - 0.96 (m, 1H), 0.84 - 0.70 (m, 1H), 0.59 - 0.46 (m, 1H), 0.17 - 0.09 (m, 1H). |
| A82 | 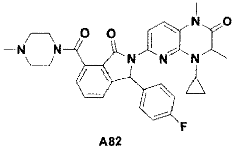 | (4-cyclopropyl-6-(1-(4-fluorophenyl) -4-(4-methylpiperazine-1-carbonyl)-3-oxoisoindolin-2-yl)-1,3-dimethyl-3,4-dihydropyrido[2,3-b]pyrazin-2(1 H)-one (A82) | **A82-P1** (white solid, 4.1mg) |
| | | | **LCMS:** *m*/*z* 568.2, 568.9(M+H); RT = 1.283 min (2.5 min). |
| | | | **¹H NMR (CD₃OD, 400 MHz)** δ 7.85 (d, *J* = 8.4 Hz, 1H), 7.71 (t, *J* = 7.7 Hz, 1H), 7.47 (dd, *J* = 14.8, 7.8 Hz, 2H), 7.35 (d, *J* = 8.4 Hz, 3H), 7.03 (dd, *J* = 16.6, 8.2 Hz, 2H), 6.73 (d, *J* = 9.6 Hz, 1H), 4.13 (d, *J* = 6.8 Hz, 1H), 3.42 (s, 2H), 3.30 (s, 3H), 3.07 - 2.29 (m, 10H), 1.11 - 0.99 (m, 3H), 0.95 - 0.86 (m, 1H), 0.85 - 0.76 (m, 1H), 0.59 (s, 2H). |
| | | | **A82-P2** (white solid, 7.5mg) |
| | | | **LCMS:** *m*/*z* 568.2, 568.9(M+H); RT = 1.280 min (2.5 min). |
| | | | **¹H NMR (CD₃OD, 400 MHz)** δ 7.74 - 7.66 (m, 2H), 7.54 (d, *J* = 8.2 Hz, 1H), 7.49 - 7.34 (m, 4H), 7.04 (dd, *J* = 17.8, 8.8 Hz, 2H), 6.85 (s, 1H), 4.16 (dd, *J* = 12.6, 6.6 Hz, 1H), 3.53 - 3.41 (m, 2H), 3.30 (d, *J* = 3.6 Hz, 3H), 3.13 - 2.53 (m, 10H), 1.23 (d, *J* = 6.8 Hz, 3H), 1.09 - 0.97 (m, 1H), 0.95 - 0.85 (m, 1H), 0.78-0.74 (m, 1H), 0.55 - 0.43 (m, 1H). |
| | | | **A82-P3** (white solid, 2.1mg) |
| | | | **LCMS:** *m*/*z* 568.2, 568.9(M+H); RT = 1.285 min (2.5 min). |
| | | | **¹H NMR (CD₃OD, 400 MHz)** δ 7.82 (d, *J* = 8.6 Hz, 1H), 7.70 (t, *J* = 7.6 Hz, 1H), 7.50 - 7.41 (m, 2H), 7.35 (d, *J* = 8.4 Hz, 3H), 7.02 (q, *J* = 8.6 Hz, 3H), 6.73 (d, *J* = 8.3 Hz, 1H), 4.17 - 4.09 (m, 1H), 4.08 - 3.98 (m, 1H), 3.93 - 3.83 (m, 1H), 3.30 (s, 3H), 2.91 - 2.34 (m, 10H), 1.03 (dd, *J* = 6.6, 3.4 Hz, 3H), 0.95 - 0.87 (m, 1H), 0.85 - 0.75 (m, 1H), 0.64 - 0.53 (m, 2H). |
| | | | **A82-P4** (white solid, 6.7mg) |
| | | | **LCMS:** *m*/*z* 568.2, 568.9(M+H); RT = 1.280 min (2.5 min). |
| | | | **¹H NMR (CD₃OD, 400 MHz)** δ 7.71 (t, *J* = 7.9 Hz, 2H), 7.59 - 7.49 (m, 2H), 7.46-7.37 (m, 4H), 7.04 (dd, *J* = 18.6, 10.0 Hz, 2H), 6.85 (s, 1H), 4.23 - 4.11 (m, 1H), 3.43 (s, 2H), 3.30 (d, *J* = 5.0 Hz, 3H), 3.07 - 2.50 (m, 1H), 1.22 (d, *J* = 6.8 Hz, 3H), 1.06 - 0.95 (m, 1H), 0.95 - 0.84 (m, 1H), 0.80 - 0.68 (m, 0H), 0.56 - 0.42 (m, 1H). |
| A83 | 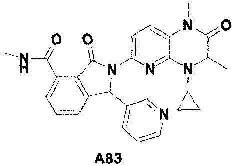 | 2-(4-cyclopropyl-1,3-dimethyl-2-oxo-1,2,3,4-tetrahydropyrido[2,3-b]pyrazin-6-yl)-N-methyl-3-oxo-1-(pyridin-3-yl)isoindoline-4-carboxamide (A83) | **A83-P1** |
| | | | **LCMS:** *m*/*z* 482.8 (M+H); RT = 1.210 min (2.5 min). |
| | | | **¹H NMR (400 MHz, CDCl₃)** δ 11.01 (s, 1H), 8.81 (s, 1H), 8.60 (d, *J* = 7.8 Hz, 1H), 8.50 (s, 1H), 7.83 (d, *J* = 8.4 Hz, 1H), 7.70 (t, *J* = 7.6 Hz, 1H), 7.37 (t, *J* = 7.2 Hz, 2H), 7.22 - 7.16 (m, 1H), 7.11 (d, *J* = 8.4 Hz, 1H), 6.65 (s, 1H), 4.14 (q, *J* = 6.8 Hz, 1H), 3.28 (s, 3H), 3.13 (d, *J=* 4.8 Hz, 3H), 2.43 - 2.37 (m, 1H), 1.17 - 1.10 (m, 1H), 1.07 (d, *J* = 6.8 Hz, 3H), 0.84 - 0.77 (m, 1H), 0.69 - 0.62 (m, 1H), 0.60 - 0.53 (m, 1H). |
| | | | **A83-P2** |
| | | | **LCMS:** *m*/*z* 482.8 (M+H); RT = 1.207 min (2.5 min). |
| | | | **¹H NMR (400 MHz, CDCl₃)** δ11.00 (s, 1H), 8.81 (s, 1H), 8.60 (d, *J* = 7.8 Hz, 1H), 8.49 (s, 1H), 7.83 (d, *J* = 8.4 Hz, 1H), 7.69 (t, *J* = 7.6 Hz, 1H), 7.37 (t, J = 8.4 Hz, 2H), 7.21 - 7.15 (m, 1H), 7.11 (d, *J* = 8.4 Hz, 1H), 6.65 (s, 1H), 4.14 (q, *J* = 6.8 Hz, 1H), 3.28 (s, 3H), 3.13 (d, *J=* 4.4 Hz, 3H), 2.43 - 2.36 (m, 1H), 1.17 - 1.11 (m, 1H), 1.07 (d, *J* = 6.8 Hz, 3H), 0.84 - 0.76 (m, 1H), 0.69 - 0.62 (m, 1H), 0.61 - 0.53 (m, 1H). |
| | | | **A83-P3** |
| | | | **LCMS:** *m*/*z* 482.8 (M+H); RT = 1.190 min (2.5 min). |
| | | | **¹H NMR (400 MHz, CDCl₃)** δ11.02 (s, 1H), 8.75 (s, 1H), 8.58 (d, *J* = 7.8 Hz, 1H), 8.51 (s, 1H), 7.69 (t, *J* = 7.8 Hz, 2H), 7.44 (d, J = 7.4 Hz, 2H), 7.23-7.17 (m, 1H), 7.14 (d, *J* = 8.4 Hz, 1H), 6.75 (s, 1H), 4.16 (q, *J* = 6.8 Hz, 1H), 3.28 (s, 3H), 3.12 (d, *J* = 4.4 Hz, 3H), 2.68 - 2.57 (m, 1H), 1.24 (d, J = 6.8 Hz, 3H), 0.92 - 0.84 (m, 1H), 0.77 - 0.68 (m, 1H), 0.55 - 0.45 (m, 1H), 0.23 - 0.15 (m, 1H). |
| | | | **A83-P4** |
| | | | **LCMS:** *m*/*z* 482.8 (M+H); RT = 1.200 min (2.5 min). |
| | | | **¹H NMR (400 MHz, CDCl₃)** δ11.02 (s, 1H), 8.75 (s, 1H), 8.58 (d, *J=* 7.8 Hz, 1H), 8.51 (d, *J* = 4.0 Hz, 1H), 7.69 (t, *J* = 7.8 Hz, 2H), 7.46 - 7.38 (m, 2H), 7.22 - 7.17 (m, 1H), 7.14 (d, *J* = 8.4 Hz, 1H), 6.75 (s, 1H), 4.15 (q, *J* = 6.8 Hz, 1H), 3.28 (s, 3H), 3.12 (d, *J=* 4.4 Hz, 3H), 2.65 - 2.57 (m, 1H), 1.24 (d, *J* = 6.8 Hz, 3H), 0.91 - 0.85 (m, 1H), 0.79 - 0.69 (m, 1H), 0.53 - 0.46 (m, 1H), 0.22 - 0.15 (m, 1H). |
| A84 | 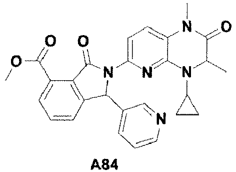 | Methyl 2-(4-cyclopropyl-1,3-dimethyl-2-oxo-1,2,3,4-tetrahydropyrido[2,3-b]pyrazin-6-yl)-3-oxo-1-(pyridin-3-yl)isoindoline-4-carboxylate (A84) | **A84-P1** |
| | | | **LCMS:** *m*/*z* 483.7 (M+H); RT = 1.277 min (2.5 min). |
| | | | **¹H NMR (400 MHz, CDCl₃)** δ8.83 (s, 1H), 8.48 (d, *J=* 4.0 Hz, 1H), 7.96 (d, *J* = 8.8 Hz, 1H), 7.66 (d, *J* = 7.4 Hz, 1H), 7.60 (t, *J* = 7.6 Hz, 1H), 7.38 (d, *J* = 7.6 Hz, 2H), 7.19 - 7.12 (m, 1H), 7.08 (d, *J* = 8.4 Hz, 1H), 6.60 (s, 1H), 4.11 (q, *J* = 6.8 Hz, 1H), 4.07 (s, 3H), 3.26 (s, 3H), 2.33 - 2.26 (m, 1H), 1.14 - 1.07 (m, 1H), 1.04 (d, J = 6.8 Hz, 3H), 0.80 - 0.70 (m, 1H), 0.69 - 0.59 (m, 1H), 0.59 - 0.51 (m, 1H). |
| | | | **A84-P2** |
| | | | **LCMS:** *m*/*z* 483.8 (M+H); RT = 1.285 min (2.5 min). |
| | | | **¹H NMR (400 MHz, CDCl₃)** δ8.84 (s, 1H), 8.48 (d, *J=* 4.4 Hz, 1H), 7.96 (d, *J* = 8.4 Hz, 1H), 7.66 (d, *J* = 7.4 Hz, 1H), 7.59 (t, *J* = 7.6 Hz, 1H), 7.38 (d, *J* = 7.6 Hz, 2H), 7.18 - 7.13 (m, 1H), 7.08 (d, *J* = 8.4 Hz, 1H), 6.60 (s, 1H), 4.11 (q, *J* = 6.8 Hz, 1H), 4.07 (s, 3H), 3.26 (s, 3H), 2.33 - 2.27 (m, 1H), 1.15 - 1.08 (m, 1H), 1.04 (d, *J* = 6.8 Hz, 3H), 0.80 - 0.73 (m, 1H), 0.66 - 0.59 (m, 1H), 0.58 - 0.48 (m, 1H). |
| A85 | | N-cyclopropyl-2-(1,3-dimethyl-2-oxo-4-(2,2,2-trifluoroethyl)-1,2,3,4-tetrahydropyrido[2,3-b]pyrazin-6-yl)-1-(4-fluorophenyl) -3-oxoisoindoline-4-carboxamide (A85) | **A85-P1** |
| | | | **LCMS:** *m*/*z* 567.1, 567.6(M+H); RT = 1.608 min (2.5 min). |
| | | | **¹H NMR (CD₃OD, 400 MHz)** δ 8.12 (d, *J* = 7.6 Hz, 1H), 7.99 (d, *J* = 8.4 Hz, 1H), 7.70 (t, *J* = 7.8 Hz, 1H), 7.50 (dd, *J* = 19.4, 8.2 Hz, 2H), 7.36 (dd, *J* = 8.6, 5.2 Hz, 2H), 7.07 (t, *J=* 8.6 Hz, 2H), 6.54 (s, 1H), 4.64 (dq, *J=* 18.8, 9.4 Hz, 1H), 4.19 (q, *J* = 6.8 Hz, 1H), 3.61 (dq, *J* = 18.0, 9.0 Hz, 1H), 3.35 (s, 3H), 3.05 - 2.98 (m, 1H), 1.04 (d, *J* = 6.8 Hz, 3H), 0.95 - 0.87 (m, 2H), 0.81 - 0.73 (m, 2H). |
| | 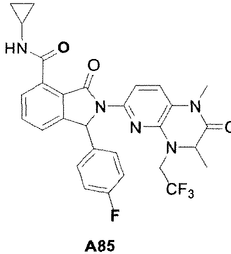 | | **A85-P2** |
| | | | **LCMS:** *m*/*z* 567.1, 567.5(M+H); RT = 1.608 min (2.5 min). |
| | | | **¹H NMR (CD₃OD, 400 MHz) δ** 8.12 (d, *J* = 7.6 Hz, 1H), 8.00 (d, *J* = 8.4 Hz, 1H), 7.71 (t, *J* = 7.8 Hz, 1H), 7.51 (dd, *J* = 17.8, 8.2 Hz, 2H), 7.37 (dd, *J* = 8.6, 5.2 Hz, 2H), 7.07 (t, *J* = 8.6 Hz, 2H), 6.54 (s, 1H), 4.64 (td, *J* = 18.8, 9.4 Hz, 1H), 4.19 (q, *J* = 6.8 Hz, 1H), 3.61 (td, *J* = 18.0, 9.0 Hz, 1H), 3.36 (s, 3H), 3.05 - 3.00 (m, 1H),1.04 (d, *J=* 6.8 Hz, 3H), 0.94 - 0.88 (m, 2H), 0.81 - 0.74 (m, 2H). |
| | | | **A85-P3** |
| | | | **LCMS:** *m*/*z* 567.1, 567.6(M+H); RT = 1.599 min (2.5 min). |
| | | | **¹H NMR (CD₃OD, 400 MHz)** δ 8.12 (d, *J* = 7.6 Hz, 1H), 8.04 (d, *J* = 8.6 Hz, 1H), 7.71 (t, *J* = 7.8 Hz, 1H), 7.51 (t, *J* = 7.8 Hz, 2H), 7.34 (dd, *J* = 8.6, 5.2 Hz, 2H), 7.00 (t, *J* = 8.6 Hz, 2H), 6.67 (s, 1H), 4.89 - 4.82 (m, 1H),4.22 (q, *J=* 6.8 Hz, 1H), 3.67 (td, *J* = 17.6, 8.8 Hz, 1H), 3.35 (s, 3H), 3.07 - 2.98 (m, 1H), 1.22 (d, *J=* 6.8 Hz, 3H), 0.95 - 0.85 (m, 2H), 0.83 - 0.74 (m, 2H). |
| | | | **A85-P4** |
| | | | **LCMS:** *m*/*z* 567.1, 567.6(M+H); RT = 1.599 min (2.5 min). |
| | | | **¹H NMR (CD₃OD, 400 MHz)** δ 8.12 (d, *J* = 7.8 Hz, 1H), 8.03 (d, *J=* 8.6 Hz, 1H), 7.75 - 7.66 (m, 1H), 7.50 (dd, *J* = 14.8, 6.2 Hz, 2H), 7.38 - 7.30 (m, 2H), 7.00 (t, *J* = 8.6 Hz, 2H), 6.66 (s, 1H), 4.92 - 4.80 (m, 1H), 4.22 (q, *J* = 6.8 Hz, 1H), 3.67 (dq, *J* = 17.4, 8.8 Hz, 1H), 3.35 (s, 3H), 3.07 - 2.97 (m, 1H), 1.22 (d, *J* = 6.8 Hz, 3H), 0.95 - 0.87 (m, 2H), 0.82 - 0.71 (m, 2H). |
| A86 | 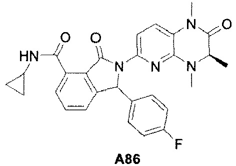 | N-cyclopropyl-1-(4-fluorophenyl)-3-oxo-2-((*R*)-1,3,4-trimethyl-2-oxo-1,2,3,4-tetrahydropyrido[2,3-b]pyrazin-6-yl)isoindoline -4-carboxamide (A86) | **A86-P1** |
| | | | **LCMS:** *m*/*z* 499.7 (M+H); RT = 1.576 min (2.5 min). |
| | | | **¹H NMR (DMSO-*d₆*, 400 MHz):** 9.82 (d, *J* = 4.0 Hz, 1H), 7.88 (d, *J* = 8.0 Hz, 1H), 7.71 - 7.65 (m, 2H), 7.45 - 7.36 (m, 3H), 7.33 (d, *J=* 8.0 Hz, 1H), 7.12 (t, *J=* 8.0 Hz, 2H), 6.70 (s, 1H), 4.12 (q, *J* = 8.0 Hz, 1H), 3.22 (s, 3H), 2.96 - 2.90 (m, 1H), 2.80 (s, 3H), 0.98 (d, *J* = 8.0 Hz, 3H), 0.82 - 0.76 (m, 2H), 0.66 - 0.60 (m, 2H). |
| | | | **A86-P2** |
| | | | **LCMS:** *m*/*z* 500.1 (M+H); RT = 1.604 min (2.5 min). |
| | | | **¹H NMR (DMSO-*d₆*, 400 MHz):** 9.82 (d, *J* = 4.0 Hz, 1H), 7.88 (d, *J=* 8.0 Hz, 1H), 7.71 - 7.65 (m, 2H), 7.45 - 7.36 (m, 3H), 7.33 (d, *J* = 8.0 Hz, 1H), 7.12 (t, *J* = 8.0 Hz, 2H), 6.70 (s, 1H), 4.12 (q, *J* = 8.0 Hz, 1H), 3.22 (s, 3H), 2.96 - 2.90 (m, 1H), 2.80 (s, 3H), 0.98 (d, *J* = 8.0 Hz, 3H), 0.82 - 0.76 (m, 2H), 0.66 - 0.60 (m, 2H). |
| A87 | 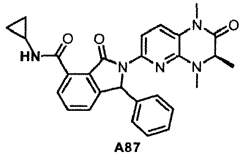 | N-cyclopropyl-3-oxo-1-phenyl-2-((*R*)-1,3,4-trimethyl-2-oxo-1,2,3,4-tetrahydropyrido[2,3-b]pyrazin-6-yl)isoindoline -4-carboxamide (A87) | **A87-P1** |
| | | | **LCMS:** *m*/*z* 481.7 (M+H); RT = 1.584 min (2.5 min). |
| | | | **¹H NMR (400 MHz, DMSO-*d*₆):** δ 9.86 (d, *J=* 4.0 Hz, 1H), 7.87 (d, *J* = 7.4 Hz, 1H), 7.70 - 7.64 (m, 2H), 7.43 (d, *J* = 7.6 Hz, 1H), 7.35 - 7.19 (m, 6H), 6.67 (s, 1H), 4.10 (q, *J* = 6.8 Hz, 1H), 3.21 (s, 3H), 2.95 - 2.91 (m, 1H), 2.77 (s, 3H), 1.47-1.45 (m, 1H), 0.95 (d, *J* = 6.8 Hz, 3H), 0.93 - 0.90 (m, 1H), 0.79 - 0.77 (m, 1H), 0.64 - 0.62 (m, 1H). |
| | | | **A87-P2** |
| | | | **LCMS:** *m*/*z* 481.7 (M+H); RT = 1.574 min (2.5 min). |
| | | | **1H NMR** (400 **MHz, DMSO-*d*₆)** δ 9.83 (d, *J* = 4.0 Hz, 1H), 7.86 (d, *J* = 7.4 Hz, 1H), 7.74 (d, *J* = 8.4 Hz, 1H), 7.66 (t, *J* = 7.6 Hz, 1H), 7.43 (d, *J* = 7.6 Hz, 1H), 7.36 - 7.19 (m, 6H), 6.68 (s, 1H), 4.12 (q, *J* = 6.8 Hz, 1H), 3.20 (s, 3H), 2.94 - 2.90 (m, 1H), 2.76 (s, 3H), 1.47-1.43 (m, 1H), 1.12 (d, *J* = 6.8 Hz, 3H), 0.95-0.90 (m, 1H), 0.79-0.76 (m, 1H), 0.65-0.62 (m, 1H). |
| A88 | 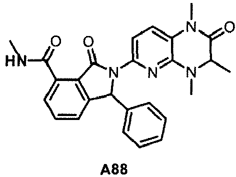 | N-methyl-3-oxo-1-phenyl-2-(1,3,4-trimethyl-2-oxo-1,2,3,4-tetrahydropyrido[2,3-b]pyrazin-6-yl)isoindoline-4-carboxamide (A88) | **A88-P1** |
| | | | **LCMS:** *m*/*z* 455.7 (M+H); RT = 1.525 min (2.5 min). |
| | | | **¹H NMR (400 MHz, DMSO-*d*₆):** δ 9.75 (d, *J=* 4.6 Hz, 1H), 7.89 (d, *J* = 7.4 Hz, 1H), 7.74 - 7.64 (m, 2H), 7.44 (d, *J* = 7.6 Hz, 1H), 7.38 - 7.18 (m, 6H), 6.69 (s, 1H), 4.10 (q, *J* = 6.8 Hz, 1H), 3.21 (s, 3H), 2.91 (d, *J=* 4.6 Hz, 3H), 2.77 (s, 3H), 0.95 (d, *J=* 6.8 Hz, 3H). |
| | | | **A88-P2** |
| | | | **LCMS:** *m*/*z* 455.7 (M+H); RT = 1.516 min (2.5 min). |
| | | | **¹H NMR (400 MHz, DMSO-*d*₆):** δ 9.73 (d, *J* = 4.4 Hz, 1H), 7.88 (d, *J* = 7.4 Hz, 1H), 7.76 (d, *J* = 8.4 Hz, 1H), 7.67 (t, *J* = 7.6 Hz, 1H), 7.44 (d, *J* = 7.6 Hz, 1H), 7.35-7.19 (m, 6H), 6.70 (s, 1H), 4.12 (q, *J* = 6.8 Hz, 1H), 3.20 (s, 3H), 2.91 (d, *J* = 4.6 Hz, 3H), 2.76 (s, 3H), 1.12 (d, *J* = 6.8 Hz, 3H). |

The following examples were synthesized analogously to the synthesis of Example A1:

| | | | |
|---|---|---|---|
| A89 | 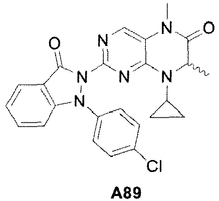 | 1-(4-chlorophenyl)-2-(1,3-dimethyl-2-oxo-4-hydropyrimido[2,3-b]pyrazin-6-yl)isoindoline -4-carboxamide (A89) | **A89-P1** |
| | | | **LCMS:** *m*/*z* 460.1,461.0 (M+H); |
| | | | RT = 1.560min (2.5 min). |
| | | | **¹H NMR (CDCl₃, 400 MHz)** δ 8.01 (d, *J=* 8.0 Hz, 1H), 7.96 (s, 1H), 7.58 (m, 1H), 7.42-7.36 (m, 4H), 7.20 (d, *J* = 8.4 Hz, 1H), 4.24 (q, *J* = 7.2 Hz, 1H), 3.32 (s, 3H), 2.65 (m, 1H), 1.38 (d, *J* = 6.8 Hz, 3H), 1.09-1.03 (m, 1H), 0.84-0.77 (m, 1H), 0.61-0.54 (m, 1H), 0.49-0.43 (m, 1H). |
| | | | **A89-P2** |
| | | | **LCMS:** *m*/*z* 460.1, 461.0 (M+H); RT = 1.560 min (2.5 min). |
| | | | **¹H NMR (CDCl₃, 400 MHz)** δ 8.01 (d, *J* = 8.0 Hz, 1H), 7.96 (s, 1H), 7.58 (m, 1H), 7.42-7.36 (m, 4H), 7.20 (d, *J* = 8.4 Hz, 1H), 4.24 (q, *J* = 7.2 Hz, 1H), 3.32 (s, 3H), 2.65 (m, 1H), 1.38 (d, *J* = 6.8 Hz, 3H), 1.09-1.03 (m, 1H), 0.84-0.77 (m, 1H), 0.61-0.54 (m, 1H), 0.49-0.43 (m, 1H). |
| | | | **A89-P3** |
| | | | **LCMS:** *m*/*z* 460.1,461.0 (M+H); RT = 1.755 min (2.5 min). |
| | | | **¹H NMR (CDCl₃, 400 MHz)** δ 7.76-7.71 (m, 4H), 7.63 (d, *J* = 8.4 Hz, 1H), 7.54-7.47 (m, 3H), 7.23 (q, *J* = 7.2 Hz, 1H), 4.27 (q, *J* = 6.8 Hz, 1H), 3.31 (s, 3H), 2.63 (m, 1H), 1.45 (d, *J* = 7.2 Hz, 3H), 0.82-0.72 (m, 2H), 0.60-0.56 (m, 2H). |
| | | | **A89-P4** |
| | | | **LCMS:** *m*/*z* 460.1, 461.0 (M+H); RT = 1.755 min (2.5 min). |
| | | | **¹H NMR (CDCl₃, 400 MHz)** δ 7.76-7.71 (m, 4H), 7.63 (d, *J* = 8.4 Hz, 1H), 7.54-7.47 (m, 3H), 7.23 (q, *J* = 7.2 Hz, 1H), 4.27 (q, *J* = 6.8 Hz, 1H), 3.31 (s, 3H),2.63 (m, 1H), 1.45 (d, *J* = 7.2 Hz, 3H), 0.82-0.72 (m, 2H), 0.60-0.56 (m, 2H). |

### Intermediate 181a: 2-bromo-5-cyclopropyl-7,8-trimethyl-7,8-dihydropyrimidin-6(5H)-one (181a)

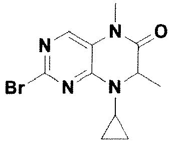

Intermediate 181a was prepared analogously to the synthetic method of (*R*)-2-bromo-5,7,8-trimethyl-7,8-dihydropyrimidin-6(5H)-one (105), starting from cyclopropylamine.

### Intermediate 184: 3-hydroxy-3-(4-(trifluoromethyl)phenyl)isoindolin-1-one (184)

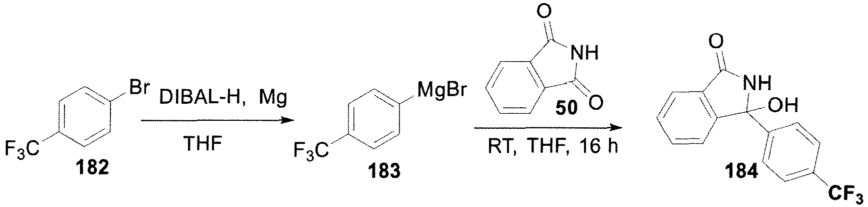

Into a dry 50 mL round-bottom flask, **182** (3.0 g, 13.4 mmol), magnesium chips (0.33 g, 13.73 mmol), tetrahydrofuran (50mL), diisobutylaluminum hydride (1mL) and iodine (one grain) were sequentially added at RT. The mixture was heated to 40°C under stirring for 1 hour. The magnesium chips gradually disappeared. Then the mixture was cooled to RT. Compound **50** (0.49 g, 3.35 mmol) was added. The mixture was stirred at RT for 12 hours, and concentrated under reduced pressure to yield 3-hydroxy-3-(4-(trifluoromethyl)phenyl)isoindolin-1-one **184** (2.1 g, yellow oil), which was directly used in the next reaction without further purification. **LCMS:** *m*/*z* 293.9 (M+H); RT = 1.311 min (2.5 min).

### Intermediate 185: Synthesis of 3-(4-(trifluoromethyl)phenyl)isoindolin-1-one (185)

Into 100 mL round-bottom flask, **184** (2.1 g, crude product), dichloromethane (30mL), trifluoroacetic acid (3mL) and triethylsilane (15mL) were sequentially added at RT. The mixture was warmed to 40°C under stirring for 16 hours, After the completion of reaction, the mixture was concentrated under reduced pressure. The obtained residue was purified by silica gel column chromatography with eluent system (ethyl acetate : petroleum ether = 1:1) to yield 3-(4-(trifluoromethyl)phenyl)isoindolin-1-one **185** (780 mg, off-white solid), total yield over two steps: 79.5 %.

The following examples were synthesized analogously to the synthesis of Example A1.

| | | | |
|---|---|---|---|
| A90 | 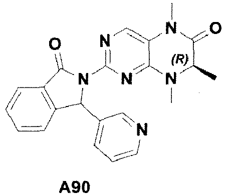 | (7*R*)-5,7,8-trimethyl-2-(1-oxo-3-(pyridin-3-yl)isoindolin-2-yl)-7,8-dihydropteridin-6(5H)-one (A90) | **A90-P1** |
| | | | **LCMS:** *m*/*z* 400.9 (M+H); RT = 0.987 min (2.5 min). |
| | | | **¹H NMR (400 MHz, CDCl₃)**δ 8.80 (s, 1H), 8.50 (s, 1H), 8.02 (d, *J* = 7.2 Hz, 1H), 7.80 (s, 1H), 7.60 - 7.49 (m, 2H), 7.41 (d, *J* = 7.8 Hz, 1H), 7.22 (d, *J* = 7.2 Hz, 1H), 7.19 - 7.14 (m, 1H), 6.46 (s, 1H), 4.17 (q, *J* = 6.8 Hz, 1H), 3.28 (s, 3H), 3.05 (s, 3H), 1.34 (d, *J* = 6.8 Hz, 3H). |
| | | | **A90-P2** |
| | | | **LCMS:** *m*/*z* 400.8 (M+H); RT = 0.997 min (2.5 min). |
| | | | **¹H NMR (400 MHz, CDCl₃)** δ 8.80 (s, 1H), 8.50 (d, *J* = 3.8 Hz, 1H), 8.02 (d, *J* = 7.2 Hz, 1H), 7.82 (s, 1H), 7.60 - 7.49 (m, 2H), 7.44 (d, *J* = 8.0 Hz, 1H), 7.23 (d, *J* = 7.2 Hz, 1H), 7.21 - 7.15 (s, 1H), 6.47 (s, 1H), 4.16 (q, *J* = 6.8 Hz, 1H), 3.28 (s, 3H), 3.01 (s, 3H), 1.40 (d, *J* = 6.8 Hz, 3H). |
| A91 | 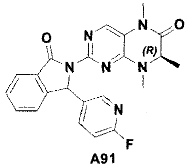 | (7*R*)-2-(1-(6-fluoropyridin-3-yl)-3-oxoisoindolin-2-yl)-5,7,8-trimethyl-7,8-dihydropteridin-6(5H)-one (A91) | **A91-P1** |
| | | | **LCMS:** *m*/*z* 418.8 (M+H); RT = 1.092 min (2.5 min). |
| | | | **¹H NMR (400 MHz, CDCl₃)** δ 8.42 (s, 1H), 8.02 (d, *J* = 7.2 Hz, 1H), 7.78 (s, 1H), 7.62 - 7.51 (m, 2H), 7.47 (t, *J* = 8.0 Hz, 1H), 7.21 (d, *J* = 7.4 Hz, 1H), 6.80 (d, *J* = 8.4 Hz, 1H), 6.48 (s, 1H), 4.20 (q, *J* = 6.4 Hz, 1H), 3.29 (s, 3H), 3.09 (s, 3H), 1.38 (d, *J* = 6.8 Hz, 3H). |
| | | | **A91-P2** |
| | | | **LCMS:** *m*/*z* 418.8 (M+H); RT = 1.100 min (2.5 min). |
| | | | **¹H NMR (400 MHz, CDCl₃)** δ8.42 (s, 1H), 8.02 (d, *J* = 6.8 Hz, 1H), 7.84 (s, 1H), 7.65 - 7.45 (m, 3H), 7.25 - 7.19 (m, 1H), 6.81 (d, *J* = 6.4 Hz, 1H), 6.48 (s, 1H), 4.19 (q, *J* = 6.8 Hz, 1H), 3.30 (s, 3H), 3.05 (s, 3H), 1.41 (d, *J* = 6.6 Hz, 3H). |
| A92 | 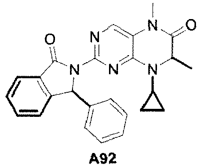 | 8-cyclopropyl-5,7-dimethyl-2-(1-oxo-3-phenylisoindolin-2-yl)-7,8-dihydropteridin-6(5H)-one (A92) | **A92-P1 ¹H NMR (CD₃OD, 400 MHz):** δ7.97 (d, *J=* 7.6 Hz, 1H), 7.92 (s, 1H), 7.65 (t, *J* = 7.6 Hz, 1H), 7.58 (d, *J* = 7.2 Hz, 1H), 7.37-7.24 (m, 6H), 6.61 (s, 1H), 4.24 (q, *J* = 6.8 Hz, 1H) 3.31 (s, 3H), 2.54 (m, 1H), 1.28 (m, 1H), 1.22 (d, *J=* 6.8 Hz, 3H), 0.87 (m, 1H), 0.71 (m, 2H). |
| | | | **A92-P2** (2.62 mg, white solid) |
| | | | **LCMS:** *m*/*z* 425.1, 425.8 (M+H); RT = 1.319 min (2.5 min). |
| | | | **¹H NMR (CD₃OD, 400 MHz):** δ7.97 (d, *J* = 7.6 Hz, 1H), 7.92 (s, 1H), 7.65 (t, *J* = 7.6 Hz, 1H), 7.58 (d, *J* = 7.2 Hz, 1H), 7.37-7.24 (m, 6H), 6.61 (s, 1H), 4.24 (q, *J* = 6.8 Hz, 1H) 3.31 (s, 3H), 2.54 (m, 1H), 1.28 (m, 1H), 1.22 (d, *J=* 6.8 Hz, 3H), 0.87 (m, 1H), 0.71 (m, 2H). |
| | | | **A92-P3** (16.58 mg, white solid) |
| | | | **LCMS:** *m*/*z* 425.1, 425.7 (M+H); RT = 1.327 min (2.5 min). |
| | | | **¹H NMR (CD₃OD, 400 MHz):** δ7.97-7.94 (m, 2H), 7.65 (t, *J* = 8.0 Hz, 1H), 7.57 (d, *J* = 7.6 Hz, 1H), 7.43-7.23 (m, 6H), 6.70 (s, 1H), 4.24 (q, *J* = 6.8 Hz, 1H) 3.31 (s, 3H), 2.77 (m, 1H), 1.39 (d, *J* = 6.8 Hz, 3H), 1.05 (m, 1H), 0.80 (m, 1H), 0.59 (m, 1H), 0.21 (m, 1H). |
| | | | **A92-P4** (24.91 mg, white solid) |
| | | | **LCMS:** *m*/*z* 425.1, 425.7 (M+H); RT = 1.327 min (2.5 min). |
| | | | **¹H NMR (CD₃OD, 400 MHz):** δ7.97-7.94 (m, 2H), 7.65 (t, *J* = 8.0 Hz, 1H), 7.57 (d, *J=* 7.6 Hz, 1H), 7.43-7.23 (m, 6H), 6.70 (s, 1H), 4.24 (q, *J* = 6.8 Hz, 1H) 3.31 (s, 3H), 2.77 (m, 1H), 1.39 (d, *J=* 6.8 Hz, 3H), 1.05 (m, 1H), 0.80 (m, 1H), 0.59 (m, 1H), 0.21 (m, 1H). |
| A93 | 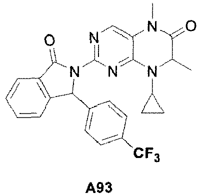 | 8-cyclopropyl-5,7-dimethyl-2-(1-oxo-3-(4-(trifluoromethyl)phenyl)isoindolin-2-yl)-7,8-dihydropteridin-6(5H)-one (A93) | **A93-P1:** HHDED0026-072-P1, 15 mg, pale yellow solid m/z 493.8 (M+H); RT = 1.360 min (2.5 min). |
| | | | ¹H NMR (CD₃OD, 400 MHz): δ7.99 (d, *J* = 7.2 Hz 1H), 7.93 (s,1H), 7.69-7.55 (m, 5H), 7.41 (s,1H), 6.70 (s, 1H), 4.24-4.23 (m, 1H), 3.05-3.00 (m, 1H), 2.46 (s, 1H), 1.30 (t,*J* = 7.2 Hz, 2H), 1.21 (d, *J* = 6.8 Hz,3H), 0.89-0.87 (m, 1H), 0.72 (s,1H). |
| | | | **A93-P2:** HHDED0026-072-P2, 20 mg, pale yellow solid m/z 494.1(M+H); RT = 1.467 min (2.5 min). |
| | | | ¹H NMR (DMSO-*d*₆, 300 MHz): δ7.93 (s,1H), 7.84 (d, *J* = 10.0 Hz, 1H), 7.65-7.51 (m, 5H), 7.35 (d, *J=* 6.0, 1H), 6.65 (s, 1H), 4.18-4.11 (m, 1H), 3.17 (s,3H), 2.92-2.90 (m, 1H), 2.41 (s, 1H), 1.17-1.11 (m, 5H), 0.74-0.71 (m, 1H), 0.65-0.52 (m, 1H). |
| | | | **A93-P3:** HHDED0026-072-P3, 20 mg, pale yellow solid m/z 493.8(M+H); RT = 1.364 min (2.5 min). |
| | | | ¹H NMR (DMSO-*d*₆, 400 MHz): δ8.01 (s,1H), 7.87 (d, *J* = 7.6 Hz, 1H), 7.71-7.55 (m, 5H), 7.42 (d, *J* = 7.2 Hz,1H), 6.77 (s,1H), 4.18-4.16 (m, 1H), 3.30 (s,1H), 3.21 (s,3H), 2.68 (s, 1H),1.29 (d, *J=* 6.8 Hz, 3H), 0.96 (s,1H), 0.67 (s,2H), 0.11 (s,1H). |
| | | | **A93-P4:** HHDED0026-072-P4, 23 mg, pale yellow solid m/z 493.8(M+H); RT = 1.361 min (2.5 min). |
| | | | ¹H NMR (DMSO-*d*₆, 400 MHz): δ8.01 (s,1H), 7.87 (d, *J* = 7.6 Hz, 1H), 7.71-7.55 (m, 5H), 7.42 (d, J = 7.6 Hz, 1H), 6.77 (s,1H), 4.18-4.16 (m, 1H), 3.30 (s,1H), 3.21 (s,3H), 2.68 (s, 1H), 1.29 (d, *J* = 6.8 Hz, 3H), 0.96 (s,1H), 0.67 (s,2H), 0.12 (s,1H). |
| A94 | 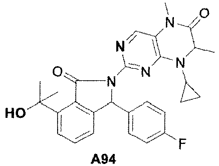 | (7*R*)-8-cyclopropyl-2-(3-oxo-1-(4-fluorophenyl)-4-(2-hydroxyprop -2-yl)isoindolin-2-yl)-5,7-dimethyl-7,8-dihydropteridin-6(5H)-one (A94) | **A94-P1** |
| | | | **LCMS:** *m*/*z* 501.2, 501.7(M+H); RT = 1.351 min (2.5 min). |
| | | | **¹H NMR (CD₃OD, 400 MHz)** δ 7.93 (s, 1H), 7.65 - 7.56 (m, 2H), 7.40 - 7.31 (m, 2H), 7.27 (d, *J* = 7.4 Hz, 1H), 7.05 (t, *J* = 8.8 Hz, 2H), 6.65 (s, 1H), 4.27 (d, *J=* 6.8 Hz, 1H), 3.32 (s, 3H), 2.65 - 2.57 (m, 1H), 1.78 (s, 3H), 1.74 (s, 3H), 1.32 (s, 1H), 1.27 (d, J = 6.8 Hz, 3H), 0.90 (s, 1H), 0.74 (s, 1H). |
| | | | **A94-P2** |
| | | | **LCMS:** *m*/*z* 501.2, 501.7(M+H); RT = 1.372 min (2.5 min) |
| | | | **¹H NMR (CD₃OD, 400 MHz)** δ 7.97 (s, 1H), 7.66 - 7.54 (m, 2H), 7.40 (dd, *J* = 8.2, 5.4 Hz, 2H), 7.32 (d, *J* = 7.4 Hz, 1H), 7.05 (t, *J* = 8.8 Hz, 2H), 6.73 (s, 1H), 4.27 (q, *J* = 6.8 Hz, 1H), 3.31 (s, 3H), 2.79 (s, 1H), 1.76 (s, 3H), 1.73 (s, 3H), 1.40 (d, *J* = 6.8 Hz, 3H), 1.19 - 1.05 (m, 1H), 0.89 - 0.77 (m, 1H), 0.69-0.63 (m, 1H), 0.35-0.28 (m, 1H). |
| | | | **A94-P3** |
| | | | **LCMS:** *m*/*z* 501.2, 501.7(M+H); RT = 1.368 min (2.5 min) |
| | | | **¹H NMR (CD₃OD, 400 MHz)** δ 7.93 (s, 1H), 7.65-7.58 (m, 2H), 7.40-7.32 (m, 2H), 7.27 (d, *J* = 7.2 Hz, 1H), 7.10 - 7.00 (m, 2H), 6.65 (s, 1H), 4.27 (q, *J* = 6.8 Hz, 1H), 3.31 (s, 3H), 2.68 -2.56 (m, 1H), 1.78 (s, 3H), 1.74 (s, 3H), 1.31 (s, 1H), 1.27 (d, *J* = 6.8 Hz, 3H), 0.93-0.83 (m, 1H), 0.75-0.72 (m, 1H). |
| | | | **A94-P4** |
| | | | **LCMS:** *m*/*z* 501.2, 501.9(M+H); RT = 1.488 min (2.5 min). |
| | | | **¹H NMR (CD₃OD, 400 MHz)** δ 7.97 (s, 1H), 7.66 - 7.55 (m, 2H), 7.40 (dd, *J* = 8.8, 5.2 Hz, 2H), 7.32 (d, *J* = 7.4 Hz, 1H), 7.05 (s, 2H), 6.73 (s, 1H), 4.27 (q, *J=* 6.8 Hz, 1H), 3.31 (s, 3H), 2.84 - 2.75 (m, 1H), 1.76 (s, 3H), 1.73 (s, 3H), 1.40 (d, *J* = 6.8 Hz, 3H), 1.18-1.09 (m, 1H), 0.89 - 0.77 (m, 1H), 0.71 - 0.60 (m, 1H), 0.35-0.28 (m, 1H). |
| A95 | 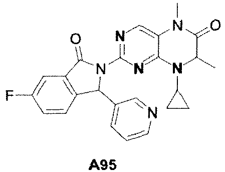 | (7*R*)-8-cyclopropyl-2-(5-fluoro-3-oxo-1-(pyridin-3-yl)isoindolin-2-yl)-5,7-dimethyl-7,8-dihydropteridin-6(5H)-one (A95) | **A95-P1** |
| | | | **LCMS:** *m*/*z* 444.2, 444.7(M+H); RT = 1.175 min (2.5 min). |
| | | | **¹H NMR (CD₃OD, 400 MHz)** δ 8.76 (s, 1H), 8.46 (d, *J* = 4.6 Hz, 1H), 7.92 (s, 1H), 7.66 (dd, *J=* 15.8, 7.6 Hz, 2H), 7.43 (s, 2H), 7.40 - 7.32 (m, 1H), 6.67 (s, 1H), 4.26 (dd, *J* = 13.2, 6.6 Hz, 1H), 3.30 (s, 3H), 2.58 - 2.44 (m, 1H), 1.30 (s, 1H), 1.24 (d, *J* = 6.8 Hz, 3H), 0.91-086 (m, 1H), 0.80 - 0.66 (m, 2H). |
| | | | **A95-P2** |
| | | | **LCMS:** *m*/*z* 444.2, 444.7(M+H); RT = 1.197 min (2.5 min). |
| | | | **¹H NMR (CD₃OD, 400 MHz)** δ 8.78 (s, 1H), 8.47 (d, *J* = 4.7 Hz, 1H), 7.97 (s, 1H), 7.69 (dd, *J* = 18.8, 7.8 Hz, 2H), 7.47 (dd, *J* = 14.0, 9.4 Hz, 2H), 7.37 (dd, *J* = 7.8, 4.8 Hz, 1H), 6.78 (s, 1H), 4.26 (q, *J* = 6.8 Hz, 1H), 3.31 (s, 3H), 2.86-2.73 (m, 1H), 1.38 (d, *J* = 6.8 Hz, 3H), 1.05 - 0.95 (m, 1H), 0.85-0.78 (m, 1H), 0.66-0.59 (m, 1H), 0.27 - 0.15 (m, 1H). |
| | | | **A95-P3** |
| | | | **LCMS:** *m*/*z* 444.2, 444.7(M+H); RT = 1.179 min (2.5 min). |
| | | | **¹H NMR (CD₃OD, 400 MHz)** δ 8.77 (s, 1H), 8.47 (s, 1H), 7.92 (s, 1H), 7.67 (dd, *J* = 19.6, 7.8 Hz, 2H), 7.46-7.44 (m, 2H), 7.39 - 7.32 (m, 1H), 6.68 (s, 1H), 4.26 (d, *J* = 7.0 Hz, 1H), 3.30 (s, 3H), 2.59 - 2.46 (m, 1H), 1.40-1.28 (m, 1H), 1.24 (d, *J=* 6.8 Hz, 3H), 0.92-0.86 (m, 1H), 0.80 - 0.69 (m, 2H). |
| | | | **A95-P4** |
| | | | **LCMS:** *m*/*z* 444.2, 444.8(M+H); RT = 1.191 min (2.5 min) |
| | | | **¹H NMR (CD₃OD, 400 MHz)** δ 8.79 (s, 1H), 8.46 (s, 1H), 7.97 (s, 1H), 7.69 (dd, *J* = 18.4, 8.4 Hz, 2H), 7.52 - 7.41 (m, 2H), 7.37 (dd, *J* = 7.8, 4.8 Hz, 1H), 6.78 (s, 1H), 4.26 (dd, *J* = 13.4, 6.0 Hz, 1H), 3.31 (s, 3H), 2.81-2.76 (m, 1H), 1.38 (d, *J* = 6.8 Hz, 3H), 1.03-0.97 (m, 1H), 0.85-0.78 (m, 1H), 0.68-0.61 (m, 1H), 0.26-0.15 (m, 1H). |
| A96 | 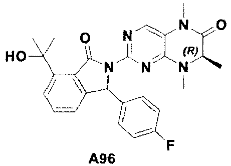 | (7*R*)-2-(1-(4-fluorophenyl)-4-(2-hydroxypropan-2-yl)-3-oxoisoindolin-2-yl)-5,7,8-trimethyl-7,8-dihydropteridin-6(5H)-one (A96) | **A96-P1** |
| | | | **LCMS:** *m*/*z* 475.8 (M+H); RT = 1.238 min (2.5 min). |
| | | | **¹H NMR (400 MHz, CDCl₃)**δ7.59 (t, *J* = 7.6 Hz, 1H), 7.51 (d, *J* = 7.6 Hz, 1H), 7.34 - 7.28 (m, 2H), 7.14 (d, *J* = 6.2 Hz, 2H), 7.08 - 7.02 (m, 3H), 4.29 (s, 1H), 3.33 (s, 3H), 3.01 (s, 3H), 1.73 (d,*J* = 15.2 Hz, 6H), 1.60 (s, 3H). |
| | | | **A96-P2** |
| | | | **LCMS:** *m*/*z* 475.8 (M+H); RT = 1.248 min (2.5 min). |
| | | | **¹H NMR (400 MHz, CDCl₃)** δ7.96 (s, 1H), 7.58 (t, *J=* 7.6 Hz, 1H), 7.50 (d, *J* = 7.6 Hz, 1H), 7.33 - 7.29 (m, 2H), 7.12 (d, *J* = 7.2 Hz, 1H), 7.05 (t, *J* = 8.4 Hz, 2H), 6.87 (s, 1H), 6.38 (s, 1H), 4.34 (q, *J* = 6.8 Hz, 1H), 3.33 (s, 3H), 3.03 (s, 3H), 1.74 (s, 3H), 1.70 (s, 3H), 1.51 (d, *J* = 6.8 Hz, 3H). |
| A97 | | (7R)-2-(5-fluoro-3-oxo-1-(pyridin-3-yl)isoindolin-2-yl)-5,7,8-trimethyl-7,8-dihydropteridin-6(5H)-one (A97) | **A97-P1** |
| | | | **LCMS:** *m*/*z* 418.1, 419.0(M+H); RT = 1.227 min (2.5 min) |
| | | | ¹H NMR (400 MHz, CD₃OD) δ 8.73 (d, *J* = 1.8 Hz, 1H), 8.46 (dd, *J* = 4.8, 1.4 Hz, 1H), 7.84 (s, 1H), 7.72-7.62 (m, 2H), 7.54 - 7.32 (m, 3H), 6.68 (s, 1H), 4.27 (q, *J* = 6.8 Hz, 1H), 3.30(s, 3H), 3.02 (s, 3H), 1.40 (d, *J* = 6.8 Hz, 3H). |
| | 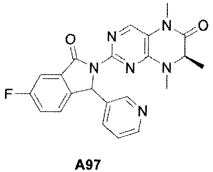 | | **A97-P2** |
| | | | **LCMS:** *m*/*z* 418.1, 418.8(M+H); RT = 1.035 min (2.5 min). ¹H NMR (400 MHz, CD₃OD) δ 8.74 (s, 1H), 8.46 (d, *J* = 3.8 Hz, 1H), 7.83 (s, 1H), 7.66 (d, *J* = 2.0 Hz, 2H), 7.41 (d, *J* = 5.4 Hz, 3H), 6.67 (s, 1H), 4.27 (q, *J* = 6.8 Hz, 1H),3.31(s,3H), 3.07 (s, 3H), 1.30 (d, *J* = 6.8 Hz, 3H). |
| A98 | 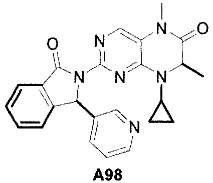 | 8-cyclopropyl-5,7-dimethyl-2-(1-oxo-3-(pyridin-3-yl)isoindolin-2-yl)-7,8-dihydropteridin-6(5H)-one (A98) | **A98-P1**(9.65 mg, white solid) |
| | | | **LCMS:** *m*/*z* 426.1, 426.7 (M+H); RT = 1.145 min (2.5 min). |
| | | | **¹H NMR (CD₃OD, 400 MHz)** δ 8.75 (s, 1H), 8.46 (d, *J* = 4.8 Hz, 1H), 8.00 (d, *J* = 7.6 Hz, 1H), 7.93 (s, 1H), 7.70 (t, *J* = 7.6 Hz, 1H), 7.63 (m, 2H), 7.41 ((d, *J* = 7.6 Hz, 1H), 7.35 (dd, *J* = 7.2 Hz, 5.2 Hz, 1H), 6.69 (s, 1H), 4.26 (q, *J* = 7.2 Hz, 1H), 3.31 (s, 3H), 2.53 (m, 1H), 1.32 (m, 1H), 1.24 (d, *J* = 6.8 Hz, 3H), 0.88 (m, 1H), 0.75 (m, 2H). |
| | | | **A98-P2**(21.55 mg, white solid) |
| | | | **LCMS:** *m*/*z* 426.1, 426.8 (M+H); RT = 1.159 min (2.5 min). . |
| | | | **¹H NMR (CD₃OD, 400 MHz)** δ 8.75 (s, 1H), 8.46 (d, *J* = 4.8 Hz, 1H), 8.00 (d, *J* = 7.6 Hz, 1H), 7.93 (s, 1H), 7.70 (t, *J* = 7.6 Hz, 1H), 7.63 (m, 2H), 7.41 ((d, *J* = 7.6 Hz, 1H), 7.35 (dd, *J=* 7.2 Hz, 5.2 Hz, 1H), 6.69 (s, 1H), 4.26 (q, *J* = 7.2 Hz, 1H), 3.31 (s, 3H), 2.53 (m, 1H), 1.32 (m, 1H), 1.24 (d, *J* = 6.8 Hz, 3H), 0.88 (m, 1H), 0.75 (m, 2H). |
| | | | **A98-P3**(22.59 mg, white solid) |
| | | | **LCMS:** *m*/*z* 426.1, 426.8 (M+H); RT = 1.159 min (2.5 min). |
| | | | **¹H NMR (CD₃OD, 400 MHz)** δ 8.78 (s, 1H), 8.46 (d, *J* = 4.8 Hz, 1H), 7.98 (d, *J* = 7.6 Hz, 1H), 7.70 (dd, *J* = 12.0 Hz, 7.6 Hz, 2H), 7.61 (t, *J* = 7.6 Hz, 1H), 7.46 ((d, *J* = 7.6 Hz, 1H), 7.36 (dd, *J=* 7.2 Hz, 5.2 Hz, 1H), 6.79 (s, 1H), 4.26 (q, *J* = 7.2 Hz, 1H), 3.31 (s, 3H), 2.79 (m, 1H), 1.38 (d, *J* = 6.8 Hz, 3H), 0.99 (m, 1H), 0.82 (m, 1H), 0.63 (m, 1H), 0.20 (m, 1H). |
| | | | **A98-P4**(44.38 mg, white solid) |
| | | | **LCMS:** *m*/*z* 426.1, 427.0 (M+H); RT = 1.239 min (2.5 min). |
| | | | **¹H NMR (CD₃OD, 400 MHz)** δ 9.00 (s, 1H), 8.68 (d, *J* = 4.0 Hz, 1H), 8.13 (d, *J* = 8.4 Hz, 1H), 8.09 (d, *J* = 7.6 Hz, 2H), 7.82 (m, 2H), 7.73-7.64 ((m, 2H), 7.59 (d, *J=* 7.6 Hz, 1H), 6.86 (s, 1H), 4.41 (q, *J* = 7.2 Hz, 1H), 2.95 (m, 1H), 1.56 (d, *J* = 6.8 Hz, 3H), 0.90 (m, 1H), 0.82 (m, 1H), 0.51 (m, 1H), 0.02 (m, 1H). |
| A99 | 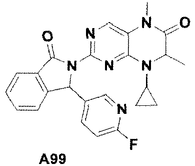 | 8-cyclopropyl-2-(1-(6-fluoropyridin-3-yl)-3-oxoisoindol-2-yl)-5,7-dimethyl-7,8-dihydropteridin-6(5H)-one (A99) | **A99-P1**(19.37 mg, white solid) |
| | | | LCMS: m/z 444.1, 445.0 (M+H); RT = 1.335 min (2.5 min). |
| | | | **¹H NMR (CD₃OD, 400 MHz)** δ8.43 (s, 1H), 8.00 (d, J = 7.6 Hz, 1H), 7.93 (s, 1H), 7.70 (q, J = 7.2 Hz, 2H), 7.64 (t, J = 7.2 Hz, 1H), 7.41 (d, J = 7.2 Hz, 1H), 6.99 (d, J = 8.8 Hz, 1H), 6.70 (s, 1H), 4.28 (q, J = 6.8 Hz, 1H), 3.31 (s, 3H), 2.60 (m, 1H), 1.32 (m, 1H), 1.29 (d, J = 6.8 Hz, 3H), 0.91 (m, 1H), 0.75(m, 2H). |
| | | | **A99-P2(15.36** mg, white solid) |
| | | | **LCMS:** m/z 444.1, 445.1(M+H); RT = 1.336 min (2.5 min) |
| | | | **¹H NMR (CD₃OD, 400 MHz)** δ8.43 (s, 1H), 8.00 (d, J = 7.6 Hz, 1H), 7.93 (s, 1H), 7.70 (q, J = 7.2 Hz, 2H), 7.64 (t, J = 7.2 Hz, 1H), 7.41 (d, J = 7.2 Hz, 1H), 6.99 (d, J = 8.8 Hz, 1H), 6.70 (s, 1H), 4.28 (q, J = 6.8 Hz, 1H), 3.31 (s, 3H), 2.60 (m, 1H), 1.32 (m, 1H), 1.29 (d, J = 6.8 Hz, 3H), 0.91 (m, 1H), 0.75(m, 2H). |
| | | | **A99-P3(26.38** mg, white solid) |
| | | | **LCMS:** m/z 444.1, 445.1(M+H); RT = 1.336 min (2.5 min). |
| | | | **¹H NMR (CD₃OD, 400 MHz)** δ8.45 (s, 1H), 7.98 (d, J = 7.6 Hz, 2H), 7.77 (t, J = 7.2 Hz, 1H), 7.70 (t, J = 7.2 Hz, 1H), 7.62 (t, J = 7.6 Hz, 1H), 7.46 (d, J = 8.0 Hz, 1H), 7.00 (d, J = 10.8 Hz, 1H), 6.79 (s, 1H), 4.28 (q, J = 6.4 Hz, 1H), 3.31 (s, 3H), 2.81 (m, 1H), 1.39 (d, J = 6.8 Hz, 3H), 1.32 (m, 1H), 1.05 (m, 1H), 0.86(m, 1H), 0.68(m, 1H), 0.29(m, 1H). |
| | | | **A99-P4**(22.71 mg, white solid) |
| | | | **LCMS:** m/z 444.1, 445.1(M+H); RT = 1.352 min (2.5 min). |
| | | | **¹H NMR (CD₃OD, 400 MHz)** δ8.45 (s, 1H), 7.98 (d, J = 7.6 Hz, 2H), 7.77 (t, J = 7.2 Hz, 1H), 7.70 (t, J = 7.2 Hz, 1H), 7.62 (t, J = 7.6 Hz, 1H), 7.46 (d, J = 8.0 Hz, 1H), 7.00 (d, J = 10.8 Hz, 1H), 6.79 (s, 1H), 4.28 (q, J = 6.4 Hz, 1H), 3.31 (s, 3H), 2.81 (m, 1H), 1.39 (d, J = 6.8 Hz, 3H), 1.32 (m, 1H), 1.05 (m, 1H), 0.86(m, 1H), 0.68(m, 1H), 0.29(m, 1H). |
| A100 | 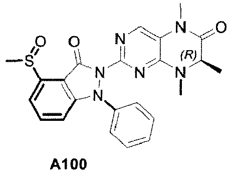 | (3R)-1,3,4-trimethyl-6-(4-(methylsulfinyl)-3-oxo-1-phenyl-1,3-dihydro-2H-indazol-2-yl)-3,4-dihydropyrido[2,3-b]pyrazin-2(1 H)-one (A100) | **A100-P1** |
| | | | LCMS: m/z 462.7 (M+H); RT = 1.379 min (2.5 min). |
| | | | **1H NMR** (400 MHz, CD3OD)δ 8.00 (dd, J = 8.0 Hz,1.2 Hz, 1H), 7.82-7.75 (m, 5H), 7.63 (t, J = 7.6 Hz, 2H), 7.48 (t, J = 7.6 Hz, 1H), 4.32 (q, J = 6.8 Hz, 1H), 3.34 (s, 3H), 2.94(s, 3H), 2.92(s, 3H), 1.45 (d, J = 6.8 Hz, 3H). |
| | | | **A100-P2** |
| | | | LCMS: m/z 462.6 (M+H); RT = 1.380 min (2.5 min). |
| | | | 1H NMR (400 MHz, CD3OD)δ 8.00 (dd, J = 8.0 Hz,1.2 Hz, 1H), 7.82-7.75 (m, 5H), 7.63 (t, J = 7.6 Hz, 2H), 7.48 (t, J = 7.6 Hz, 1H), 4.32 (q, J = 6.8 Hz, 1H), 3.34 (s, 3H), 2.94(s, 3H), 2.92(s, 3H), 1.45 (d, J = 6.8 Hz, 3H). |
| | | | **A100-P3** |
| | | | LCMS: m/z 462.6 (M+H); RT = 1.260 min (2.5 min). |
| | | | 1H NMR (400 MHz, CD3OD)δ 7.89 (m, 2H), 7.78 (d, J = 7.2 Hz, 1H), 7.49-7.41 (m, 5H), 7.37 (m, 1H), 4.29 (q, J = 6.8 Hz, 1H), 3.31 (s, 3H), 3.09(s, 3H), 2.97(s, 3H), 1.37 (d, J = 6.8 Hz, 3H). |
| | | | **A100-P4** |
| | | | LCMS: m/z 462.7 (M+H); RT = 1.259 min (2.5 min). |
| | | | 1H NMR (400 MHz, CD3OD)δ 7.89 (m, 2H), 7.78 (d, J = 7.2 Hz, 1H), 7.49-7.41 (m, 5H), 7.37 (m, 1H), 4.29 (q, *J* = 6.8 Hz, 1H), 3.31 (s, 3H), 3.09(s, 3H), 2.97(s, 3H), 1.37 (d, J = 6.8 Hz, 3H). |
| A101 | 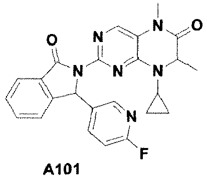 | 8-cyclopropyl-2-(1-(6-fluoropyridin-3-yl)-3-oxoisoindol-2-yl)-5,7-dimethyl-7,8-dihydropteridin-6(5H)-one (**A101**) | **A101-P1**(19.37 mg, white solid) |
| | | | LCMS: m/z 444.1, 445.0 (M+H); RT = 1.335 min (2.5 min). |
| | | | 1H NMR (CD3OD, 400 MHz) δ8.43 (s, 1H), 8.00 (d, J = 7.6 Hz, 1H), 7.93 (s, 1H), 7.70 (q, J = 7.2 Hz, 2H), 7.64 (t, J = 7.2 Hz, 1H), 7.41 (d, J = 7.2 Hz, 1H), 6.99 (d, J = 8.8 Hz, 1H), 6.70 (s, 1H), 4.28 (q, J = 6.8 Hz, 1H), 3.31 (s, 3H), 2.60 (m, 1H), 1.32 (m, 1H), 1.29 (d, J = 6.8 Hz, 3H), 0.91 (m, 1H), 0.75(m, 2H). |
| | | | **A101-P2**(15.36 mg, white solid) |
| | | | LCMS: m/z 444.1, 445.1(M+H); RT = 1.336 min (2.5 min). |
| | | | 1H NMR (CD3OD, 400 MHz) δ8.43 (s, 1H), 8.00 (d, J = 7.6 Hz, 1H), 7.93 (s, 1H), 7.70 (q, J = 7.2 Hz, 2H), 7.64 (t, J = 7.2 Hz, 1H), 7.41 (d, J = 7.2 Hz, 1H), 6.99 (d, J = 8.8 Hz, 1H), 6.70 (s, 1H), 4.28 (q, J = 6.8 Hz, 1H), 3.31 (s, 3H), 2.60 (m, 1H), 1.32 (m, 1H), 1.29 (d, J = 6.8 Hz, 3H), 0.91 (m, 1H), 0.75(m, 2H). |
| | | | **A101-P3**(26.38 mg, white solid) |
| | | | LCMS: m/z 444.1, 445.1(M+H); RT = 1.336 min (2.5 min). |
| | | | 1H NMR (CD3OD, 400 MHz) δ8.45 (s, 1H), 7.98 (d, J = 7.6 Hz, 2H), 7.77 (t, J = 7.2 Hz, 1H), 7.70 (t, J = 7.2 Hz, 1H), 7.62 (t, J = 7.6 Hz, 1H), 7.46 (d, J = 8.0 Hz, 1H), 7.00 (d, J = 10.8 Hz, 1H), 6.79 (s, 1H), 4.28 (q, J = 6.4 Hz, 1H), 3.31 (s, 3H), 2.81 (m, 1H), 1.39 (d, J = 6.8 Hz, 3H), 1.32 (m, 1H), 1.05 (m, 1H), 0.86(m, 1H), 0.68(m, 1H), 0.29(m, 1H). |
| | | | **A101-P4**(22.71 mg, white solid) |
| | | | LCMS: m/z 444.1, 445.1(M+H); RT = 1.352 min (2.5 min). |
| | | | 1H NMR (CD3OD, 400 MHz) δ8.45 (s, 1H), 7.98 (d, J = 7.6 Hz, 2H), 7.77 (t, J = 7.2 Hz, 1H), 7.70 (t, J = 7.2 Hz, 1H), 7.62 (t, J = 7.6 Hz, 1H), 7.46 (d, J = 8.0 Hz, 1H), 7.00 (d, J = 10.8 Hz, 1H), 6.79 (s, 1H), 4.28 (q, J = 6.4 Hz, 1H), 3.31 (s, 3H), 2.81 (m, 1H), 1.39 (d, J = 6.8 Hz, 3H), 1.32 (m, 1H), 1.05 (m, 1H), 0.86(m, 1H), 0.68(m, 1H), 0.29(m, 1H). |
| A102 | 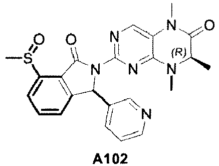 | (7R)-5,7,8-trimethyl-2-(4-(methylsulfinyl)-3-oxo-1-(pyridin-3-yl)isoindolin-2-yl)-7,8-dihydropteridin-6(5H)-one (**A102**) | **A102-P1** |
| | | | **LCMS:** *m*/*z* 462.8 (M+H); RT = 1.050 min (2.5 min). |
| | | | **¹H NMR (400 MHz, CD₃OD)**δ8.92 (s, 1H), 8.61 (s, 1H), 8.18 (d, *J* = 7.6 Hz, 1H), 8.03 (d, *J* = 7.6 Hz, 1H), 7.91 (s, 1H), 7.68 (s, 1H), 7.60 (d, *J* = 7.2 Hz, 1H), 7.53 (s, 1H), 6.83 (s, 1H), 4.47 (q, *J* = 6.4 Hz, 1H), 3.11 (s, 3H), 3.06 (s, 3H), 1.46 (d, *J* = 6.8 Hz, 3H). |
| | | | **A102-P2** |
| | | | **LCMS:** *m*/*z* 463.0 (M+H); RT = 1.062 min (2.5 min). |
| | | | **¹H NMR (400 MHz, CD₃OD)**δ8.78 (d, *J* = 1.6 Hz, 1H), 8.48 (dd, *J=* 5.2 Hz,1.6 Hz,1H), 8.10 (d, *J* = 8.0 Hz, 1H), 7.92 (t, *J* = 8.0 Hz, 1H), 7.83 (s, 1H), 7.76 (m, 1H), 7.56 (d, *J=* 7.2 Hz, 1H), 7.39 (dd, *J=* 7.6 Hz, 4.8 Hz, 1H), 6.81 (s, 1H), 4.28 (q, *J* = 6.4 Hz, 1H), 3.30 (s, 3H), 3.14 (s, 3H), 3.01 (s, 3H), 1.41 (d, *J=* 6.8 Hz, 3H). |
| | | | **A102-P3** |
| | | | **LCMS:** *m*/*z* 463.0 (M+H); RT = 1.046 min (2.5 min). |
| | | | **¹H NMR (400 MHz, CD₃OD)**δ8.78 (d, *J=* 1.6 Hz, 1H), 8.48 (dd, *J* = 5.2 Hz,1.6 Hz,1H), 8.10 (d, *J* = 8.0 Hz, 1H), 7.92 (t, *J* = 8.0 Hz, 1H), 7.83 (s, 1H), 7.76 (m, 1H), 7.56 (d, *J* = 7.2 Hz, 1H), 7.39 (dd, *J* = 7.6 Hz, 4.8 Hz, 1H), 6.81 (s, 1H), 4.28 (q, *J* = 6.4 Hz, 1H), 3.30 (s, 3H), 3.14 (s, 3H), 3.01 (s, 3H), 1.41 (d, *J* = 6.8 Hz, 3H). |
| | | | **A102-P4** |
| | | | **LCMS:** *m*/*z* 462.8 (M+H); RT = 1.052 min (2.5 min). |
| | | | **¹H NMR (400 MHz, CD₃OD)**δ8.92 (s, 1H), 8.61 (s, 1H), 8.18 (d,*J* = 7.6 Hz, 1H), 8.03 (d,*J* = 7.6 Hz, 1H), 7.91 (s, 1H), 7.68 (s, 1H), 7.60 (d, *J* = 7.2 Hz, 1H), 7.53 (s, 1H), 6.83 (s, 1H), 4.47 (q, *J* = 6.4 Hz, 1H), 3.11 (s, 3H), 3.06 (s, 3H), 1.46 (d, *J* = 6.8 Hz, 3H). |
| A103 | 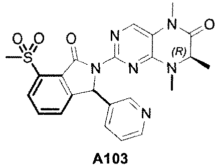 | (7*R*)-5,7,8-trimethyl-2-(4-(methylsulfonyl)-3-oxo-1-(pyridin-3-yl)isoindolin-2-yl)-7,8-dihydropteridin-6(5H)-one **(A103)** | **A103** |
| | | | **LCMS:** *m*/*z* 478.6 (M+H); RT = 1.071 min (2.5 min). |
| | | | **¹H NMR (400 MHz, CD₃OD)**δ 8.80 (s, 1H), 8.48 (d, *J* = 4.4 Hz, 1H), 8.26 (d, *J* = 7.6 Hz, 1H), 7.89 (t, *J* = 7.6 Hz, 1H), 7.85 (s, 1H), 7.73 (m, 2H), 7.38 (dd, *J* = 8.0 Hz, 5.2 Hz, 1H), 6.79 (s, 1H), 4.28 (q, *J* = 6.4 Hz, 1H), 3.67 (s, 3H), 3.31 (s, 3H), 3.03 (s, 3H), 1.42 (d, *J=* 6.8 Hz, 3H). |
| A104 | 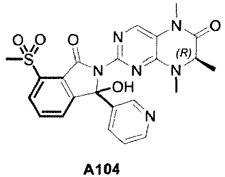 | (7*R*)-2-(1-hydroxy-4-(methylsulfonyl)-3-oxo-1-(pyridin-3-yl)isoindolin-2-yl)-5,7,8-trim ethyl-7,8-dihydropteridin-6(5H)-one **(A104)** | **A104** |
| | | | **LCMS:** *m*/*z* 494.6 (M+H); RT = 1.069 min (2.5 min). |
| | | | **¹H NMR (400 MHz, CD₃OD)**δ 9.03 (s, 1H), 8.62 (s, 1H), 8.34 (t, *J* = 7.2 Hz, 1H), 8.19 (s, 1H), 8.02 (t, *J=* 7.6 Hz, 1H), 7.82 (d, *J* = 7.2 Hz, 1H), 7.70 (s, 1H), 7.60 (s, 1H), 4.39 (q, *J=* 6.4 Hz, 1H), 3.65 (s, 3H), 2.76-2.71 (s, 3H), 1.53-1.42 (d, *J* = 6.8 Hz, 3H). |

### Example A105: Synthesis of (7R)-2-(1-(6-fluoropyridin-3-yl)-3-oxoisoindolin-2-yl)-5,7-dimet hyl-5H-pyrimido[4,5-b][1,4]oxazin-6(7H)-one (A105)

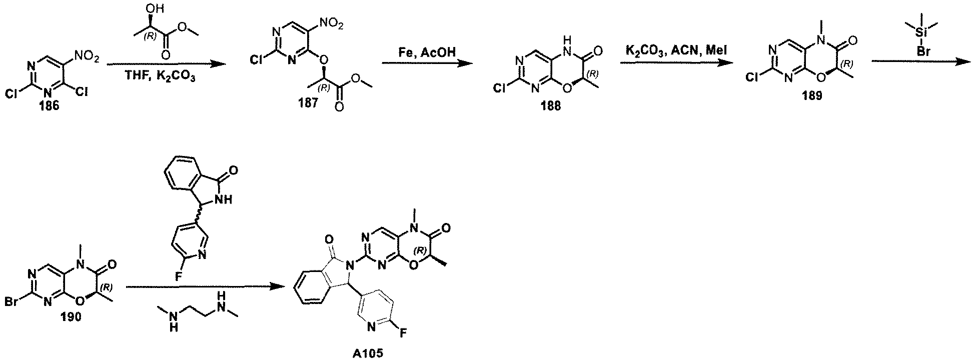

### Step 1: Synthesis of Methyl (R)-2-((2-chloro-5-nitropyrimidin-4-yl)oxy)propionate (187)

Into a dry 250 mL round-bottom flask, compound **186** (6.0 g, 0.031 mol), compound **2** (3.25 g, 0.031 mol), potassium carbonate (8.53 g, 0.062 mol) and tetrahydrofuran (100mL) were sequentially added at RT. The reaction was stirred at RT for 16 hours. After the completion of the reaction as monitored by LCMS, the mixture was concentrated under reduced pressure. The obtained residue was purified by silica gel column chromatography (ethyl acetate : petroleum ether = 1:4) to yield methyl (R)-2-((2-chloro-5-nitropyrimidin-4-yl)oxy)propionate **187** (3 g, pale yellow oil), yield: 37%. **LCMS:** *m*/*z* 261.8 (M+H); RT = 1.495 min (2.5 min).

### Step 2: Synthesis of (R)-2-chloro-7-methyl-5H-pyrimido[4,5-b][1,4]oxazin-6(7H)-one (188)

Into a dry 100 mL round-bottom flask, compound **187** (2 g, 7.66 mmol), iron powder (1.0 g) and acetic acid (30mL) were sequentially added at RT. The mixture was heated to 80°C under stirring for 2 hours. After the completion of the reaction as monitored by TLC, the reaction system was concentrated under reduced pressure. The obtained residue was purified by silica gel column chromatography (ethyl acetate : petroleum ether = 1:3) to yield (*R*)-2-chloro-7-methyl-5H-pyrimido[4,5-b][1,4]oxazin-6(7H)-one **188** (1.5 g, white solid), yield: 98%. **LCMS:** *m*/*z* 199.9 (M+H); RT = 1.181 min (2.5 min).

### Step 3: Synthesis of (R)-2-chloro-5,7-dimethyl-5H-pyrimido[4,5-b][1,4]oxazin-6(7H)-one (189)

Into a dry 100 mL round-bottom flask, compound **188** (1.0 g, 5.02 mmol), acetonitrile (50mL), potassium carbonate (2.08 g, 15.07 mmol) and methyl iodide (1.43 g, 10.07 mmol) were sequentially added under ice bath. The mixture was warmed to RT under stirring for 16 hours. After the completion of the reaction as monitored by LCMS, the mixture was concentrated under reduced pressure. The obtained residue was purified by silica gel column chromatography (ethyl acetate : petroleum ether = 1:2) to yield (*R*)-2-chloro-5,7-dimethyl-5H-pyrimido[4,5-b][1,4]oxazin-6(7H)-one **189** (1.0 g, white solid), yield: 93%. **LCMS:** *m*/*z* 213.9 (M+H); RT = 1.221 min (2.5 min).

### Step 4: Synthesis of (R)-2-bromo-5,7-dimethyl-5H-pyrimido[4,5-b][1,4]oxazin-6(7H)-one (190)

Into a dry 100 mL sealed tube, compound **189** (1.3 g, 6.07 mmol), acetonitrile (20mL) and compound 6 (4.65 g, 30.4 mmol) were sequentially added at RT. The mixture was warmed to 80°C under stirring for 16 hours. After the completion of the reaction as monitored by LCMS, the mixture was concentrated under reduced pressure. The obtained residue was purified by silica gel column chromatography (ethyl acetate : petroleum ether = 1:1) to yield (*R*)-2-bromo-5,7-dimethyl-5H-pyrimido[4,5-b][1,4]oxazin-6(7H)-one **190** (1.0 g, white solid), yield: 63.8%. **LCMS:** *m*/*z* 257.9/259.8 (M+H); RT = 1.241 min (2.5 min).

### Step 5: Synthesis of (7R)-2-(1-(6-fluoropyridin-3-yl)-3-oxoisoindolin-2-yl)-5,7-dimethyl-5H-pyrimido[4,5-b][1,4]oxazin-6(7H)-one (A105)

Into a dry 100 mL round-bottom flask, compound **190** (80 mg, 0.31 mmol), compound **8** (101 mg, 0.44 mmol), potassium phosphate (164 mg, 0.77 mmol), N¹,N²-dimethylethane-1,2-diamine (68 mg, 0.77 mmol), copper iodide (88 mg, 0.46 mmol) and 1,4-dioxane (20mL) were added at RT. The mixture was purged with nitrogen gas for three times, and heated to 100°C under stirring for 16 hours. The mixture was cooled to RT and filtered. The filtrate was concentrated under reduced pressure. The obtained residue was purified by pre-TLC (pure ethyl acetate) to yield (7*R*)-2-(1-(6-fluoropyridin-3-yl)-3-oxoisoindolin-2-yl)-5,7-dimethyl-5H-pyrimido[4,5-b][1,4]oxazin-6(7H)-one **A105** (70 mg)

### Resolution of (7R)-2-(1-(6-fluoropyridin-3-yl)-3-oxoisoindolin-2-yl)-5,7-dimethyl-5H-pyrimid o[4,5-b][1,4]oxazin-6(7H)-one (A105)

Chiral resolution of A105 gave A105-P1: 26 mg, white solid, A126-P2: 16 mg, white solid.

Resolution conditions:
Chiral column: OJ-H
Mobile phase: 70% carbon dioxide + 30% ethanol (0.2% diethylamine)
Flow rate: 40 g/min

**A105-P1: LCMS:** *m*/*z* 405.7 (M+H); RT = 1.372 min (2.5 min).

**¹H NMR (400 MHz, DMSO-*d₆*)** δ 8.39 (s, 1H), 8.35 (s, 1H), 7.89 (d, *J=* 8.0 Hz, 1H), 7.75-7.65 (m, 2H), 7.62-7.56 (m, 1H), 7.39 (d, *J=* 7.6 Hz, 1H), 7.08 (d, *J=* 8.8 Hz, 1H), 6.69 (s, 1H), 5.15 (q, *J* = 7.6 Hz, 1H), 3.23 (s, 3H), 1.52 (d, *J* = 6.8 Hz, 3H)

**A105-P2: LCMS:** *m*/*z* 405.7 (M+H); RT = 1.369 min (2.5 min).

**¹H NMR (400 MHz, DMSO-*d₆*)** δ8.39 (s, 1H), 8.35 (s, 1H), 7.90 (d, *J* = 7.6 Hz, 1H), 7.76-7.66 (m, 2H), 7.63-7.57 (m, 1H), 7.40 (d, *J=* 7.6 Hz, 1H), 7.08 (d, *J=* 8.8 Hz, 1H), 6.69 (s, 1H), 5.16 (q, *J* = 6.8 Hz, 1H), 3.23 (s, 3H), 1.52 (d, *J=* 6.8 Hz, 3H)

The following examples were synthesized analogous to the synthesis of Example A105.

| | | | |
|---|---|---|---|
| A106 | 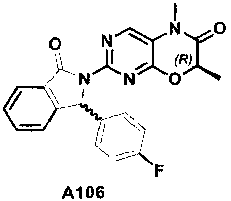 | (*R*)-2-(1-(4-fluorophenyl) -3-oxoisoindolin -2-yl)-5,7-dimethyl-5H-pyrimido[4,5-b][1,4]oxazin -6(7H)-one (A106) | **A106-P1** |
| | | | **LCMS:** *m*/*z* 404.7 (M+H); RT = 1.463 min (2.5 min). |
| | | | **¹H NMR (400 MHz, DMSO-*d₆*)** δ 8.34 (s, 1H), 7.87 (d, *J* = 7.6 Hz, 1H), 7.65 (t, *J* = 7.6 Hz, 1H), 7.57 (t, *J* = 7.6 Hz, 1H), 7.34 (t, *J* = 7.6 Hz, 3H), 7.13 (t, *J* = 8.4 Hz, 2H), 6.62 (s, 1H), 5.18 (q, *J* = 6.8 Hz, 1H), 3.23 (s, 3H), 1.52 (d, *J* = 6.8 Hz, 3H) |
| | | | **A106-P2** |
| | | | **LCMS:** *m*/*z* 404.7 (M+H); RT = 1.464 min (2.5 min). |
| | | | **¹H NMR (400 MHz, CD₃Cl)** δ8.16 (s, 1H), 8.02 (d, *J* = 7.6 Hz, 1H), 7.60-7.49 (m, 2H), 7.30-7.28 (m, 2H), 7.22 (d, *J* = 7.2 Hz, 1H), 6.95 (t, *J* = 8.0 Hz, 2H), 6.45 (s, 1H), 4.96 (q, *J* = 6.8 Hz, 1H), 3.32 (s, 3H), 1.64 (d, *J* = 6.8 Hz, 3H) |

### Intermediate 191: Synthesis of (R)-6-bromo-1-ethyl-3-methyl-1H-pyrido[2,3-B][1,4]oxazin-2(3H)-one (191)

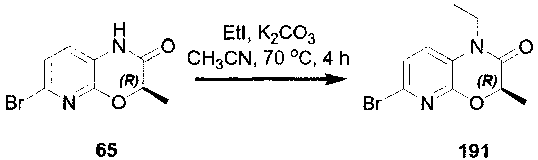

Into a dry 100 mL round-bottom flask, compound **65** (0.23 mg, 0.92 mmol), acetonitrile (20mL), potassium carbonate (0.254 g, 1.84 mmol) and ethyl iodide (1.28 g, 12.72 mmol) were sequentially added at RT. The mixture was warmed to 70°C under stirring for 4 hours. The mixture was cooled to RT, and concentrated under reduced pressure. The obtained residue was purified by column chromatography (petroleum ether:ethyl acetate = 1:1) to yield (*R*)-6-bromo-1-ethyl-3-methyl-1H-pyrido[2,3-B][1,4]oxazin-2(3H)-one **191** (0.18 g, yellow solid), yield: 72 %. **LCMS:** *m*/*z* 270.0, 270.7 (M+H).

### Intermediate 192: Synthesis of (R)-6-bromo-1-(2,2,2-trifluoroethyl)-3-methyl-1H-pyrido[2,3 -B][1,4]oxazin-2(3H)-one (192)

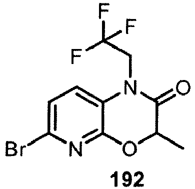

Intermediate 192 was prepared analogous to the the synthetic method of (R)-6-bromo-1-ethyl-3-methyl-1H-pyrido[2,3-B][1,4]oxazin-2(3H)-one (191), starting from trifluoroethyl iodide.

### Intermediate 196: Synthesis of (R)-7-bromo-2,4-dimethyl-2H-pyrido[4,3-b][1,4]oxazin-3(4H) -one (196)

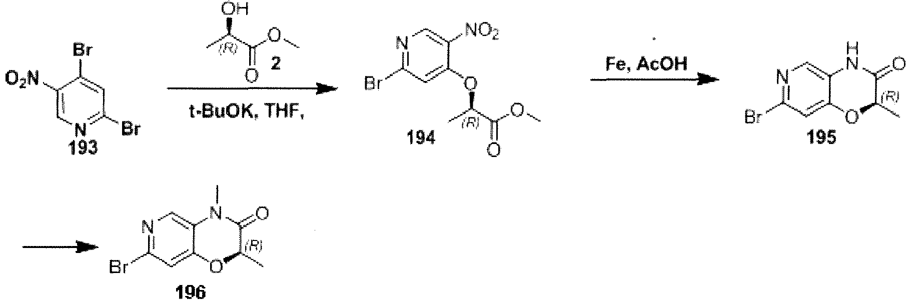

### Step 1: Synthesis of Methyl (R)-2-((2-bromo-5-nitropyridin-4-yl)oxy)propionate (194)

Into a dry 100 mL round-bottom flask, compound **193** (1.7 g, 6.77 mmol), compound **2** (1.06 g, 10.19 mmol), tetrahydrofuran (50mL) and potassium tert-butoxide (1.52 g, 13.57 mmol) were sequentially added at RT. The reaction was warmed to 50°C under stirring for 16 hours. After the completion of the reaction as monitored by LCMS, the mixture was concentrated under reduced pressure. The obtained residue was purified by silica gel column chromatography (ethyl acetate : petroleum ether = 1:10) to yield methyl (R)-2-((2-bromo-5-nitropyridin-4-yl)oxy)propionate **194** (700 mg, pale yellow oil), yield: 38%. **LCMS:** *m*/*z* 304.9/307.0 (M+H); RT = 1.528 min (2.5 min).

### Step 2: Synthesis of (R)-7-bromo-2-methyl-2H-pyrido[4,3-b][1,4]oxazin-3(4H)-one (195)

Into a dry 100 mL round-bottom flask, compound **194** (900 mg, 2.95 mmol), iron powder (1.0 g) and acetic acid (20mL) were sequentially added at RT. The reaction was warmed to 80°C under stirring for 2 hours. After the completion of the reaction as monitored by TLC, the mixture was concentrated under reduced pressure. The obtained residue was purified by silica gel column chromatography (ethyl acetate : petroleum ether = 1:5) to yield (*R*)-7-bromo-2-methyl-2H-pyrido [4,3-b][1,4]oxazin-3(4H)-one **195** (400 mg, white solid), yield: 56%.

### Step 3: Synthesis of (R)-7-bromo-2,4-dimethyl-2H-pyrido[4,3-b][1,4]oxazin-3(4H)-one (196)

Into a dry 100 mL round-bottom flask, compound **195** (300 mg, 1.23 mmol), tetrahydrofuran (20mL) and sodium hydride (197 mg, 4.92 mmol) were sequentially under ice bath. The mixture was stirred for 30 minutes under ice bath. Methyl iodide (351 mg, 2.47 mmol) was added, and the mixture was warmed to RT under stirring for 16 hours. After the completion of the reaction as monitored by LCMS, the mixture was quenched by addition of ice water (50mL), and extracted with ethyl acetate (50mL×3). The combined organic phase was dried over anhydrous Na₂SO₄ and filtered. The filtrate was concentrated under reduced pressure. The obtained residue was purified by silica gel column chromatography (ethyl acetate : petroleum ether = 1:5) to yield (*R*)-7-bromo-2,4-dimethyl-2H-pyrido[4,3-b][1,4]oxazin-3(4H)-one **196** (300 mg, white solid), yield: 63.7%. **LCMS:** *m*/*z* 257.0/259.0 (M+H); RT = 1.629 min (2.5 min).

### Intermediate 201: Synthesis of (R)-6-bromo-7-fluoro-1,3-dimethyl-1H-pyrido[2,3-b][1,4]oxa zin-2(3H)-one (201)

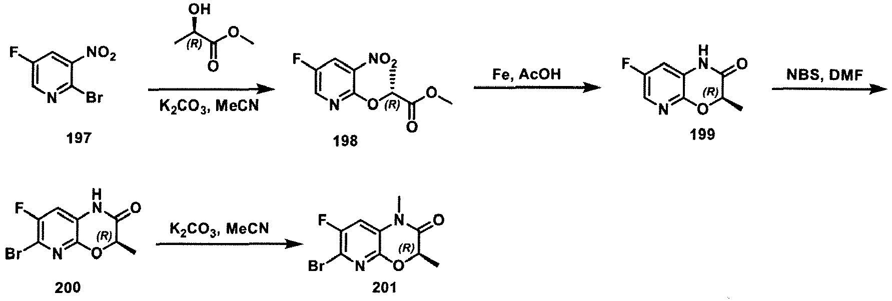

### Step 1: Synthesis of Methyl (R)-2-((5-fluoro-3-nitropyridin-2-yl)oxy)propionate (198)

Into a dry 100 mL round-bottom flask, compound **197** (5.0 g, 0.02 mol), compound **2** (9.4 g, 0.09 mol), potassium carbonate (9.3 g, 0.067 mol) and acetonitrile (50mL) were sequentially added at RT. The mixture was warmed to 90°C under stirring for 48 hours. After the completion of the reaction as monitored by LCMS, the mixture was diluted with water (100mL), and extracted with ethyl acetate (80mL×3). The combined organic phase was dried over anhydrous Na₂SO₄ and filtered. The filtrate was concentrated under reduced pressure. The obtained residue was purified by silica gel column chromatography (ethyl acetate : petroleum ether = 1:10) to yield methyl (R)-2-((5-fluoro-3-nitropyridin-2-yl)oxy)propionate **198** (2.0 g, pale yellow oil), yield: 36.0%. **LCMS:** *m*/*z* 245.0 (M+H); RT = 1.532 min (2.5 min).

### Step 2: Synthesis of (R)-7-fluoro-3-methyl-1H-pyrido[2,3-b][1,4]oxazin-2(3H)-one (199)

Into a dry 100 mL round-bottom flask, compound **198** (2 g, 8.2 mmol), iron powder (1.0 g) and acetic acid (30mL) were sequentially added at RT. The mixture was warmed to 80°C under stirring for 2 hours. After the completion of the reaction as monitored by LCMS, the mixture was concentrated under reduced pressure, diluted with saturated aqueous sodium bicarbonate solution (80mL), and extracted with ethyl acetate (80mL×3). The combined organic phase was dried over anhydrous Na₂SO₄ and filtered. The filtrate was concentrated under reduced pressure. The obtained residue was purified by silica gel column chromatography (ethyl acetate : petroleum ether = 1:3) to yield (*R*)-1-fluoro-3-methyl-1H-pyrido[2,3-b][1,4]oxazin-2(3H)-one **199** (1.0 g, white solid), yield: 66.7%. **LCMS:** *m*/*z* 183.0 (M+H); RT = 0.708 min (2.5 min).

### Step 3: Synthesis of (R)-6-bromo-7-fluoro-3-methyl-1H-pyrido[2,3-b][1,4]oxazin-2(3H)-one (200)

At RT into a dry 100 mL round-bottom flask, compound **199** (780 mg, 4.3 mmol) was added, followed by addition of N-bromosuccinimide (4.6 g, 25.8 mmol) and N,N-dimethylformamide (30mL) in portions. The mixture was warmed to 100°C under stirring for 16 hours. After the completion of the reaction as monitored by TLC, the mixture was diluted with water (100mL), and extracted with ethyl acetate (80mL×3). The organic phases were combined, washed with saturated brine (200mL×2), dried over anhydrous Na₂SO₄ and filtered. The filtrate was concentrated under reduced pressure. The obtained residue was purified by silica gel column chromatography (ethyl acetate : petroleum ether = 1:4) to yield (*R*)-6-bromo-7-fluoro-3-methyl-1H-pyrido[2,3-b][1,4]oxazin-2(3H)-one **200** (1.0 g, white solid), yield: 89.3%. **LCMS:** *m*/*z* 260.9/263.0 (M+H); RT = 1.397 min (2.5 min).

### Step 4: Synthesis of (R)-6-bromo-7-fluoro-1,3-dimethyl-1H-pyrido[2,3-b][1,4]oxazin-2(3H)-one (201)

Into a dry 100 mL round-bottom flask, under ice bath, compound **200** (300 mg, 1.0 mmol), acetonitrile (30mL), potassium carbonate (529 mg, 3.8 mmol) and methyl iodide (816 mg, 5.75 mmol) were sequentially added. The mixture was warmed to RT under stirring for 16 hours. After the completion of the reaction as monitored by LCMS, the mixture was concentrated under reduced pressure. The obtained residue was purified by silica gel column chromatography (ethyl acetate : petroleum ether = 1:5) to yield (*R*)-6-bromo-7-fluoro-1,3-dimethyl-1H-pyrido[2,3-b][1,4]oxazin - 2(3H)-one **201** (550 mg, white solid). **LCMS:** *m*/*z* 275.0/277.0 (M+H); RT = 1.484 min (2.5 min).

### Intermediate 205: Synthesis of 3-(6-methylpyridin-3-yl)isoindolin-1-one (205)

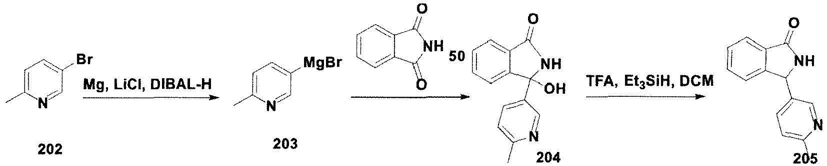

### Step 1: Synthesis of (6-methylpyridin-3-yl)magnesium bromide (203)

Into a dry 100 mL three-necked flask, magnesium (1.14 g, 47.5 mmol), lithium chloride (998 mg, 23.75 mmol) and dry tetrahydrofuran (50mL) were added at RT. The mixture was purged with nitrogen gas for three times, and a solution of diisopropylaluminum hydride in tetrahydrofuran (1.0M, 0.2 mL, 0.2 mmol) was added. The mixture was stirred at RT for 30 minutes. Compound **202** (3.26 g, 19 mmol) was added. The mixture was warmed to 40°C under stirring for 3 hours to yield the solution of (6-methylpyridin-3-yl)magnesium bromide **203,** which was directly used in the next reaction after cooling to RT.

### Step 2: Synthesis of 3-hydroxy-3-(6-methylpyridin-3-yl)isoindolin-1-one (204)

Into a dry 250 mL three-necked flask, compound **203** (698 mg, 4.75 mmol) and dry dichloromethane (20mL) were added. The mixture was purged with nitrogen gas for three times. A solution of compound **50** in tetrahydrofuran (50 mL, 19 mmol) was added. The mixture was stirred at RT for 16 hours, and concentrated under reduced pressure to yield 3-hydroxy-3-(6-methylpyridin-3-yl)isoindolin-1-one **204** (5 g, crude containing the salt, brown solid). **LCMS:** *m*/*z* 241.1 (M+H); RT = 0.973 min (2.5 min).

### Step 3: Synthesis of 3-(6-methylpyridin-3-yl)isoindolin-1-one (205)

Into a dry 250 mL three-necked flask, compound **204** (5 g crude, 4.75 mmol), dichloromethane (50mL), trifluoroacetic acid (20mL) and triethylsilane (20mL) were sequentially added at RT. The mixture was heated to 40°C under stirring for 16 hours. After the completion of the reaction, the mixture was adjusted to pH 8-9 with 5 N s NaOH olution, and extracted with dichloromethane (50mL×3). The combined organic phase was dried over anhydrous Na₂SO₄ and filtered. The filtrate was concentrated under reduced pressure. The obtained residue was purified by column chromatography (dichloromethane : methanol = 20:1) to yield 3-(6-methylpyridin-3-yl)isoindolin-1-one **205** (950 mg, yellow solid), yield: 89%. **LCMS:** *m*/*z* 225.2 (M+H); RT = 1.037 min (2.5 min).

Analogously to the synthesis of Example A105, the following examples were synthesized according to general synthetic procedure A, with replacing Cs₂CO₃ with K₂CO₃:

| | | | |
|---|---|---|---|
| A107 | 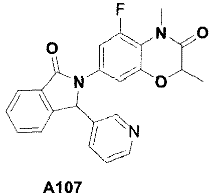 | 1-methyl-3-methyl-6-(1-oxo-3-(pyridin-3-yl)isoindolin-2-yl)-8-fluoro-1 H-benzo[2,3-B][1,4]oxazin-2(3H)-one (A108) | **A107-P3** |
| | | | **LCMS:** *m*/*z* 403.1, 403.8(M+H); RT = 1.330 min (2.5 min). |
| | | | **¹H NMR (CD₃OD, 400 MHz)**δ8.65 (s, 1H), 8.49 (d, *J* = 3.8 Hz, 1H), 7.97 (d, *J* = 7.4 Hz, 1H), 7.65 (ddd, *J=* 18.4, 12.6, 7.4 Hz, 3H), 7.42-7.35 (m, 3H), 7.26-7.21 (m, 1H), 6.55 (s, 1H), 4.60 (q, *J* = 6.8 Hz, 1H), 3.42 (d, *J* = 5.6 Hz, 3H), 1.49 (d, *J* = 6.8 Hz, 3H). |
| | | | **A107-P4** |
| | | | **LCMS:** *m*/*z* 403.1, 403.8(M+H); RT = 1.331 min (2.5 min). |
| | | | **¹H NMR (CD₃OD, 400 MHz)** δ 8.64 (s, 1H), 8.48 (d, *J=* 4.8 Hz, 1H), 7.97 (d, *J* = 7.6 Hz, 1H), 7.74-7.56 (m, 3H), 7.42-7.33 (m, 3H), 7.26-7.22 (m, 1H), 6.54 (s, 1H), 4.57 (q, *J* = 6.8 Hz, 2H), 3.41 (d, *J* = 5.6 Hz, 3H), 1.49 (d, *J* = 6.8 Hz, 3H). |
| A108 | 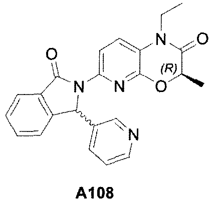 | (*R*)-1-ethyl-3-methyl-6-(1-oxo-3-(pyridin-3-yl)isoindolin-2-yl)-1H-pyrido[2,3-B][1,4]oxazin-2(3H)-one (A108) | **A108-P1** |
| | | | **LCMS:** *m*/*z* 400.1, 401.0 (M+H); RT = 1.407 min (2.5 min). |
| | | | (s, 1H), **NMR (CD₃OD, 400 MHz)** δ 8.91 (s, 1H), 8.60 s, 8.23 (d, *J* = 8.8 Hz, 1H), 8.01 (t, *J* = 7.6 Hz, 1H), 7.72-7.64 (m, 4H), 7.43 (d, *J* = 7.6 Hz, 1H), 6.78 (s, 1H), 4.80 (q, *J* = 6.8 Hz, 1H), 3.96 (q, *J* = 7.2 Hz, 1H), 1.53 (d, *J* = 6.8 Hz, 3H), 1.21 (t, *J* = 6.8 Hz, 3H). |
| | | | **A108-P2** |
| | | | **LCMS:** *m*/*z* 400.1, 400.9 (M+H); RT = 1.272 min (2.5 min). |
| | | | **¹H NMR (CDCl₃, 400 MHz)** δ 8.72 (s, 1H), 8.52 (d, *J* = 4.0 Hz, 1H), 8.18 (d, *J* = 8.8 Hz, 1H), 8.02 (d, *J* = 8.0 Hz, 1H), 7.63-7.56 (m, 4H), 7.33 (d, *J* = 8.8 Hz, 1H), 6.63 (s, 1H), 4.80 (q, *J* = 6.8 Hz, 1H), 3.98-3.86 (m, 2H), 1.55 (d, *J* = 6.8 Hz, 3H), 1.25 (t, *J=* 7.2 Hz, 3H). |
| A109 | 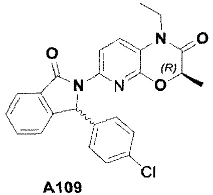 | (*R*)-6-(1-(4-chlorophenyl)-3-oxoisoindolin-2-yl)-1-ethyl-3-methyl-1H-pyrido[2,3-B][1,4]oxazin-2-(3H)-one (A109) | **A109-P1** |
| | | | **LCMS:** *m*/*z* 433.1, 434.1 (M+H); RT = 1.548 min (2.5 min). |
| | | | **¹H NMR (CD₃OD, 400 MHz)** δ8.07 (d, *J* = 8.4 Hz, 1H), 7.94 (d, *J* = 7.6 Hz, 1H), 7.67-7.57 (m, 3H), 7.37 (d, *J* = 7.2 Hz, 1H), 7.28 (s, 4H), 6.61 (s, 1H), 4.84 (q, *J* = 6.8 Hz, 1H), 3.95 (m, 2H), 1.48 (d, *J* = 6.8 Hz, 3H), 1.22 (t, *J* = 6.8 Hz, 3H). |
| | | | **A109-P2** |
| | | | **LCMS:** *m*/*z* 433.1, 433.8(M+H); RT = 1.692 min (2.5 min). |
| | | | **1H NMR (CD₃OD, 400 MHz)** δ8.07 (d, *J* = 8.4 Hz, 1H), 7.94 (d, *J* = 7.6 Hz, 1H), 7.67-7.57 (m, 3H), 7.37 (d, *J* = 7.2 Hz, 1H), 7.28 (s, 4H), 6.61 (s, 1H), 4.84 (q, *J* = 6.8 Hz, 1H), 3.95 (m, 2H), 1.48 (d, *J* = 6.8 Hz, 3H), 1.22 (t, *J* = 6.8 Hz, 3H). |
| A110 | 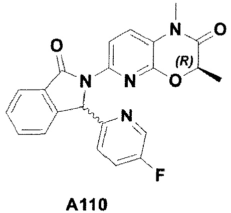 | (*R*)-6-(1-(5-fluoropyridin-2-yl)-3-oxoisoindolin-2-yl)-1,3-dimethyl-1H-pyrido[2,3-b][1,4]oxazin-2(3H)-one (A110) | **A110-P1** |
| | | | **LCMS:** *m*/*z* 405.0 (M+H); RT = 1.528 min (2.5 min). |
| | | | **¹H NMR (400 MHz, CD₃OD)** δ 8.29 (s, 1H), 8.23 (d, *J* = 8.6 Hz, 1H), 7.90 (d, *J* = 7.6 Hz, 1H), 7.64 - 7.50 (m, 5H), 7.42 (d, *J* = 7.2 Hz, 1H), 6.68 (s, 1H), 4.78 (q, *J* = 7.6 Hz, 1H), 3.31 (s, 3H), 1.49 (d, *J* = 6.8 Hz, 3H). |
| | | | **A110-P2** |
| | | | **LCMS:** *m*/*z* 405.0 (M+H); RT = 1.538 min (2.5 min). |
| | | | **¹H NMR (400 MHz, CD₃OD)** δ 8.29 (s, 1H), 8.24 (d, *J* = 8.6 Hz, 1H), 7.90 (d, *J=* 7.6 Hz, 1H), 7.67 - 7.48 (m, 5H), 7.42 (d, *J=* 7.2 Hz, 1H), 6.68 (s, 1H), 4.79 (d, *J=* 7.6 Hz, 1H), 3.31 (s, 3H), 1.43 (d, *J* = 6.8 Hz, 3H). |
| A111 | 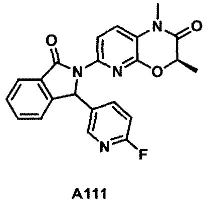 | (*R*)-6-(1-(6-fluoropyridin-3-yl)-3-oxoisoindolin-2-yl)-1,3-dimethyl-1H-pyrido[2,3-b][1,4]oxazin-2(3H)-one (A110) | **A111-P1** |
| | | | **LCMS:** *m*/*z* 404.8 (M+H); RT = 1.487 min (2.5 min). |
| | | | **¹H NMR (DMSO-*d₆*, 400 MHz):** δ8.40 (s, 1H), 8.09 (d, *J* = 8.0 Hz, 1H), 7.90 (d, *J* = 8.0 Hz, 1H), 7.76 (t, *J* = 7.9 Hz, 0H), 7.79 - 7.73 (m, 1H), 7.68 (t, *J=* 8.0 Hz, 2H), 7.40 (d, *J* = 8.0 Hz, 1H), 7.09 - 7.04 (m, 1H), 6.73 (s, 1H), 4.94 (q, *J* = 6.8 Hz, 1H), 3.23 (s, 3H), 1.44 (d, *J=* 4.0 Hz, 3H). |
| | | | **A111-P2** |
| | | | **LCMS:** *m*/*z* 404.8 (M+H); RT = 1.488 min (2.5 min). |
| | | | **¹H NMR (DMSO-*d₆*, 400 MHz):** δ8.38 (s, 1H), 8.09 (d, *J* = 8.0 Hz, 1H), 7.89 (d, *J* = 8.0 Hz, 1H), 7.76 - 7.69 (m, 1H), 7.67 (d, *J* = 8.0 Hz, 2H), 7.60 (t, *J* = 8.0 Hz, 1H), 7.41 (d, *J* = 8.0 Hz, 1H), 7.07 - 7.02 (m, 1H), 6.72 (s, 1H), 4.91 (q, *J* = 4.0 Hz, 1H), 3.22 (s, 3H), 1.41 (d, *J* = 8.0 Hz, 3H). |
| A112 | 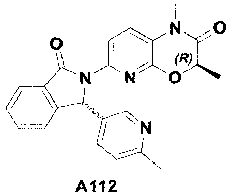 | (*R*)-1,3-dimethyl-6-(1-(6-methylpyridin-3-yl)-3-oxoisoindolin-2-yl)-1H-pyrido[2,3-b][1,4]oxazin-2(3H)-one (A112) | **A112-P1** |
| | | | **LCMS:** *m*/*z* 401.1 (M+H); RT = 1.308 min (2.5 min). |
| | | | **¹H NMR (400 MHz, CD₃OD)** δ 8.51 (s, 1H), 8.08 (d, *J* = 8.6 Hz, 1H), 7.94 (d, *J=* 7.6 Hz, 1H), 7.68 - 7.53 (m, 3H), 7.44 (d, *J* = 8.0 Hz, 1H), 7.35 (d, *J=* 7.6 Hz, 1H), 7.17 (d, *J* = 8.0 Hz, 1H), 6.64 (s, 1H), 4.83 (q, *J* = 6.8 Hz, 1H), 3.30 (s, 3H), 2.45 (s, 3H), 1.53 (d, *J=* 6.8 Hz, 3H). |
| | | | **A112-P2** |
| | | | **LCMS:** *m*/*z* 401.1 (M+H); RT = 1.306 min (2.5 min). |
| | | | **¹H NMR (400 MHz, CD₃OD)** δ 8.50 (s, 1H), 8.08 (d, *J* = 8.6 Hz, 1H), 7.94 (d, *J* = 8.0 Hz, 1H), 7.68 - 7.53 (m, 3H), 7.43 (d, *J* = 8.0 Hz, 1H), 7.36 (d, *J=* 7.6 Hz, 1H), 7.16 (d, *J* = 8.1 Hz, 1H), 6.63 (s, 1H), 4.84 (q, *J* = 6.8 Hz, 1H), 3.30 (s, 3H), 2.44 (s, 3H), 1.47 (d, *J=* 6.8 Hz, 3H). |
| A113 | | (R)-2,4-dimethyl- 7-(1-(6-methylpyridin -3-yl)-3-oxoisoindolin-2-yl)-2H-pyrido[4,3-b][1,4]oxazin-3(4H)-one (A113) | **A113-P1** |
| | | | **LCMS:** *m*/*z* 401.1 (M+H); RT = 1.312 min (2.5 min). |
| | | | **¹H NMR (400 MHz, CDCl₃)** δ 8.66 (s, 1H), 8.12 (s, 1H), 7.98 (d, *J* = 7.4 Hz, 1H), 7.85 (s, 1H), 7.60 - 7.50 (m, 2H), 7.25 - 7.20 (m, 2H), 7.01 (d, *J* = 8.0 Hz, 1H), 6.57 (s, 1H), 4.76 (q, *J* = 6.8 Hz, 1H), 3.32 (s, 3H), 2.50 (s, 3H), 1.57 (d, *J* = 6.8 Hz, 3H). |
| | 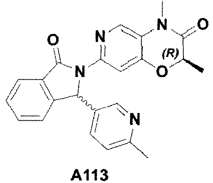 | | **A113-P2** |
| | | | **LCMS:** *m*/*z* 401.1 (M+H); RT = 1.315 min (2.5 min). |
| | | | **¹H NMR (400 MHz, CDCl₃)** δ 8.65 (s, 1H), 8.12 (s, 1H), 7.98 (d, *J=* 7.4 Hz, 1H), 7.85 (s, 1H), 7.60-7.49 (m, 2H), 7.26 - 7.20 (m, 2H), 7.00 (d, *J* = 8.0 Hz, 1H), 6.57 (s, 1H), 4.74 (q, *J=* 6.8 Hz, 1H), 3.32 (s, 3H), 2.49 (s, 3H), 1.58 (d, *J* = 6.8 Hz, 3H). |
| A114 | 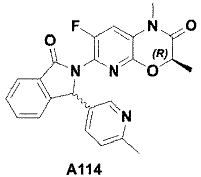 | (*R*)-7-fluoro-1,3-dimethyl-6-(1-(6-methylpyridin-3-yl)-3-oxoisoindolin-2-yl)-1H-pyrido[2,3-b][1,4]oxazin-2(3H)-one (A114) | **A114-P1** |
| | | | **LCMS:** *m*/*z* 419.0 (M+H); RT = 1.290 min (2.5 min). |
| | | | **¹H NMR (400 MHz, CDCl₃)** δ 8.54 (s, 1H), 7.99 (d, *J* = 7.6 Hz, 1H), 7.61 - 7.51 (m, 2H), 7.32 (d, *J* = 8.0 Hz, 1H), 7.24 (d, *J* = 7.4 Hz, 1H), 7.11 - 7.03 (m, 2H), 6.46 (s, 1H), 4.84 (q, *J=* 6.8 Hz, 1H), 3.29 (s, 3H), 2.49 (s, 3H), 1.63 (d, *J* = 6.8 Hz, 3H). |
| | | | **A114-P2** |
| | | | **LCMS:** *m*/*z* 419.0 (M+H); RT = 1.291 min (2.5 min). |
| | | | **¹H NMR (400 MHz, CDCl₃)** δ 8.51 (s, 1H), 7.99 (d, *J* = 7.4 Hz, 1H), 7.61 - 7.52 (m, 2H), 7.33 (d, *J* = 8.0 Hz, 1H), 7.24 (d, *J=* 7.4 Hz, 1H), 7.11 - 7.02 (m, 2H), 6.45 (d, *J* = 6.8 Hz, 1H), 4.82 (q, *J* = 6.8 Hz, 1H), 3.29 (s, 3H), 2.49 (s, 3H), 1.63 (d, *J* = 6.8 Hz, 3H). |
| A115 | 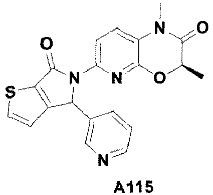 | (3*R*)-1,3-dimethyl-6-(6-oxo-4-(pyridin-3-yl)-4,6-dihydro-5H-thieno[2,3-c]pyrrol-5-yl)-1H-pyrido[2,3-b][1,4]oxazin-2(3H)-oneA115 | **A115-P1** |
| | | | **LCMS:** *m*/*z* 393.0 (M+H); RT = 1.153 min (2.5 min). |
| | | | **¹H NMR (CDCl₃, 400 MHz):** δ8.69 (s, 1H), 8.48 (s, 1H), 8.02 (d, *J* = 8.0 Hz, 1H), 7.74 (d, *J* = 4.0 Hz, 1H), 7.51 (d, *J* = 8.0 Hz, 1H), 7.26 - 7.23 (m, 1H), 7.20 - 7.16 (m, 1 H), 6.90 (d, *J* = 8.0 Hz, 1H), 6.56 (s, 1H), 4.82 - 4.77 (m, 1H), 3.29 (s, 3H), 1.53 (d, *J* = 4.0 Hz, 3H). |
| | | | **A115-P2** |
| | | | **LCMS:** *m*/*z* 393.0 (M+H); RT = 1.152 min (2.5 min). |
| | | | **¹H NMR (CDCl₃, 400 MHz):** δ8.69 (s, 1H), 8.48 (s, 1H), 8.02 (d, *J* = 8.0 Hz, 1H), 7.74 (d, *J* = 4.0 Hz, 1H), 7.51 (d, *J* = 8.0 Hz, 1H), 7.26 - 7.23 (m, 1H), 7.20 - 7.16 (m, 1H), 6.90 (d, *J* = 8.0 Hz, 1H), 6.56 (s, 1H), 4.82 - 4.77 (m, 1H), 3.29 (s, 3H), 1.53 (d, *J* = 4.0 Hz, 3H). |
| A116 | 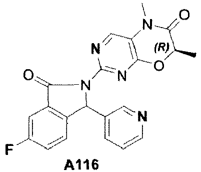 | (7*R*)-2-(5-fluoro-3-oxo-1-(pyridin-3-yl)isoindolin-2-yl)-5,7-dimethyl-5H-pyrimido[4,5-B][1,4]oxazin-6(7H)-one (A116) | **A116-P1** |
| | | | **LCMS:** *m*/*z* 405.1, 406.0(M+H); RT = 1.236 min (2.5 min). |
| | | | **¹H NMR (CD₃OD, 400 MHz)** δ 8.70 (s, 1H), 8.44 (s, 1H), 8.27 (s, 1H), 7.70 (dd, *J=* 7.6, 2.4 Hz, 1H), 7.65 (dd, *J=* 8.2, 1.6 Hz, 1H), 7.50 - 7.33 (m, 3H), 6.67 (s, 1H), 5.09 (q, *J=* 6.8 Hz, 1H), 3.32 (s, 3H), 1.59 (d, *J* = 6.8 Hz, 3H). |
| | | | **A116-P2** |
| | | | **LCMS:** *m*/*z* 405.1, 405.7(M+H); RT = 1.183 min (2.5 min). |
| | | | **¹H NMR (CD₃OD, 400 MHz)** δ 8.70 (s, 1H), 8.45 (d, *J* = 4.6 Hz, 1H), 8.27 (s, 1H), 7.73 - 7.63 (m, 2H), 7.48-7.34 (m, 3H), 6.67 (s, 1H), 5.08 (dd, *J* = 13.8, 6.8 Hz, 1H), 3.33 (s, 3H), 1.61 (t, *J* = 8.8 Hz, 3H). |
| A117 | | (2R)-7-(5-fluoro-3-oxo-1-(pyridin-3-yl)isoindolin-2-yl)-2,4-dimethyl-2H-pyrido[4,3-b][1,4]oxazin-3(4H)-one (A117) | **A117-P2** |
| | | | **LCMS:** *m*/*z* 404.1, 404.7(M+H); RT = 1.289 min (2.5 min). |
| | | | **¹H NMR (CD₃OD, 400 MHz)** δ 8.68 (s, 1H), 8.43 (d, *J* = 4.4 Hz, 1H), 8.07 (s, 1H), 8.01 (s, 1H), 7.71 - 7.58 (m, 2H), 7.42 (d, *J* = 5.8 Hz, 2H), 7.39 - 7.28 (m, 1H), 6.72 (s, 1H), 4.87 - 4.81 (m, 1H), 3.33 (s, 3H), 1.53 (d, *J* = 6.8 Hz, 3H). |
| | 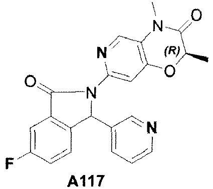 | | **A117-P1** |
| | | | **LCMS:** *m*/*z* 404.1, 404.6(M+H); RT = 1.296 min (2.5 min). |
| | | | **¹H NMR (CD₃OD, 400 MHz)** δ 8.67 (s, 1H), 8.42 (d, *J=* 4.6 Hz, 1H), 8.03 (d, *J* = 17.0 Hz, 2H), 7.62 (d, *J* = 8.2 Hz, 2H), 7.42 (d, *J* = 5.4 Hz, 2H), 7.36 - 7.28 (m, 1H), 6.73 (s, 1H), 4.81 (d, *J* = 5.6 Hz, 1H), 3.33 (s, 3H), 1.57 (d, *J* = 6.8 Hz, 3H). |
| A118 | 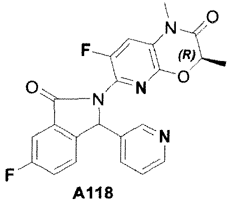 | (3*R*)-7-fluoro-6-(5-fluoro-3-oxo-1-(pyridin-3-yl)isoindolin-2-yl)-1,3-dimethyl-1H-pyrido[2,3-B][1,4]oxazin-2(3H)-one (A118) | **A118-P1** |
| | | | **LCMS:** *m*/*z* 422.1, 422.9(M+H); RT = 1.351 min (2.5 min). |
| | | | **¹H NMR (CD₃OD, 400 MHz)** δ 8.56 (s, 1H), 8.48 (d, *J=* 4.8 Hz, 1H), 7.71 - 7.63 (m, 2H), 7.55 (d, *J* = 9.8 Hz, 1H), 7.46 (td, J = 8.8, 3.0 Hz, 2H), 7.39 (dd, *J* = 7.8, 4.8 Hz, 1H), 6.57 (s, 1H), 4.63 (s, 1H), 3.30 (s, 3H), 1.56 (d, *J=* 6.8 Hz, 3H). |
| | | | **A118-P2** |
| | | | **LCMS:** *m*/*z* 422.1, 422.9(M+H); RT = 1.352 min (2.5 min). |
| | | | **¹H NMR (CD₃OD, 400 MHz)** δ 8.57 (s, 1H), 8.48 (d, *J* = 3.8 Hz, 1H), 7.70 - 7.65 (m, 2H), 7.56 (d, *J* = 9.8 Hz, 1H), 7.49 - 7.43 (m, 2H), 7.39 (dd, *J* = 7.8, 4.8 Hz, 1H), 6.58 (s, 1H), 4.63 (s, 1H), 3.31 (s, 3H), 1.58 (d, *J* = 6.8 Hz, 3H). |
| A119 | 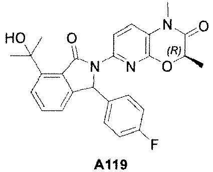 | (*3 R*)-6-(1-(4-fluorophenyl) -4-(2-hydroxyprop-2-yl)-3-oxoisoindolin-2-yl)-1,3-dimethyl-1H-pyrido[2,3b][ 1,4]oxazin-2(3H)-one (A119) | **A119-P1** |
| | | | **LCMS:** *m*/*z* 461.1, 461.7(M+H); RT = 1.594 min (2.5 min). |
| | | | **¹H NMR (CDCl₃, 400 MHz)** δ 8.01 (d, *J=* 8.4 Hz, 1H), 7.51 (m, 2H), 7.32-7.29 (m, 1H), 7.27-7.26 (m, 2H), 7.14 (d, *J* = 7.2 Hz, 1H), 6.97 (t, *J* = 8.8 Hz, 2H), 6.61 (s, 1H), 4.88 (q, *J* = 6.8 Hz, 1H), 3.33 (s, 3H), 1.79 (s, 3H), 1.75 (s, 3H), 1.63 (d, *J* = 6.8 Hz, 3H). |
| | | | **A119-P2** |
| | | | **LCMS:** *m*/*z* 461.1, 461.7(M+H); RT = 1.600 min (2.5 min). |
| | | | **¹H NMR (CDCl₃, 400 MHz)** δ 8.02 (d, *J=* 8.4 Hz, 1H), 7.51 (m, 2H), 7.31 (s, 1H), 7.27-7.25 (m, 2H), 7.15 (d, *J* = 7.2 Hz, 1H), 6.97 (t, *J* = 8.8 Hz, 2H), 6.60 (s, 1H), 4.86 (q, *J=* 6.8 Hz, 1H), 3.33 (s, 3H), 1.80 (s, 3H), 1.72 (s, 3H), 1.60 (d, *J* = 6.8 Hz, 3H). |
| A120 | 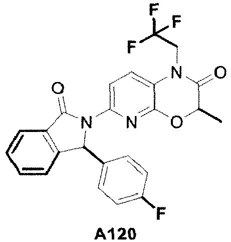 | 6-(1-(4-fluorophenyl)-3-oxoisoindol-2-yl)-3-methyl-1-(2,2,2-trifluoroethyl) -1H-pyrido[2,3-B][1,4]oxazin-2(3H)-one (A120) | **A120-P1(6.4** mg, white solid) |
| | | | **LCMS:** *m*/*z* 471.1, 472.0 (M+H); RT = 1.684 min (2.5 min). |
| | | | **¹H NMR (CD₃OD, 400 MHz)** δ8.10 (d, *J* = 8.8 Hz,1H), 7.94 (d, *J* = 7.6 Hz, 1H), 7.73 (d, *J* = 8.4 Hz, 1H), 7.65 (t, *J* = 7.6 Hz, 1H), 7.58 (t, *J=* 7.2 Hz, 1H), 7.36-7.30 (m, 3H), 7.02 (t, *J* = 8.4 Hz, 2H), 6.63 (s, 1H), 4.90 (m, 1H), 4.75 (m, 2H), 1.58 (d, *J* = 6.8 Hz, 3H). |
| | | | **A120-P2(7.3** mg, white solid) |
| | | | **LCMS:** *m*/*z* 471.1, 472.0(M+H); RT = 1.684 min (2.5 min). |
| | | | **¹H NMR (CD₃OD, 400 MHz)** δ8.09 (d, *J=* 8.8 Hz,1H), 7.94 (d, *J* = 7.6 Hz, 1H), 7.73 (d, *J* = 8.4 Hz, 1H), 7.65 (t, *J* = 7.6 Hz, 1H), 7.58 (t, *J* = 7.2 Hz, 1H), 7.36 (d, *J* = 7.2 Hz, 1H), 7.30 (m, 2H), 7.02 (t, *J* = 8.4 Hz, 2H), 6.63 (s, 1H), 4.90 (m, 1H), 4.75 (m, 2H), 1.51 (d, *J=* 6.8 Hz, 3H). |
| | | | **A120-P3(7.12** mg, white solid) |
| | | | **LCMS:** *m*/*z* 471.1, 472.0(M+H); RT = 1.683 min (2.5 min). |
| | | | **¹H NMR (CD₃OD, 400 MHz)** δ8.10 (d, *J* = 8.8 Hz,1H), 7.94 (d, *J* = 7.6 Hz, 1H), 7.73 (d, *J* = 8.4 Hz, 1H), 7.65 (t, *J* = 7.6 Hz, 1H), 7.58 (t, *J* = 7.2 Hz, 1H), 7.36 (d, *J* = 7.2 Hz, 1H), 7.30 (m, 2H), 7.02 (t, *J* = 8.4 Hz, 2H), 6.63 (s, 1H), 4.90 (m, 1H), 4.75 (m, 2H), 1.58 (d, *J* = 6.8 Hz, 3H). |
| | | | **A120-P4(7.35** mg, white solid) |
| | | | **LCMS:** *m*/*z* 471.1, 472.0(M+H); RT = 1.684 min (2.5 min). |
| | | | **¹H NMR (CD₃OD, 400 MHz)** δ8.09 (d, *J* = 8.8 Hz,1H), 7.94 (d, *J* = 7.6 Hz, 1H), 7.73 (d, *J* = 8.4 Hz, 1H), 7.65 (t, *J* = 7.6 Hz, 1H), 7.58 (t, *J* = 7.2 Hz, 1H), 7.36 (d, *J* = 7.2 Hz, 1H), 7.30 (m, 2H), 7.02 (t, *J* = 8.4 Hz, 2H), 6.63 (s, 1H), 4.90 (m, 1H), 4.75 (m, 2H), 1.51 (d, *J* = 6.8 Hz, 3H). |
| A121 | 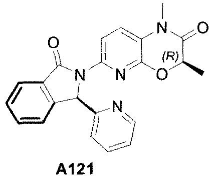 | (3*R*)-1,3-dimethyl-6-(1-oxo-3-(pyridin-2-yl)isoindolin-2-yl)-1H-pyrido[2,3-B][1,4]oxazin-2(3H)-one (A121) | **A121-P1(5.5** mg, white solid) |
| | | | **LCMS:** *m*/*z* 386.1, 387.1 (M+H); RT = 1.469 min (2.5 min). |
| | | | **¹H NMR (CD₃OD, 400 MHz)** δ8.42 (d, *J* = 4.4 Hz, 1H), 8.27 (d, *J* = 8.4 Hz, 1H), 7.94 (d, *J* = 7.6 Hz, 1H), 7.78 (t, *J* = 7.6 Hz, 1H), 7.64-7.55 (m, 3H), 7.49 (d, *J* = 8.0 Hz, 1H), 7.44 (d, *J* = 7.2 Hz, 1H), 7.28 (t, *J* = 6.4 Hz, 1H), 6.69 (s, 1H), 4.79 (q, *J* = 6.8 Hz, 1H), 1.51 (d, *J* = 6.8 Hz, 3H). |
| | | | **A121-P2**(7.8 mg, white solid) |
| | | | **LCMS:** *m*/*z* 386.1, 387.1 (M+H); RT = 1.471 min (2.5 min). |
| | | | **¹H NMR (CD₃OD, 400 MHz)** δ8.40 (d, *J=* 4.4 Hz,1H), 8.24 (d, *J* = 8.4 Hz, 1H), 7.91 (d, *J* = 7.6 Hz, 1H), 7.76 (t, *J* = 7.6 Hz, 1H), 7.61-7.52 (m, 3H), 7.46 (d, *J* = 8.0 Hz, 1H), 7.42 (d, *J* = 7.2 Hz, 1H), 7.26 (t, *J* = 6.4 Hz, 1H), 6.62 (s, 1H), 4.77 (q, *J* = 6.8 Hz, 1H), 1.40 (d, *J* = 6.8 Hz, 3H). |
| A121 -1 | 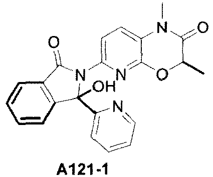 | 6-(1-hydroxy-3-oxo-1-(pyridin-2-yl)isoindolin-2-yl)-1,3-dimethyl-1H-pyrido[2,3-B][1,4]oxazin-2(3H)-one (A121-1) | **A121-1-P3**(9.65 mg, white solid) |
| | | | **LCMS:** *m*/*z* 402.1, 403.1 (M+H); RT = 1.247 min (2.5 min). |
| | | | **¹H NMR (CDCl₃, 400 MHz)** δ8.46 (s,1H), 8.28 (d, *J* = 8.8 Hz, 1H), 7.97 (d, *J* = 7.2 Hz, 1H), 7.79-7.70 (m, 2H), 7.56 (m, 2H), 7.39 (d, *J* = 6.0 Hz, 1H), 7.31 (d, *J* = 8.8 Hz, 1H), 7.17 (q, *J* = 4.4 Hz, 1H), 4.76 (q, *J* = 6.8 Hz, 1H), 3.31 (s, 3H), 1.57 (d, *J* = 6.8 Hz, 3H). |
| | | | **A121-1-P4**(7.81 mg, white solid) |
| | | | **LCMS:** *m*/*z* 402.1, 402.7 (M+H); RT = 1.337 min (2.5 min). |
| | | | **¹H NMR (CDCl₃, 400 MHz)** δ8.46 (s,1H), 8.28 (d, *J* = 8.8 Hz, 1H), 7.97 (d, *J* = 7.2 Hz, 1H), 7.79-7.70 (m, 2H), 7.56 (m, 2H), 7.39 (d, *J* = 6.0 Hz, 1H), 7.31 (d, *J* = 8.8 Hz, 1H), 7.17 (q, *J* = 4.4 Hz, 1H), 4.76 (q, *J* = 6.8 Hz, 1H), 3.31 (s, 3H), 1.46 (d, *J* = 6.8 Hz, 3H). |
| A122 | | (3*R*)-6-(1-(6-chloropyridin-3-yl)-3-oxoisoindol-2-yl)-1,3-dimethyl-1H-pyrido[2,3-B][1,4]oxazin-2(3H)-one (A122) | **A122-P1** |
| | | | **LCMS:** *m*/*z* 420.1, 421.0 (M+H); RT = 1.387 min (2.5 min). |
| | | | **¹H NMR (DMSO, 400 MHz)** 8.56 (s, 1H), 8.12 (d, *J=* 8.4 Hz, 1H), 7.90 (d, *J* = 7.6 Hz, 1H), 7.69-7.58 (m, 4H), 7.42 (d, *J* = 7.6 Hz, 1H), 7.39 (d, *J* = 8.0 Hz, 1H), 6.71 (s, 1H), 4.92 (q, *J* = 6.8 Hz, 1H), 3.23 (s, 3H), 1.42 (d, *J* = 6.8 Hz, 3H). |
| | 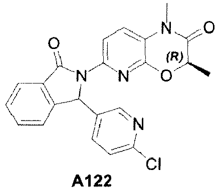 | | **A122-P2** |
| | | | **LCMS:** *m*/*z* 420.1, 420.7 (M+H); RT = 1.550 min (2.5 min). |
| | | | **¹H NMR (DMSO, 400 MHz)** 8.57 (d, *J* = 6.0 Hz, 1H), 8.12 (dd, *J* = 8.4 Hz, *J* = 5.2 Hz, 1H), 7.90 (d, *J* = 6.8 Hz, 1H), 7.69-7.58 (m, 4H), 7.48-7.38 (m, 2H), 6.71 (s, 1H), 4.93 (m, 1H), 3.23 (s, 3H), 1.43 (t, *J* = 6.4 Hz, 3H). |
| A123 | 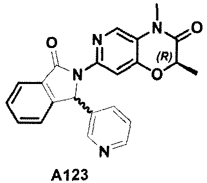 | (*R*)-2,4-dimethyl-7-(1-oxo-3-(pyridin-3-yl)isoindolin-2-yl)-2H-pyrido[4,3-b][1,4]oxazin-3(4H)-one (A123) | **A123-P1** |
| | | | **LCMS:** *m*/*z* 386.8 (M+H); RT = 1.277 min (2.5 min). |
| | | | **¹H NMR (400 MHz, CD₃OD)** δ 8.66 (s, 1H), 8.41 (d, *J=* 5.2 Hz, 1H), 8.08 (s, 1H), 8.02 (s, 1H),7.98 (d, *J=* 7.2 Hz, 1H), 7.71-7.58(m, 3H), 7.40 (d, *J* = 7.2 Hz, 1H), 7.35-7.29 (m, 1H), 6.76 (s, 1H), 4.83-4.78 (m, 1H), 3.34 (s, 3H), 1.58 (d, *J* = 6.8 Hz, 3H) |
| | | | **A123-P2** |
| | | | **LCMS:** *m*/*z* 386.8 (M+H); RT = 1.270 min (2.5 min). |
| | | | **¹H NMR (400 MHz, CD₃OD)** δ 8.67 (s, 1H), 8.42 (d, *J=* 4.8 Hz, 1H), 8.10 (s, 1H), 8.02 (s, 1H),7.97 (d, *J=* 7.6 Hz, 1H), 7.71-7.58(m, 3H), 7.39 (d, *J* = 7.6 Hz, 1H), 7.35-7.31 (m, 1H), 6.75 (s, 1H), 4.92-4.90 (m, 1H), 3.33 (s, 3H), 1.54 (d, *J* = 6.8 Hz, 3H) |
| A124 | 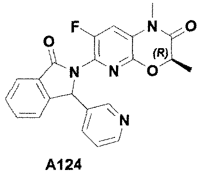 | (*R*)-7-fluoro-1,3-dimethyl-6-(1-oxo-3-(pyridin-3-yl)isoindolin-2-yl)-1 H-pyrido[2,3-b][1,4]oxazin-2(3H)-one (A124) | **A124-P1** |
| | | | **LCMS:** *m*/*z* 404.8 (M+H); RT = 1.256 min (2.5 min). |
| | | | **¹H NMR (400 MHz, CD₃OD)** δ 8.56 (s, 1H), 8.47 (d, *J=* 4.8 Hz, 1H), 7.98 (d, *J* = 7.6 Hz, 1H), 7.73-7.64(m, 3H), 7.56 (d, *J* = 9.6 Hz, 1H), 7.42-7.36 (m, 2H), 6.58 (s, 1H), 4.93 (q, *J* = 6.8 Hz, 1H), 3.32 (s, 3H), 1.58 (d, *J* = 6.8 Hz, 3H) |
| | | | **A124-P2** |
| | | | **LCMS:** *m*/*z* 404.8(M+H); RT = 1.256 min (2.5 min). |
| | | | **¹H NMR (400 MHz, CD₃OD)**δ 8.54 (s, 1H), 8.47 (d, *J* = 4.8 Hz, 1H), 7.98 (d, *J* = 7.6 Hz, 1H), 7.74-7.64(m, 3H), 7.54 (d, *J=* 9.6 Hz, 1H), 7.42-7.36 (m, 2H), 6.57 (s, 1H), 4.93 (q, *J* = 6.8 Hz, 1H), 3.30 (s, 3H), 1.58 (d, *J* = 6.8 Hz, 3H) |
| A125 | 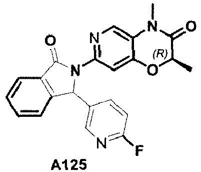 | (2*R*)-7-(1-(6-fluoropyridin-3-yl)-3-oxoisoindolin-2-yl)-2,4-dimethyl-2H-pyrido[4,3-b][1,4]oxazin-3 (4H)-one (A125) | **A125-P1** |
| | | | **LCMS:** *m*/*z* 404.8 (M+H); RT = 1.531 min (2.5 min). |
| | | | **¹H NMR (400 MHz, CD₃OD)** δ 8.34 (s, 1H), 8.02 (d, *J* = 6.4 Hz, 1H), 7.95 (d, *J=* 7.6 Hz, 1H), 7.68-7.58 (m, 3H), 7.38 (d, *J* = 6.8 Hz, 1H), 6.93(dd, *J* = 8.4, 2.0 Hz, 1H), 6.72 (s, 1H), 4.79 (q*, J* = 6.8 Hz, 1H), 3.33 (s, 3H), 1.56 (d, *J* = 6.8 Hz, 3H) |
| | | | **A125-P2** |
| | | | **LCMS:** *m*/*z* 404.9 (M+H); RT = 1.529 min (2.5 min). |
| | | | **¹H NMR (400 MHz, CD₃OD)** δ8.37 (s, 1H), 8.06 (d, *J* = 16.4 Hz, 1H), 7.97 (d, *J* = 8.0 Hz, 1H), 7.71-7.60 (m, 3H), 7.41 (d, *J* = 7.6 Hz, 1H), 6.95(d, *J* = 8.8 Hz, 1H), 6.76 (s, 1H), 4.84 (m, 1H), 3.33 (s, 3H), 1.54 (d, *J* = 6.8 Hz, 3H) |
| A126 | | (*R*)-7-fluoro-6-(1-(6-fluoropyridin-3-yl)-3-oxoisoindolin -2-yl)-1,3-dimethyl-1H-pyrido[2,3-b][1,4]oxazin-2(3H)-one (A126) | **A126-P1** |
| | | | **LCMS:** *m*/*z* 422.8 (M+H); RT = 1.465 min (2.5 min). |
| | | | **¹H NMR (400 MHz, CD₃OD)**δ 8.25 (s, 1H), 7.98 (d, *J* = 7.6 Hz, 1H), 7.74-7.64 (m, 3H), 7.56 (d, *J* = 9.6 Hz, 1H), 7.42 (d, *J* = 7.6 Hz, 1H), 7.02(dd, *J* = 8.8, 2.0 Hz, 1H), 6.58 (s, 1H), 5.15-5.08 (m, 1H), 3.32 (s, 3H), 1.58 (d, *J* = 6.8 Hz, 3H) |
| | 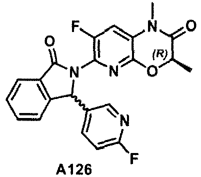 | | **A126-P2** |
| | | | **LCMS:** *m*/*z* 422.8 (M+H); RT = 1.469 min (2.5 min). |
| | | | **¹H NMR (400 MHz, CD₃OD)** δ 8.24 (s, 1H), 7.98 (d, *J* = 7.6 Hz, 1H), 7.74-7.64 (m, 3H), 7.55 (d, *J* = 10.0 Hz, 1H), 7.42 (d, *J* = 7.6 Hz, 1H), 7.01 (dd, *J* = 8.8, 2.4 Hz, 1H), 6.57 (s, 1H), 4.93 (q, *J* = 6.8 Hz, 1H), 3.31 (s, 3H), 1.57 (d, *J=* 6.8 Hz, 3H) |
| A127 | 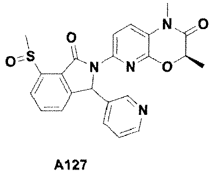 | (3*R*)-1,3-dimethyl-6-(4-(methylsulfinyl)-3-oxo-1-(pyridin-3-yl)isoindolin-2-yl)-1H-pyrido[2,3-B][1,4]oxazin-2(3H)-one (A127) | **A127-P1** |
| | | | **LCMS:** *m*/*z* 448.8 (M+H); RT = 1.175 min (2.5 min). |
| | | | **¹H NMR (400 MHz, CD₃OD)**δ 8.14 (t, *J* = 8.4 Hz, 3H), 8.05 (d, *J* = 8.8 Hz, 1H), 7.94 (t, *J* = 7.6 Hz, 1H), 7.62-7.57 (m, 4H), 6.89 (s, 1H), 4.84 (q, *J* = 6.4 Hz, 1H), 3.04 (s, 3H), 3.30 (s, 3H), 1.54 (d, *J* = 6.8 Hz, 3H). |
| | | | **A127-P2** |
| | | | **LCMS:** *m*/*z* 449.0 (M+H); RT = 1.292 min (2.5 min). |
| | | | **¹H NMR (400 MHz, CDCl₃**)δ 8.74 (d, *J* = 2.0 Hz, 1H), 8.44 (dd, *J* = 5.2, 1.6 Hz, 1H), 8.11 (d, *J* = 7.6 Hz, 1H), 8.08 (d, *J* = 8.4 Hz, 1H), 7.92 (t, *J* = 7.6 Hz, 1H), 7.65 (dq, *J=* 8.4, 1.6 Hz, 1H), 7.59 (d, *J* = 8.4 Hz, 1H), 7.56 (d, *J* = 7.6 Hz, 1H), 7.35 (dd, *J* = 8.0, 4.8 Hz, 1H), 6.82 (s, 1H), 4.88 (q, *J* = 6.8 Hz, 1H), 3.04 (s, 3H), 1.47 (d, *J=* 6.8 Hz, 3H). |
| | | | **A127-P3** |
| | | | **LCMS:** *m*/*z* 449.0 (M+H); RT = 1.298 min (2.5 min). |
| | | | **¹H NMR (400 MHz, CDCl₃)**δ 8.74 (d, *J* = 2.0 Hz, 1H), 8.44 (dd, *J* = 5.2, 1.6 Hz, 1H), 8.13 (d, *J* = 8.4 Hz, 1H), 8.08 (d, *J* = 7.6 Hz, 1H), 7.92 (t, *J* = 7.6 Hz, 1H), 7.65 (dq, *J* = 8.4, 1.6 Hz, 1H), 7.59 (d, *J* = 8.4 Hz, 1H), 7.56 (d, *J* = 7.6 Hz, 1H), 7.35 (dd, *J* = 8.0, 4.8 Hz, 1H), 6.82 (s, 1H), 4.85 (q, *J* = 6.8 Hz, 1H), 3.15 (s, 3H), 1.55 (d, *J* = 6.8 Hz, 3H). |
| | | | **A127-P4** |
| | | | **LCMS:** *m*/*z* 449.0 (M+H); RT = 1.298 min (2.5 min). |
| | | | **¹H NMR (400 MHz, CD₃OD)**δ 8.72 (s, 1H), 8.44 (d, *J* = 4.8 Hz, 1H), 8.11 (t, *J* = 8.4 Hz, 2H), 7.91 (t, *J* = 7.2 Hz, 1H), 7.70 (d, *J* = 8.4 Hz, 1H), 7.58 (t, *J* = 6.4 Hz, 2H), 7.36 (dd, *J* = 7.6 Hz, 4.8 Hz, 1H), 6.80 (s, 1H), 4.86 (q, *J* = 6.4 Hz, 1H), 3.31 (s, 3H), 3.15 (s, 3H), 1.47 (d, *J* = 6.8 Hz, 3H). |
| A128 | 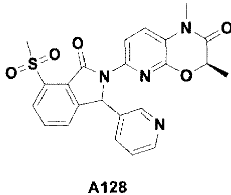 | (3*R*)-1,3-dimethyl-6-(4-(methylsulfon yl)-3-oxo-1-(pyridin-3-yl)isoindolin-2-yl)-1H-pyrido[2,3-B][1,4]oxazin-2(3H)-one (A128) | **A128-P1** |
| | | | **LCMS:** *m*/*z* 465.0 (M+H); RT = 1.327 min (2.5 min). |
| | | | **¹H NMR (400 MHz, CDCl₃)** δ8.90 (s, 1H), 8.65 (d, *J* = 4.4 Hz, 1H), 8.38 (d, *J* = 8.0 Hz, 1H), 8.27 (d, *J* = 8.4 Hz, 1H), 8.13 (m, 1H), 7.85 (t, *J* = 7.6 Hz, 1H), 7.72 (m, 1H), 7.57 (d, *J* = 10.4 Hz, 1H), 7.34 (d, *J* = 8.4 Hz, 1H), 6.76 (s, 1H), 4.87 (q, *J* = 6.8 Hz, 1H), 3.67 (s, 3H), 3.34 (s, 3H), 1.64 (d, *J* = 6.8 Hz, 3H). |
| | | | **A128-P2** |
| | | | **LCMS:** *m*/*z* 465.0 (M+H); RT = 1.327 min (2.5 min). |
| | | | **¹H NMR (400 MHz, CDCl₃)** δ8.94 (s, 1H), 8.68 (d, *J* = 4.4 Hz, 1H), 8.38 (d, *J* = 8.0 Hz, 1H), 8.27 (d, *J* = 8.4 Hz, 1H), 8.13 (m, 1H), 7.85 (t*, J* = 7.6 Hz, 1H), 7.72 (m, 1H), 7.57 (d, *J* = 10.4 Hz, 1H), 7.34 (d, *J* = 8.4 Hz, 1H), 6.75 (s, 1H), 4.85 (q, *J* = 6.8 Hz, 1H), 3.66 (s, 3H), 3.33 (s, 3H), 1.58 (d, *J* = 6.8 Hz, 3H). |
| A129 | 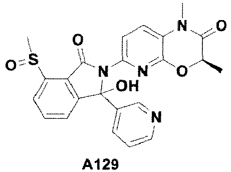 | (3*R*)-6-(1-hydroxy4-(methylsulfinyl)-3-oxo-1-(pyridin-3-yl)isoindolin-2-yl)-1,3-dimethyl-1H-pyrido[2,3-B][1,4]oxazin-2(3H)-one (A129) | **A129-P2** |
| | | | **LCMS:** *m*/*z* 465.1 (M+H); RT = 1.251 min (2.5 min). |
| | | | **¹H NMR (400 MHz, CD₃OD)** δ8.77 (m, 1H), 8.46 (m, 1H), 8.13 (m, 1H), 7.94-7.79 (m, 3H), 7.60-7.56 (m, 2H), 7.38 (m, 1H), 4.87 (q, *J=* 6.8 Hz, 1H), 3.12-3.06 (s, 3H), 1.53-1.46 (d, *J* = 6.8 Hz, 3H). |
| | | | **A129-P3** |
| | | | **LCMS:** *m*/*z* 465.1 (M+H); RT = 1.250 min (2.5 min). |
| | | | **¹H NMR (400 MHz, CD₃OD)** δ8.95 (m, 1H), 8.60 (m, 1H), 8.14 (m, 2H), 7.98-7.94 (m, 1H), 7.88-7.83 (m, 1H), 7.62-7.58 (m, 3H), 4.87 (q, *J* = 6.8 Hz, 1H), 3.12-3.06 (s, 3H), 1.53-1.46 (d, *J* = 6.8 Hz, 3H). |

### Effect Examples: In vitro Enzyme Activity Assay

Half inhibitory activity (IC₅₀) of the compounds of the invention against the bromodomain protein BRD4 BD1 was determined in the present Examples.

### 1. Method of the Assay

The compounds of the invention were tested for inhibitory activity against BRD4 BD1 with Homogeneous Time-Resolved Fluorescence (HTRF).

### 2. Reagents, Consumable Materials and Instruments:

Protein BRD4 BD1 used in the assay was purchased from Active Motif (cat. 31380); streptavidin XL-665 (# 610SAXLA) and EPIgeneous Binding Domain Kit A (# 62BDAPEG) were both purchased from Cisbio Bioassays; [Lys(5,8,12,16)Ac] H4(1-21)-biotin (#64989), [Lys(5,8,12,16)Ac] H4(1-25) were available from Anaspec; OTX015 and ABBV-075 were purchased from Selleckchem Co.. The 384-well ProxiPlate (#6008280) was purchased from Perkinelmer Co.. The Laboratory microplate reader (Envision 2104) was available from PerkinElmer Co.. The water used in the assay was Millipore-Q pure water.

### 3. Formulation of the Compound

10 mM DMSO stock solution of the test compound was diluted to 1mM in DMSO, and then diluted 10-fold in Binding Domain dilution buffer (purchased from Cisbio Bioassays) for use (DMSO concentration in the final system was 0.1%), including 5 concentrations in 1:5 gradient.

### 4. Assay Protocol: 1 batch, 2 repeats

1) 5 µL of test compound was transferred to 45 µL Binding Domain dilution buffer.
2) 2 µL of 10x compound (4 µL Binding Domain dilution buffer: positive control; 6 µL enzyme buffer (purchased from Cisbio Bioassays): negative control) was transferred to a reaction plate.
3) 4 µL of 5xBRD 4(1) was added to the reaction plate (30 nM in the final system).
4) 4 µL of 5x[Lys(5,8,12,16)Ac] H4(1-21)-biotin was added to the reaction plate, and the plate was covered with a film and incubated at 37°C for 30 min.
5) A detection mixture of SA-XL665 (2x) and anti-H3K9me0-Eu(K) (2x) was prepared in detection buffer (purchased from Cisbio Bioassays).
6) 10 µL of detection mixture (2x) was added to each well, incubated at RT for 3h. The mp value was read by microplate reader Envision with the following parameters:

| Top mirror | LANCE/DELFIA Dual / Bias(446) |
|---|---|
| Exc. Filter | UV2 (TRF) 320 |
| Ems. Filter | APC 665 |
| 2^{nd} Ems. Filter | Europium 615 |

### 5. Results

All the compounds of the Examples of the invention have inhibitory activity data (IC₅₀) of less than or equal to 1 µM against the bromodomain protein BRD4 BD1, preferably less than or equal to 100nM against the bromodomain protein BRD4 BD1. For example, the inhibitory activity data (IC₅₀) of some compounds of the invention against the bromodomain protein BRD4 BD1 were shown in the following table, wherein:
A: represents that the IC₅₀ of the compound was less than or equal to 10 nM;
B: represents that the IC₅₀ of the compound was more than 10 nM and less than or equal to 100 nM;
C: represents that the IC₅₀ of the compound was more than 100 nM and less than or equal to 1 µM.

**Table 1: IC₅₀ values (nM) of the compounds of the invention against BRD4 BD1 binding**

| **Compound No** | **BRD4 IC₅₀** | **Compound No** | **BRD4 IC₅₀** | **Compound No** | **BRD4 IC₅₀** |
|---|---|---|---|---|---|
| A1-P2-2 | A | A2-P2-2 | A | A3-P3 | A |
| A4-P2 | A | A5-P1 | A | A6-P1 | A |
| A7-P3 | A | A8-P3 | A | A9-P1-2 | B |
| A10-P2 | B | A11-P3 | A | A12-P1 | A |
| A13-P1 | A | A14-P1 | A | A15-P1-1 | A |
| A16-P1 | A | A17-P1 | C | A18-P4 | A |
| A19-P3 | A | A20-P1 | B | A21-P1 | A |
| A22-P1 | B | A23P4 | A | A24 | B |
| A25 | B | A26-P2 | A | A27-P1 | A |
| A28 | B | A29-P4 | A | A30 | B |
| A31-P3 | A | A32-P3 | B | A33-P1 | A |
| A34 | A | A35 | B | A36-P3 | A |
| A37 | C | A38 | B | A38-P2 | A |
| A39-P1 | A | A40-P1 | A | A41-P3 | A |
| A42-P2 | A | A43-P4 | A | A44 | A |
| A45-P4 | A | A46-P4 | A | A47-P4 | A |
| A48-P4 | A | A49-P4 | A | A50-P1 | A |
| A50-P4 | A | A50-P3 | A | A51-P3 | A |
| A52-P4 | A | A53-P4 | A | A54-P4 | A |
| A55-P2 | A | A56-P1 | A | A57-P3 | A |
| A58-P1 | A | A59-P2 | A | A60-P1 | A |
| A60-P2 | A | A60-P3 | A | A61 (the racemate) | A |
| A62-P1 | A | A63-P3 | A | A63-P4 | A |
| A64-P3 | A | A65-P3 | A | A66-P3 | A |
| A67-P3 | A | A68-P3 | A | A69-P3 | A |
| A70-P3 | A | A71-P4 | A | A72-P4 | A |
| A73 | A | A74-P4 | A | A75-P3 | A |
| A76-P1 | A | A77-P1/P4 | A | A78-P4 | A |
| A79-P4 | A | A80-P3 | A | A81-P4 | A |
| A82-P4 | A | A83-P3 | A | A84-P4 | A |
| A85-P4 | A | A86-P3 | A | A87-P4 | A |
| A88-P4 | A | A89-P3 | A | A90-P4 | A |
| A91-P2 | A | A92-P4 | A | A93-P4 | A |
| A94-P4 | A | A95-P4 | A | A96-P1 | A |
| A97-P1 | A | A98-P3 | A | A99-P3/P4 | A |
| A100-P3/P4 | A | A101-P3 | A | A102-P2/P3 | A |
| A103-P1 | A | A104-P3 | A | A105-P1 | B |
| A106-P1 | A | A107-P3 | B | A108-P3 | B |
| A109-P3 | B | A110-P1 | A | A111-P1 | A |
| A112-P1 | A | A113-P1 | C | A114-P3 | C |
| A115-P1 | A | A116-P3 | C | A117 | C |
| A118 | C | A119-P1 | A | A120-P1 | C |
| A121-P1 | A | A122-P1 | B | A123-P2 | C |
| A124-P1 | B | A125-P1 | C | A126-P1 | B |
| A127-P1 | A | A128-P1 | B | A129-P3 | B |

It can be seen that the compounds of the present application have excellent BRDs inhibitory activity, thus providing a new and effective therapeutic option.

All documents mentioned in the present application are incorporated herein by reference in their entirety, as if they are individually listed. Furthermore, it shall be understood that, based on the present disclosure, various changes or modifications to the invention are obvious to those skilled in the art, and these equivalent forms also fall within the scope defined by the claims appended to this application.

## Claims

1. Compound of formula (I), or a stereoisomer, racemate, geometric isomer, tautomer, prodrug, hydrate, solvate, or pharmaceutically acceptable salt thereof, 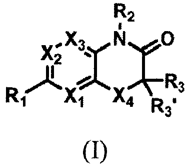 wherein, R₁ is selected from 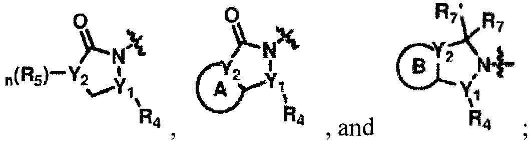 wherein,
Y₁ is selected from CR₄' and N; wherein R₄' is selected from H, D, hydroxy, halo (e.g., F, Cl, Br, I), cyano and optionally substituted alkyl (e.g., C₁₋₆alkyl);
R₄ is selected from optionally substituted aryl (e.g., C₆₋₁₄aryl), optionally substituted heteroaryl (e.g., 5- to 12-membered heteroaryl), optionally substituted cycloalkyl (e.g., C₃₋₁₂cycloalkyl), optionally substituted arylalkylene (e.g., C₆₋₁₄arylC₁₋₃alkylene), optionally substituted heteroarylalkylene (e.g., 5- to 12-membered heteroarylC₁₋₃alkylene), and optionally substituted cycloalkylalkylene (e.g., C₃₋₁₂cycloalkylC₁₋₃alkylene);
Y₂ is selected from C, O and N; n represents 1 or 2, and each R₅ is independently selected from H and optionally substituted alkyl (e.g., C₁₋₈alkyl);
Ring A is selected from optionally substituted aromatic ring (e.g., C₆₋₁₄aromatic ring), optionally substituted heteroaromatic ring (e.g., 5- to 12-membered heteroaromatic ring), and optionally substituted heterocycle (e.g., 3- to 12-membered heterocycle);
Ring B is selected from optionally substituted aromatic ring (e.g., C₆₋₁₄aromatic ring), optionally substituted heteroaromatic ring (e.g., 5- to 12-membered heteroaromatic ring), and optionally substituted heterocycle (e.g., 3- to 12-membered heterocycle);
R₇ and R₇' are each independently selected from H, D, CN and optionally substituted alkyl (e.g., C₁₋₈alkyl);
R₂ is selected from H, D, optionally substituted alkyl (e.g., C₁₋₈alkyl) and optionally substituted cycloalkyl (e.g., C₃₋₁₂cycloalkyl);
R₃ and R₃' are each independently selected from H, D, and optionally substituted alkyl (e.g., C₁₋₈alkyl), and at least one of R₃ and R₃' is not H; or R₃ and R₃', together with the carbon to which they are bound, form a cycloalkyl (e.g., C₃₋₁₂cycloalkyl, e.g. C₃₋₈cycloalkyl, such as cyclopropyl);
X₁ is selected from CR₆ₐ and N;
X₂ is selected from CR_{6b} and N;
X₃ is selected from CR_{6c} and N, and at most two of X₁, X₂ and X₃ are N;
X₄ is selected from NR₈ and O;
R₆ₐ, R_{6b} and R_{6c} are each independently selected from H, D, halo (e.g., F, Cl, Br, I) and optionally substituted alkyl (e.g., C₁₋₈alkyl);
R₈ is selected from H, D, oxygen, hydroxy, optionally substituted alkyl (e.g., C₁₋₈alkyl), optionally substituted alkanoyl (e.g., C₁₋₈alkanoyl), optionally substituted alkoxycarbonyl (C₁₋₈alkoxycarbonyl), optionally substituted cycloalkyl (e.g., C₃₋₁₂cycloalkyl), optionally substituted heterocyclyl (e.g., 3- to 20-membered heterocyclyl), optionally substituted aryl (e.g., C₆₋₁₄aryl), optionally substituted heteroaryl (e.g., 5- to 12-membered heteroaryl), optionally substituted cycloalkylalkylene- (e.g., C₃₋₁₂cycloalkylC₁₋₃alkylene-), optionally substituted heterocyclylalkylene-(e.g., 3- to 20-membered heterocyclylC₁₋₃alkylene-), optionally substituted arylalkylene- (e.g., C₆₋₁₄arylC₁₋₃alkylene-), and optionally substituted heteroarylalkylene- (e.g., C₅₋₁₂heteroarylC₁₋₃alkylene-).

2. The compound according to claim 1, or a stereoisomer, racemate, geometric isomer, tautomer, prodrug, hydrate, solvate, or pharmaceutically acceptable salt thereof, wherein: R₁ is selected from 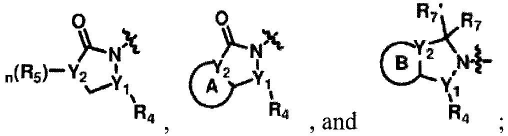 wherein,
Y₁ is selected from CR₄' and N; wherein R₄' is selected from H, D, hydroxy, F, Cl, Br, I, cyano, and C₁₋₆alkyl (e.g., C₁₋₃alkyl) optionally substituted with 1-3 halo (e.g., F, Cl, Br, I); preferably, R₄' is selected from H, D, hydroxy, F, Cl, cyano, methyl, ethyl, methyl substituted with 1-3 fluoro and ethyl substituted with 1-3 fluoro; more preferably, R₄' is selected from H, D, hydroxy, F, cyano, methyl, and methyl substituted with 1-3 fluoro; most preferably, R₄' is selected from H, D and hydroxy;
R₄ is selected from optionally substituted C₆₋₁₀aryl, optionally substituted 5- to 10-membered heteroaryl, optionally substituted C₃₋₈cycloalkyl, optionally substituted C₆₋₁₀arylC₁₋₃alkylene, optionally substituted 5- to 10-membered heteroarylC₁₋₃alkylene, and optionally substituted C₃₋₈cycloalkylC₁₋₃alkylene; preferably, R₄ is selected from optionally substituted phenyl, optionally substituted pyridinyl, optionally substituted pyrazinyl, optionally substituted pyridazinyl, optionally substituted pyrimidinyl, optionally substituted cyclopropyl, optionally substituted cyclobutyl, optionally substituted benzyl, optionally substituted pyridinylmethylene, optionally substituted pyrazinylmethylene, optionally substituted pyridazinylmethylene, optionally substituted pyrimidinylmethylene, optionally substituted cyclopropylmethylene, and optionally substituted cyclobutylmethylene; more preferably, R₄ is selected from optionally substituted phenyl and optionally substituted pyridinyl; for the substitution on R₄, it is preferable that the substituent is one or more groups independently selected from halo (e.g., F, Cl, Br, I), C₁₋₆alkyl (e.g., C₁₋₄alkyl), C₁₋₆alkoxy (e.g., C₁₋₄alkoxy), haloC₁₋₆alkyl (e.g., haloC₁₋₄alkyl), haloC₁₋₆alkoxy (e.g., haloC₁₋₄alkoxy), methanesulfonyl and cyano; more preferably, the substituent is one or more groups independently selected from halo (e.g., F, Cl, Br, I), C₁₋₂alkyl, C₁₋₂alkoxy, haloC₁₋₂alkyl, haloC₁₋₂alkoxy, methanesulfonyl and cyano; further preferably, the substituent is one or more groups independently selected from fluoro, chloro, methyl, ethyl, methoxy, ethoxy, methyl substituted with 1-3 fluoro, ethyl substituted with 1-3 fluoro, methoxy substituted with 1-3 fluoro, ethoxy substituted with 1-3 fluoro, methanesulfonyl and cyano;
Most preferably, R₄ is selected from phenyl optionally substituted with one or more halo, C₁₋₆alkyl or cyano; and heteroaryl such as pyridinyl, optionally substituted with one or more halo, C₁₋₆alkyl or cyano;
Y₂ is selected from C, O and N; n represents 1 or 2, and each R₅ is independently selected from H and optionally substituted C₁₋₆alkyl; preferably, each R₅ is independently selected from H and optionally substituted C₁₋₃alkyl; more preferably, each R₅ is independently selected from H and methyl;
Ring A is selected from optionally substituted 5- to 6-membered aromatic ring, optionally substituted 5- to 6-membered heteroaromatic ring, and optionally substituted 3- to 8-membered heterocycle; preferably, ring A is selected from optionally substituted benzene ring, optionally substituted pyridine, optionally substituted pyrrole, optionally substituted furan, optionally substituted thiophene, optionally substituted piperazine, and optionally substituted pyrazine;
Ring B is selected from optionally substituted 5- to 6-membered aromatic ring, optionally substituted 5- to 6-membered heteroaromatic ring, and optionally substituted 3- to 8-membered heterocycle; preferably, ring B is selected from optionally substituted benzene ring, optionally substituted pyridine, optionally substituted pyrrole, optionally substituted furan, optionally substituted thiophene, optionally substituted piperazine, and optionally substituted pyrazine;
For the substitution on ring A and ring B, it is preferable that the substituent is one or more, e.g, one or two groups independently selected from halo, carboxyl, C₁₋₈alkyl, -OR_{d}, 5- to 10-membered heterocyclylC₁₋₃alkylene unsubstituted or substituted with 1-3 C₁₋₃alkyl, C₁₋₃alkyleneNRₐR_{b}, 5- to 10-membered heterocyclylcarbonyl unsubstituted or substituted with 1-3 C₁₋₃alkyl, haloC₁₋₈alkyl, cyano, -C(O)NRₐR_{b}, -NRₐR_{b}, -S(O)₂C₁₋₆alkyl, -N(Rₐ)S(O)₂R_{b}, - N(Rₐ)C(O)R_{b} and -C(O)OC₁₋₆alkyl; R_{d} is H, C₆₋₁₀aryl, C₁₋₈alkyl, haloC₁₋₈alkyl, C₁₋₈alkyl substituted with -NRₐR_{b}, C₁₋₈alkyl substituted with hydroxy, or C₁₋₈alkyl substituted with C₁₋₆alkoxy; wherein Rₐ and R_{b} are each independently selected from hydrogen, C₁₋₈alkyl, and haloC₁₋₈alkyl;
R₇ and R₇' are each independently selected from H, D, CN and optionally substituted C₁₋₆alkyl (e.g., C₁₋₃alkyl); preferably, R₇ and R₇' are each independently selected from H, D, CN, C₁₋₃alkyl and haloC₁₋₃alkyl; more preferably, R₇ and R₇' are each independently selected from H, D, CN, methyl, and methyl substituted with 1-3 halo such as fluoro;
Preferably, R₁ is selected from 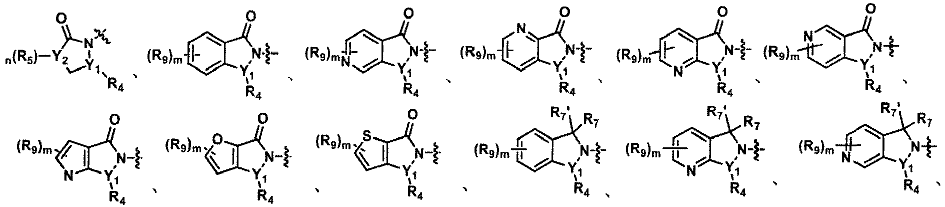 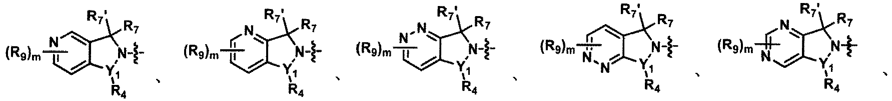 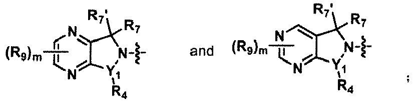
More preferably, R₁ is selected from 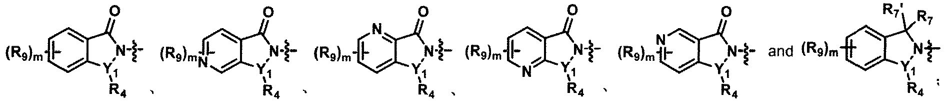
wherein,
Y₁ is selected from CR₄' and N; wherein R₄' is selected from H, D, hydroxy, F, Cl, Br, I, cyano, and C₁₋₆alkyl (e.g., C₁₋₃alkyl) optionally substituted with 1-3 halo (e.g., F, Cl, Br, I); preferably, R₄' is selected from H, D, hydroxy, F, Cl, cyano, methyl, ethyl, methyl substituted with 1-3 fluoro and ethyl substituted with 1-3 fluoro; more preferably, R₄' is selected from H, D, hydroxy, F, cyano, methyl, and methyl substituted with 1-3 fluoro; most preferably, R₄' is selected from H, D and hydroxy;
R₄ is selected from optionally substituted C₆₋₁₀aryl, optionally substituted 5- to 10-membered heteroaryl, optionally substituted C₃₋₈cycloalkyl, optionally substituted C₆₋₁₀arylC₁₋₃alkylene, optionally substituted 5- to 10-membered heteroarylC₁₋₃alkylene, and optionally substituted C₃₋₈cycloalkylC₁₋₃alkylene; preferably, R₄ is selected from optionally substituted phenyl, optionally substituted pyridinyl, optionally substituted pyrazinyl, optionally substituted pyridazinyl, optionally substituted pyrimidinyl, optionally substituted cyclopropyl, optionally substituted cyclobutyl, optionally substituted benzyl, optionally substituted pyridinylmethylene, optionally substituted pyrazinylmethylene, optionally substituted pyridazinylmethylene, optionally substituted pyrimidinylmethylene, optionally substituted cyclopropylmethylene and optionally substituted cyclobutylmethylene; more preferably, R₄ is selected from optionally substituted phenyl and optionally substituted pyridinyl; for the substitution on R₄, it is preferable that the substituent is one or more groups independently selected from halo (e.g., F, Cl, Br, I), C₁₋₆alkyl (e.g., C₁₋₄alkyl), C₁₋₆alkoxy (e.g., C₁₋₄alkoxy), haloC₁₋₆alkyl (e.g., haloC₁₋₄alkyl), haloC₁₋₆alkoxy (e.g., haloC₁₋₄alkoxy), methanesulfonyl and cyano; more preferably, the substituent is one or more groups independently selected from halo (e.g., F, Cl, Br, I), C₁₋₂alkyl, C₁₋₂alkoxy, haloC₁₋₂alkyl, haloC₁₋₂alkoxy, methanesulfonyl and cyano; further preferably, the substituent is one or more groups independently selected from fluoro, chloro, methyl, ethyl, methoxy, ethoxy, methyl substituted with 1-3 fluoro, ethyl substituted with 1-3 fluoro, methoxy substituted with 1-3 fluoro, ethoxy substituted with 1-3 fluoro, methanesulfonyl and cyano;
Most preferably, R₄ is selected from phenyl optionally substituted with one or more halo, C₁₋₆alkyl or cyano; and heteroaryl such as pyridinyl, optionally substituted with one or more halo, C₁₋₆alkyl or cyano;
Y₂ is selected from C, O and N; n represents 1 or 2, and each R₅ is independently selected from H and optionally substituted C₁₋₆alkyl; preferably, each R₅ is independently selected from H and optionally substituted C₁₋₃alkyl; more preferably, each R₅ is independently selected from H and methyl;
R₉ is selected from halo, carboxyl, C₁₋₈alkyl, -OR_{d}, 5- to 10-membered heterocyclylC₁₋₃alkylene unsubstituted or substituted with 1-3 C₁₋₃alkyl, C₁₋₃alkyleneNRₐR_{b}, 5- to 10-membered heterocyclylcarbonyl unsubstituted or substituted with 1-3 C₁₋₃alkyl, haloC₁₋₈alkyl, cyano, - C(O)NRₐR_{b}, -NRₐR_{b}, -S(O)₂C₁₋₆alkyl, -N(Rₐ)S(O)₂R_{b}, -N(Rₐ)C(O)R_{b} and -C(O)OC₁₋₆alkyl; R_{d} is H, C₆₋₁₀aryl, C₁₋₈alkyl, haloC₁₋₈alkyl, C₁₋₈alkyl substituted with -NRₐR_{b}, C₁₋₈alkyl substituted with hydroxy, or C₁₋₈alkyl substituted with C₁₋₆alkoxy; wherein Rₐ and R_{b} are each independently selected from hydrogen, C₁₋₈alkyl and haloC₁₋₈alkyl;
Preferably, R₉ is selected from halo, carboxyl, C₁₋₆alkyl, -OR_{d}, 5- to 8-membered heterocyclylC₁₋₃alkylene unsubstituted or substituted with 1-3 C₁₋₃alkyl, C₁₋₃alkyleneNRₐR_{b}, 5-to 8-membered heterocyclylcarbonyl unsubstituted or substituted with 1-3 C₁₋₃alkyl, haloC₁₋₆alkyl, cyano, -C(O)NRₐR_{b}, -NRₐR_{b}, -S(O)₂C₁₋₄alkyl, -N(Rₐ)S(O)₂R_{b}, -N(Rₐ)C(O)R_{b} and - C(O)OC₁₋₄alkyl; R_{d} is H, C₆₋₁₀aryl, C₁₋₆alkyl, haloC₁₋₆alkyl, C₁₋₆alkyl substituted with -NRₐR_{b}, C₁₋₆alkyl substituted with hydroxy, or C₁₋₆alkyl substituted with C₁₋₃alkoxy; wherein Rₐ and R_{b} are each independently selected from hydrogen, C₁₋₆alkyl, and haloC₁₋₆alkyl;
Preferably, R₉ is selected from halo, carboxyl, C₁₋₄alkyl, C₁₋₄alkoxy, C₁₋₄alkyl substituted with halo, C₁₋₄alkoxy substituted with halo, cyano, -C(O)NRₐR_{b}, -N(Rₐ)C(O)R_{b} and C(O)OC₁₋₄alkyl, Rₐ and R_{b} are each independently selected from hydrogen, C₁₋₄alkyl, and C₁₋₄alkyl substituted with halo, and
m is the number of R₉, and m is selected from 0, 1, 2 and 3; preferably, m is 0, 1 or 2;
Most preferably, R₁ is selected from

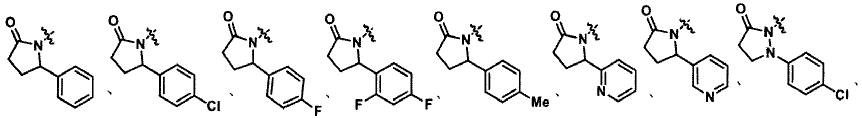

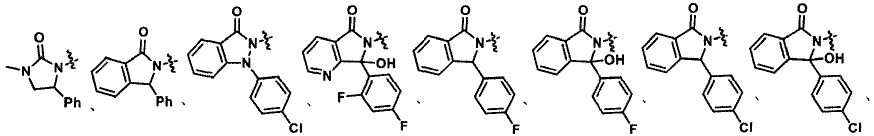

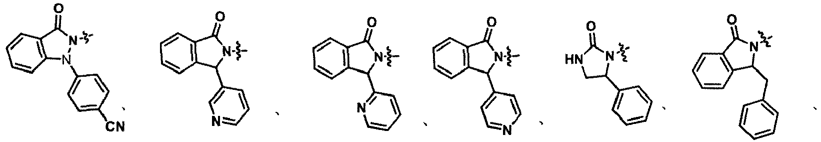

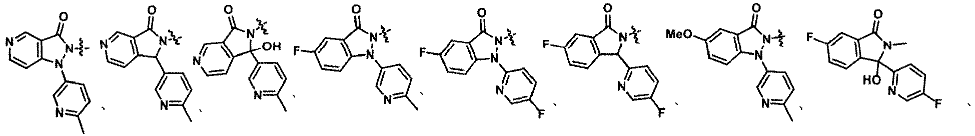

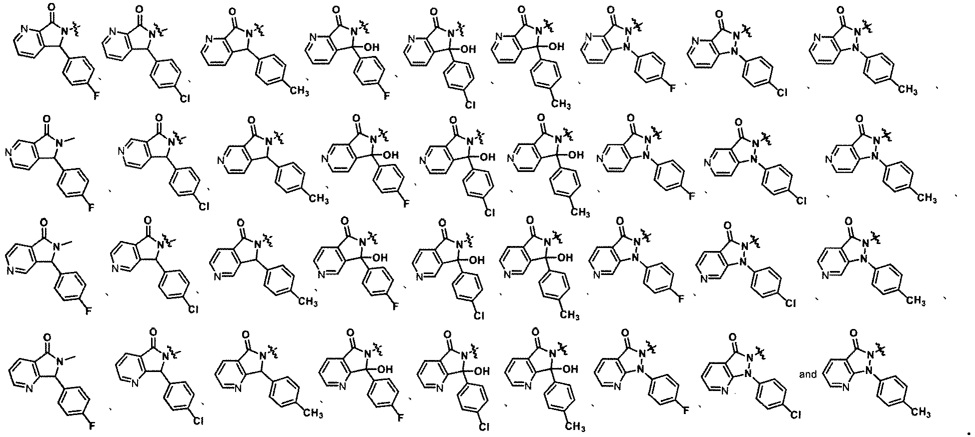

3. The compound according to any of preceding claims, or a stereoisomer, racemate, geometric isomer, tautomer, prodrug, hydrate, solvate, or pharmaceutically acceptable salt thereof, **characterized in that**:
X₁ is N or CH;
X₂ is CR_{6b} or N;
X₃ is CR_{6c};
R_{6b} and R_{6c} are each independently selected from H, D, halo and optionally substituted C₁₋₆alkyl such as C₁₋₃alkyl; preferably, R_{6b} and R_{6c} are each independently selected from H, D, fluoro, methyl and methyl substituted with 1-3 halo such as fluoro; more preferably, R₆ₐ, R_{6b} and R_{6c} are each independently selected from H, D, fluoro and methyl.

4. The compound according to any of preceding claims, or a stereoisomer, racemate, geometric isomer, tautomer, prodrug, hydrate, solvate, or pharmaceutically acceptable salt thereof, **characterized in that**:
X₄ is selected from NR₈ and O, wherein
R₈ is selected from H, D, oxygen, hydroxy, optionally substituted C₁₋₄alkyl, optionally substituted C₁₋₄alkanoyl, optionally substituted C₁₋₄alkoxycarbonyl, optionally substituted C₃₋₈cycloalkyl, optionally substituted 3- to 8-membered heterocyclyl (e.g., 3- to 8-membered heterocyclyl containing oxygen or nitrogen as a heteroatom), optionally substituted C₆₋₁₀aryl, optionally substituted 5- to 10-membered heteroaryl, optionally substituted C₃₋₈cycloalkylC₁₋₃alkylene- (e.g., C₃₋₈cycloalkylmethylene), optionally substituted 3- to 8-membered heterocyclylC₁₋₃alkylene-, optionally substituted C₆₋₁₀arylalkylene- (e.g., benzyl), and optionally substituted 5- to 10-membered heteroarylC₁₋₃alkylene- (e.g., pyridinylC₁₋₃alkylene-, such as pyridinylmethylene-);
Preferably, R₈ is selected from H, D, oxygen, hydroxy, methyl, ethyl, propyl such as isopropyl hydroxyethyl, propyl substituted with hydroxy, C₂₋₄alkyl substituted with C₁₋₂alkoxy, methoxycarbonyl, cyclopropyl, cyclobutyl, cyclopentyl, oxetanyl, oxolanyl, tetrahydropyranyl, cyclopropanemethylene-, benzyl and pyridinylmethylene;
It is preferable for the substitution on R₈ that the substituent is one or more groups independently selected from -OH, F, CN, -NH₂, C₁₋₆alkoxy (e.g., C₁₋₂alkoxy), -NH(C₁₋₃alkoxy), and -N(C₁₋₃alkoxy)₂.

5. The compound according to any of preceding claims, or a stereoisomer, racemate, geometric isomer, tautomer, prodrug, hydrate, solvate, or pharmaceutically acceptable salt thereof, **characterized in that**:
R₂ is selected from H, D, optionally substituted C₁₋₆alkyl (e.g., C₁₋₄alkyl) and optionally substituted C₃₋₈cycloalkyl (e.g., C₃₋₆cycloalkyl); it is preferable for the substitution on R₂ that the substituent is one,two, three or more groups independently selected from D, halo (e.g., F, Cl, Br and I) and hydroxy;
More preferably, R₂ is selected from H, D, C₁₋₃alkyl optionally substituted with 1-3 D, halo such as fluoro and/or hydroxyl, and C₃₋₄cycloalkyl optionally substituted with 1-3 halo such as fluoro and/or hydroxy;
Further preferably, R₂ is selected from H, D, methyl, ethyl, propyl, hydroxyethyl, -CHF₂, -CH₂F, -CF₃, CH₂CF₃ and cyclopropyl.

6. The compound according to any of preceding claims, or a stereoisomer, racemate, geometric isomer, tautomer, prodrug, hydrate, solvate, or pharmaceutically acceptable salt thereof, **characterized in that**:
R₃ and R₃' are each independently selected from H, D, and optionally substituted C₁₋₆alkyl (e.g., C₁₋₃alkyl), and at least one of R₃ and R₃' is not H;
Preferably, R₃ and R₃' are each independently selected from H, D, and C₁₋₃alkyl (e.g., methyl, ethyl) optionally substituted with one, two or more halo (e.g., F, Cl, Br, and I), and at least one of R₃ and R₃' is not H;
Further preferably, R₃ and R₃' are each independently selected from H, methyl and ethyl, and at least one of R₃ and R₃' is not H;
Or further preferably, either of R₃ and R₃' is methyl, and the other is H.

7. The compound according to claim 1, or a stereoisomer, racemate, geometric isomer, tautomer, prodrug, hydrate, solvate, or pharmaceutically acceptable salt thereof, wherein:
R₁ is selected from 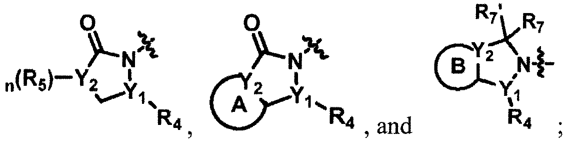 wherein,
Y₁ is selected from CR₄' and N; wherein R₄' is selected from H, D, hydroxy, F, Cl, cyano, methyl, ethyl, methyl substituted with 1-3 fluoro, and ethyl substituted with 1-3 fluoro;
R4 is selected from optionally substituted phenyl, optionally substituted heteroaryl, optionally substituted C₃₋₈cycloalkyl, optionally substituted benzyl, optionally substituted heteroarylalkylene, and optionally substituted C₃₋₈cycloalkylalkylene;
Y₂ is selected from C, O and N; n represents 1 or 2, and each R₅ is independently selected from H and optionally substituted C₁₋₃alkyl;
Ring A is selected from optionally substituted benzene ring, and optionally substituted 5- to 6-membered heteroaromatic ring;
Ring B is selected from optionally substituted benzene ring, and optionally substituted 5- to 6-membered heteroaromatic ring such as pyridine ring;
R₇ and R₇' are each independently selected from H, D, CN and optionally substituted C₁₋₃alkyl;
R2 is selected from H, D, optionally substituted C₁₋₄alkyl and optionally substituted C₃₋₄cycloalkyl;
R₃ and R₃' are each independently selected from H, D, and optionally substituted C₁₋₆alkyl (e.g., C₁₋₃alkyl), preferably each independently selected from H, D, methyl, and ethyl, and at least one of R3 and R₃' is not H; or R₃ and R₃' together with the carbon to which they are bound form a cyclopropyl;
X₁ is selected from CR₆ₐ and N;
X₂ is selected from CR_{6b} and N;
X₃ is selected from CR_{6c} and N, and at most two of X₁, X₂ and X₃ are N;
X₄ is selected from NR₈ and O;
R₆ₐ, R_{6b} and R_{6c} are each independently selected from H, D, halo and optionally substituted C₁₋₃alkyl;
R₈ is selected from H, D, oxygen, hydroxy, optionally substituted C₁₋₆alkyl, optionally substituted C₁₋₆alkanoyl, optionally substituted C₁₋₆alkylcarbonyl, optionally substituted C₃₋₈cycloalkyl, optionally substituted 3- to 8-membered heterocyclyl and optionally substituted benzyl.

8. The compound according to claim 1, or a stereoisomer, racemate, geometric isomer, tautomer, prodrug, hydrate, solvate, or pharmaceutically acceptable salt thereof, **characterized in that**:
R₁ is selected from 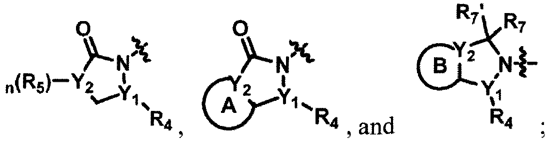 wherein,
Y₁ is selected from CR₄' and N; wherein R₄' is selected from H, D, hydroxy, F, Cl, cyano, methyl, ethyl, methyl substituted with 1-3 fluoro and ethyl substituted with 1-3 fluoro;
R₄ is selected from optionally substituted phenyl, optionally substituted pyridinyl, optionally substituted pyrazinyl, optionally substituted pyridazinyl, optionally substituted pyrimidinyl, optionally substituted C₃₋₄cycloalkyl, optionally substituted benzyl, optionally substituted pyridinylmethylene and optionally substituted C₃₋₄cycloalkylmethylene; it is preferable for the substitution on R₄ that the substituent is one or more groups independently selected from halo, C₁₋₄alkyl, C₁₋₄alkoxy, haloC₁₋₄alkyl, haloC₁₋₄alkoxy, methanesulfonyl and - CN;
Y₂ is selected from C, O and N; n represents 1 or 2, and each R₅ is independently selected from H and optionally substituted C₁₋₃alkyl;
Ring A is selected from optionally substituted benzene ring, optionally substituted pyridine, optionally substituted pyrrole, optionally substituted furan and optionally substituted thiophene;
Ring B is selected from optionally substituted benzene ring, and optionally substituted pyridine;
It is preferable for the substitution on ring A and ring B that the substituent is one or more, for example one or two groups independently selected from halo, carboxyl, C₁₋₈alkyl, haloC₁₋₈alkyl, cyano, -C(O)NRₐR_{b}, -OR_{d}, 5- to 10-membered heterocyclylC₁₋₃alkylene unsubstituted or substituted with 1-3 C₁₋₃alkyl, C₁₋₃alkyleneNRₐR_{b}, 5- to 10-membered heteroarylcarbonyl unsubstituted or substituted with 1-3 C₁₋₃alkyl, -NRₐR_{b}, -S(O)₂C₁₋₆alkyl, - N(Rₐ)S(O)₂R_{b}, -N(Rₐ)C(O)R_{b} and -C(O)OC₁₋₈alkyl, wherein Rₐ and R_{b} are each independently selected from hydrogen, C₁₋₈alkyl and haloC₁₋₈alkyl; R_{d} is H, C₆₋₁₀aryl, C₁₋₈alkyl, haloC₁₋₈alkyl, C₁₋₈alkyl substituted with -NRₐR_{b}, C₁₋₈alkyl substituted with hydroxy or C₁₋₈alkyl substituted with C₁₋₃alkoxy;
R₇ and R₇' are each independently selected from H, D, CN, C₁₋₃alkyl and haloC₁₋₃alkyl;
R₂ is selected from H, D, C₁₋₂alkyl and C₁₋₂alkyl substituted with 1-3 fluoro;
R₃ and R₃' are each independently selected from H, D, and optionally substituted C₁₋₆alkyl (e.g., C₁₋₃alkyl), preferably each independently selected from H, D, methyl, and ethyl, and at least one of R₃ and R₃' is not H;
X₁ is selected from CR₆ₐ and N;
X₂ is selected from CR_{6b} and N;
X₃ is selected from CR_{6c} and N, and at most two of X₁, X₂ and X₃ are N;
X₄ is selected from NR₈ and O;
R₆ₐ, R_{6b} and R_{6c} are each independently selected from H, D, F, Cl, methyl and methyl substituted with 1-3 fluoro;
R₈ is selected from H, D, oxygen, hydroxy, optionally substituted C₁₋₄alkyl, optionally substituted C₁₋₆alkanoyl, optionally substituted C₃₋₆cycloalkyl, optionally substituted 3- to 6-membered heterocyclyl containing oxygen or nitrogen as a heteroatom, optionally substituted C₃-₆cycloalkylmethylene, optionally substituted benzyl, and optionally substituted pyridinylmethylene; it is preferable for the substituents on R₈ that the substituent is one or more, for example 1-3 groups independently selected from -OH, halo (e.g., F, Cl, Br and I), CN and C₁₋₆alkoxy (e.g., C₁₋₂alkoxy).

9. The compound according to claim 1, or a stereoisomer, racemate, geometric isomer, tautomer, prodrug, hydrate, solvate, or pharmaceutically acceptable salt thereof, **characterized in that**:
R₁ is selected from 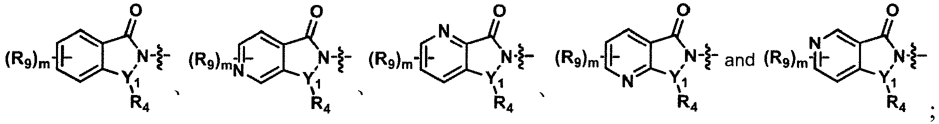
Y₁ is selected from CR₄' and N; wherein R₄' is selected from H, D and hydroxy;
R₄ is selected from optionally substituted phenyl, optionally substituted pyridinyl and optionally substituted benzyl; it is preferable for the substitution on R₄ that the substituent is one or more groups independently selected from F, Cl, C₁₋₂alkyl, C₁₋₂alkyl substituted with 1-3 fluoro, and -CN;
R₉ is selected from fluoro, chloro, carboxyl, methyl, methoxy, fluoromethyl and fluoromethoxy; m is the number of R₉, and m is 0, 1 or 2;
R₂ is selected from methyl, ethyl, -CHF₂, -CH₂F and -CF₃;
R₃ and R₃' are each independently selected from H, D, methyl, and ethyl, and at least one of R₃ and R₃' is not H;
X₁ is N;
X₂ is CR_{6b} or N;
X₃ is CR_{6c};
X₄ is NR₈ or O;
R_{6b} and R_{6c} are each independently selected from H and F;
R₈ is selected from H, D, oxygen, hydroxy, methyl, ethyl, propyl such as isopropyl, hydroxyethyl, acetyl, cyclopropyl, cyclobutyl, cyclopentyl, oxetanyl, furyl, pyranyl, tetrahydropyranyl, cyclopropanemethylene, benzyl and pyridinylmethylene; it is preferable for the substitution on R₈ that the substituent is one or more groups independently selected from halo (e.g., F, Cl, Br and I), CN and C₁₋₆alkoxy such as methoxy.

10. The compound according to preceeding claims, or a stereoisomer, racemate, geometric isomer, tautomer, prodrug, hydrate, solvate, or pharmaceutically acceptable salt thereof, R₁ is selected from 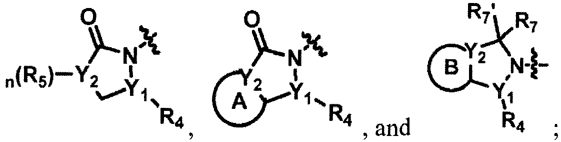 wherein,
Y₁ is selected from CR₄' and N; wherein R₄' is selected from H, D, hydroxy, F, Cl, cyano, methyl, ethyl, methyl substituted with 1-3 fluoro, and ethyl substituted with 1-3 fluoro;
R₄ is selected from optionally substituted phenyl, optionally substituted pyridinyl, optionally substituted pyrazinyl, optionally substituted pyridazinyl, optionally substituted pyrimidinyl, optionally substituted C₃₋₄cycloalkyl, optionally substituted benzyl, optionally substituted pyridinylmethylene, and optionally substituted C₃₋₄cycloalkylmethylene; it is preferable for the substitution on R₄ that the substituent is one or more groups independently selected from halo, C₁₋₄alkyl, C₁₋₄alkoxy, haloC₁₋₄alkyl, haloC₁₋₄alkoxy, methanesulfonyl and - CN;
Y₂ is selected from C, O and N; n represents 1 or 2, and each R₅ is independently selected from H and optionally substituted C₁₋₃alkyl;
Ring A is selected from optionally substituted benzene ting, optionally substituted pyridine, optionally substituted pyrrole, optionally substituted furan, and optionally substituted thiophene;
Ring B is selected from optionally substituted benzene ring, and optionally substituted pyridine;
It is preferable for the substitution on ring A and ring B that the substituent is one or more, for example one or two groups independently selected from halo, carboxyl, C₁₋₈alkyl, haloC₁₋₈alkyl, -C₁-C₆alkyl-OH, C₁₋₈alkylthio, cyano, -C(O)NRₐR_{b}, -OR_{d}, 5- to 10-membered heterocyclylC₁₋₃alkylene unsubstituted or substituted with 1-3 C₁₋₃alkyl, C₁₋₃alkyleneNRₐR_{b}, 5-to 10-membered heteroarylcarbonyl unsubstituted or substituted with 1-3 C₁₋₃alkyl, -NRₐR_{b}, - S(O)₂C₁₋₆alkyl, -S(O)C₁₋₆alkyl, -N(Rₐ)S(O)₂R_{b}, -N(Rₐ)C(O)R_{b} and -C(O)OC₁₋₈alkyl, wherein Rₐ and R_{b} are each independently selected from hydrogen, C₁₋₈alkyl, haloC₁₋₈alkyl and C₃₋₈cycloalkyl, or Rₐ and R_{b} together form C₃₋₈heterocyclyl optionally substituted with C₁₋₄alkyl; R_{d} is H, C₆₋₁₀aryl, C₁₋₈alkyl, haloC₁₋₈alkyl, C₁₋₈alkyl substituted with -NRₐR_{b}, C₁₋₈alkyl substituted with hydroxy, or C₁₋₈alkyl substituted with C₁₋₃alkoxy;
R₇ and R₇' are each independently selected from H, D, CN, C₁₋₃alkyl and haloC₁₋₃alkyl;
R₂ is selected from H, D, C₁₋₂alkyl, C₁₋₃alkyl substituted with one or more deuterium, C₁₋₂alkyl substituted with 1-3 fluoro;
R₃ and R₃' are each independently selected from H, D and optionally substituted C₁₋₆alkyl (e.g., C₁₋₃alkyl), preferably each independently selected from H, D, methyl and ethyl, and at least one of R₃ and R₃' is not H;
X₁ is selected from CR₆ₐ and N;
X₂ is selected from CR_{6b} and N;
X₃ is selected from CR_{6c} and N, and at most two of X₁, X₂ and X₃ are N;
X₄ is selected from NR₈ and O;
R₆ₐ, R_{6b} and R_{6c} are each independently selected from H, D, F, Cl, methyl and methyl substituted with 1-3 fluoro;
R₈ is selected from H, D, oxygen, hydroxy, optionally substituted C₁₋₄alkyl, optionally substituted C₁₋₆alkanoyl, optionally substituted C₃₋₆cycloalkyl, optionally substituted 3- to 6-membered heterocyclyl containing oxygen or nitrogen as a heteroatom, optionally substituted C₃₋₆cycloalkylmethylene, optionally substituted benzyl, and optionally substituted pyridinylmethylene; it is preferable for the substituents on R₈ that the substituent is one or more, for example 1-3 groups independently selected from -OH, halo (e.g., F, Cl, Br and I), CN, C₁₋₆alkyl and C₁₋₆alkoxy (e.g., C₁₋₂alkoxy).

11. The compound according to preceeding claims, or a stereoisomer, racemate, geometric isomer, tautomer, prodrug, hydrate, solvate, or pharmaceutically acceptable salt thereof, R₁ is selected from 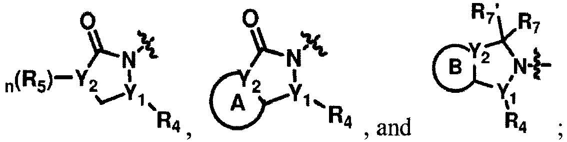 wherein,
Y₁ is selected from CR₄' and N; wherein R₄' is selected from H, D, hydroxy, F, Cl, cyano, methyl, ethyl, methyl substituted with 1-3 fluoro, and ethyl substituted with 1-3 fluoro;
R4 is selected from optionally substituted phenyl, optionally substituted pyridinyl, optionally substituted pyrazinyl, optionally substituted pyridazinyl, optionally substituted pyrimidinyl, optionally substituted C₃₋₄cycloalkyl, optionally substituted benzyl, optionally substituted pyridinylmethylene, and optionally substituted C₃₋₄cycloalkylmethylene; it is preferable for the substitution on R₄ that the substituent is one or more groups independently selected from halo, C₁₋₄alkyl, C₁₋₄alkoxy, haloC₁₋₄alkyl, haloC₁₋₄alkoxy, methanesulfonyl and - CN;
Y₂ is selected from C, O and N; n represents 1 or 2, and each R₅ is independently selected from H and optionally substituted C₁₋₃alkyl;
Ring A is selected from optionally substituted benzene ring, optionally substituted pyridine, optionally substituted pyrrole, optionally substituted furan, and optionally substituted thiophene;
Ring B is selected from optionally substituted benzene ring, and optionally substituted pyridine;
It is preferable for the substitution on ring A and ring B that the substituent is one or more, for example one or two groups independently selected from halo, carboxyl, C₁₋₈alkyl, haloC₁₋₈alkyl, -C₁-C₆alkyl-OH, cyano, -C(O)NRₐR_{b}, -OR_{d}, 5- to 10-membered heterocyclylC₁₋₃alkylene unsubstituted or substituted with 1-3 C₁₋₃alkyl, C₁₋₃alkyleneNRₐR_{b}, 5- to 10-membered heteroarylcarbonyl unsubstituted or substituted with 1-3 C₁₋₃alkyl, -NRₐR_{b}, -S(O)₂C₁₋₆alkyl, - S(O)C₁₋₆alkyl, -N(Rₐ)S(O)₂R_{b}, -N(Rₐ)C(O)R_{b} and -C(O)OC₁₋₈alkyl, wherein Rₐ and R_{b} are each independently selected from hydrogen, C₁₋₈alkyl, haloC₁₋₈alkyl; R_{d} is H, C₆₋₁₀aryl, C₁₋₈alkyl, haloC₁₋₈alkyl, C₁₋₈alkyl substituted with -NRaRb, C₁₋₈alkyl substituted with hydroxy, or C₁₋₈alkyl substituted with C₁₋₃alkoxy;
R₇ and R₇' are each independently selected from H, D, CN, C₁₋₃alkyl and haloC₁₋₃alkyl;
R₂ is selected from H, D, C₁₋₂alkyl, C₁₋₃alkyl substituted with one or more deuterium, C₁₋₂alkyl substituted with 1-3 fluoro;
R₃ and R₃' are each independently selected from H, D and optionally substituted C₁₋₆alkyl (e.g., C₁₋₃alkyl), preferably each independently selected from H, D, methyl and ethyl, and at least one of R₃ and R₃' is not H;
X₁ is selected from CR₆ₐ and N;
X₂ is selected from CR_{6b} and N;
X₃ is selected from CR_{6c} and N, and at most two of X₁, X₂ and X₃ are N;
X₄ is selected from NR₈ and O;
R₆ₐ, R_{6b} and R_{6c} are each independently selected from H, D, F, Cl, methyl and methyl substituted with 1-3 fluoro;
R₈ is selected from H, D, oxygen, hydroxy, optionally substituted C₁₋₄alkyl, optionally substituted C₁₋₆alkanoyl, optionally substituted C₃₋₆cycloalkyl, optionally substituted 3- to 6-membered heterocyclyl containing oxygen or nitrogen as a heteroatom, optionally substituted C₃₋₆cycloalkylmethylene, optionally substituted benzyl, and optionally substituted pyridinylmethylene; it is preferable for the substituents on R₈ that the substituent is one or more, for example 1-3 groups independently selected from -OH, halo (e.g., F, Cl, Br and I), CN, C₁₋₆alkyl and C₁₋₆alkoxy (e.g., C₁₋₂alkoxy).

12. The compound according to preceeding claims, or a stereoisomer, racemate, geometric isomer, tautomer, prodrug, hydrate, solvate, or pharmaceutically acceptable salt thereof, **characterized in that**: Formula (I) is : 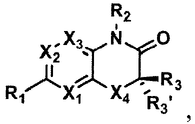 wherein each variable is correspondingly defined in each claim;
Preferably, R₃ and R₃' are each independently selected from H, D, and optionally substituted C₁₋₆alkyl (e.g., C₁₋₃alkyl), and at least one of R₃ and R₃' is not H;
Preferably, R₃ and R₃' are each independently selected from H, D, and C₁₋₃alkyl (e.g., methyl, ethyl) optionally substituted with one, two or more halo (e.g., F, Cl, Br, and I), and at least one of R₃ and R₃' is not H;
Further preferably, R₃ is methyl or ethyl, and R₃' is H; more preferably, R₃ is methyl, and R₃' is H.

13. The compound selected from the examples, or a stereoisomer, racemate, geometric isomer, tautomer, prodrug, hydrate, solvate, or pharmaceutically acceptable salt thereof.

14. A pharmaceutical composition comprising the compound according to any of claims 1-13, or a stereoisomer, racemate, geometric isomer, tautomer, prodrug, hydrate, solvate, or pharmaceutically acceptable salt thereof, and optionally a pharmaceutically acceptable carrier.

15. Use of the compound according to any of claims 1-13, or a stereoisomer, racemate, geometric isomer, tautomer, prodrug, hydrate, solvate, or pharmaceutically acceptable salt thereof, for the manufacture of a medicament for the treatment and/or prevention of a disease mediated by bromodomain protein, or a product as a bromodomain protein inhibitor.

16. The use according to claim 15, wherein the disease mediated by bromodomain protein includes cancer such as hematological malignant tumor, midline carcinoma, inflammatory diseases, cardiovascular diseases, viral infections, fibrotic diseases, metabolic diseases, radiation poisoning, acute rejection of transplanted organs or multiorgan dysfunction syndrome and Alzheimer's disease.

17. A method for non-therapeutically inhibiting bromodomain protein activity, comprising contacting an effective amount of the compound according to any of claims 1-13, or a stereoisomer, racemate, geometric isomer, tautomer, prodrug, hydrate, solvate, or pharmaceutically acceptable salt thereof, with the bromodomain proteins, thereby inhibiting the bromodomain proteins.
